# EUROPEAN PATENT APPLICATION

(11) **EP 2 450 352 A1**
(43) Date of publication of application: **09.05.2012**
(21) Application number: 10806500.4
(22) Date of filing: 04.08.2010
(51) Int. Cl.: C07D 233/96, A61K 31/4166, A61K 31/426, A61K 31/427, A61P 3/06, A61P 3/10, A61P 9/10, A61P 9/12, A61P 19/00, A61P 19/02, A61P 19/06, A61P 25/00, A61P 25/16, A61P 25/18, A61P 25/22, A61P 25/24, A61P 25/28, A61P 25/30, A61P 29/00, A61P 35/00

(54) **PROPHYLACTIC OR THERAPEUTIC AGENT FOR CANCER**

(30) Priority: 04.08.2009 JP 2009181936
(71) Applicant: Takeda Pharmaceutical Company Limited, Osaka-shi, Osaka 541-0045 (JP)
(72) Inventor: HASUOKA, Atsushi, Fujisawa Kanagawa 251-0012 (JP); ONO, Koji, Fujisawa Kanagawa 251-0012 (JP); KAWASAKI, Masanori, Fujisawa Kanagawa 251-0012 (JP)
(74) Representative: König, Gregor Sebastian
(86) International application number: PCT/JP2010/063227
(87) International publication number: WO 2011/016501

(57) **Abstract**

A compound represented by the formula (I): 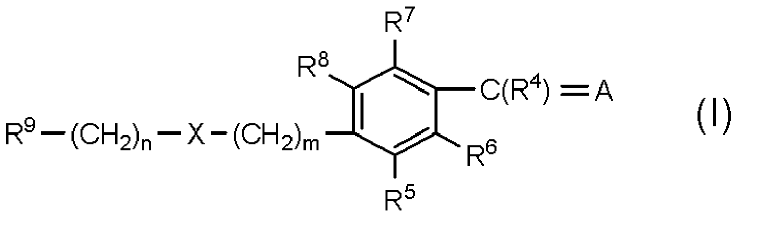 wherein each symbol is as described in the specification, or a salt thereof has a superior activity as an estrogen-related receptor-α modulator, and is useful as a prophylactic or therapeutic agent for cancer.

## Description

### Technical Field

The present invention relates to a novel compound having a superior activity as an estrogen-related receptor-α (ERR-α) modulator and useful as a drug for the prophylaxis or treatment of ERR-α associated diseases such as breast cancer and the like.

### (Background of the Invention)

Nuclear receptors are transcription regulators that respond to various stimuli such as physiological factors (e.g., development and differentiation), environmental factors and the like, and regulate gene expression in a ligand dependent manner. They form a gene superfamily based on their structural characteristics. Among them are included classic nuclear receptors such as estrogen receptor (ER) with estrogen as a ligand, and the like, and orphan nuclear receptors whose ligands and physiological functions are unknown.

Estrogen-related receptor-α (ERRα, NR3B1) belongs to the estrogen-related receptor subfamily (ERR subfamily) (orphan nuclear receptor closely related to ER). ERRβ and ERRγ have additionally been identified as the members of the ERR subfamily, and all the members show high homology with ER in the DNA-binding domain and the ligand-binding domain. ER and the ERR subfamily are known to have common target genes such as estrogen-responsive genes and the like.

Crosstalks occur between the signal transduction pathways of ER and the ERR subfamily (non-patent documents 1 - 4). Therefore, a compound capable of modulating the activity of ERRα (ERRα modulator) could provide a therapeutic effect for both the diseases related to ERRα and the diseases related to ER, through a direct modulation of the ERRα transcriptional effect or an indirect effect on the ER signal transduction pathway.

In consideration of the wide activity of ERRα, an ERRα modulator is expected to be useful for the treatment or prophylaxis of various disease conditions (e.g., cancers such as breast cancer and the like, diabetes, hyperlipidemia, obesity, metabolic syndrome, arthritis, atherosclerosis, rheumatoid arthritis, osteoporosis, anxiety, depression, Parkinson's disease, Alzheimer's disease etc.) (patent documents 1, 2, non-patent document 5).
In recent years, it has been reported that siRNA and a low-molecular-weight compound having an ERRα inhibitory activity can provide a good antitumor effect against estrogen receptor-positive and -negative breast cancers in mouse models (non-patent documents 6, 7).

As ERRα modulators, the following compounds are known. (1) As an ERRα inverse agonist etc., patent document 3 (WO2006/047269) describes a compound represented by the formula:

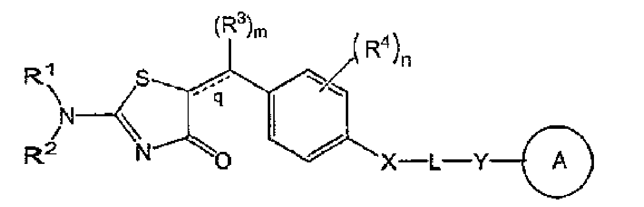

wherein
bond q is a single bond or double bond;
R¹ and R² are, each independently, alkyl substituted as necessary, alkenyl substituted as necessary, alkynyl substituted as necessary and the like; R² may further be hydrogen; or
R¹ and R² form, together with the nitrogen atom bonded thereto, heterocycle or heteroaryl ring substituted as necessary;
R³ is hydrogen, halo or alkyl substituted as necessary;
each R⁴ is, independently, halo, cyano, nitro, alkyl substituted as necessary, alkenyl substituted as necessary and the like;
m is an integer of 1 - 2;
n is an integer of 0 - 4;
X is -O-, -NR⁸-, -S(O)ᵤ- (wherein u is an integer of 0 - 2) or a direct bond;
L is branched or straight chain alkylene chain substituted as necessary and having 1 - 6 carbons, cycloalkyl substituted as necessary and having 3 - 6 carbons and the like;
Y is -O-, -NR⁸-, -S(O)ᵤ- (wherein u is an integer of 0 - 2) or a direct bond;
A is aryl substituted as necessary or heteroaryl ring substituted as necessary; and
each R⁸ is, independently, hydrogen or alkyl substituted as necessary,
which is useful for the prophylaxis or treatment of ERR-associated diseases such as cancer, inflammatory disease, neuropathy, hyperglycemia and the like.

(2) Patent document 4 (WO2008/109727) describes a compound represented by the formula:

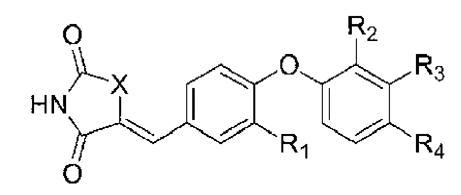

wherein
R₁ is halo, optionally substituted C₁₋₄ alkyl, optionally substituted C₁₋₄ alkoxy or hydroxyl;
R₂ is selected from halo-substituted C₁₋₃ alkyl, cyano, halo, - C(O)NH₂ and -C(O)O-C₁₋₄ alkyl, or R₂ form, together with R₃, aryl fused with a phenyl ring bonded thereto;
R₃ is H or R₃ form, together with R₂, aryl fused with a phenyl ring bonded thereto;
R₄ is halo, cyano, -C≡CH, halo-substituted C₁₋₃ alkyl, -C(O)O-C₁₋₄ alkyl, -C(O)NH₂ or -S(O₂)-C₁₋₄ alkyl; and
X is S or O,
which is useful for the prophylaxis or treatment of ERR-associated diseases such as breast cancer, diabetes and the like.

(3) Patent document 5 (WO2008/109731) describes a compound represented by the formula:

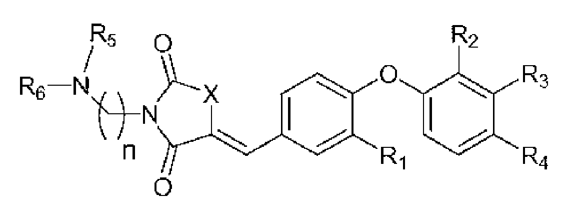

wherein
X is S or O;
N is 1 - 4;
R₁ is halo, optionally substituted C₁₋₄ alkyl, optionally substituted C₁₋₄ alkoxy or hydroxyl;
R₂ is selected from halo-substituted C₁₋₃ alkyl, cyano, halo, - C(O)NH2 and -C(O)O-C₁₋₄ alkyl, or R₂ form, together with R₃, aryl fused with a phenyl ring bonded thereto;
R₃ is H, or R₃ form, together with R₂, aryl fused with a phenyl ring bonded thereto;
R₄ is halo, cyano, halo-substituted C₁₋₃ alkyl, -C≡CH, -C(O)O-C₁₋₄ alkyl, -C(O)NH₂ or -S(O₂)-C₁₋₄ alkyl; and
R₅ and R₆ are each independently H or optionally substituted C₁₋₄ alkyl, or R₅ and R₆ form, together with the nitrogen atom bonded thereto, optionally substituted 5- to 9-membered nitrogen-containing heteroaryl or optionally substituted 5- to 7-membered nitrogen-containing heterocyclyl,
which is useful for the prophylaxis or treatment of ERR-associated diseases such as breast cancer, diabetes and the like.

(4) As an ERR-α inverse agonist, patent document 6 (WO2008/109737) describes a compound represented by the formula:

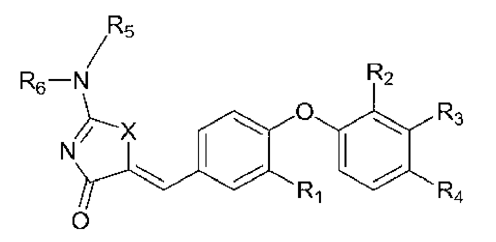

wherein
X is S or O;
R₁ is halo, optionally substituted C₁₋₄ alkyl, optionally substituted C₁₋₄ alkoxy or hydroxyl;
R₂ is selected from halo-substituted C₁₋₃ alkyl, cyano, halo, - C(O)NH₂ and -C(O)O-C₁₋₄ alkyl, or R₂ form, together with R₃, aryl fused with a phenyl ring bonded thereto;
R₃ is H, or R₃ form, together with R₂, aryl fused with a phenyl ring bonded thereto;
R₄ is halo, cyano, halo-substituted C₁₋₃ alkyl, -C≡CH, -C(O)O-C₁₋₄ alkyl, -C(O)NH₂ or -S(O₂)-C₁₋₄ alkyl; and
R₅ and R₆ are each independently selected from H, optionally substituted C₁₋₄ alkyl, optionally substituted heteroaryl, and optionally substituted heterocyclyl, or R₅ and R₆ form, together with the nitrogen atom bonded thereto, optionally substituted nitrogen-containing heterocyclyl,
which is useful for the prophylaxis or treatment of ERR-α associated diseases such as breast cancer, metabolic disease and the like.

(5) As an ERR-α antagonist, patent document 7 (WO2006/019741) describes a compound represented by the formula:

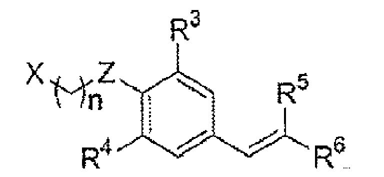

wherein
n is 0 or 1;
Z is -O-, -S-, >NH or >NR^{a} (wherein R^{a} is alkyl, cycloalkyl, phenyl or heterocycloalkyl);
X is an aryl or heteroaryl group;
R³ is -H or unsubstituted or substituted -O-alkyl;
R⁴ is selected from the group consisting of -H, halo, -O-alkyl, -CN, -NO₂ and -COOH; and
R⁵ and R⁶ are each independently -CN; -COOH; or -COO-alkyl etc.; or R⁵ and R⁶ form, together with the carbon bonded thereto, optionally benzocondensed heterocycloalkyl or cycloalkyl moiety,
which is useful for the prophylaxis or treatment of ERR-α associated diseases such as breast cancer, metabolic disease and the like.

(6) Patent document 8 (US-A-2008-0221179) describes a compound represented by the formula:

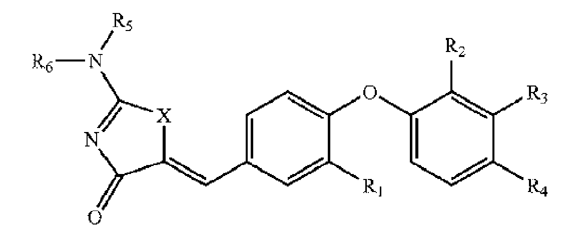

wherein
X is S or O;
R₁ is halo, optionally substituted C₁₋₄ alkyl, optionally substituted C₁₋₄ alkoxy or hydroxyl;
R₂ is selected from halo-substituted C₁₋₃ alkyl, cyano, halo, - C(O)NH₂ and -C(O)O-C₁₋₄ alkyl, or R₂ form, together with R₃, aryl fused with a phenyl ring bonded thereto;
R₃ is H, or R₃ form, together with R₂, aryl fused with a phenyl ring bonded thereto;
R₄ is halo, cyano, halo-substituted C₁₋₃ alkyl, -C≡CH, -C(O)O-C₁₋₄ alkyl, -C(O)NH₂ or -S(O₂)-C₁₋₄ alkyl; and
R₅ and R₆ are independently selected from H, optionally substituted C₁₋₄ alkyl, optionally substituted heteroaryl, and optionally substituted heterocyclyl, or R₅ and R₆ form, together with the nitrogen atom bonded thereto, optionally substituted nitrogen-containing heterocyclyl,
which is useful for the prophylaxis or treatment of ERR-α associated diseases such as breast cancer, chronic bronchitis and the like.

(7) Patent document 9 (US-A-2006-0014812) describes a compound represented by the formula:

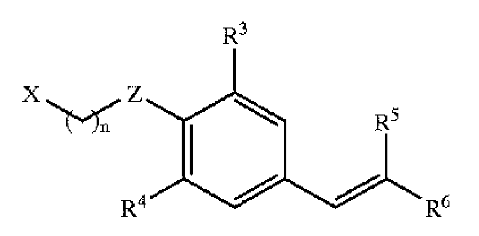

wherein
n is 0 or 1;
Z is -O-, -S-, >NH or >NR^{a} (wherein R^{a} is alkyl, cycloalkyl, phenyl or heterocycloalkyl);
X is an aryl or heteroaryl group;
R³ is -H or unsubstituted or substituted -O-alkyl;
R⁴ is selected from the group consisting of -H, halo, -O-alkyl, -CN, -NO₂ and -COOH; and
R⁵ and R⁶ are each independently -CN; -COOH; or -COO-alkyl etc.; or R⁵ and R⁶ form, together with the carbon bonded thereto, optionally benzocondensed heterocycloalkyl or cycloalkyl moiety,
which is useful for the prophylaxis or treatment of ERR-α associated diseases such as breast cancer, diabetes and the like.

(8) As an ERR-α inverse agonist, patent document 10 (WO2005/072731) describes a compound represented by the formula:

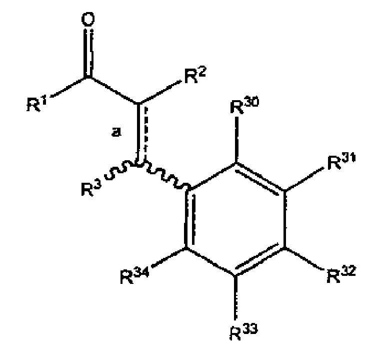

wherein
bond a is a single bond or double bond;
R¹ is selected from the group consisting of hydrogen, optionally substituted haloalkyl, optionally substituted haloalkenyl, optionally substituted alkyl etc.;
R² is selected from the group consisting of hydrogen, halo, cyano, nitro, azido, optionally substituted alkyl, optionally substituted alkenyl etc.;
R³ is selected from the group consisting of hydrogen, optionally substituted alkyl, optionally substituted alkenyl etc.;
R³⁰, R³¹, R³², R³³ and R³⁴ are each independently selected from the group consisting of hydrogen, halo, cyano, nitro, azido, optionally substituted alkyl, optionally substituted alkenyl etc.; or
a pair of adjacent substituents selected from R³⁰ and R³¹, R³¹ and R³², R³² and R³³, and R³³ and R³⁴ form, together with the carbon bonded thereto, optionally substituted cycloalkyl, optionally substituted heterocyclyl, optionally substituted aryl or optionally substituted heteroaryl; and the remaining unpaired substituents R³⁰, R³¹, R³², R³³ and R³⁴ are as mentioned above,
which is useful for the prophylaxis or treatment of ERR-α associated diseases such as cancer, inflammatory disease, Alzheimer's disease and the like.

Moreover, as a histamine H3 receptor modulator, patent document 11 (WO02/24695) describes a compound represented by the formula:

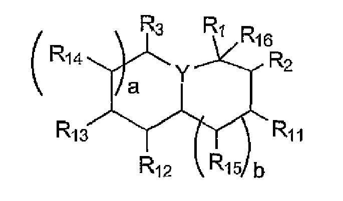

wherein
a is 0, and b is 0; a is 1, and b is 0; or a is 1, and b is 1; Y is selected from N and N→O;
one of R₁, R₂ and R₃ is a ring moiety selected from C₄₋₆ cycloalkyl, phenyl, naphthyl, C₁₋₅ heterocyclyl etc.;
the remaining two from R₁, R₂ and R₃ are independently selected from hydrogen, halogen and C₁₋₆ alkyl;
R₁₁. R₁₂, R₁₄ and R₁₅ are each independently selected from hydrogen, halogen, C₁₋₆ alkyl and C₁₋₆ alkoxy;
R₁₃ is selected from hydrogen, oxo and phenyl; and
R₁₆ is selected from hydrogen, cyano, C₁₋₆ alkyl and C₁₋₆ alkylamino,
which is useful for the prophylaxis or treatment of Alzheimer's disease, eating disorder and the like.

In addition, as a γ-secretase modulator, patent document 12 (WO2009/005729) describes a compound represented by the formula:

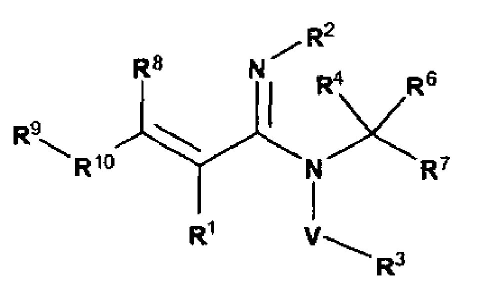

wherein
a ring moiety may be formed when
(i) R¹ and R³ are joined to form an optionally substituted 5 - 8-membered heterocyclyl moiety and the like;
(ii) R³ and R⁴ are joined to form an optionally substituted 5 - 8-membered heterocyclyl moiety; and the like; V is selected from the group consisting of a bond, -O-, -S(O₂)-, -S(O)- etc.;
R¹ (when not bonded to R³) is selected from the group consisting of H, alkyl, aryl etc.; or R¹ (when not bonded to R³) is joined with R⁸ to form a bond;
R² is selected from the group consisting of H, alkyl, cycloalkyl etc.;
R³ (when not bonded to R¹) is selected from the group consisting of H, alkyl, aryl etc.;
R ⁴ R⁶ and R⁷ are each independently selected from the group consisting of H, alkyl, aryl etc.;
R⁸ is selected from the group consisting of H, alkyl, aryl etc.;
R⁹ is selected from the group consisting of alkyl, aryl etc.; and
R¹⁰ is selected from the group consisting of a bond, alkyl, aryl etc.,
which is useful for the prophylaxis or treatment of Alzheimer's disease and the like.

As a light-absorbing compound, patent document 13 (WO02/080160) describes a compound represented by the formula:

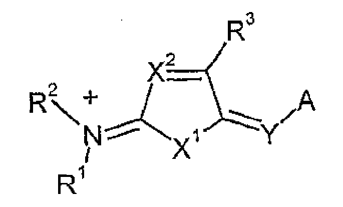

wherein
X¹ is O or S;
X² is N and the like;
R³ is hydrogen, NR⁸R⁹ and the like;
R¹, R², R⁸ and R⁹ are each independently hydrogen, C₁₋₆ alkyl and the like;
Y is =Y¹-(Y²=Y³)ₙ-;
Y¹ - Y³ are each independently N or C-R¹⁸;
n is 0 or 1;
R¹⁸ is hydrogen, cyano or C₁₋₃ alkyl; and
A is a group represented by the formula:

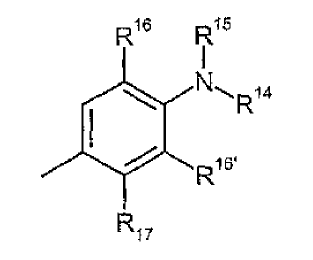

wherein
R¹⁴ and R¹⁵ are each independently hydrogen, C₁₋₁₆ alkyl, C₄₋₇ cycloalkyl, C₇₋₁₆ aralkyl, C₆₋₁₀ aryl and the like;
R¹⁶ and R¹⁶' are each independently hydrogen, C₁₋₆ alkyl and the like; and
R¹⁷ is hydrogen, C₁₋₆ alkyl and the like, and the like.

The following compounds are registered in Chemical Abstract:

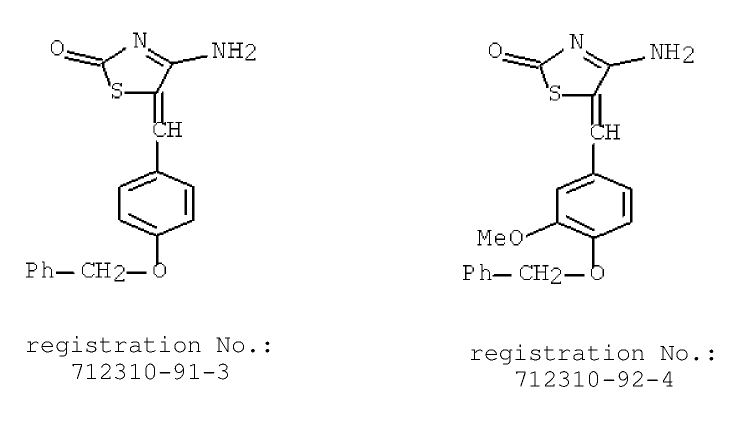

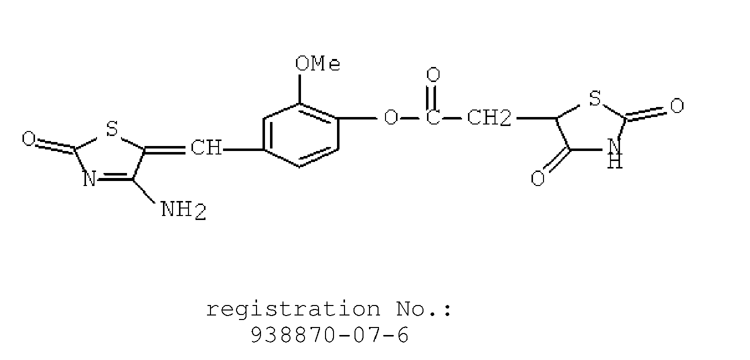

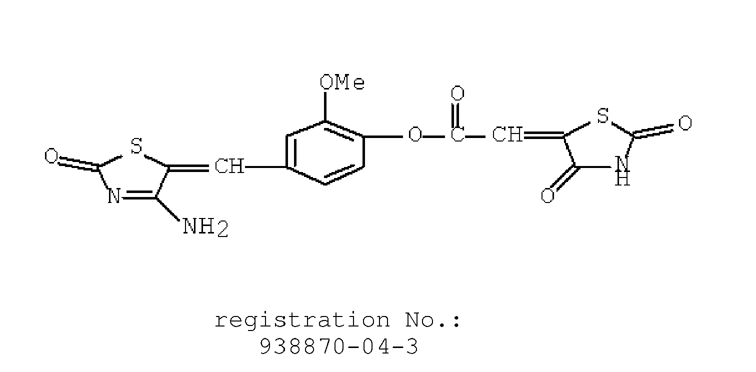

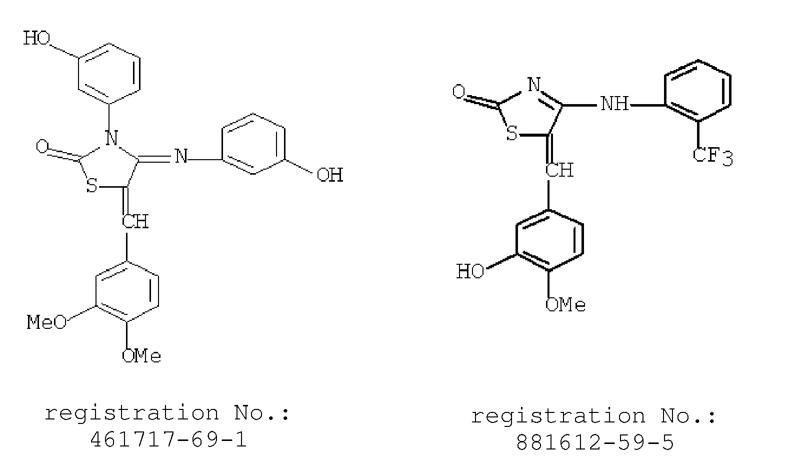

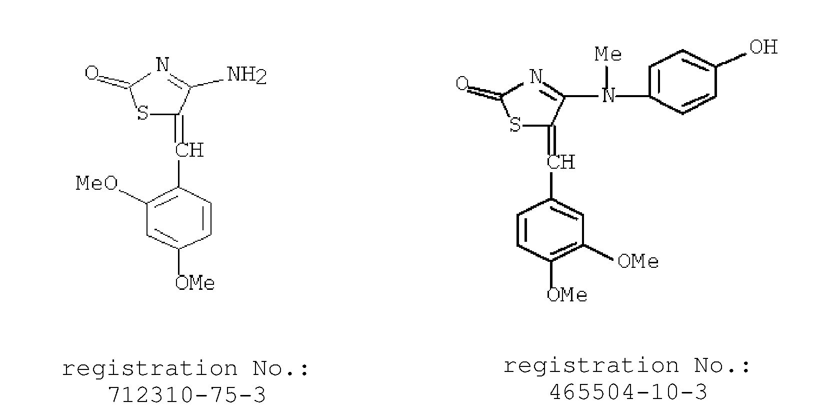

patent document 1: US-A-2003-0152959
patent document 2: US-A-2002-0187953
patent document 3: WO2006/047269
patent document 4: WO2008/109727
patent document 5: WO2008/109731
patent document 6: WO2008/109737
patent document 7: WO2006/019741
patent document 8: US-A-2008-0221179
patent document 9: US-A-2006-0014812
patent document 10: WO2005/072731
patent document 11: WO02/24695
patent document 12: WO2009/005729
patent document 13: WO02/080160
non-patent document 1: J. Clin. Endocrinol. Metab., 2005, vol. 90, p.3115-21
non-patent document 2: EMBO J., 1999, vol. 18, p.4270-9 non-patent document 3: Mol. Cell Endocrinol., 2001, vol. 172, p.223-33
non-patent document 4: J. Biol. Chem., 1996, vol. 271, 5795-804
non-patent document 5: Circulation, 2004, vol. 109, p.433-8 non-patent document 6: Cancer Research, 2008, vol. 68, p.8805-12
non-patent document 7: Mol. Cancer Ther., 2009, vol. 8, p.672-81

### [SUMMARY OF THE INVENTION]

### Problems to be Solved by the Invention

There is a demand for the development of a novel compound having a superior activity as an ERR-α modulator and useful as a drug for the prophylaxis or treatment of ERR-α associated diseases such as cancer and the like, and the like.

### Means of Solving the Problems

The present inventors have conducted intensive studies in an attempt to solve the above-mentioned problem and found that the following compound represented by the formula (I) or a salt thereof has a superior activity as an ERR-α modulator, and conducted further studies, which resulted in the completion of the present invention.

Accordingly, the present invention relates to [1] a compound represented by the formula

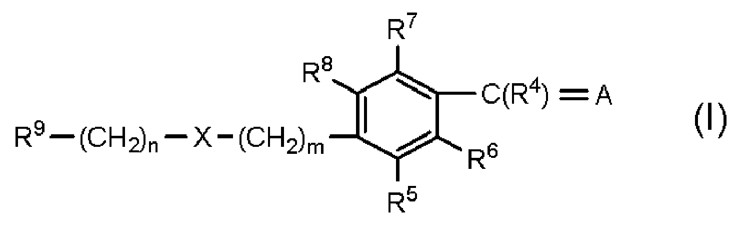

wherein,
A is a group represented by

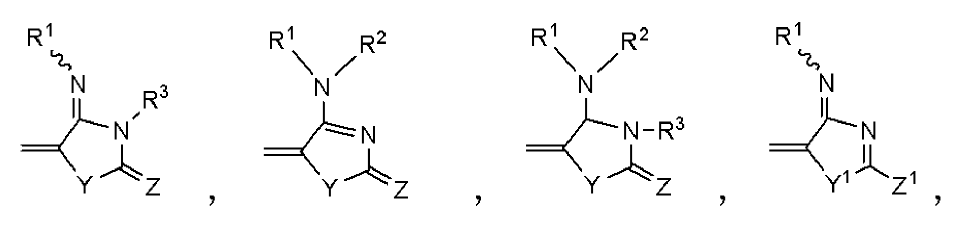

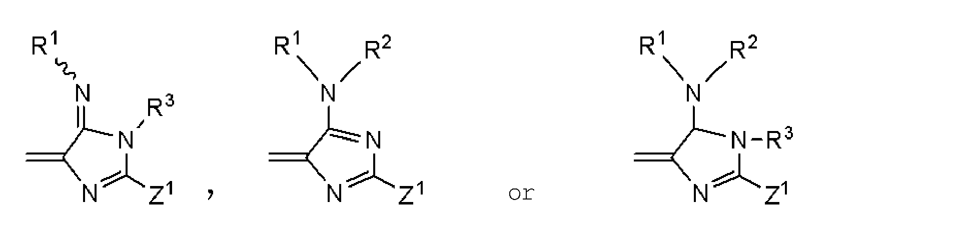

wherein
R¹, R² and R³ are each independently a hydrogen atom, a hydrocarbon group optionally having substituent(s), a heterocyclic group optionally having substituent(s), an acyl group or a hydroxyl group optionally substituted by an alkyl group optionally having substituent(s);
Y is -O-, -S- or -NR^{a}- wherein R^{a} is a hydrogen atom, a hydrocarbon group optionally having substituent(s), a heterocyclic group optionally having substituent(s), an acyl group or a hydroxyl group optionally substituted by an alkyl group optionally having substituent(s);
Z is =O, =S or =NR^{b} wherein R^{b} is a hydrogen atom, a hydrocarbon group optionally having substituent(s), a heterocyclic group optionally having substituent(s), an acyl group or a hydroxyl group optionally substituted by an alkyl group optionally having substituent(s);
Y¹ is -O-, -S- or -NR^{c}- wherein R^{c} is a hydrogen atom, a hydrocarbon group optionally having substituent(s), a heterocyclic group optionally having substituent(s) or an acyl group; and
Z¹ is -OR^{d}, -SR^{d} or -NHR^{d} wherein R^{d} are each independently a hydrogen atom, a hydrocarbon group optionally having substituent(s), a heterocyclic group optionally having substituent(s) or an acyl group;
R⁴ is a hydrogen atom or an alkyl group optionally having substituent(s);
R⁵, R⁶, R⁷ and R⁸ are each independently a hydrogen atom, a hydrocarbon group optionally having substituent(s), an amino group optionally having substituent(s), a hydroxyl group optionally having a substituent, a thiol group optionally having a substituent, a carbamoyl group optionally having substituent(s), an alkyloxycarbonyl group optionally having substituent(s), a halogen atom, a cyano group or a nitro group;
m and n are each independently an integer of 0 - 3;
X is -O-, -CO-, -O-CO-, -CO-O-, -SO₂-, -SO-, -S-, -SO₂-O-, - NR^{c1}-, -CO-NR^{c1}-, -NR^{c1}-CO-, -NR^{c1}-CO-NR^{c2}-, -O-CO-NR^{c1}-, -NR^{c1}-COO-, -SO₂-NR^{c1}-, -NR^{c1}-SO₂- or -NR^{c1}-SO₂-NR^{c2}-
wherein R^{c1} and R^{c2} are each independently a hydrogen atom, a hydrocarbon group optionally having substituent(s), a heterocyclic group optionally having substituent(s), an acyl group or a hydroxyl group optionally substituted by an alkyl group optionally having substituent(s); and
R⁹ is an aromatic ring group optionally having substituent(s), excluding the following two compounds:

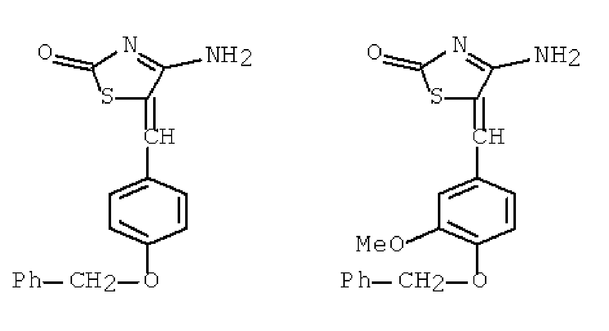

or a salt thereof (hereinafter sometimes to be abbreviated as compound (I));

[2] the compound according to the above-mentioned [1], wherein
A is

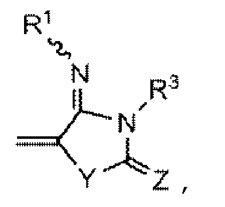

R¹, Y and Z are as defined in the above-mentioned [1], and
R³ is a hydrocarbon group optionally having substituent(s), a heterocyclic group optionally having substituent(s), an acyl group or a hydroxyl group optionally substituted by an alkyl group optionally having substituent(s),
or a salt thereof; [3] the compound according to the above-mentioned [1], wherein
A is

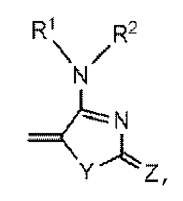

R¹ is a hydrocarbon group optionally having substituent(s) or a heterocyclic group optionally having substituent(s),
R² is a hydrogen atom, and
Y and Z are as defined in the above-mentioned [1],
or a salt thereof;
[4] the compound according to any of the above-mentioned [1] - [3], wherein R⁵, R⁶, and R⁷ are each a hydrogen atom, and R⁸ is a hydroxyl group substituted by a C₁₋₁₀ alkyl group optionally having substituent(s), or a salt thereof;
[5] the compound according to any of the above-mentioned [1] - [4], wherein X is -O-, or a salt thereof;
[6] the compound according to any of the above-mentioned [1] - [5], wherein R⁹ is C₆₋₁₀ aryl optionally having substituent(s), or a salt thereof;
[7] the compound according to any of the above-mentioned [1] - [6], wherein R⁴ is a hydrogen atom, or a salt thereof;
[8] (5Z)-5-({4-[2-chloro-4-(trifluoromethyl)phenoxy]-3-methoxyphenyl}methylidene)-4-{[2-(diethylamino)ethyl]amino}-1,3-thiazol-2(5H)-one or a salt thereof;
[9] 4-(2-methoxy-4-{(Z)-[4-{[2-(1-methylpyrrolidin-2-yl)ethyl]aminol-2-oxo-1,3-thiazol-5(2H)-ylidene]methyl}phenoxy)naphthalene-1-carbonitrile or a salt thereof;
[10] 4-{4-[(Z)-{4-[(2-hydroxy-2-methylpropyl)amino]-2-oxo-1,3-thiazol-5(2H)-ylidene}methyl]-2-methoxyphenoxy}-3-(trifluoromethyl)benzonitrile or a salt thereof;
[11] 4-{4-[(Z)-(1-ethyl-5-imino-3-methyl-2-oxoimidazolidin-4-ylidene)methyl]-2-methoxyphenoxy}-3-(trifluoromethyl)benzonitrile or a salt thereof;
[12] a prodrug of the compound according to the above-mentioned [1] or a salt thereof;
[13] a medicament containing the compound according to the above-mentioned [1] or a salt thereof or a prodrug thereof;
[14] the medicament according to the above-mentioned [13], which is an estrogen-related receptor-α inverse agonist;
[15] the medicament according to the above-mentioned [13], which is a prophylactic or therapeutic drug for cancer;
[16] a method for the prophylaxis or treatment of cancer in a mammal, comprising administering an effective amount of the compound according to the above-mentioned [1] or a salt thereof or a prodrug thereof to the mammal;
[17] use of the compound according to the above-mentioned [1] or a salt thereof or a prodrug thereof for the production of a prophylactic or therapeutic agent for cancer;
[18] the compound according to the above-mentioned [1], wherein A is

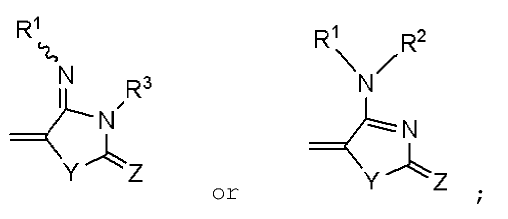

[19] the compound according to the above-mentioned [1], wherein at least one of R⁵, R⁶ R⁷ and R⁸ is a hydrocarbon group optionally having substituent(s), an amino group optionally having substituent(s), a hydroxyl group optionally having a substituent, a thiol group optionally having a substituent, a carbamoyl group optionally having substituent(s), an alkyloxycarbonyl group optionally having substituent(s), a halogen atom, a cyano group or a nitro group;
[20] the medicament according to the above-mentioned [15], wherein the cancer is a solid tumor confirmed to show promoted expression of ERR-α;
[21] the medicament according to the above-mentioned [15], wherein the cancer is lung cancer, prostate cancer, ovarian cancer, endometrial carcinoma or breast cancer;
and the like.

### (Detailed Description of the Invention)

In the present specification, unless otherwise specified, the "halogen atom" means a fluorine atom, a chlorine atom, a bromine atom or an iodine atom.
In the present specification, unless otherwise specified, the "C₁₋₆ alkyl group" means methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl and the like.
In the present specification, unless otherwise specified, the "C₁₋₆ alkoxy group" means methoxy, ethoxy, propoxy, isopropoxy, butoxy, isobutoxy, sec-butoxy, tert-butoxy, pentyloxy, hexyloxy and the like.
In the present specification, unless otherwise specified, the "C₁₋₆ alkoxy-carbonyl group" means methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl and the like.
In the present specification, unless otherwise specified, the "C₁₋₆ alkyl-carbonyl group" means acetyl, propanoyl, butanoyl, 2-methylpropanoyl, pentanoyl, 3-methylbutanoyl, 2-methylbutanoyl, 2,2-dimethylpropanoyl, hexanoyl, heptanoyl and the like.

The definition of each symbol in the formula (I) is described in detail in the following.
A is a group represented by

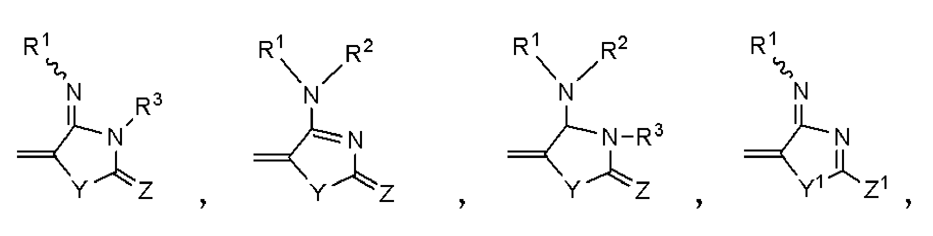

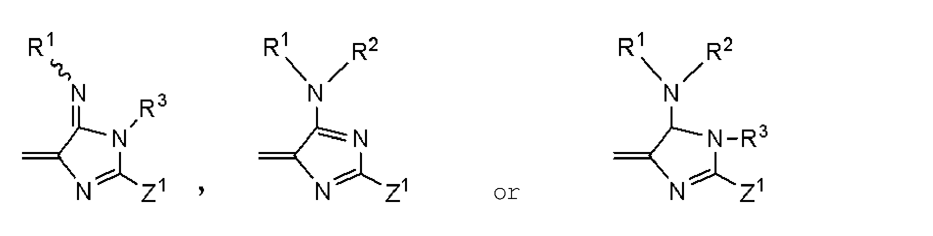

wherein each symbol is as defined above.

These groups may contain a tautomer. For example,

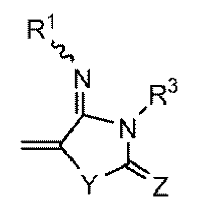

wherein R³ is a hydrogen atom contains a tautomer such as

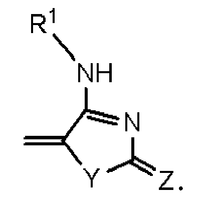

In the present specification, when A is, for example, a group represented by

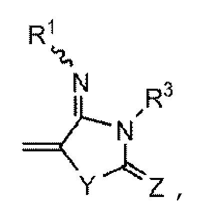

the group contains any of the above-mentioned tautomers. Similarly, various groups mentioned above may contain any possible tautomer.

R¹, R² and R³ are each independently a hydrogen atom, a hydrocarbon group optionally having substituent(s), a heterocyclic group optionally having substituent(s), an acyl group or a hydroxyl group optionally substituted by an alkyl group optionally having substituent(s).

Examples of the "hydrocarbon group" of the "hydrocarbon group optionally having substituent(s)" for R¹, R² or R³ include a C₁₋₁₀ alkyl group, a C₂₋₁₀ alkenyl group, a C₂₋₁₀ alkynyl group, a C₃₋₁₀ cycloalkyl group, a C₃₋₁₀ cycloalkenyl group, a C₄₋₁₀ cycloalkadienyl group, a C₆₋₁₄ aryl group, a C₇₋₁₃ aralkyl group, a C₈₋₁₃ arylalkenyl group, a C₃₋₁₀ cycloalkyl-C₁₋₆ alkyl group and the like.

Here, examples of the C₁₋₁₀ alkyl group include methyl, ethyl, propyl, isopropyl, butyl, isobutyl, sec-butyl, tert-butyl, pentyl, isopentyl, neopentyl, tert-pentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl and the like.
Examples of the C₂₋₁₀ alkenyl group include ethenyl, 1-propenyl, 2-propenyl, 2-methyl-l-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octenyl and the like.
Examples of the C₂₋₁₀ alkynyl group include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 1-octynyl and the like.

Examples of the C₃₋₁₀ cycloalkyl group include cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl, adamantyl and the like.
Examples of the C₃₋₁₀ cycloalkenyl group include 2-cyclopenten-1-yl, 3-cyclopenten-1-yl, 2-cyclohexen-1-yl, 3-cyclohexen-1-yl and the like.
Examples of the C₄₋₁₀ cycloalkadienyl group include 2, 4-cyclopentadien-1-yl, 2,4-cyclohexadien-1-yl, 2,5-cyclohexadien-1-yl and the like.
The above-mentioned C₃₋₁₀ cycloalkyl group, C₃₋₁₀ cycloalkenyl group and C₄₋₁₀ cycloalkadienyl group each may be fused with a benzene ring, and examples of such fused ring group include indanyl, dihydronaphthyl, tetrahydronaphthyl, fluorenyl and the like.

Examples of the C₆₋₁₄ aryl group include phenyl, naphthyl, anthryl, phenanthryl, acenaphthylenyl, biphenylyl and the like.
Examples of the C₇₋₁₃ aralkyl group include benzyl, phenethyl, naphthylmethyl, biphenylylmethyl, phenylpropyl and the like.
Examples of the C₈₋₁₃ arylalkenyl group include styryl and the like.
Examples of the C₃₋₁₀ cycloalkyl-C₁₋₆ alkyl group include cyclopropylmethyl, cyclohexylmethyl, 1-cyclohexylethyl, 2-cyclohexylethyl and the like.

The C₁₋₁₀ alkyl group, C₂₋₁₀ alkenyl group and C₂₋₁₀ alkynyl group exemplified as the aforementioned "hydrocarbon group" optionally have 1 to 3 substituents at substitutable position(s).
Examples of such substituent include
(1) a C₃₋₁₀ cycloalkyl group (e.g., cyclopropyl, cyclohexyl) optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group, and
   (d) a halogen atom;
(2) a C₆₋₁₄ aryl group (e.g., phenyl, naphthyl) optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group, and
   (d) a halogen atom;
(3) an aromatic heterocyclic group (e.g., thienyl, furyl, pyrrolyl, pyridyl, oxazolyl, thiazolyl, tetrazolyl, oxadiazolyl, pyrazinyl, quinolyl, indolyl, pyrimidinyl, imidazolyl, pyrazolyl, thiadiazolyl, isoxazolyl) optionally substituted by 1 to 3 substituents selected from
   (a) a C₁₋₆ alkyl group optionally substituted by 1 to 3 substituents selected from a halogen atom and a hydroxy group,
   (b) a hydroxy group,
   (c) a C₁₋₆ alkoxy group, and
   (d) a halogen atom;
(4) a non-aromatic heterocyclic group (e.g., tetrahydrofuryl, morpholinyl, thiomorpholinyl, piperidinyl, pyrrolidinyl, piperazinyl, dioxolyl, dioxolanyl, 1,3-dihydro-2-benzofuranyl, thiazolidinyl, oxooxadiazolyl, tetrahydropyranyl) optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group,
   (d) a halogen atom, and
   (e) a C₁₋₆ alkyl-carbonyl group;
(5) an amino group optionally mono- or di-substituted by substituent(s) selected from
   (a) a C₁₋₆ alkyl group optionally substituted by 1 to 3 substituents selected from
      (i) a C₁₋₆ alkoxy group,
      (ii) a C₆₋₁₄ aryloxy group (e.g., phenoxy),
      (iii) a carboxyl group,
      (iv) a C₁₋₆ alkoxy-carbonyl group, and
      (v) a hydroxy group,
   (b) a C₇₋₁₃ aralkyloxy group (e.g., benzyloxy),
   (c) a C₁₋₆ alkyl-carbonyl group optionally substituted by 1 to 3 substituents selected from
      (i) a hydroxy group, and
      (ii) an aromatic heterocyclic group (e.g., thienyl, furyl, pyridyl, oxazolyl, thiazolyl, tetrazolyl, oxadiazolyl, pyrazinyl, quinolyl, indolyl, imidazolyl, pyrazolyl, thiadiazolyl, isoxazolyl),
   (d) a C₃₋₁₀ cycloalkyl-carbonyl group (e.g., cyclopropylcarbonyl, cyclopentylcarbonyl, cyclohexylcarbonyl),
   (e) a C₁₋₆ alkoxy-carbonyl group,
   (f) a C₆₋₁₄ aryl-carbonyl group (e.g., benzoyl) optionally substituted by 1 to 3 substituents selected from
      (i) a C₁₋₆ alkyl group optionally substituted by 1 to 3 halogen atoms (e.g., trifluoromethyl), and
      (ii) a halogen atom,
   (g) a C₇₋₁₃ aralkyl-carbonyl group (e.g., benzylcarbonyl, phenethylcarbonyl),
   (h) an aromatic heterocyclyl-carbonyl group (e.g., thienylcarbonyl, furylcarbonyl, pyridylcarbonyl, oxazolylcarbonyl, thiazolylcarbonyl, tetrazolylcarbonyl, oxadiazolylcarbonyl, pyrazinylcarbonyl, quinolylcarbonyl, indolylcarbonyl, imidazolylcarbonyl, pyrazolylcarbonyl, thiadiazolylcarbonyl, isoxazolylcarbonyl) optionally substituted by 1 to 3 C₁₋₆ alkyl groups,
   (i) an aromatic heterocyclyl-carbamoyl group (e.g., thienylcarbamoyl, furylcarbamoyl, pyridylcarbamoyl, oxazolylcarbamoyl, thiazolylcarbamoyl, tetrazolylcarbamoyl, oxadiazolylcarbamoyl, pyrazinylcarbamoyl, quinolylcarbamoyl, indolylcarbamoyl, imidazolylcarbamoyl, pyrazolylcarbamoyl, thiadiazolylcarbamoyl, isoxazolylcarbamoyl),
   (j) a C₁₋₆ alkyl-carbamoyl group (e.g., methylcarbamoyl, ethylcarbamoyl),
   (k) a C₆₋₁₄ aryl-carbamoyl group (e.g., phenylcarbamoyl, 1-naphthylcarbamoyl, 2-naphthylcarbamoyl),
   (l) a C₇₋₁₃ aralkyl-carbamoyl group (e.g., benzylcarbamoyl),
   (m) a C₁₋₆ alkyl-sulfonyl group (e.g., methylsulfonyl, ethylsulfonyl, isopropylsulfonyl),
   (n) a C₆₋₁₄ aryl-sulfonyl group (e.g., benzenesulfonyl, toluenesulfonyl, 1-naphthalenesulfonyl, 2-naphthalenesulfonyl), and
   (o) a C₇₋₁₃ aralkyl-sulfonyl group (e.g., benzylsulfonyl);
(6) an amidino group;
(7) a C₁₋₆ alkyl-carbonyl group optionally substituted by 1 to 3 halogen atoms;
(8) a C₁₋₆ alkoxy-carbonyl group optionally substituted by 1 to 3 halogen atoms;
(9) a C₁₋₆ alkylsulfonyl group (e.g., methylsulfonyl) optionally substituted by 1 to 3 halogen atoms;
(10) a carbamoyl group optionally mono- or di-substituted by substituents selected from
   (a) a C₁₋₆ alkyl group optionally substituted by 1 to 3 substituents selected from
      (i) a halogen atom,
      (ii) a cyano group,
      (iii) a hydroxy group, and
      (iv) an aromatic heterocyclic group (e.g., thienyl, furyl, pyridyl, oxazolyl, thiazolyl, tetrazolyl, oxadiazolyl, pyrazinyl, quinolyl, indolyl, imidazolyl, pyrazolyl, thiadiazolyl, isoxazolyl),
   (b) a C₆₋₁₄ aryl group (e.g., phenyl),
   (c) a C₇₋₁₃ aralkyl group (e.g., benzyl), and
   (d) an aromatic heterocyclyl-C₁₋₆ alkyl group (e.g., furfuryl);
(11) a thiocarbamoyl group optionally mono- or di-substituted by C₁₋₆ alkyl groups optionally substituted by 1 to 3 halogen atoms;
(12) a sulfamoyl group optionally mono- or di-substituted by C₁₋₆ alkyl groups optionally substituted by 1 to 3 halogen atoms;
(13) a carboxyl group;
(14) a hydroxy group;
(15) a C₁₋₆ alkoxy group optionally substituted by 1 to 3 substituents selected from
   (a) a halogen atom,
   (b) a carboxyl group,
   (c) a C₁₋₆ alkoxy group, and
   (d) a C₁₋₆ alkoxy-carbonyl group;
(16) a C₂₋₆ alkenyloxy group (e.g., ethenyloxy) optionally substituted by 1 to 3 halogen atoms;
(17) a C₃₋₁₀ cycloalkyloxy group (e.g., cyclohexyloxy);
(18) a C₇₋₁₃ aralkyloxy group (e.g., benzyloxy);
(19) a C₆₋₁₄ aryloxy group (e.g., phenoxy, naphthyloxy) optionally substituted by 1 to 3 halogen atoms;
(20) a C₁₋₆ alkyl-carbonyloxy group (e.g., acetyloxy, tert-butylcarbonyloxy);
(21) a mercapto group;
(22) a C₁₋₆ alkylthio group (e.g., methylthio, ethylthio) optionally substituted by 1 to 3 halogen atoms;
(23) a C₇₋₁₃ aralkylthio group (e.g., benzylthio);
(24) a C₆₋₁₄ arylthio group (e.g., phenylthio, naphthylthio);
(25) a sulfo group;
(26) a cyano group;
(27) an azido group;
(28) a nitro group;
(29) a nitroso group;
(30) a halogen atom;
(31) a C₁₋₆ alkylsulfinyl group (e.g., methylsulfinyl);
(32) a C₃₋₁₀ cycloalkyl-C₁₋₆ alkyloxy group (e.g., cyclopropylmethyloxy);
(33) a C₁₋₃ alkylenedioxy group;
(34) an N-hydroxycarbamoyl group;
(35) a mono or di-C₁₋₆ alkylphosphoryl group (e.g., diethylphosphoryl);
(36) a C₆₋₁₄ arylsulfonyl group (e.g., phenylsulfonyl);
(37) a non-aromatic heterocyclyl-carbonyl group (e.g., tetrahydrofurylcarbonyl, morpholinylcarbonyl, thiomorpholinylcarbonyl, piperidinylcarbonyl, pyrrolidinylcarbonyl, piperazinylcarbonyl, dioxolylcarbonyl, dioxolanylcarbonyl, 1,3-dihydro-2-benzofuranylcarbonyl, thiazolidinylcarbonyl) optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group, and
   (d) a halogen atom;
and the like. When the number of substituents is two or more, the substituents may be the same or different.

The C₆₋₁₄ aryl group, C₇₋₁₃ aralkyl group, and C₈₋₁₃ arylalkenyl group exemplified as the aforementioned "hydrocarbon group" optionally have 1 to 3 substituents at substitutable position(s).
Examples of such substituent include
(1) the aforementioned groups exemplified as the substituents that C₁₋₁₀ alkyl group and the like may have;
(2) a C₁₋₆ alkyl group optionally substituted by 1 to 3 substituents selected from
   (a) a halogen atom,
   (b) a carboxyl group,
   (c) a hydroxy group optionally substituted by a non-aromatic heterocyclic group (e.g., piperidino, tetrahydropyranyl),
   (d) an amino group optionally mono- or di-substituted by C₁₋₆ alkyl groups,
   (e) a C₆₋₁₄ aryl group (e.g., phenyl),
   (f) a C₁₋₆ alkoxy-carbonyl group,
   (g) a C₁₋₆ alkyl-carbonyloxy group (e.g., acetyloxy, tert-butylcarbonyloxy),
   (h) a carbamoyl group,
   (i) an aromatic heterocyclic group (e.g., thienyl, furyl, pyridyl, oxazolyl, thiazolyl, tetrazolyl, oxadiazolyl, pyrazinyl, quinolyl, indolyl, imidazolyl, pyrazolyl, thiadiazolyl, isoxazolyl) optionally substituted by 1 to 3 C₁₋₆ alkyl groups optionally substituted by 1 to 3 hydroxy groups (e.g., hydroxymethyl), and
   (j) a non-aromatic heterocyclic group (e.g., piperidino, tetrahydropyranyl);
(3) a C₂₋₆ alkenyl group (e.g., ethenyl, 1-propenyl) optionally substituted by 1 to 3 substituents selected from
   (a) a halogen atom,
   (b) a carboxyl group,
   (c) a C₁₋₆ alkoxy-carbonyl group, and
   (d) a carbamoyl group;
(4) a C₇₋₁₃ aralkyl group (e.g., benzyl) optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group, and
   (d) a halogen atom;
and the like. When the number of substituents is two or more, the substituents may be the same or different.

The C₃₋₁₀ cycloalkyl group, C₃₋₁₀ cycloalkenyl group, C₄₋₁₀ cycloalkadienyl group and C₃₋₁₀ cycloalkyl-C₁₋₆ alkyl group exemplified as the aforementioned "hydrocarbon group" optionally have 1 to 3 substituents at substitutable position(s).
Examples of such substituent include
(1) the groups exemplified as the substituent that the aforementioned C₆₋₁₄ aryl group and the like optionally have;
(2) an oxo group;
and the like. When the number of substituents is two or more, the substituents may be the same or different.

Examples of the "heterocyclic group" of the "heterocyclic group optionally having substituent(s)" for R¹, R² or R³ include an aromatic heterocyclic group and a non-aromatic heterocyclic group.
Here, examples of the aromatic heterocyclic group include a 4- to 7-membered (preferably 5- or 6-membered) monocyclic aromatic heterocyclic group containing, as a ring constituting atom besides carbon atom, 1 to 4 hetero atoms selected from an oxygen atom, a sulfur atom and a nitrogen atom, and a fused aromatic heterocyclic group. Examples of the fused aromatic heterocyclic group include a group wherein such 4- to 7-membered monocyclic aromatic heterocyclic group is fused with one or two selected from 5- or 6-membered aromatic heterocycle containing 1 or 2 nitrogen atoms (e.g., pyrrole, imidazole, pyrazole, pyrazine, pyridine, pyrimidine), a 5-membered aromatic heterocycle containing one sulfur atom (e.g., thiophene), a benzene ring and the like, and the like.

Preferable examples of the aromatic heterocyclic group include
monocyclic aromatic heterocyclic groups such as furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl (e.g., 2-pyrimidinyl, 4-pyrimidinyl, 5-pyrimidinyl), pyridazinyl (e.g., 3-pyridazinyl, 4-pyridazinyl), pyrazinyl (e.g., 2-pyrazinyl), pyrrolyl (e.g., 2-pyrrolyl, 3-pyrrolyl), imidazolyl (e.g., 1-imidazolyl, 2-imidazolyl, 4-imidazolyl, 5-imidazolyl), pyrazolyl (e.g., 1-pyrazolyl, 3-pyrazolyl, 4-pyrazolyl, 5-pyrazolyl), thiazolyl (e.g., 2-thiazolyl, 4-thiazolyl, 5-thiazolyl), isothiazolyl (e.g., 3-isothiazolyl, 4-isothiazolyl, 5-isothiazolyl), oxazolyl (e.g., 2-oxazolyl, 4-oxazolyl, 5-oxazolyl), isoxazolyl (e.g., 3-isoxazolyl, 4-isoxazolyl, 5-isoxazolyl), oxadiazolyl (e.g., 1,2,4-oxadiazol-5-yl, 1,3,4-oxadiazol-2-yl), thiadiazolyl (e.g., 1,3,4-thiadiazol-2-yl), triazolyl (e.g., 1,2,4-triazol-1-yl, 1,2,4-triazol-3-yl, 1,2,3-triazol-1-yl, 1,2,3-triazol-2-yl, 1,2,3-triazol-4-yl), tetrazolyl (e.g., tetrazol-1-yl, tetrazol-5-yl), triazinyl (e.g., 1,2,4-triazin-3-yl, 1,2,4-triazin-5-yl, 1,2,4-triazin-6-yl) and the like;
fused aromatic heterocyclic groups such as quinolyl (e.g., 2-quinolyl, 3-quinolyl, 4-quinolyl, 6-quinolyl), isoquinolyl (e.g., 3-isoquinolyl), quinazolyl (e.g., 2-quinazolyl, 4-quinazolyl), quinoxalyl (e.g., 2-quinoxalyl, 6-quinoxalyl), benzofuranyl (e.g., 2-benzofuranyl, 3-benzofuranyl, 4-benzofuranyl, 5-benzofuranyl, 6-benzofuranyl, 7-benzofuranyl), benzothienyl (e.g., 2-benzothienyl, 3-benzothienyl), benzoxazolyl (e.g., 2-benzoxazolyl), benzisoxazolyl (e.g., 7-benzisoxazolyl), benzothiazolyl (e.g., 2-benzothiazolyl), benzimidazolyl (e.g., benzimidazol-1-yl, benzimidazol-2-yl, benzimidazol-5-yl), benzotriazolyl (e.g., 1H-1,2,3-benzotriazol-5-yl), benzothiadiazolyl (e.g., 1,2,3-benzothiadiazol-5-yl), indolyl (e.g., indol-1-yl, indol-2-yl, indol-3-yl, indol-5-yl), indazolyl (e.g., 1H-indazol-3-yl), pyrrolopyrazinyl (e.g., 1H-pyrrolo[2,3-b]pyrazin-2-yl, 1H-pyrrolo[2,3-b]pyrazin-6-yl), imidazopyridinyl (e.g., 1H-imidazo[4,5-b]pyridin-2-yl, 1H-imidazo[4,5-c]pyridin-2-yl, 2H-imidazo[1,2-a]pyridin-3-yl), imidazopyrazinyl (e.g., 1H-imidazo[4,5-b]pyrazin-2-yl), pyrazolopyridinyl (e.g., 1H-pyrazolo[4,3-c]pyridin-3-yl), pyrazolothienyl (e.g., 2H-pyrazolo[3,4-b]thiophen-2-yl), pyrazolotriazinyl (e.g., pyrazolo[5,1-c][1,2,4]triazin-3-yl) and the like;
and the like.

Examples of the non-aromatic heterocyclic group include a 4- to 7-membered (preferably 5- or 6-membered) monocyclic non-aromatic heterocyclic group, containing, as a ring constituting atom besides carbon atom, 1 to 4 hetero atoms selected from an oxygen atom, a sulfur atom and a nitrogen atom, and a fused non-aromatic heterocyclic group. Examples of the fused non-aromatic heterocyclic group include a group wherein such 4- to 7-membered monocyclic non-aromatic heterocyclic group is fused with one or two selected from a 5-or 6-membered aromatic or non-aromatic heterocycle containing 1 or 2 nitrogen atoms (e.g., pyrrole, imidazole, pyrazole, pyrazine, pyridine, pyrimidine), a 5-membered aromatic or non-aromatic heterocycle containing one sulfur atom (e.g., thiophene), a benzene ring and the like, and the like.
Preferable examples of the non-aromatic heterocyclic group include
monocyclic non-aromatic heterocyclic groups such as pyrrolidinyl (e.g., 1-pyrrolidinyl, 2-pyrrolidinyl), piperidinyl (e.g., piperidino, 2-piperidinyl, 3-piperidinyl, 4-piperidinyl), homopiperidinyl (e.g., homopiperidino, 2-homopiperidinyl, 3-homopiperidinyl, 4-homopiperidinyl), tetrahydropyridyl (e.g., 1,2,3,6-tetrahydropyridin-1-yl), morpholinyl (e.g., morpholino), thiomorpholinyl (e.g., thiomorpholino), piperazinyl (e.g., 1-piperazinyl, 2-piperazinyl, 3-piperazinyl), hexamethyleniminyl (e.g., hexamethylenimin-1-yl), oxazolidinyl (e.g., oxazolidin-2-yl), thiazolidinyl (e.g., thiazolidin-2-yl), imidazolidinyl (e.g., imidazolidin-2-yl, imidazolidin-3-yl), oxazolinyl (e.g., oxazolin-2-yl), thiazolinyl (e.g., thiazolin-2-yl), imidazolinyl (e.g., imidazolin-2-yl, imidazolin-3-yl), dioxolyl (e.g., 1,3-dioxol-4-yl), dioxolanyl (e.g., 1,3-dioxolan-4-yl), dihydrooxadiazolyl (e.g., 4,5-dihydro-1,2,4-oxadiazol-3-yl), 2-thioxo-1,3-oxazolidin-5-yl, pyranyl (e.g., 4-pyranyl), tetrahydropyranyl (e.g., 2-tetrahydropyranyl, 3-tetrahydropyranyl, 4-tetrahydropyranyl), thiopyranyl (e.g., 4-thiopyranyl), tetrahydrothiopyranyl (e.g., 2-tetrahydrothiopyranyl, 3-tetrahydrothiopyranyl, 4-tetrahydrothiopyranyl), 1-oxidetetrahydrothiopyranyl (e.g., 1-oxidetetrahydrothiopyran-4-yl), 1,1-dioxidetetrahydrothiopyranyl (e.g., 1,1-dioxidetetrahydrothiopyran-4-yl), tetrahydrofuryl (e.g., tetrahydrofuran-3-yl, tetrahydrofuran-2-yl), pyrazolidinyl (e.g., pyrazolidin-1-yl, pyrazolidin-3-yl), pyrazolinyl (e.g., pyrazolin-1-yl), tetrahydropyrimidinyl (e.g., tetrahydropyrimidin-1-yl), dihydrotriazolyl (e.g., 2,3-dihydro-1H-1,2,3-triazol-1-yl), tetrahydrotriazolyl (e.g., 2,3,4,5-tetrahydro-1H-1,2,3-triazol-1-yl), oxodihydrooxadiazolyl (e.g., 5-oxo-4,5-dihydro-1,2,4-oxadiazol-3-yl) and the like;
fused non-aromatic heterocyclic groups such as dihydroindolyl (e.g., 2,3-dihydro-1H-indol-1-yl), dihydroisoindolyl (e.g., 1,3-dihydro-2H-isoindol-2-yl), dihydrobenzofuranyl (e.g., 2,3-dihydro-1-benzofuran-5-yl), dihydrobenzodioxinyl (e.g., 2,3-dihydro-1,4-benzodioxinyl), dihydrobenzodioxepinyl (e.g., 3,4-dihydro-2H-1,5-benzodioxepinyl), tetrahydrobenzofuranyl (e.g., 4,5,6,7-tetrahydro-1-benzofuran-3-yl), chromenyl (e.g., 4H-chromen-2-yl, 2H-chromen-3-yl, 2H-chromen-7-yl), dihydroquinolinyl (e.g., 1,2-dihydroquinolin-4-yl), tetrahydroquinolinyl (e.g., 1,2,3,4-tetrahydroquinolin-4-yl), dihydroisoquinolinyl (e.g., 1,2-dihydroisoquinolin-4-yl), tetrahydroisoquinolinyl (e.g., 1,2,3,4-tetrahydroisoquinolin-4-yl, 1,2,3,4-tetrahydroisoquinolin-2-yl), dihydrophthalazinyl (e.g., 1,4-dihydrophthalazin-4-yl), tetrahydrobenzoazepinyl (e.g., 2,3,4,5-tetrahydro-1H-benzo[c]azepin-1-yl) and the like;
and the like.

The aforementioned "aromatic heterocyclic group" and "non-aromatic heterocyclic group" optionally have 1 to 3 substituents at substitutable position(s).
Examples of such substituent of the "aromatic heterocyclic group" include those exemplified as the substituents that C₆₋₁₄ aryl and the like exemplified as the "hydrocarbon group" of the aforementioned "hydrocarbon group optionally having substituent(s)" may have. When the number of substituents is two or more, the substituents may be the same or different.
Examples of such substituent of the "non-aromatic heterocyclic group" include those exemplified as the substituents that C₃₋₁₀ cycloalkyl group and the like exemplified as the "hydrocarbon group" of the aforementioned "hydrocarbon group optionally having substituent(s)" may have. When the number of substituents is two or more, the substituents may be the same or different.

Examples of the "acyl group" for R¹, R² or R³ include groups represented by the formulas: -COR^{A}, -CO-OR^{A}, -SO₂R^{A}, - SOR^{A}, -CO-NR^{A}'R^{B}', -SO₂-NR^{A}'R^{B}', -SO₂-NR^{A}' (COR^{B}' ) or -CS-NR^{A}'R^{B}' wherein R^{A} is a hydrogen atom, a hydrocarbon group optionally having substituent(s) or a heterocyclic group optionally having substituent(s), R^{A}' is a hydrogen atom, a hydroxy group, a hydrocarbon group optionally having substituent(s) or a heterocyclic group optionally having substituent(s), and R^{B}' is a hydrogen atom, a hydrocarbon group optionally having substituent(s) or a heterocyclic group optionally having substituent(s), or R^{A}' and R^{B}' form, together with the adjacent nitrogen atom, a nitrogen-containing heterocycle optionally having substituent(s), and the like.
Examples of the "hydrocarbon group optionally having substituent(s)" and "heterocyclic group optionally having substituent(s)" for R^{A}, R^{A}' or R^{B}' include those similar to the "hydrocarbon group optionally having substituent(s)" and "heterocyclic group optionally having substituent(s)" for R¹ and the like.

Examples of the "nitrogen-containing heterocycle" of the "nitrogen-containing heterocycle optionally having substituent(s)" formed by R^{A}' and R^{B}' together with the adjacent nitrogen atom include a 5- to 7-membered nitrogen-containing heterocycle containing, as a ring constituting atom besides carbon atom, at least one nitrogen atom, and optionally further containing 1 or 2 hetero atoms selected from an oxygen atom, a sulfur atom and a nitrogen atom. Preferable examples of the nitrogen-containing heterocycle include pyrrolidine, imidazolidine, pyrazolidine, piperidine, piperazine, morpholine, thiomorpholine, oxopiperazine and the like.
The nitrogen-containing heterocycle optionally has 1 to 3 (preferably 1 or 2) substituents at substitutable position(s). Examples of such substituent include those exemplified as the substituent that the C₃₋₁₀ cycloalkyl group and the like exemplified as the "hydrocarbon group" of the aforementioned "hydrocarbon group optionally having substituent(s)" may have. When the number of substituents is two or more, the substituents may be the same or different.

Specific examples of the aforementioned "acyl group" include
(1) a formyl group,
(2) a C₁₋₆ alkyl-carbonyl group optionally having substituent(s),
(3) a C₂₋₆ alkenyl-carbonyl group optionally having substituent(s),
(4) a C₂₋₆ alkynyl-carbonyl group optionally having substituent(s),
(5) a C₃₋₆ cycloalkyl-carbonyl group optionally having substituent(s),
(6) a C₃₋₆ cycloalkenyl-carbonyl group optionally having substituent(s),
(7) a C₆₋₁₀ aryl-carbonyl group optionally having substituent(s),
(8) a heterocyclylcarbonyl group optionally having substituent(s),
(9) a carboxy group,
(10) a C₁₋₆ alkoxy-carbonyl group optionally having substituent(s),
(11) a C₂₋₆ alkenyloxy-carbonyl group optionally having substituent(s),
(12) a C₂₋₆ alkynyloxy-carbonyl group optionally having substituent(s),
(13) a C₃₋₆ cycloalkyloxy-carbonyl group optionally having substituent(s),
(14) a C₃₋₆ cycloalkenyloxy-carbonyl group optionally having substituent(s),
(15) a C₆₋₁₀ aryloxy-carbonyl group optionally having substituent(s),
(16) a heterocyclyloxycarbonyl group optionally having substituent(s),
(17) carbamoyl optionally having substituent(s),
(18) a C₁₋₆ alkyl-sulfonyl group optionally having substituent(s)
and the like.

Examples of the "C₂₋₆ alkenyl-carbonyl group" of the "C₂₋₆ alkenyl-carbonyl group optionally having substituent(s)" include ethenylcarbonyl, 1-propenylcarbonyl, 2-propenylcarbonyl, 2-methyl-1-propenylcarbonyl, 1-butenylcarbonyl, 2-butenylcarbonyl, 3-butenylcarbonyl, 3-methyl-2-butenylcarbonyl, 1-pentenylcarbonyl, 2-pentenylcarbonyl, 3-pentenylcarbonyl, 4-pentenylcarbonyl, 4-methyl-3-pentenylcarbonyl, 1-hexenylcarbonyl, 2-hexenylcarbonyl, 3-hexenylcarbonyl, 4-hexenylcarbonyl, 5-hexenylcarbonyl and the like.
Examples of the "C₂₋₆ alkynyl-carbonyl group" of the "C₂₋₆ alkynyl-carbonyl group optionally having substituent(s)" include ethynylcarbonyl, 1-propynylcarbonyl, 2-propynylcarbonyl, 1-butynylcarbonyl, 2-butynylcarbonyl, 3-butynylcarbonyl, 1-pentynylcarbonyl, 2-pentynylcarbonyl, 3-pentynylcarbonyl, 4-pentynylcarbonyl, 1-hexynylcarbonyl, 2-hexynylcarbonyl, 3-hexynylcarbonyl, 4-hexynylcarbonyl, 5-hexynylcarbonyl and the like.

Examples of the "C₃₋₆ cycloalkyl-carbonyl group" of the "C₃₋₆ cycloalkyl-carbonyl group optionally having substituent(s)" include cyclopropylcarbonyl, cyclobutylcarbonyl, cyclopentylcarbonyl, cyclohexylcarbonyl and the like.
Examples of the "C₃₋₆ cycloalkenyl-carbonyl group" of the "C₃₋₆ cycloalkenyl-carbonyl group optionally having substituent(s)" include 2-cyclopropen-1-ylcarbonyl, 2-cyclobuten-1-ylcarbonyl, 2-cyclopenten-1-ylcarbonyl, 3-cyclopenten-1-ylcarbonyl, 2-cyclohexen-1-ylcarbonyl, 3-cyclohexen-1-ylcarbonyl and the like.

Examples of the "C₆₋₁₀ aryl-carbonyl group" of the "C₆₋₁₀ aryl-carbonyl group optionally having substituent(s)" include benzoyl, 1-naphthoyl, 2-naphthoyl and the like.
Examples of the "heterocyclylcarbonyl group" of the "heterocyclylcarbonyl group optionally having substituent(s)" include (1) 5- or 6-membered monocyclic aromatic heterocycle (e.g., furan, thiophene, pyrrole, oxazole, isoxazole, thiazole, isothiazole, imidazole, pyridine, pyrazole etc.)-carbonyl, (2) 8- to 12-membered condensed aromatic heterocycle (e.g., benzofuran, isobenzofuran, benzothiophene, isobenzothiophene, indole, isoindole, 1H-indazole, benzimidazole, benzoxazole etc.)-carbonyl, (3) 3 to 6-membered non-aromatic heterocycle (e.g., oxirane, azetidine, oxetane, pyrrolidine, tetrahydrofuran, thioran, piperidine etc.)-carbonyl and the like.

Examples of the "C₂₋₆ alkenyloxy-carbonyl group" of the "C₂₋₆ alkenyloxy-carbonyl group optionally having substituent(s)" include ethenyloxycarbonyl, 1-propenyloxycarbonyl, 2-propenyloxycarbonyl, 1-butenyloxycarbonyl, 2-butenyloxycarbonyl, 3-butenyloxycarbonyl, 3-methyl-2-butenyloxycarbonyl, 1-pentenyloxycarbonyl, 2-pentenyloxycarbonyl, 3-pentenyloxycarbonyl, 4-pentenyloxycarbonyl, 1-hexenyloxycarbonyl, 2-hexenyloxycarbonyl, 3-hexenyloxycarbonyl, 4-hexenyloxycarbonyl, 5-hexenyloxycarbonyl and the like.
Examples of the "C₂₋₆ alkynyloxy-carbonyl group" of the "C₂₋₆ alkynyloxy-carbonyl group optionally having substituent(s)" include ethynyloxycarbonyl, 1-propynyloxycarbonyl, 2-propynyloxycarbonyl, 1-butynyloxycarbonyl, 2-butynyloxycarbonyl, 3-butynyloxycarbonyl, 1-pentynyloxycarbonyl, 2-pentynyloxycarbonyl, 3-pentynyloxycarbonyl, 4-pentynyloxycarbonyl, 1-hexynyloxycarbonyl, 2-hexynyloxycarbonyl, 3-hexynyloxycarbonyl, 4-hexynyloxycarbonyl, 5-hexynyloxycarbonyl and the like.

Examples of the "C₃₋₆ cycloalkyloxy-carbonyl group" of the "C₃₋₆ cycloalkyloxy-carbonyl group optionally having substituent(s)" include cyclopropyloxycarbonyl, cyclobutyloxycarbonyl, cyclopentyloxycarbonyl, cyclohexyloxycarbonyl and the like.
Examples of the "C₃₋₆ cycloalkenyloxy-carbonyl group" of the "C₃₋₆ cycloalkenyloxy-carbonyl group optionally having substituent(s)" include 2-cyclopropen-1-yloxycarbonyl, 2-cyclobuten-1-yloxycarbonyl, 2-cyclopenten-1-yloxycarbonyl, 3-cyclopenten-1-yloxycarbonyl, 2-cyclohexen-1-yloxycarbonyl, 3-cyclohexen-1-yloxycarbonyl and the like.

Examples of the "C₆₋₁₀ aryloxy-carbonyl group" of the "C₆₋₁₀ aryloxy-carbonyl group optionally having substituent(s)" include phenoxycarbonyl, 1-naphthyloxycarbonyl, 2-naphthyloxycarbonyl and the like.
Examples of the "heterocyclyloxycarbonyl group" of the "heterocyclyloxycarbonyl group optionally having substituent(s)" include (1) 5- or 6-membered monocyclic aromatic heterocycle (e.g., furan, thiophene, pyrrole, oxazole, isoxazole, thiazole, isothiazole, imidazole, pyridine, pyrazole etc.)-oxycarbonyl, (2) 8- to 12-membered condensed aromatic heterocycle (e.g., benzofuran, isobenzofuran, benzothiophene, isobenzothiophene, indole, isoindole, 1H-indazole, benzimidazole, benzoxazole etc.)-oxycarbonyl, (3) 3 to 6-membered non-aromatic heterocycle (e.g., oxirane, azetidine, oxetane, pyrrolidine, tetrahydrofuran, thioran, piperidine etc.)-oxycarbonyl and the like.
Examples of the "C₁₋₆ alkyl-sulfonyl group" of the "C₁₋₆ alkyl-sulfonyl group optionally having substituent(s)" include, unless otherwise specified, methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, isopentylsulfonyl, neopentylsulfonyl, 1-ethylpropylsulfonyl, hexylsulfonyl, isohexylsulfonyl, 1,1-dimethylbutylsulfonyl, 2,2-dimethylbutylsulfonyl, 3,3-dimethylbutylsulfonyl, 2-ethylbutylsulfonyl and the like.

These C₁₋₆ alkyl-carbonyl group, C₂₋₆ alkenyl-carbonyl group, C₂₋₆ alkynyl-carbonyl group, C₃₋₆ cycloalkyl-carbonyl group, C₃₋₆ cycloalkenyl-carbonyl group, C₆₋₁₀ aryl-carbonyl group, heterocyclylcarbonyl group, C₁₋₆ alkoxy-carbonyl group, C₂₋₆ alkenyloxy-carbonyl group, C₂₋₆ alkynyloxy-carbonyl group, C₃₋₆ cycloalkyloxy-carbonyl group, C₃₋₆ cycloalkenyloxy-carbonyl group, C₆₋₁₀ aryloxy-carbonyl group, C₁₋₆ alkyl-sulfonyl group and heterocyclyloxycarbonyl group optionally have 1 to 3 substituents at substitutable position(s). When the number of substituents is two or more, the substituents may be the same or different.
Examples of the substituent of the C₁₋₆ alkyl-carbonyl group, C₂₋₆ alkenyl-carbonyl group, C₂₋₆ alkynyl-carbonyl group, C₁₋₆ alkoxy-carbonyl group, C₂₋₆ alkenyloxy-carbonyl group, C₂₋₆ alkynyloxy-carbonyl group and C₁₋₆ alkyl-sulfonyl group include those exemplified as the substituent that the C₁₋₁₀ alkyl group and the like exemplified as the "hydrocarbon group" of the "hydrocarbon group optionally having substituent(s)" for R¹ and the like optionally have.
Examples of the substituent of the C₃₋₆ cycloalkyl-carbonyl group, C₃₋₆ cycloalkenyl-carbonyl group, heterocyclylcarbonyl group, C₃₋₆ cycloalkyloxy-carbonyl group, C₃₋₆ cycloalkenyloxy-carbonyl group and heterocyclyloxycarbonyl group include those exemplified as the substituent that the C₃₋₁₀ cycloalkyl group and the like exemplified as the "hydrocarbon group" of the "hydrocarbon group optionally having substituent(s)" for R¹ and the like optionally have, excluding oxo when the heterocycle of the heterocyclylcarbonyl group and heterocyclyloxycarbonyl group are each an aromatic heterocycle.
Examples of the substituent of the C₆₋₁₀ aryl-carbonyl group and C₆₋₁₀ aryloxy-carbonyl group include those exemplified as the substituent that the C₆₋₁₄ aryl group and the like exemplified as the "hydrocarbon group" of the "hydrocarbon group optionally having substituent(s)" for R¹ and the like optionally have.

Examples of the "carbamoyl optionally having substituent(s)" include those similar to the "carbamoyl optionally having substituent(s)" for the below-mentioned R⁵ and the like.

Examples of the "hydroxyl group optionally substituted by an alkyl group optionally having substituent(s)" for R¹, R² or R³ include a hydroxyl group optionally substituted by a C₁₋₁₀ alkyl group optionally having substituent(s) and the like. Here, examples of the C₁₋₁₀ alkyl group include those exemplified as the "hydrocarbon group" of the "hydrocarbon group optionally having substituent(s)" for R¹ and the like.
The aforementioned C₁₋₁₀ alkyl group may have 1 to 3 substituents at substitutable position(s). When the number of substituents is two or more, the substituents may be the same or different.
Examples of the substituent of the C₁₋₁₀ alkyl group include those exemplified as the substituent that the C₁₋₁₀ alkyl group and the like exemplified as the "hydrocarbon group" of the "hydrocarbon group optionally having substituent(s)" for R¹ and the like optionally have.

Y is -O-, -S- or -NR^{a}- wherein R^{a} is a hydrogen atom, a hydrocarbon group optionally having substituent(s), a heterocyclic group optionally having substituent(s), an acyl group or a hydroxyl group optionally substituted by an alkyl group optionally having substituent(s).
Examples of the "hydrocarbon group optionally having substituent(s)", "heterocyclic group optionally having substituent(s)", "acyl group" and "hydroxyl group optionally substituted by an alkyl group optionally having substituent(s)" for R^{a} include those similar to the "hydrocarbon group optionally having substituent(s)", "heterocyclic group optionally having substituent(s)", "acyl group" and "hydroxyl group optionally substituted by an alkyl group optionally having substituent(s)" for R¹ and the like.

Z is =O, =S or =NR^{b} wherein R^{b} is a hydrogen atom, a hydrocarbon group optionally having substituent(s), a heterocyclic group optionally having substituent(s), an acyl group or a hydroxyl group optionally substituted by an alkyl group optionally having substituent(s).
Examples of the "hydrocarbon group optionally having substituent(s)", "heterocyclic group optionally having substituent(s)", "acyl group" and "hydroxyl group optionally substituted by an alkyl group optionally having substituent(s)" for R^{b} include those similar to the "hydrocarbon group optionally having substituent(s)", "heterocyclic group optionally having substituent(s)", "acyl group" and "hydroxyl group optionally substituted by an alkyl group optionally having substituent(s)" for R¹ and the like.

Y¹ is -O-, -S- or -NR^{c}- wherein R^{c} is a hydrogen atom, a hydrocarbon group optionally having substituent(s), a heterocyclic group optionally having substituent(s) or an acyl group.
Examples of the "hydrocarbon group optionally having substituent(s)", "heterocyclic group optionally having substituent(s)" and "acyl group" for R^{c} include those similar to the "hydrocarbon group optionally having substituent(s)", "heterocyclic group optionally having substituent(s)" and "acyl group" for R¹ and the like.

Z¹ is -OR^{d}, -SR^{d} or -NHR^{d} wherein R^{d} are each independently a hydrogen atom, a hydrocarbon group optionally having substituent(s), a heterocyclic group optionally having substituent(s) or an acyl group.
Examples of the "hydrocarbon group optionally having substituent(s)", "heterocyclic group optionally having substituent(s)" and "acyl group" for R^{d} include those similar to the "hydrocarbon group optionally having substituent(s)", "heterocyclic group optionally having substituent(s)" and "acyl group" for R¹ and the like.

R⁴ is a hydrogen atom or an alkyl group optionally having substituent(s).
Examples of the "alkyl group" of the "alkyl group optionally having substituent(s)" for R⁴ include a C₁₋₁₀ alkyl group and the like. Examples of the C₁₋₁₀ alkyl group include those exemplified as the "hydrocarbon group" of the "hydrocarbon group optionally having substituent(s)" for R¹ and the like.
The aforementioned C₁₋₁₀ alkyl group optionally has 1 to 3 substituents at substitutable position(s). When the number of substituents is two or more, the substituents may be the same or different.
Examples of the substituent of the C₁₋₁₀ alkyl group include those exemplified as the substituent that the C₁₋₁₀ alkyl group and the like exemplified as the "hydrocarbon group" of the "hydrocarbon group optionally having substituent(s)" for R¹ and the like optionally have.

R⁵, R⁶, R⁷ and R⁸ are each independently a hydrogen atom, a hydrocarbon group optionally having substituent(s), an amino group optionally having substituent(s), a hydroxyl group optionally having a substituent, a thiol group optionally having a substituent, a carbamoyl group optionally having substituent(s), an alkyloxycarbonyl group optionally having substituent(s), a halogen atom, a cyano group or a nitro group.

Examples of the "hydrocarbon group optionally having substituent(s)" for R⁵, R⁶, R⁷ or R⁸ include those similar to the "hydrocarbon group optionally having substituent(s)" for R¹ and the like.

Examples of the "amino group optionally having substituent(s)" for R⁵, R⁶, R⁷ or R⁸ include an amino group optionally substituted by 1 or 2 substituents selected from a C₁₋₁₀ alkyl group, a C₂₋₁₀ alkenyl group, a C₃₋₁₀ cycloalkyl group, a C₃₋₁₀ cycloalkenyl group, a C₆₋₁₄ aryl group, a C₇₋₁₃ aralkyl group, a C₈₋₁₃ arylalkenyl group and a heterocyclic group, each of which optionally have substituent(s); and an acyl group and the like.
Examples of the C₁₋₁₀ alkyl group, C₂₋₁₀ alkenyl group, C₃₋₁₀ cycloalkyl group, C₃₋₁₀ cycloalkenyl group, C₆₋₁₄ aryl group, C₇₋₁₃ aralkyl group and C₈₋₁₃ arylalkenyl group include those exemplified as the "hydrocarbon group" of the "hydrocarbon group optionally having substituent(s)" for R¹ and the like.
Examples of the heterocyclic group include the "aromatic heterocyclic group" and "non-aromatic heterocyclic group" exemplified as the "heterocyclic group" of the "heterocyclic group optionally having substituent(s)" for R¹ and the like.
These C₁₋₁₀ alkyl group, C₂₋₁₀ alkenyl group, C₃₋₁₀ cycloalkyl group, C₃₋₁₀ cycloalkenyl group, C₆₋₁₄ aryl group, C₇₋₁₃ aralkyl group, C₈₋₁₃ arylalkenyl group, aromatic heterocyclic group and non-aromatic heterocyclic group optionally have 1 to 3 substituents at substitutable position(s). When the number of substituents is two or more, the substituents may be the same or different.
Examples of the substituent of the C₁₋₁₀ alkyl group and C₂₋₁₀ alkenyl group include those exemplified as the substituent that the C₁₋₁₀ alkyl group and the like exemplified as the "hydrocarbon group" of the "hydrocarbon group optionally having substituent(s)" for R¹ and the like optionally have.
Examples of the substituent of the C₃₋₁₀ cycloalkyl group, C₃₋₁₀ cycloalkenyl group and non-aromatic heterocyclic group include those exemplified as the substituent that the C₃₋₁₀ cycloalkyl group and the like exemplified as the "hydrocarbon group" of the "hydrocarbon group optionally having substituent(s)" for R¹ etc. may have.
Examples of the substituent of the C₆₋₁₄ aryl group, C₇₋₁₃ aralkyl group, C₈₋₁₃ arylalkenyl group and aromatic heterocyclic group include those exemplified as the substituent the C₆₋₁₄ aryl group and the like exemplified as the "hydrocarbon group" of the "hydrocarbon group optionally having substituent(s)" for R¹ and the like optionally have.
Examples of the acyl group include those similar to the "acyl group" for R¹ and the like.

Examples of the "hydroxyl group optionally having a substituent" for R⁵, R⁶, R⁷ or R⁸ include a hydroxyl group optionally substituted by a substituent selected from C₁₋₁₀ alkyl group, C₂₋₁₀ alkenyl group, C₃₋₁₀ cycloalkyl group, C₃₋₁₀ cycloalkenyl group, C₆₋₁₄ aryl group, C₇₋₁₃ aralkyl group, C₈₋₁₃ arylalkenyl group, C₃₋₁₀ cycloalkyl-C₁₋₆ alkyl group, C₁₋₆ alkyl-carbonyl group, heterocyclic group and the like, each of which optionally have substituent(s).
Examples of the C₁₋₁₀ alkyl group, C₂₋₁₀ alkenyl group, C₃₋₁₀ cycloalkyl group, C₃₋₁₀ cycloalkenyl group, C₆₋₁₄ aryl group, C₇₋₁₃ aralkyl group, C₈₋₁₃ arylalkenyl group and C₃₋₁₀ cycloalkyl-C₁₋₆ alkyl group include those exemplified as the "hydrocarbon group" of the "hydrocarbon group optionally having substituent(s)" for R¹ and the like.
Examples of the heterocyclic group include the "aromatic heterocyclic group" and "non-aromatic heterocyclic group" exemplified as the "heterocyclic group" of the "heterocyclic group optionally having substituent(s)" for R¹ and the like.
These C₁₋₁₀ alkyl group, C₂₋₁₀ alkenyl group, C₃₋₁₀ cycloalkyl group, C₃₋₁₀ cycloalkenyl group, C₆₋₁₄ aryl group, C₇₋₁₃ aralkyl group, C₈₋₁₃ arylalkenyl group, C₃₋₁₀ cycloalkyl-C₁₋₆ alkyl group, C₁₋₆ alkyl-carbonyl group, aromatic heterocyclic group and non-aromatic heterocyclic group optionally have 1 to 3 substituents at substitutable position(s). When the number of substituents is two or more, the substituents may be the same or different.
Examples of the substituent of the C₁₋₁₀ alkyl group, C₂₋₁₀ alkenyl group and C₁₋₆ alkyl-carbonyl group include those exemplified as the substituent that the C₁₋₁₀ alkyl group and the like exemplified as the "hydrocarbon group" of the "hydrocarbon group optionally having substituent(s)" for R¹ and the like optionally have.
Examples of the substituent of the C₃₋₁₀ cycloalkyl group, C₃₋₁₀ cycloalkenyl group, C₃₋₁₀ cycloalkyl-C₁₋₆ alkyl group and non-aromatic heterocyclic group include those exemplified as the substituent that the C₃₋₁₀ cycloalkyl group and the like exemplified as the "hydrocarbon group" of the "hydrocarbon group optionally having substituent(s)" for R¹ and the like optionally have.
Examples of the substituent of the C₆₋₁₄ aryl group, C₇₋₁₃ aralkyl group, C₈₋₁₃ arylalkenyl group and aromatic heterocyclic group include those exemplified as the substituent that the C₆₋₁₄ aryl group and the like exemplified as the "hydrocarbon group" of the "hydrocarbon group optionally having substituent(s)" for R¹ and the like optionally have.

Examples of the "thiol group optionally having a substituent" for R⁵, R⁶ R⁷ or R⁸ include a thiol group optionally substituted by a substituent selected from C₁₋₁₀ alkyl group, C₂₋₁₀ alkenyl group, C₃₋₁₀ cycloalkyl group, C₃₋₁₀ cycloalkenyl group, C₆₋₁₄ aryl group, C₇₋₁₃ aralkyl group, C₈₋₁₃ arylalkenyl group, C₁₋₆ alkyl-carbonyl group, heterocyclic group and the like, each of which optionally have substituent(s).
Examples of the C₁₋₁₀ alkyl group, C₂₋₁₀ alkenyl group, C₃₋₁₀ cycloalkyl group, C₃₋₁₀ cycloalkenyl group, C₆₋₁₄ aryl group, C₇₋₁₃ aralkyl group and C₈₋₁₃ arylalkenyl group include those exemplified as the "hydrocarbon group" of the "hydrocarbon group optionally having substituent(s)" for R¹ and the like.
Examples of the heterocyclic group include the "aromatic heterocyclic group" and "non-aromatic heterocyclic group" exemplified as the "heterocyclic group" of the "heterocyclic group optionally having substituent(s)" for R¹ and the like.
These C₁₋₁₀ alkyl group, C₂₋₁₀ alkenyl group, C₃₋₁₀ cycloalkyl group, C₃₋₁₀ cycloalkenyl group, C₆₋₁₄ aryl group, C₇₋₁₃ aralkyl group, C₈₋₁₃ arylalkenyl group, C₁₋₆ alkyl-carbonyl group, aromatic heterocyclic group and non-aromatic heterocyclic group optionally have 1 to 3 substituents at substitutable position(s). When the number of substituents is two or more, the substituents may be the same or different.
Examples of the substituent of the C₁₋₁₀ alkyl group, C₂₋₁₀ alkenyl group and C₁₋₆ alkyl-carbonyl group include those exemplified as the substituent that the C₁₋₁₀ alkyl group and the like exemplified as the "hydrocarbon group" of the "hydrocarbon group optionally having substituent(s)" for R¹ and the like optionally have.
Examples of the substituent of the C₃₋₁₀ cycloalkyl group, C₃₋₁₀ cycloalkenyl group and non-aromatic heterocyclic group include those exemplified as the substituent that the C₃₋₁₀ cycloalkyl group and the like exemplified as the "hydrocarbon group" of the "hydrocarbon group optionally having substituent(s)" for R¹ and the like optionally have.
Examples of the substituent of the C₆₋₁₄ aryl group, C₇₋₁₃ aralkyl group, C₈₋₁₃ arylalkenyl group and aromatic heterocyclic group include those exemplified as the substituent that the C₆₋₁₄ aryl group and the like exemplified as the "hydrocarbon group" of the "hydrocarbon group optionally having substituent(s)" for R¹ and the like optionally have.

Examples of the "carbamoyl group optionally having substituent(s)" for R⁵, R⁶, R⁷ or R⁸ include a carbamoyl group optionally substituted by 1 or 2 substituents selected from a C₁₋₁₀ alkyl group, a C₂₋₁₀ alkenyl group, a C₃₋₁₀ cycloalkyl group, a C₃₋₁₀ cycloalkenyl group, a C₆₋₁₄ aryl group, a C₇₋₁₃ aralkyl group, a C₈₋₁₃ arylalkenyl group and a heterocyclic group, each of which optionally have substituent(s); and an acyl group and the like.
Examples of the C₁₋₁₀ alkyl group, C₂₋₁₀ alkenyl group, C₃₋₁₀ cycloalkyl group, C₃₋₁₀ cycloalkenyl group, C₆₋₁₄ aryl group, C₇₋₁₃ aralkyl group, and C₈₋₁₃ arylalkenyl group include those exemplified as the "hydrocarbon group" of the "hydrocarbon group optionally having substituent(s)" for R¹ and the like.
Examples of the heterocyclic group include the "aromatic heterocyclic group" and "non-aromatic heterocyclic group" exemplified as the "heterocyclic group" of the "heterocyclic group optionally having substituent(s)" for R¹ and the like.
These C₁₋₁₀ alkyl group, C₂₋₁₀ alkenyl group, C₃₋₁₀ cycloalkyl group, C₃₋₁₀ cycloalkenyl group, C₆₋₁₄ aryl group, C₇₋₁₃ aralkyl group, C₈₋₁₃ arylalkenyl group, aromatic heterocyclic group and non-aromatic heterocyclic group optionally have 1 to 3 substituents at substitutable position(s). When the number of substituents is two or more, the substituents may be the same or different.
Examples of the substituent of the C₁₋₁₀ alkyl group and C₂₋₁₀ alkenyl group include those exemplified as the substituent that the C₁₋₁₀ alkyl group and the like exemplified as the "hydrocarbon group" of the "hydrocarbon group optionally having substituent(s)" for R¹ and the like optionally have.
Examples of the substituent of the C₃₋₁₀ cycloalkyl group, C₃₋₁₀ cycloalkenyl group and non-aromatic heterocyclic group include those exemplified as the substituent that the C₃₋₁₀ cycloalkyl group and the like exemplified as the "hydrocarbon group" of the "hydrocarbon group optionally having substituent(s)" for R¹ and the like optionally have.
Examples of the substituent of the C₆₋₁₄ aryl group, C₇₋₁₃ aralkyl group and C₈₋₁₃ arylalkenyl group and aromatic heterocyclic group include those exemplified as the substituent that the C₆₋₁₄ aryl group and the like exemplified as the "hydrocarbon group" of the "hydrocarbon group optionally having substituent(s)" for R¹ and the like optionally have.
Examples of the acyl group include those similar to the "acyl group" for R¹ and the like.

Examples of the "alkyloxycarbonyl group optionally having substituent(s)" for R⁵, R⁶, R⁷ or R⁸ include a carboxyl group substituted by a C₁₋₁₀ alkyl group and the like optionally having substituent(s).
Examples of the "C₁₋₁₀ alkyl group" of the C₁₋₁₀ alkyl group optionally having substituent(s)" include those exemplified as the "hydrocarbon group" of the "hydrocarbon group optionally having substituent(s)" for R¹ and the like.
The aforementioned C₁₋₁₀ alkyl group optionally has 1 to 3 substituents at substitutable position(s). When the number of substituents is two or more, the substituents may be the same or different.
Examples of the substituent of the C₁₋₁₀ alkyl group include those exemplified as the substituent that the C₁₋₁₀ alkyl group and the like exemplified as the "hydrocarbon group" of the "hydrocarbon group optionally having substituent(s)" for R¹ and the like optionally have.

The m and n are each independently an integer of 0 - 3.

X is -O-, -CO-, -O-CO-, -CO-O-, -SO₂-, -SO-, -S-, -SO₂-O-, -NR^{c1}-, -CO-NR^{c1}-, -NR^{c1}-CO-, -NR^{c1}-CO-NR^{c2}-, -O-CO-NR^{c1}-, -NR^{c1}-CO-O-, -SO₂NR^{c1}-, -NR^{c1}-SO₂- or -NR^{c1}-SO₂-NR^{c2}-wherein R^{c1} and R^{c2} are each independently a hydrogen atom, a hydrocarbon group optionally having substituent(s), a heterocyclic group optionally having substituent(s), an acyl group or a hydroxyl group optionally substituted by an alkyl group optionally having substituent(s).
Examples of the "hydrocarbon group optionally having substituent(s)", "heterocyclic group optionally having substituent(s)", "acyl group" and "hydroxyl group optionally substituted by an alkyl group optionally having substituent(s)" for R^{c1} or R^{c2} include those similar to the "hydrocarbon group optionally having substituent(s)", "heterocyclic group optionally having substituent(s)", "acyl group" and "hydroxyl group optionally substituted by an alkyl group optionally having substituent(s)" for R¹ and the like.

R⁹ is an aromatic ring group optionally having substituent(s).
Examples of the "aromatic ring group" of the "aromatic ring group optionally having substituent(s)" for R⁹ include an aromatic hydrocarbon group and an aromatic heterocyclic group.
Examples of the aromatic hydrocarbon group include monocyclic or condensed polycyclic aromatic hydrocarbon group, preferably, a C₆₋₁₄ aryl group and the like. Examples of the C₆₋₁₄ aryl group include those exemplified as the "hydrocarbon group" of the "hydrocarbon group optionally having substituent(s)" for R¹ and the like.
Examples of the aromatic heterocyclic group include those exemplified as the "heterocyclic group" of the "heterocyclic group optionally having substituent(s)" for R¹ and the like.

These aromatic hydrocarbon group and aromatic heterocyclic group optionally have 1 to 3 substituents at substitutable position(s). When the number of substituents is two or more, the substituents may be the same or different.
Examples of the substituent of the aromatic hydrocarbon group and aromatic heterocyclic group include those exemplified as the substituent that the C₆₋₁₄ aryl group and the like exemplified as the "hydrocarbon group" of the "hydrocarbon group optionally having substituent(s)" for R¹ and the like optionally have.

A is preferably a group represented by

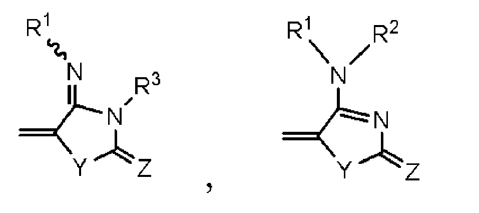

wherein each symbol is as defined above.

R¹ is preferably a hydrogen atom, a hydrocarbon group optionally having substituent(s), a heterocyclic group optionally having substituent(s) or a hydroxyl group optionally substituted by an alkyl group optionally having substituent(s), more preferably a hydrogen atom, a C₁₋₁₀ alkyl group optionally having substituent(s) (preferably, a C₁₋₆ alkyl group optionally having substituent(s)), a C₃₋₁₀ cycloalkyl group optionally having substituent(s), a C₇₋₁₃ aralkyl group optionally having substituent(s), a C₃₋₁₀ cycloalkyl-C₁₋₆ alkyl group optionally having substituent(s), an aromatic heterocyclic group optionally having substituent(s), a hydroxyl group optionally substituted by a C₁₋₁₀ alkyl group optionally having substituent(s) and the like.
R¹ is more preferably
(1) a hydrogen atom;
(2) a C₁₋₆ alkyl group (e.g., methyl, ethyl, propyl, isopropyl, butyl, isobutyl, sec-butyl, tert-butyl, pentyl, isopentyl, 1-ethylpropyl) optionally substituted by 1 to 3 substituents selected from
   (a) an aromatic heterocyclic group (e.g., furyl, pyridyl, pyrazinyl, imidazolyl) optionally substituted by 1 to 3 C₁₋₆ alkyl groups (e.g., methyl);
   (b) a non-aromatic heterocyclic group (e.g., tetrahydrofuryl, morpholinyl, piperidinyl, pyrrolidinyl) optionally substituted by 1 to 3 C₁₋₆ alkyl groups (e.g., methyl);
   (c) an amino group optionally mono- or di-substituted by C₁₋₆ alkyl groups (e.g., methyl, ethyl);
   (d) a carbamoyl group;
   (e) a hydroxyl group;
   (f) a C₁₋₆ alkoxy group (e.g., methoxy, ethoxy, isopropoxy);
   (g) a C₆₋₁₄ aryloxy group (e.g., phenyloxy);
   (h) a C₁-₆ alkylthio group (e.g., methylthio); and
   (i) a C₁₋₆ alkoxy-carbonyl group (e.g., methoxycarbonyl);
(3) a C₃₋₁₀ cycloalkyl group (e.g., cyclopropyl, cyclopentyl, cyclohexyl);
(4) a C₇₋₁₃ aralkyl group (e.g., benzyl, phenethyl) optionally substituted by 1 to 3 substituents selected from
   (a) a C₁₋₆ alkoxy group (e.g., methoxy);
   (b) a hydroxyl group;
   (c) a halogen atom (e.g., fluorine atom, chlorine atom); and
   (d) a C₁₋₆ alkyl group (e.g., methyl);
(5) a C₃₋₁₀ cycloalkyl-C₁₋₆ alkyl group (e.g., cyclohexylmethyl);
(6) an aromatic heterocyclic group (e.g., pyridyl);
(7) a C₁₋₆ alkoxy group (e.g., methoxy)
and the like.
R² is preferably a hydrogen atom or C₁₋₆ alkyl group (e.g., methyl).

R³ is preferably a hydrogen atom, a hydrocarbon group optionally having substituent(s), an acyl group, more preferably a hydrogen atom, a C₁₋₁₀ alkyl group optionally having substituent(s) (preferably, a C₁₋₆ alkyl group optionally having substituent(s)), a C₇₋₁₃ aralkyl group optionally having substituent(s), a C₆₋₁₀ aryl-carbonyl group optionally having substituent(s) and the like.
R³ is more preferably
(1) a hydrogen atom;
(2) a C₁₋₆ alkyl group (e.g., methyl, ethyl);
(3) a C₇₋₁₃ aralkyl group (e.g., benzyl);
(4) a C₆₋₁₀ aryl-carbonyl group (e.g., benzoyl)
and the like.

Y is preferably -S-, -NR^{a}- wherein R^{a} is as defined above; preferably a hydrogen atom, a hydrocarbon group optionally having substituent(s); more preferably a hydrogen atom, a C₁₋₁₀ alkyl group optionally having substituent(s) (preferably, a C₁₋₆ alkyl group optionally having substituent(s)), a C₃₋₁₀ cycloalkyl group optionally having substituent(s), a C₇₋₁₃ aralkyl group optionally having substituent(s), a C₃₋₁₀ cycloalkyl-C₁₋₆ alkyl group optionally having substituent(s); still more preferably
(1) a hydrogen atom,
(2) a C₁₋₆ alkyl group (e.g., methyl, ethyl, propyl, isopropyl, butyl, isobutyl, pentyl, isopentyl) optionally substituted by 1 to 3 substituents selected from
   (a) an aromatic heterocyclic group (e.g., pyrrolyl, pyridyl);
   (b) a non-aromatic heterocyclic group (e.g., tetrahydropyranyl, piperidinyl, piperazinyl, morpholinyl) optionally substituted by 1 to 3 C₁₋₆ alkyl-carbonyl groups (e.g., acetyl);
   (c) an amino group optionally mono- or di-substituted by C₁₋₆ alkyl groups (e.g., methyl);
   (d) a carbamoyl group;
   (e) a hydroxyl group;
   (f) a C₁₋₆ alkoxy group (e.g., methoxy);
   (g) a C₆₋₁₄ aryloxy group (e.g., phenyloxy) optionally substituted by 1 to 3 halogen atoms (e.g., fluorine atom);
   (h) a C₁₋₆ alkoxy-carbonyl group (e.g., ethoxycarbonyl);
   (i) a C₇₋₁₃ aralkyloxy group (e.g., benzyloxy); and
   (j) a halogen atom (e.g., fluorine atom);
(3) a C₃₋₁₀ cycloalkyl group (e.g., cyclopentyl),
(4) a C₇₋₁₃ aralkyl group (e.g., benzyl, phenethyl, naphthylmethyl, phenylpropyl) optionally substituted by 1 to 3 substituents selected from (a) a C₁₋₆ alkyl group (e.g., methyl, tert-butyl) optionally substituted by 1 to 3 halogen atoms (e.g., fluorine atom) (e.g., trifluoromethyl); (b) a cyano group; and (c) a C₆₋₁₄ aryl group (e.g., phenyl),
(5) a C₃₋₁₀ cycloalkyl-C₁₋₆ alkyl group (e.g., cyclopropylmethyl, 2-cyclohexylethyl) and the like, and the like.
Z is preferably =O.

Another preferable embodiment of A is

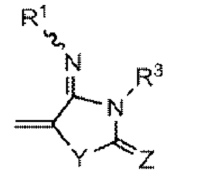

wherein
R¹ is a hydrogen atom, a hydrocarbon group optionally having substituent(s), a heterocyclic group optionally having substituent(s), an acyl group or a hydroxyl group optionally substituted by an alkyl group optionally having substituent(s);
Y is **-0-,** -S- or -NR^{a}- wherein R^{a} is a hydrogen atom, a hydrocarbon group optionally having substituent(s), a heterocyclic group optionally having substituent(s), an acyl group or a hydroxyl group optionally substituted by an alkyl group optionally having substituent(s);
Z is =O, =S or =NR^{b} wherein R^{b} is a hydrogen atom, a hydrocarbon group optionally having substituent(s), a heterocyclic group optionally having substituent(s), an acyl group or a hydroxyl group optionally substituted by an alkyl group optionally having substituent(s); and
R³ is a hydrocarbon group optionally having substituent(s), a heterocyclic group optionally having substituent(s), an acyl group or a hydroxyl group optionally substituted by an alkyl group optionally having substituent(s).

In this case, R¹ is preferably a hydrogen atom, a hydrocarbon group optionally having substituent(s) or a hydroxyl group optionally substituted by an alkyl group optionally having substituent(s) and the like, more preferably a hydrogen atom, a C₁₋₁₀ alkyl group optionally having substituent(s) (preferably, a C₁₋₆ alkyl group optionally having substituent(s)), a C₇₋₁₃ aralkyl group optionally having substituent(s), a hydroxyl group optionally substituted by a C₁₋₁₀ alkyl group optionally having substituent(s) and the like.
R¹ is still more preferably
(1) a hydrogen atom;
(2) a C₁₋₆ alkyl group (e.g., methyl);
(3) a C₇₋₁₃ aralkyl group (e.g., benzyl);
(4) a C₁₋₆ alkoxy group (e.g., methoxy)
and the like.

R³ is preferably a hydrocarbon group optionally having substituent(s), an acyl group and the like, more preferably a C₁₋₁₀ alkyl group optionally having substituent(s) (preferably, a C₁₋₆ alkyl group optionally having substituent(s)), a C₇₋₁₃ aralkyl group optionally having substituent(s), a C₆₋₁₀ aryl-carbonyl group optionally having substituent(s) and the like.
R³ is still more preferably
(1) a C₁₋₆ alkyl group (e.g., methyl, ethyl);
(2) a C₇₋₁₃ aralkyl group (e.g., benzyl);
(3) a C₆₋₁₀ aryl-carbonyl group (e.g., benzoyl)
and the like.

Y is preferably -S-, -NR^{a}- wherein R^{a} is as defined above; preferably a hydrogen atom, a hydrocarbon group optionally having substituent(s); more preferably a hydrogen atom, a C₁₋₁₀ alkyl group optionally having substituent(s) (preferably, a C₁₋₆ alkyl group optionally having substituent(s)), a C₃₋₁₀ cycloalkyl group optionally having substituent(s), a C₇₋₁₃ aralkyl group optionally having substituent(s), a C₃₋₁₀ cycloalkyl-C₁₋₆ alkyl group optionally having substituent(s); more preferably
(1) a hydrogen atom;
(2) a C₁₋₆ alkyl group (e.g., methyl, ethyl, propyl, isopropyl, butyl, isobutyl, pentyl, isopentyl) optionally substituted by 1 to 3 substituents selected from
   (a) an aromatic heterocyclic group (e.g., pyrrolyl, pyridyl);
   (b) a non-aromatic heterocyclic group (e.g., tetrahydropyranyl, piperidinyl, piperazinyl, morpholinyl) optionally substituted by 1 to 3 C₁₋₆ alkyl-carbonyl groups (e.g., acetyl);
   (c) an amino group optionally mono- or di-substituted by C₁₋₆ alkyl groups (e.g., methyl);
   (d) a carbamoyl group;
   (e) a hydroxyl group;
   (f) a C₁₋₆ alkoxy group (e.g., methoxy);
   (g) a C₆₋₁₄ aryloxy group (e.g., phenyloxy) optionally substituted by 1 to 3 halogen atoms (e.g., fluorine atom);
   (h) a C₁₋₆ alkoxy-carbonyl group (e.g., ethoxycarbonyl);
   (i) a C₇₋₁₃ aralkyloxy group (e.g., benzyloxy); and
   (j) a halogen atom (e.g., fluorine atom);
(3) a C₃₋₁₀ cycloalkyl group (e.g., cyclopentyl);
(4) a C₇₋₁₃ aralkyl group (e.g., benzyl, phenethyl, naphthylmethyl, phenylpropyl) optionally substituted by 1 to 3 substituents selected from (a) a C₁₋₆ alkyl group (e.g., methyl, tert-butyl) optionally substituted by 1 to 3 halogen atoms (e.g., fluorine atom) (e.g., trifluoromethyl); (b) a cyano group; and (c) a C₆₋₁₄ aryl group (e.g., phenyl);
(5) a C₃₋₁₀ cycloalkyl-C₁₋₆ alkyl group (e.g., cyclopropylmethyl, 2-cyclohexylethyl) and the like, and the like.
Z is preferably =O.

A still another preferable embodiment of A is a group represented by

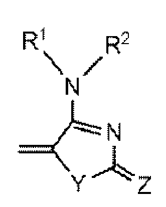

wherein R¹ is a hydrocarbon group optionally having substituent(s) or a heterocyclic group optionally having substituent(s),
R² is a hydrogen atom,
Y is **-0-,** -S- or -NR^{a}- wherein R^{a} is a hydrogen atom, a hydrocarbon group optionally having substituent(s), a heterocyclic group optionally having substituent(s), an acyl group or a hydroxyl group optionally substituted by an alkyl group optionally having substituent(s), and
Z is =O, =S or =NR^{b} wherein R^{b} is a hydrogen atom, a hydrocarbon group optionally having substituent(s), a heterocyclic group optionally having substituent(s), an acyl group or a hydroxyl group optionally substituted by an alkyl group optionally having substituent(s).

In this case, R¹ is preferably a C₁₋₁₀ alkyl group optionally having substituent(s) (preferably, a C₁₋₆ alkyl group optionally having substituent(s)), a C₃₋₁₀ cycloalkyl group optionally having substituent(s), a C₇₋₁₃ aralkyl group optionally having substituent(s), a C₃₋₁₀ cycloalkyl-C₁₋₆ alkyl group optionally having substituent(s), an aromatic heterocyclic group optionally having substituent(s) and the like.
R¹ is more preferably
(1) a C₁₋₆ alkyl group (e.g., methyl, ethyl, propyl, isopropyl, butyl, isobutyl, sec-butyl, tert-butyl, pentyl, isopentyl, 1-ethylpropyl) optionally substituted by 1 to 3 substituents selected from
   (a) an aromatic heterocyclic group (e.g., furyl, pyridyl, pyrazinyl, imidazolyl) optionally substituted by 1 to 3 C₁₋₆ alkyl groups (e.g., methyl);
   (b) a non-aromatic heterocyclic group (e.g., tetrahydrofuryl, morpholinyl, piperidinyl, pyrrolidinyl) optionally substituted by 1 to 3 C₁₋₆ alkyl groups (e.g., methyl);
   (c) an amino group optionally mono- or di-substituted by C₁₋₆ alkyl groups (e.g., methyl, ethyl);
   (d) a carbamoyl group;
   (e) a hydroxyl group;
   (f) a C₁₋₆ alkoxy group (e.g., methoxy, ethoxy, isopropoxy);
   (g) a C₆₋₁₄ aryloxy group (e.g., phenyloxy);
   (h) a C₁₋₆ alkylthio group (e.g., methylthio); and
   (i) a C₁₋₆ alkoxy-carbonyl group (e.g., methoxycarbonyl);
(2) a C₃₋₁₀ cycloalkyl group (e.g., cyclopropyl, cyclopentyl, cyclohexyl);
(3) a C₇₋₁₃ aralkyl group (e.g., benzyl, phenethyl) optionally substituted by 1 to 3 substituents selected from
   (a) a C₁₋₆ alkoxy group (e.g., methoxy);
   (b) a hydroxyl group;
   (c) a halogen atom (e.g., fluorine atom, chlorine atom); and
   (d) a C₁₋₆ alkyl group (e.g., methyl);
(4) a C₃₋₁₀ cycloalkyl-C₁₋₆ alkyl group (e.g., cyclohexylmethyl);
(5) an aromatic heterocyclic group (e.g., pyridyl)
and the like.

Y is preferably -S-, -NR^{a}- wherein R^{a} is as defined above; preferably a hydrogen atom, a hydrocarbon group optionally having substituent(s); more preferably a hydrogen atom, a C₁₋₁₀ alkyl group optionally having substituent(s) (preferably, a C₁₋₆ alkyl group optionally having substituent(s)); still more preferably
(1) a hydrogen atom,
(2) a C₁₋₆ alkyl group (e.g., methyl) and the like, and the like.
Z is preferably =O.

R⁴ is preferably a hydrogen atom.

Preferably, R⁵ and R⁸ are each independently a hydrogen atom, a hydrocarbon group optionally having substituent(s), an amino group optionally having substituent(s), a hydroxyl group optionally having a substituent, a carbamoyl group optionally having substituent(s), an alkyloxycarbonyl group optionally having substituent(s), a halogen atom, a nitro group and the like, more preferably a hydrogen atom, a C₁₋₁₀ alkyl group optionally having substituent(s) (preferably, a C₁₋₆ alkyl group optionally having substituent(s)), an amino group optionally having substituent(s), a hydroxyl group optionally having a substituent, a carbamoyl group optionally having substituent(s), an alkyloxycarbonyl group optionally having substituent(s), a halogen atom, a nitro group and the like.
Still more preferably, R⁵ and R⁸ are each independently
(1) a hydrogen atom;
(2) a C₁₋₆ alkyl group (e.g., methyl) optionally substituted by 1 to 3 halogen atoms (e.g., fluorine atom) (e.g., trifluoromethyl);
(3) an amino group optionally substituted by 1 or 2 substituents selected from (a) a C₁₋₆ alkyl group (e.g., methyl, ethyl, propyl, isopropyl, isobutyl), (b) a C₇₋₁₃ aralkyl group (e.g., benzyl), (c) a C₁₋₆ alkyl-carbonyl group (e.g., acetyl), and (d) a C₁₋₆ alkyl-sulfonyl group (e.g., methanesulfonyl);
(4) a hydroxyl group optionally substituted by a C₁₋₆ alkyl group (e.g., methyl, ethyl, propyl, isopropyl, butyl, isobutyl), a C₇₋₁₃ aralkyl group (e.g., benzyl) or a C₃₋₁₀ cycloalkyl-C₁₋₆ alkyl group (e.g., cyclohexylmethyl);
(5) a carbamoyl group optionally mono- or di-substituted by C₁₋₆ alkyl groups (e.g., methyl);
(6) a C₁₋₁₀ alkyl-oxycarbonyl group (e.g., methoxycarbonyl);
(7) a halogen atom (e.g., fluorine atom, chlorine atom);
(8) a nitro group and the like.
Preferably, R⁶ and R⁷ are each independently a hydrogen atom, a hydrocarbon group optionally having substituent(s), a hydroxyl group optionally having a substituent, a halogen atom and the like, more preferably a hydrogen atom, a C₁₋₁₀ alkyl group optionally having substituent(s) (preferably, a C₁₋₆ alkyl group optionally having substituent(s)), a hydroxyl group optionally having a substituent, a halogen atom and the like. Still more preferably, R⁶ and R⁷ are each independently a hydrogen atom, a C₁₋₆ alkyl group (e.g., methyl), a hydroxyl group optionally substituted by a C₁₋₆ alkyl group (e.g., methyl) (preferably, C₁₋₆ alkoxy group), a halogen atom (e.g., chlorine atom, bromine atom) and the like.
In another embodiment, at least one of R⁵, R⁶, R⁷ and R⁸ is preferably a hydrocarbon group optionally having substituent(s), an amino group optionally having substituent(s), a hydroxyl group optionally having a substituent, a thiol group optionally having a substituent, a carbamoyl group optionally having substituent(s), an alkyloxycarbonyl group optionally having substituent(s), a halogen atom, a cyano group or a nitro group.
In this case, more preferably,
R⁵, R⁶, and R⁷ are each a hydrogen atom,
R⁸ is a hydroxyl group substituted by a C₁₋₁₀ alkyl group optionally having substituent(s),
more preferably,
R⁵, R⁶, and R⁷ are each a hydrogen atom,
R⁸ is a hydroxyl group substituted by a C₁₋₆ alkyl group (e.g., methyl, ethyl, propyl, isopropyl, butyl, isobutyl) (i.e., a C₁₋₆ alkoxy group).

m is preferably 0.
n is preferably 0, 1 or 2.
X is preferably -O- or -SO₂-O-. X is more preferably -OR⁹ is preferably a C₆₋₁₄ aryl group optionally having substituent(s), an aromatic heterocyclic group optionally having substituent(s) and the like, more preferably (1) a C₆₋₁₄ aryl group (preferably, a C₆₋₁₀ aryl group (e.g., phenyl, naphthyl)) optionally substituted by 1 to 3 substituents selected from
(a) a C₁₋₆ alkyl group (e.g., methyl, isopropyl) optionally substituted by 1 to 3 halogen atoms (e.g., fluorine atom)(e.g., trifluoromethyl);
(b) a C₁₋₆ alkoxy group (e.g., methoxy) optionally substituted by 1 to 3 halogen atoms (e.g., fluorine atom) (e.g., trifluoromethoxy);
(c) a cyano group;
(d) a halogen atom (e.g., fluorine atom, chlorine atom, bromine atom);
(e) a nitro group:
(f) a carbamoyl group;
(g) a C₆₋₁₄ aryloxy group (e.g., phenyloxy); and
(h) a C₆₋₁₄ aryl group (e.g., phenyl) (when the C₆₋₁₄ aryl group has 2 substituents, these substituents are preferably present at the ortho-position and para-position relative to the bond at R⁹);
(2) an aromatic heterocyclic group (e.g., pyridyl, benzothiadiazolyl) optionally substituted by 1 to 3 substituents selected from
(a) a C₁₋₆ alkyl group (e.g., methyl) optionally substituted by 1 to 3 halogen atoms (e.g., fluorine atom) (e.g., trifluoromethyl); and
(b) a halogen atom (e.g., chlorine atom);
and the like.
In another embodiment, R⁹ is preferably C₆₋₁₀ aryl optionally having substituent(s).

The following 2 compounds are excluded from compound (I).

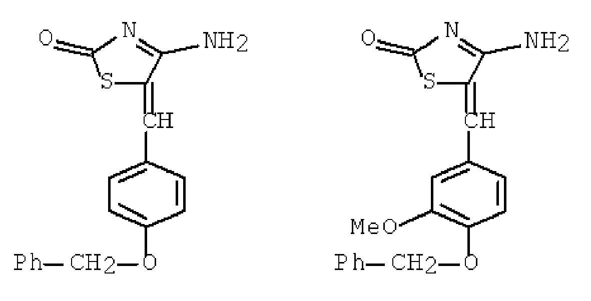

Compound (I) is preferably a compound
wherein
A is a group represented by

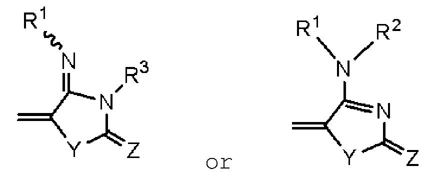

wherein
R¹ is a hydrogen atom, a hydrocarbon group optionally having substituent(s), a heterocyclic group optionally having substituent(s) or a hydroxyl group optionally substituted by an alkyl group optionally having substituent(s);
R² is a hydrogen atom or a C₁₋₆ alkyl group;
R³ is a hydrogen atom, a hydrocarbon group optionally having substituent(s) or an acyl group;
Y is -S- or -NR^{a}- wherein R^{a} is a hydrogen atom or a hydrocarbon group optionally having substituent(s); and Z is =O;
R⁴ is a hydrogen atom;
R⁵ and R⁸ are each independently a hydrogen atom, a hydrocarbon group optionally having substituent(s), an amino group optionally having substituent(s), a hydroxyl group optionally having a substituent, a carbamoyl group optionally having substituent(s), an alkyloxycarbonyl group optionally having substituent(s), a halogen atom or a nitro group;
R⁶ and R⁷ are each independently a hydrogen atom, a hydrocarbon group optionally having substituent(s), a hydroxyl group optionally having a substituent or a halogen atom;
m is 0;
n is 0, 1 or 2;
X is -O- or -SO₂-O-; and
R⁹ is a C₆₋₁₄ aryl group optionally having substituent(s) or an aromatic heterocyclic group optionally having substituent(s), or a salt thereof.

Compound (I) is more preferably a compound
wherein
A is a group represented by

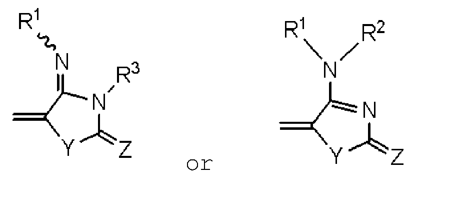

wherein
R¹ is a hydrogen atom, a C₁₋₁₀ alkyl group optionally having substituent(s) (preferably, a C₁₋₆ alkyl group optionally having substituent(s)), a C₃₋₁₀ cycloalkyl group optionally having substituent(s), a C₇₋₁₃ aralkyl group optionally having substituent(s), a C₃₋₁₀ cycloalkyl-C₁₋₆ alkyl group optionally having substituent(s), an aromatic heterocyclic group optionally having substituent(s) or a hydroxyl group optionally substituted by a C₁₋₁₀ alkyl group optionally having substituent(s);
R² is a hydrogen atom or a C₁₋₆ alkyl group;
R³ is a hydrogen atom, a C₁₋₁₀ alkyl group optionally having substituent(s) (preferably, a C₁₋₆ alkyl group optionally having substituent(s)), a C₇₋₁₃ aralkyl group optionally having substituent(s) or a C₆₋₁₀ aryl-carbonyl group optionally having substituent(s);
Y is -S- or -NR^{a} -wherein R^{a} is a hydrogen atom, a C₁₋₁₀ alkyl group optionally having substituent(s) (preferably, a C₁₋₆ alkyl group optionally having substituent(s)), a C₃₋₁₀ cycloalkyl group optionally having substituent(s), a C₇₋₁₃ aralkyl group optionally having substituent(s) or a C₃₋₁₀ cycloalkyl-C₁₋₆ alkyl group optionally having substituent(s); and
Z is =O;
R⁴ is a hydrogen atom;
R⁵ and R⁸ are each independently a hydrogen atom, a C₁₋₁₀ alkyl group optionally having substituent(s) (preferably, a C₁₋₆ alkyl group optionally having substituent(s)), an amino group optionally having substituent(s), a hydroxyl group optionally having a substituent, a carbamoyl group optionally having substituent(s), an alkyloxycarbonyl group optionally having substituent(s), a halogen atom or a nitro group;
R⁶ and R⁷ are each independently a hydrogen atom, a C₁₋₁₀ alkyl group optionally having substituent(s) (preferably, a C₁₋₆ alkyl group optionally having substituent(s)), a hydroxyl group optionally having a substituent or a halogen atom;
m is 0;
n is 0, 1 or 2;
X is -O- or -SO₂-O-; and
R⁹ is a C₆₋₁₄ aryl group optionally having substituent(s) or an aromatic heterocyclic group optionally having substituent(s), or a salt thereof.
In the above, preferably, R⁵, R⁶ and R⁷ are each a hydrogen atom; and R⁸ is a hydroxyl group optionally having a substituent (particularly, a hydroxyl group substituted by a C₁₋₁₀ alkyl group optionally having substituent(s)).

Specific preferable examples of compound (I) include the following compound.

### [Compound I-A]

A compound wherein
A is a group represented by

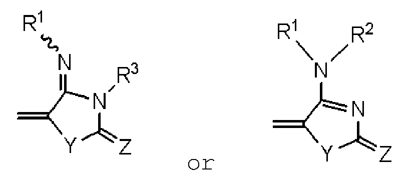

wherein,
R¹ is
(1) a hydrogen atom;
(2) a C₁₋₆ alkyl group (e.g., methyl, ethyl, propyl, isopropyl, butyl, isobutyl, sec-butyl, tert-butyl, pentyl, isopentyl, 1-ethylpropyl) optionally substituted by 1 to 3 substituents selected from
   (a) an aromatic heterocyclic group (e.g., furyl, pyridyl, pyrazinyl, imidazolyl) optionally substituted by 1 to 3 C₁₋₆ alkyl groups (e.g., methyl);
   (b) a non-aromatic heterocyclic group (e.g., tetrahydrofuryl, morpholinyl, piperidinyl, pyrrolidinyl) optionally substituted by 1 to 3 C₁₋₆ alkyl groups (e.g., methyl);
   (c) an amino group optionally mono- or di-substituted by C₁₋₆ alkyl groups (e.g., methyl, ethyl);
   (d) a carbamoyl group;
   (e) a hydroxyl group;
   (f) a C₁₋₆ alkoxy group (e.g., methoxy, ethoxy, isopropoxy);
   (g) a C₆₋₁₄ aryloxy group (e.g., phenyloxy);
   (h) a C₁₋₆ alkylthio group (e.g., methylthio); and
   (i) a C₁₋₆ alkoxy-carbonyl group (e.g., methoxycarbonyl);
(3) a C₃₋₁₀ cycloalkyl group (e.g., cyclopropyl, cyclopentyl, cyclohexyl);
(4) a C₇₋₁₃ aralkyl group (e.g., benzyl, phenethyl) optionally substituted by 1 to 3 substituents selected from
   (a) a C₁₋₆ alkoxy group (e.g., methoxy);
   (b) a hydroxyl group;
   (c) a halogen atom (e.g., fluorine atom, chlorine atom); and
   (d) a C₁₋₆ alkyl group (e.g., methyl);
(5) a C₃₋₁₀ cycloalkyl-C₁₋₆ alkyl group (e.g., cyclohexylmethyl);
(6) an aromatic heterocyclic group (e.g., pyridyl); or
(7) a C₁₋₆ alkoxy group (e.g., methoxy);
R² is a hydrogen atom or a C₁₋₆ alkyl group (e.g., methyl);
R³ is
(1) a hydrogen atom;
(2) a C₁₋₆ alkyl group (e.g., methyl, ethyl);
(3) a C₇₋₁₃ aralkyl group (e.g., benzyl); or
(4) a C₆₋₁₀ aryl-carbonyl group (e.g., benzoyl);
Y is -S- or -NR^{a}- wherein R^{a} is
(1) a hydrogen atom,
(2) a C₁₋₆ alkyl group (e.g., methyl, ethyl, propyl, isopropyl, butyl, isobutyl, pentyl, isopentyl) optionally substituted by 1 to 3 substituents selected from
   (a) an aromatic heterocyclic group (e.g., pyrrolyl, pyridyl);
   (b) a non-aromatic heterocyclic group (e.g., tetrahydropyranyl, piperidinyl, piperazinyl, morpholinyl) optionally substituted by 1 to 3 C₁₋₆ alkyl-carbonyl groups (e.g., acetyl);
   (c) an amino group optionally mono- or di-substituted by C₁₋₆ alkyl groups (e.g., methyl);
   (d) a carbamoyl group;
   (e) a hydroxyl group;
   (f) a C₁₋₆ alkoxy group (e.g., methoxy);
   (g) a C₆₋₁₄ aryloxy group (e.g., phenyloxy) optionally substituted by 1 to 3 halogen atoms (e.g., fluorine atom);
   (h) a C₁₋₆ alkoxy-carbonyl group (e.g., ethoxycarbonyl);
   (i) a C₇₋₁₃ aralkyloxy group (e.g., benzyloxy); and
   (j) a halogen atom (e.g., fluorine atom),
(3) a C₃₋₁₀ cycloalkyl group (e.g., cyclopentyl),
(4) a C₇₋₁₃ aralkyl group (e.g., benzyl, phenethyl, naphthylmethyl, phenylpropyl) optionally substituted by 1 to 3 substituents selected from (a) a C₁₋₆ alkyl group (e.g., methyl, tert-butyl) optionally substituted by 1 to 3 halogen atoms (e.g., fluorine atom) (e.g., trifluoromethyl); (b) a cyano group; and (c) a C₆₋₁₄ aryl group (e.g., phenyl), or
(5) a C₃₋₁₀ cycloalkyl-C₁₋₆ alkyl group (e.g., cyclopropylmethyl, 2-cyclohexylethyl); and
Z is =O;

R⁴ is a hydrogen atom;
R⁵ and R⁸ are each independently
(1) a hydrogen atom;
(2) a C₁₋₆ alkyl group (e.g., methyl) optionally substituted by 1 to 3 halogen atoms (e.g., fluorine atom)(e.g., trifluoromethyl);
(3) an amino group optionally substituted by 1 or 2 substituents selected from (a) a C₁₋₆ alkyl group (e.g., methyl, ethyl, propyl, isopropyl, isobutyl), (b) a C₇₋₁₃ aralkyl group (e.g., benzyl), (c) a C₁₋₆ alkyl-carbonyl group (e.g., acetyl), and (d) C₁₋₆ alkyl-sulfonyl group (e.g., methanesulfonyl);
(4) a hydroxyl group optionally substituted by a C₁₋₆ alkyl group (e.g., methyl, ethyl, propyl, isopropyl, butyl, isobutyl), a C₇₋₁₃ aralkyl group (e.g., benzyl) or a C₃₋₁₀ cycloalkyl-C₁₋₆ alkyl group (e.g., cyclohexylmethyl);
(5) a carbamoyl group optionally mono- or di-substituted by C₁₋₆ alkyl groups (e.g., methyl);
(6) a C₁₋₁₀ alkyl-oxycarbonyl group (e.g., methoxycarbonyl);
(7) a halogen atom (e.g., fluorine atom, chlorine atom); or
(8) a nitro group;
R⁶ and R⁷ are each independently a hydrogen atom, a C₁₋₆ alkyl group (e.g., methyl), a hydroxyl group optionally substituted by a C₁₋₆ alkyl group (e.g., methyl) (preferably, C₁₋₆ alkoxy group) or a halogen atom (e.g., chlorine atom, bromine atom);
m is 0;
n is 0, 1 or 2;
X is -O- or -SO₂-O-; and
R⁹ is
(1) a C₆₋₁₄ aryl group (preferably, C₆₋₁₀ aryl group (e.g., phenyl, naphthyl)) optionally substituted by 1 to 3 substituents selected from
   (a) a C₁₋₆ alkyl group (e.g., methyl, isopropyl) optionally substituted by 1 to 3 halogen atoms (e.g., fluorine atom)(e.g., trifluoromethyl);
   (b) a C₁₋₆ alkoxy group (e.g., methoxy) optionally substituted by 1 to 3 halogen atoms (e.g., fluorine atom) (e.g., trifluoromethoxy);
   (c) a cyano group;
   (d) a halogen atom (e.g., fluorine atom, chlorine atom, bromine atom);
   (e) a nitro group:
   (f) a carbamoyl group;
   (g) a C₆₋₁₄ aryloxy group (e.g., phenyloxy); and
   (h) a C₆₋₁₄ aryl group (e.g., phenyl)
   (when the C₆₋₁₄ aryl group has 2 substituents, these substituents are preferably present at the ortho-position and para-position relative to the bond at R⁹); or
(2) an aromatic heterocyclic group (e.g., pyridyl, benzothiadiazolyl) optionally substituted by 1 to 3 substituents selected from
   (a) a C₁₋₆ alkyl group (e.g., methyl) optionally substituted by 1 to 3 halogen atoms (e.g., fluorine atom) (e.g., trifluoromethyl); and
   (b) a halogen atom (e.g., chlorine atom),
or a salt thereof.
In the above, preferably, R⁵, R⁶ and R⁷ are each a hydrogen atom; and R⁸ is a hydroxyl group substituted by a C₁₋₆ alkyl group.

Specific preferable examples of compound (I) include the following compound.

### [Compound I-A']

A compound wherein
A is a group represented by

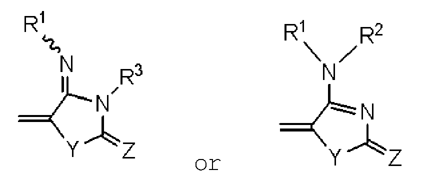

wherein,
R¹ is
(1) a hydrogen atom;
(2) a C₁₋₆ alkyl group (e.g., methyl, ethyl, propyl, isopropyl, butyl, isobutyl, sec-butyl, tert-butyl, pentyl, isopentyl, 1-ethylpropyl) optionally substituted by 1 to 3 substituents selected from
   (a) an aromatic heterocyclic group (e.g., furyl, pyridyl, pyrazinyl, imidazolyl) optionally substituted by 1 to 3 C₁₋₆ alkyl groups (e.g., methyl);
   (b) a non-aromatic heterocyclic group (e.g., tetrahydrofuryl, morpholinyl, piperidinyl, pyrrolidinyl) optionally substituted by 1 to 3 C₁₋₆ alkyl groups (e.g., methyl);
   (c) an amino group optionally mono- or di-substituted by C₁₋₆ alkyl groups (e.g., ethyl);
   (d) a carbamoyl group;
   (e) a hydroxyl group;
   (f) a C₁₋₆ alkoxy group (e.g., methoxy, ethoxy, isopropoxy);
   (g) a C₆₋₁₄ aryloxy group (e.g., phenyloxy); and
   (h) a C₁₋₆ alkylthio group (e.g., methylthio);
(3) a C₃₋₁₀ cycloalkyl group (e.g., cyclopropyl, cyclopentyl, cyclohexyl);
(4) a C₇₋₁₃ aralkyl group (e.g., benzyl, phenethyl) optionally substituted by 1 to 3 substituents selected from
   (a) a C₁₋₆ alkoxy group (e.g., methoxy);
   (b) a hydroxyl group;
   (c) a halogen atom (e.g., fluorine atom, chlorine atom); and
   (d) a C₁₋₆ alkyl group (e.g., methyl);
(5) a C₃₋₁₀ cycloalkyl-C₁₋₆ alkyl group (e.g., cyclohexylmethyl); or
(6) an aromatic heterocyclic group (e.g., pyridyl);
R² is a hydrogen atom;
R³ is
(1) a hydrogen atom;
(2) a C₁₋₆ alkyl group (e.g., ethyl);
(3) a C₇₋₁₃ aralkyl group (e.g., benzyl); or
(4) a C₆₋₁₀ aryl-carbonyl group (e.g., benzoyl);
Y is -S- or -NR^{a}- wherein R^{a} is a hydrogen atom, a C₁₋₆ alkyl group (e.g., methyl) or a C₇₋₁₃ aralkyl group (e.g., benzyl) optionally substituted by C₁₋₆ alkyl group(s) (e.g., methyl) optionally substituted by 1 to 3 halogen atoms (e.g., fluorine atom)(e.g., trifluoromethyl); and
Z is =O;

R⁴ is a hydrogen atom;
R⁵ and R⁸ are each independently
(1) a hydrogen atom;
(2) a C₁₋₆ alkyl group (e.g., methyl);
(3) a hydroxyl group optionally substituted by a C₁₋₆ alkyl group (e.g., methyl, ethyl, propyl, isopropyl, butyl, isobutyl), a C₇₋₁₃ aralkyl group (e.g., benzyl) or a C₃₋₁₀ alkyl-C₁₋₆ alkyl group (e.g., cyclohexylmethyl); or
(4) a halogen atom (e.g., fluorine atom, chlorine atom);
   R⁶ and R⁷ are each independently a hydrogen atom, a C₁₋₆ alkyl group (e.g., methyl), a hydroxyl group optionally substituted by a C₁₋₆ alkyl group (e.g., methyl) or a halogen atom (e.g., chlorine atom);
   m is 0;
   n is 0 or 1;
   X is -O- or -SO₂-O-; and
   R⁹ is a C₆₋₁₄ aryl group (preferably, a C₆₋₁₀ aryl group (e.g., phenyl, naphthyl)) optionally substituted by 1 to 3 substituents selected from
   (a) a C₁₋₆ alkyl group (e.g., methyl) optionally substituted by 1 to 3 halogen atoms (e.g., fluorine atom)(e.g., trifluoromethyl);
   (b) a C₁₋₆ alkoxy group (e.g., methoxy); and
   (c) a cyano group
(when the C₆₋₁₄ aryl group has 2 substituents, these substituents are preferably present at the ortho-position and para-position),
or a salt thereof.
In the above, preferably, R⁵, R⁶ and R⁷ are each a hydrogen atom; and R⁸ is a hydroxyl group substituted by a C₁₋₆ alkyl group.

In another embodiment, compound (I) is preferably a compound wherein
A is a group represented by

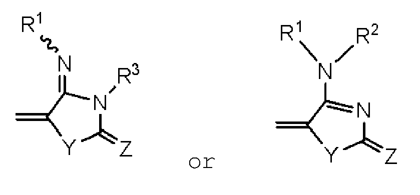

wherein
R¹ is a hydrogen atom, a hydrocarbon group optionally having substituent(s), a heterocyclic group optionally having substituent(s) or a hydroxyl group optionally substituted by an alkyl group optionally having substituent(s);
R² is a hydrogen atom or a C₁₋₆ alkyl group (e.g., methyl);
R³ is a hydrogen atom, a hydrocarbon group optionally having substituent(s) or an acyl group;
Y is -S- or -NR^{a}- wherein R^{a} is a hydrogen atom or a hydrocarbon group optionally having substituent(s); and Z is =O;
R⁴ is a hydrogen atom;
at least one of R⁵, R⁶, R⁷ and R⁸ is a hydrocarbon group optionally having substituent(s), an amino group optionally having substituent(s), a hydroxyl group optionally having a substituent, a thiol group optionally having a substituent, a carbamoyl group optionally having substituent(s), an alkyloxycarbonyl group optionally having substituent(s), a halogen atom, a cyano group or a nitro group;
m is 0;
n is 0, 1 or 2;
X is -O- or -SO₂-O-; and
R⁹ is a C₆₋₁₄ aryl group optionally having substituent(s) or an aromatic heterocycle optionally having substituent(s),
or a salt thereof.

Specific preferable examples of compound (I) also include the following compounds.

### [Compound I-A-1]

The aforementioned [compound I-A] wherein
A is a group represented by

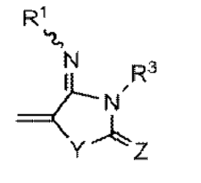

wherein,
R¹ is
(1) a hydrogen atom;
(2) a C₁₋₆ alkyl group (e.g., methyl);
(3) a C₇₋₁₃ aralkyl group (e.g., benzyl); or
(4) a C₁₋₆ alkoxy group (e.g., methoxy);
R³ is
(1) a C₁₋₆ alkyl group (e.g., methyl, ethyl);
(2) a C₇₋₁₃ aralkyl group (e.g., benzyl); or
(3) a C₆₋₁₀ aryl-carbonyl group (e.g., benzoyl);
Y is -S- or -NR^{a}- wherein R^{a} is
(1) a hydrogen atom;
(2) a C₁₋₆ alkyl group (e.g., methyl, ethyl, propyl, isopropyl, butyl, isobutyl, pentyl, isopentyl) optionally substituted by 1 to 3 substituents selected from
   (a) an aromatic heterocyclic group (e.g., pyrrolyl, pyridyl);
   (b) a non-aromatic heterocyclic group (e.g., tetrahydropyranyl, piperidinyl, piperazinyl, morpholinyl) optionally substituted by 1 to 3 C₁₋₆ alkyl-carbonyl groups (e.g., acetyl);
   (c) an amino group optionally mono- or di-substituted by C₁₋₆ alkyl groups (e.g., methyl);
   (d) a carbamoyl group;
   (e) a hydroxyl group;
   (f) a C₁₋₆ alkoxy group (e.g., methoxy);
   (g) a C₆₋₁₄ aryloxy group (e.g., phenyloxy) optionally substituted by 1 to 3 halogen atoms (e.g., fluorine atom);
   (h) a C₁₋₆ alkoxy-carbonyl group (e.g., ethoxycarbonyl);
   (i) a C₇₋₁₃ aralkyloxy group (e.g., benzyloxy); and
   (j) a halogen atom (e.g., fluorine atom);
(3) a C₃₋₁₀ cycloalkyl group (e.g., cyclopentyl);
(4) a C₇₋₁₃ aralkyl group (e.g., benzyl, phenethyl, naphthylmethyl, phenylpropyl) optionally substituted by 1 to 3 substituents selected from (a) a C₁₋₆ alkyl group (e.g., methyl, tert-butyl) optionally substituted by 1 to 3 halogen atoms (e.g., fluorine atom)(e.g., trifluoromethyl); (b) a cyano group; and (c) a C₆₋₁₄ aryl group (e.g., phenyl); or
(5) a C₃₋₁₀ cycloalkyl-C₁₋₆ alkyl group (e.g., cyclopropylmethyl, 2-cyclohexylethyl); and
Z is =O.

### [Compound I-A-2]

The aforementioned [compound I-A] wherein
A is a group represented by

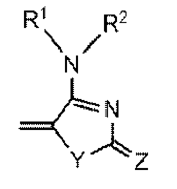

wherein,
R¹ is
(1) a C₁₋₆ alkyl group (e.g., methyl, ethyl, propyl, isopropyl, butyl, isobutyl, sec-butyl, tert-butyl, pentyl, isopentyl, 1-ethylpropyl) optionally substituted by 1 to 3 substituents selected from
   (a) an aromatic heterocyclic group (e.g., furyl, pyridyl, pyrazinyl, imidazolyl) optionally substituted by 1 to 3 C₁₋₆ alkyl groups (e.g., methyl);
   (b) a non-aromatic heterocyclic group (e.g., tetrahydrofuryl, morpholinyl, piperidinyl, pyrrolidinyl) optionally substituted by 1 to 3 C₁₋₆ alkyl groups (e.g., methyl);
   (c) an amino group optionally mono- or di-substituted by C₁₋₆ alkyl groups (e.g., methyl, ethyl);
   (d) a carbamoyl group;
   (e) a hydroxyl group;
   (f) a C₁₋₆ alkoxy group (e.g., methoxy, ethoxy, isopropoxy);
   (g) a C₆₋₁₄ aryloxy group (e.g., phenyloxy);
   (h) a C₁₋₆ alkylthio group (e.g., methylthio); and
   (i) a C₁₋₆ alkoxy-carbonyl group (e.g., methoxycarbonyl);
(2) a C₃₋₁₀ cycloalkyl group (e.g., cyclopropyl, cyclopentyl, cyclohexyl);
(3) a C₇₋₁₃ aralkyl group (e.g., benzyl, phenethyl) optionally substituted by 1 to 3 substituents selected from
   (a) a C₁₋₆ alkoxy group (e.g., methoxy);
   (b) a hydroxyl group;
   (c) a halogen atom (e.g., fluorine atom, chlorine atom); and
   (d) a C₁₋₆ alkyl group (e.g., methyl);
(4) a C₃₋₁₀ cycloalkyl-C₁₋₆ alkyl group (e.g., cyclohexylmethyl); or
(5) an aromatic heterocyclic group (e.g., pyridyl);
R² is a hydrogen atom;
Y is -S- or -NR^{a}- wherein R^{a} is
(1) a hydrogen atom, or
(2) a C₁₋₆ alkyl group (e.g., methyl); and
Z is =O.

### [Compound I-B]

The aforementioned [compound I-A], [compound I-A-1] or [compound I-A-2] wherein R⁵, R⁶, and R⁷ are each a hydrogen atom, and R⁸ is a C₁₋₆ alkoxy group.

### [Compound I-C]

The aforementioned [compound I-A], [compound I-A-1] or [compound I-A-2] or [compound I-B] wherein X is -O-.

### [Compound I-D]

The aforementioned [compound I-A], [compound I-A-1], [compound I-A-2], [compound I-B] or [compound I-C] wherein R⁹ is a C₆₋₁₀ aryl group (preferably, phenyl, naphthyl) optionally substituted by 1 to 3 substituents selected from
(a) a C₁₋₆ alkyl group (e.g., methyl, isopropyl) optionally substituted by 1 to 3 halogen atoms (e.g., fluorine atom) (e.g., trifluoromethyl);
(b) a C₁₋₆ alkoxy group (e.g., methoxy) optionally substituted by 1 to 3 halogen atoms (e.g., fluorine atom) (e.g., trifluoromethoxy);
(c) a cyano group;
(d) a halogen atom (e.g., fluorine atom, chlorine atom, bromine atom);
(e) a nitro group:
(f) a carbamoyl group;
(g) a C₆₋₁₄ aryloxy group (e.g., phenyloxy); and
(h) a C₆₋₁₄ aryl group (e.g., phenyl).

### [Compound I-E]

The compounds according to the following Examples 1 - 219 or salts thereof. Preferably,
(5Z)-5-({4-[2-chloro-4-(trifluoromethyl)phenoxy]-3-methoxyphenyl}methylidene)-4-{[2-(diethylamino)ethyl]amino}-1,3-thiazol-2(5H)-one (Example 94);
4-(2-methoxy-4-{(Z)-[4-{[2-(1-methylpyrrolidin-2-yl)ethyl]aminol-2-oxo-1,3-thiazol-5(2H)-ylidene]methyl}phenoxy)naphthalene-1-carbonitrile (Example 96);
4-{4-[(Z)-{4-[(2-hydroxy-2-methylpropyl)amino]-2-oxo-1,3-thiazol-5(2H)-ylidene}methyl]-2-methoxyphenoxy}-3-(trifluoromethyl)benzonitrile (Example 116);
4-{4-[(Z)-(1-ethyl-5-imino-3-methyl-2-oxoimidazolidin-4-ylidene)methyl]-2-methoxyphenoxy}-3-(trifluoromethyl)benzonitrile (Example 145); or a salt thereof.

As a salt of compound (I), a pharmacologically acceptable salt is preferable. Examples of such salt include a salt with inorganic base, a salt with organic base, a salt with inorganic acid, a salt with organic acid, a salt with basic or acidic amino acid and the like.
Preferable examples of the salt with inorganic base include alkali metal salts such as sodium salt, potassium salt and the like, alkaline earth metal salts such as calcium salt, magnesium salt and the like, aluminum salt, ammonium salt and the like.
Preferable examples of the salt with organic base include salts with trimethylamine, triethylamine, pyridine, picoline, ethanolamine, diethanolamine, triethanolamine, dicyclohexylamine, N,N-dibenzylethylenediamine and the like.
Preferable examples of the salt with inorganic acid include salts with hydrochloric acid, hydrobromic acid, nitric acid, sulfuric acid, phosphoric acid and the like.
Preferable examples of the salt with organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like.
Preferable examples of the salt with basic amino acid include salts with arginine, lysine, ornithine and the like.
Preferable examples of the salt with acidic amino acid include salts with aspartic acid, glutamic acid and the like.
A prodrug of compound (I) refers to a compound which is converted to compound (I) as a result of a reaction with an enzyme, gastric acid, etc. under physiological conditions in vivo, thus a compound that undergoes enzymatic oxidation, reduction, hydrolysis, etc. to convert into compound (I) and a compound that undergoes hydrolysis and the like by gastric acid, etc. to convert into compound (I). Examples of the prodrug for compound (I) include a compound obtained by subjecting an amino group in compound (I) to acylation, alkylation or phosphorylation (e.g., a compound obtained by subjecting an amino group in compound (I) to eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation, tert-butylation, etc.); a compound obtained by subjecting a hydroxy group in compound (I) to acylation, alkylation, phosphorylation or boration (e.g., a compound obtained by subjecting a hydroxy group in compound (I) to acetylation, palmitoylation, propanoylation, pivaloylation, succinylation, fumarylation, alanylation, dimethylaminomethylcarbonylation, etc.); a compound obtained by subjecting a carboxyl group in compound (I) to esterification or amidation (e.g., a compound obtained by subjecting a carboxyl group in compound (I) to ethylesterification, phenylesterification, carboxymethylesterification, dimethylaminomethylesterification, pivaloyloxymethylesterification, ethoxycarbonyloxyethylesterification, phthalidylesterification, (5-methyl-2-oxo-1,3-dioxolen-4-yl)methylesterification, cyclohexyloxycarbonylethylesterification or methylamidation) and the like. Any of these compounds can be produced from compound (I) by a method known *per* se.
A prodrug for compound (I) may also be one which is converted to compound (I) under physiological conditions as described in "IYAKUHIN no KAIHATSU (Development of Pharmaceuticals)", Vol. 7, Design of Molecules, p. 163-198, Published by HIROKAWA SHOTEN (1990).
Compound (I) may be labeled with an isotope (e.g., ³H, ¹⁴C, ³⁵S, ¹²⁵I, ¹¹C, ¹⁸F) and the like.
Compound (I) may be an anhydrate or a hydrate.
In addition, compound (I) may be a solvate or non-solvate. Furthermore, compound (I) may be a deuterium converter.
Compound (I) may be a crystal, and a single crystal form and a mixture of crystal forms are both encompassed in compound (I). The crystal can be produced by crystallization by a crystallization method known per se.
In addition, compound (I) may be a pharmaceutically acceptable cocrystal or cocrystal salt. The cocrystal or cocrystal salt means a crystalline substance constituted with two or more special solids at room temperature, each having different physical properties (e.g., structure, melting point, melting heat, hygroscopicity and stability). The cocrystal or cocrystal salt can be produced by a cocrystallization method known per se.

When compound (I) contains an isomer such as an optical isomer, a stereoisomer, a regioisomer, a rotamer, a geometric isomer or the like, any one isomer and mixtures thereof are also encompassed in compound (I). Specifically, compound (I) contains geometric isomers based on the double bond of the - C(R⁴)=A moiety, and both the geometric isomers (E form and Z form) based on the double bond and a mixture thereof are also encompassed in compound (I). When A is a group represented by the formula

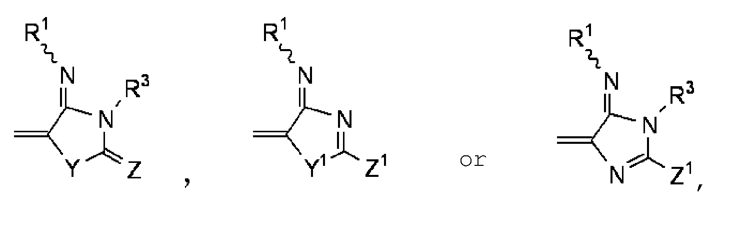

geometric isomers based on the double bond of the imino group (=NR¹) moiety is present, and the wavy line in the above-mentioned formulas means that both the geometric isomers (E form and Z form) based on the double bond and a mixture thereof are also encompassed in compound (I).

When isomers due to conformation are present, such isomers and a mixture thereof are also encompassed in compound (I) of the present invention. These isomers are also encompassed in compound (I), and can be obtained as a single product according to synthesis methods or separation methods known *per* se (e.g., concentration, solvent extraction, column chromatography, recrystallization, etc.). For example, when compound (I) has optical isomers, an optical isomer resolved from this compound is also encompassed in compound (I).
The optical isomer can be produced by a method known per se. To be specific, an optically active synthetic intermediate is used, or the final racemate product is subjected to optical resolution according to a conventional method to give an optical isomer.

The method of optical resolution may be a method known *per* se, such as a fractional recrystallization method, a chiral column method, a diastereomer method, etc.

### 1) Fractional recrystallization method

A method wherein a salt of a racemate with an optically active compound (e.g., (+)-mandelic acid, (-)-mandelic acid, (+)-tartaric acid, (-)-tartaric acid, (+)-1-phenethylamine, (-)-1-phenethylamine, cinchonine, (-)-cinchonidine, brucine, etc.) is formed, which is separated by a fractional recrystallization method, and if desired, a free optical isomer is obtained by a neutralization step.

### 2) Chiral column method

A method wherein a racemate or a salt thereof is applied to a column for separation of an optical isomer (a chiral column) to allow separation. In the case of a liquid chromatography, for example, a mixture of the optical isomers is applied to a chiral column such as ENANTIO-OVM (manufactured by Tosoh Corporation), CHIRAL series (manufactured by Daicel Chemical Industries, Ltd.) and the like, and developed with water, various buffers (e.g., phosphate buffer, etc.) and organic solvents (e.g., ethanol, methanol, isopropanol, acetonitrile, trifluoroacetic acid, diethylamine, etc.) solely or in admixture to separate the optical isomers. In the case of a gas chromatography, for example, a chiral column such as CP-Chirasil-DeX CB (manufactured by GL Sciences Inc.) and the like is used to allow separation.

### 3) Diastereomer method

A method wherein a racemic mixture is prepared into a diastereomeric mixture by chemical reaction with an optically active reagent, which is made into a single substance by a typical separation means (e.g., a fractional recrystallization method, a chromatography method, etc.) and the like, and is subjected to a chemical treatment such as hydrolysis and the like to separate an optically active reagent moiety, whereby an optical isomer is obtained. For example, when compound (I) contains a hydroxyl group, or a primary or secondary amino group in a molecule, the compound and an optically active organic acid (e.g., MTPA [α-methoxy-α-(trifluoromethyl)phenylacetic acid], (-)-menthoxyacetic acid etc.) and the like are subjected to condensation reaction to give diastereomers in the ester form or in the amide form, respectively. When compound (I) has a carboxyl group in molecule, this compound and an optically active amine or an optically active alcohol are subjected to condensation reaction to give diastereomers in the amide form or in the ester form, respectively. The separated diastereomer is converted to an optical isomer of the original compound by acid hydrolysis or base hydrolysis.

Compound (I) may be a crystal, and both a single crystal form and a crystal form mixture are encompassed in compound (I).
The crystal of compound (I) can be produced by crystallization of compound (I) according to crystallization methods known *per* se.

Compound (I) or a prodrug thereof (hereinafter sometimes to be simply abbreviated as the compound of the present invention) has low toxicity, and can be used as it is or in the form of a pharmaceutical composition by mixing with a pharmacologically acceptable carrier etc. to mammals (e.g., human, mouse, rat, rabbit, dog, cat, bovine, horse, swine, monkey) as an agent for the prophylaxis or treatment of various diseases mentioned below.
As pharmacologically acceptable carriers, various organic or inorganic carrier substances conventionally used as preparation materials can be used. These are incorporated as excipient, lubricant, binder and disintegrant for solid preparations, or solvent, solubilizing agent, suspending agent, isotonicity agent, buffer and soothing agent for liquid preparations, and the like and preparation additives such as preservative, antioxidant, colorant, sweetening agent and the like can be added as necessary.
Preferable examples of the excipient include lactose, sucrose, D-mannitol, D-sorbitol, starch, gelatinated starch, dextrin, crystalline cellulose, low-substituted hydroxypropylcellulose, sodium carboxymethylcellulose, gum arabic, pullulan, light anhydrous silicic acid, synthesis aluminum silicate, magnesium alumino metasilicate and the like.
Preferable examples of the lubricant include magnesium stearate, calcium stearate, talc, colloidal silica and the like.
Preferable examples of the binder include gelatinated starch, sucrose, gelatin, gum arabic, methylcellulose, carboxymethylcellulose, sodium carboxymethylcellulose, crystalline cellulose, sucrose, D-mannitol, trehalose, dextrin, pullulan, hydroxypropylcellulose, hydroxypropylmethylcellulose, polyvinylpyrrolidone and the like.
Preferable examples of the disintegrant include lactose, sucrose, starch, carboxymethylcellulose, calcium carboxymethylcellulose, croscarmellose sodium, sodium carboxymethyl starch, light anhydrous silicic acid, low-substituted hydroxypropylcellulose and the like.

Preferable examples of the solvent include water for injection, physiological brine, Ringer's solution, alcohol, propylene glycol, polyethylene glycol, sesame oil, corn oil, olive oil, cottonseed oil and the like.
Preferable examples of the solubilizing agents include polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like.
Preferable examples of the suspending agent include surfactants such as stearyltriethanolamine, sodium lauryl sulfate, lauryl aminopropionate, lecithin, benzalkonium chloride, benzethonium chloride, glycerol monostearate and the like; hydrophilic polymers such as polyvinyl alcohol, polyvinylpyrrolidone, carboxymethylcellulose sodium, methylcellulose, hydroxymethylcellulose, hydroxyethylcellulose, hydroxypropylcellulose and the like, polysorbates, polyoxyethylene hydrogenated castor oil and the like.
Preferable examples of the isotonicity agent include sodium chloride, glycerol, D-mannitol, D-sorbitol, glucose and the like.
Preferable examples of the buffer include buffers of phosphate, acetate, carbonate, citrate etc. and the like.
Preferable examples of the soothing agent include benzyl alcohol and the like.
Preferable examples of the preservative include p-oxybenzoates, chlorobutanol, benzyl alcohol, phenethyl alcohol, dehydroacetic acid, sorbic acid and the like.
Preferable examples of the antioxidant include sulfite, ascorbic acid salt and the like.
Preferable examples of the colorant include aqueous food tar color (e.g., food colors such as Food Color Red Nos. 2 and 3, Food Color Yellow Nos. 4 and 5, Food Color Blue Nos. 1 and 2 and the like food colors), water insoluble lake dye (e.g., aluminum salt of the aforementioned aqueous food tar color), natural dye (e.g., β-carotene, chlorophyll, ferric oxide red) and the like.
Preferable examples of the sweetening agent include saccharin sodium, dipotassium glycyrrhizinate, aspartame, stevia and the like.

Examples of the dosage form of the aforementioned pharmaceutical composition include oral preparations such as tablet (including sublingual tablet, orally disintegrating tablet), capsule (including soft capsule, microcapsule), granule, powder, troche, syrup, emulsion, suspension and the like; and parenteral agents such as injection (e.g., subcutaneous injection, intravenous injection, intramuscular injection, intraperitoneal injection, drip infusion), external preparation (e.g., dermal preparation, ointment), suppository (e.g., rectal suppository, vaginal suppository), pellet, nasal preparation, pulmonary preparation (inhalant), eye drop and the like, which can be respectively safely administered orally or parenterally.
These preparations may be a release control preparation (e.g., sustained-release microcapsule) such as an immediate-release preparation, a sustained-release preparation and the like.
The pharmaceutical composition can be produced by a method conventionally used in the field of preparation formulation, for example, the method described in the Japanese Pharmacopoeia, and the like.
While the content of the compound of the present invention in the pharmaceutical composition varies depending on the dosage form, dose of the compound of the present invention and the like, it is, for example, about 0.1 - 100 wt%.

Since the compound of the present invention shows a superior activity as an estrogen-related receptor-α (ERR-α) modulator (particularly, inverse agonist), it is also useful as a safe pharmaceutical product based on such activity.
The "ERR-α modulator" means a compound having a function to control various actions of ERR-α, and includes ERR-α agonist (agonist), ERR-α antagonist (antagonist), ERR-α inverse agonist (inverse agonist) and the like.
The "ERR-α inverse agonist" means a compound that inhibits the inherent function of ERR-α.
For example, a medicament containing the compound of the present invention is useful for the prophylaxis or treatment of ERR-α associated diseases in mammals (e.g., mouse, rat, hamster, rabbit, cat, dog, bovine, sheep, monkey, human, etc.).
That is, the compound of the present invention has high metabolic stability and, for example, is not easily conjugated by glutathione and the like.

Examples of the "ERR-α associated disease" include
(a) diseases or pathology relating to cancer (e.g., lung cancer, prostate cancer, ovarian cancer, endometrial carcinoma, breast cancer etc.);
(b) diseases or pathology relating to metabolic syndrome including hyperglycemia, insulin insensitivity, diabetes, obesity, hyperlipidemia, hypercholesterolemia, hyperlipoproteinemia, hypertriglyceridemia, dyslipidemia, hypertension, hyperinsulinemia, hyperuricemia or a combination thereof;
(c) diseases or pathology relating to bone or joint including arthritis, osteoarthritis and rheumatoid arthritis;
(d) inflammatory disease, condition or pathology caused by the release of inflammation-inducing cytokine, including rheumatoid arthritis and atherosclerosis; and
(e) mental disease and neurodegenerative disorder or stressrelated disorder including Parkinson's disease, Alzheimer's disease, depression, anxiety and chemical substance addiction; and the like.
The compound of the present invention is particularly effective for the prophylaxis or treatment of solid cancers (e.g., lung cancer, prostate cancer, ovarian cancer, endometrial carcinoma, breast cancer etc.) confirmed to show promoted ERR-α expression.

As used herein, the "prophylaxis" of the above-mentioned diseases means, for example, administration of a medicament containing the compound of the present invention to patients before onset of a disease but having a high risk of the onset due to some factor associated with the disease or patients who developed the disease but without subjective symptoms, or administration of a medicament containing the compound of the present invention to patients having a risk of recurrence of disease after treatment of the disease.

The dose of the compound of the present invention varies depending on the administration subject, route of administration, target disease, symptoms, etc. For example, when it is administered orally to an adult patient with diabetes, its dose is about 0.01 to 100 mg/kg body weight per dose, preferably 0.05 to 30 mg/kg body weight per dose, more preferably 0.1 to 10 mg/kg body weight per dose and still more preferably 0.5 to 10 mg/kg body weight per dose. This amount is desirably administered in one to 3 portions daily.
Moreover, when it is administered orally to an adult patient with prostate cancer, its dose is, for example, about 0.01 to 100 mg/kg body weight per dose, preferably 0.1 to 30 mg/kg body weight per dose and more preferably 0.5 to 10 mg/kg body weight per dose. This amount is desirably administered in one to 3 portions daily.

The compound of the present invention can be used in combination with a medicament such as therapeutic agent for diabetes, therapeutic agents for diabetic complications, therapeutic agent for hyperlipidemia, antihypertensive agent, antiobesity agent, diuretic, chemotherapeutic agent, immunotherapeutic agent, antithrombotic agent, therapeutic agent for osteoporosis or antidementia agent (hereinafter to be abbreviated as a concomitant drug). The time of administration of the compound of the present invention and that of the concomitant drug are not limited, and they may be administered simultaneously or in a staggered manner to the administration subject. Furthermore, the compound of the present invention and a concomitant drug may be administered as two kinds of preparations containing each active ingredient, or may be administered as a single preparation containing both active ingredients.
The dose of the concomitant drug can be appropriately determined based on the dose employed in clinical situations. The mixing ratio of the compound of the present invention and a concomitant drug can be appropriately determined depending on the administration subject, administration route, target disease, symptom, combination and the like. When the administration subject is human, for example, a concomitant drug can be used in 0.01 - 100 parts by weight relative to 1 part by weight of the compound of the present invention.

Examples of the therapeutic agent for diabetes include insulin preparations (e.g., animal insulin preparations extracted from pancreas of bovine and swine; human insulin preparations genetically synthesized using *Escherichia coli* or yeast; zinc insulin; protamine zinc insulin; fragments or derivatives of insulin (e.g., INS-1), oral insulin preparations), insulin sensitizers (e.g., pioglitazone or a salt thereof (preferably hydrochloride), rosiglitazone or a salt thereof (preferably maleate), Netoglitazone, Edaglitazone (BM-13.1258), Rivoglitazone (CS-011), FK-614, compounds described in WO01/38325, Tesaglitazar (AZ-242), Ragaglitazar (NN-622), Muraglitazar (BMS-298585), Metaglidasen (MBX-102), Naveglitazar (LY-519818), MX-6054, LY-510929, Balaglitazone (NN-2344), T-131 or a salt thereof, THR-0921), PPARγ agonists, PPARγ antagonists, PPARγ/a dual agonists, α-glucosidase inhibitors (e.g., voglibose, acarbose, miglitol, emiglitate), biguanides (e.g., phenformin, metformin, buformin or a salt thereof (e.g., hydrochloride, fumarate, succinate)), insulin secretagogue [sulfonylurea (e.g., tolbutamide, glibenclamide, gliclazide, chlorpropamide, tolazamide, acetohexamide, glyclopyramide, glimepiride, glipizide, glybuzole), repaglinide, senaglinide, nateglinide, mitiglinide or calcium hydrate thereof], GPR40 agonists, GLP-1 receptor agonists [e.g., GLP-1, GLP-1MR agents, NN-2211, AC-2993 (exendin-4), BIM-51077, Aib(8,35)hGLP-1(7,37)NH₂, CJC-1131], amylin agonists (e.g., pramlintide), phosphotyrosine phosphatase inhibitors (e.g., sodium vanadate), dipeptidyl peptidase IV inhibitors (e.g., NVP-DPP-278, PT-100, P32/98, Vildagliptin (LAF-237), P93/01, TS-021, Sitagliptin phosphate (MK-431), Saxagliptin (BMS-477118), E-3024, T-6666 (TA-6666), 823093, 825964, 815541), β3 agonists (e.g., AJ-9677), gluconeogenesis inhibitors (e.g., glycogen phosphorylase inhibitors, glucose-6-phosphatase inhibitors, glucagon antagonists), SGLT (sodium-glucose cotransporter) inhibitors (e.g., T-1095), 11β-HSD1 inhibitors (e.g., BVT-3498), adiponectin or agonists thereof, IKK inhibitors (e.g., AS-2868), leptin resistance improving drugs, somatostatin receptor agonists (compounds described in WO01/25228, WO03/42204, WO98/44921, WO98/45285 or WO99/22735), and the like.

Examples of the therapeutic agent for diabetic complications include aldose reductase inhibitors (e.g., Tolrestat, Epalrestat, Zenarestat, Zopolrestat, minalrestat, fidarestat, CT-112), neurotrophic factors and increasing drugs thereof (e.g., NGF, NT-3, BDNF, neurotrophin production-secretion promoters described in WO01/14372 (e.g., 4-(4-chlorophenyl)-2-(2-methyl-1-imidazolyl)-5-[3-(2-methylphenoxy)propyl]oxazole)), nerve regeneration promoters (e.g., Y-128), PKC inhibitors (e.g., ruboxistaurin mesylate), AGE inhibitors (e.g., ALT946, pimagedine, N-phenacylthiazolium bromide (ALT766), ALT-711, EXO-226, Pyridorin, pyridoxamine), active oxygen scavengers (e.g., thioctic acid), cerebral vasodilators (e.g., tiapuride, mexiletine), somatostatin receptor agonists (e.g., BIM23190), and apoptosis signal regulating kinase-1 (ASK-1) inhibitors.
Examples of the therapeutic agent for hyperlipidemia include HMG-CoA reductase inhibitors (e.g., pravastatin, simvastatin, lovastatin, atorvastatin, fluvastatin, pitavastatin, rosuvastatin or a salt thereof (e.g., sodium salt, calcium salt)), squalene synthase inhibitors (e.g., compounds described in WO97/10224, for example, N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid), fibrate compounds (e.g., bezafibrate, clofibrate, simfibrate, clinofibrate), ACAT inhibitors (e.g., Avasimibe, Eflucimibe), anion exchange resins (e.g., colestyramine), probucol, nicotinic acid drugs (e.g., nicomol, niceritrol), ethyl icosapentate, plant sterols (e.g., soysterol, γ-oryzanol) and the like.

Examples of the antihypertensive agent include angiotensin converting enzyme inhibitors (e.g., captopril, enalapril, delapril), angiotensin II antagonists (e.g., candesartan cilexetil, losartan, eprosartan, valsartan, telmisartan, irbesartan, tasosartan, 1-[[2'-(2,5-dihydro-5-oxo-4H-1,2,4-oxadiazol-3-yl)biphenyl-4-yl]methyl]-2-ethoxy-1H-benzimidazole-7-carboxylic acid), calcium antagonists (e.g., manidipine, nifedipine, amlodipine, efonidipine, nicardipine), potassium channel openers (e.g., levcromakalim, L-27152, AL 0671, NIP-121), clonidine and the like.
Examples of the antiobesity agent include antiobesity agents acting on the central nervous system (e.g., dexfenfluramine, fenfluramine, phentermine, sibutramine, amfepramone, dexamphetamine, mazindol, phenylpropanolamine, clobenzorex; MCH receptor antagonists (e.g., SB-568849; SNAP-7941; compounds described in WO01/82925 and WO01/87834); neuropeptide Y antagonists (e.g., CP-422935); cannabinoid receptor antagonists (e.g., SR-141716, SR-147778); ghrelin antagonists), pancreatic lipase inhibitors (e.g., orlistat, ATL-962), β3 agonists (e.g., AJ-9677), peptide anorexiants (e.g., leptin, CNTF (Ciliary Neurotropic Factor)), cholecystokinin agonists (e.g., lintitript, FPL-15849), anorexigenic agents (e.g., P-57) and the like.

Examples of the diuretics include xanthine derivatives (e.g., sodium salicylate and theobromine, calcium salicylate and theobromine), thiazide preparations (e.g., ethiazide, cyclopenthiazide, trichloromethiazide, hydrochlorothiazide, hydroflumethiazide, benzylhydrochlorothiazide, penflutizide, polythiazide, methyclothiazide), antialdosterone preparations (e.g., spironolactone, triamterene), carbonate dehydratase inhibitors (e.g., acetazolamide), chlorobenzenesulfonamide preparations (e.g., chlortalidone, mefruside, indapamide), azosemide, isosorbide, etacrynic acid, piretanide, bumetanide, furosemide and the like.
Examples of the chemotherapeutic agents include alkylating agents (e.g., cyclophosphamide, ifosfamide), metabolic antagonists (e.g., methotrexate, 5-fluorouracil or a derivative thereof), antitumor antibiotics (e.g., mitomycin, adriamycin), plant-derived antitumor agents (e.g., vincristine, vindesine, Taxol), cisplatin, carboplatin, etoposide and the like.
Examples of the immunotherapeutic agent include microorganism or bacterial components (e.g., muramyl dipeptide derivatives, Picibanil), polysaccharides having immunity potentiating activity (e.g., lentinan, schizophyllan, krestin), cytokines obtained by genetic engineering techniques (e.g., interferon, interleukin (IL)), colony stimulating factors (e.g., granulocyte colony stimulating factors, erythropoietin) and the like, with preference given to interleukins such as IL-1, IL-2, IL-12 and the like.

Examples of the antithrombotic agent include heparin (e.g., heparin sodium, heparin calcium, dalteparin sodium), warfarin (e.g., warfarin potassium), anti-thrombin drugs (e.g., aragatroban), thrombolytic agents (e.g., urokinase, tisokinase, alteplase, nateplase, monteplase, pamiteplase), platelet aggregation inhibitors (e.g., ticlopidine hydrochloride, cilostazol, ethyl icosapentate, beraprost sodium, sarpogrelate hydrochloride) and the like.
Examples of the therapeutic agent for osteoporosis include alfacalcidol, calcitriol, elcatonin, calcitonin salmon, estriol, ipriflavone, risedronate disodium, pamidronate disodium, alendronate sodium hydrate, incadronate disodium and the like.
Examples of the antidementia agent include tacrine, donepezil, rivastigmine, galanthamine and the like.

Furthermore, drugs having a cachexia-improving action established in animal models or clinical situations, such as cyclooxygenase inhibitors (e.g., indomethacin), progesterone derivatives (e.g., megestrol acetate), glucosteroids (e.g., dexamethasone), metoclopramide agents, tetrahydrocannabinol agents, fat metabolism improving agents (e.g., eicosapentanoic acid), growth hormones, IGF-1, or antibodies to a cachexia-inducing factor such as TNF-α, LIF, IL-6, oncostatin M and the like, can be used in combination with the compound of the present invention.

The above-mentioned concomitant drug may be used in a combination of two or more kinds at an appropriately ratio.
When the compound of the present invention and a combination drug are used in combination, the amount of each agent can be reduced within a safe range in consideration of the opposite effect of the agents. As a result, the opposite effect caused by these agents can be prevented safely.

The production method of compound (I) of the present invention is explained in the following.
Compound (I) of the present invention can be produced, for example, by the following method A, method B, method D, method E, method F, method G or a method analogous thereto.
In the following each production method, the starting compound and a production intermediate may be in the form of a salt, and examples of the salt include those similar to the aforementioned salt of compound (I) and the like.
The production intermediate obtained by each production method can be used for the next reaction in the form of a reaction mixture itself or a crude product. It may also be isolated and purified from a reaction mixture by a conventional method and using a known means such as phase transfer, concentration, solvent extraction, fractionation, liquid conversion, crystallization, recrystallization, chromatography and the like, and used.
When the compound of each formula is commercially available, a commercially available product may also be used as it is.

A compound represented by the formula (Ia), which is compound (I) wherein A is a group represented by

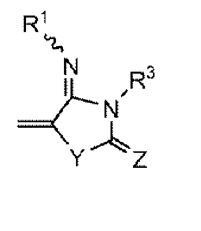

wherein each symbol is as defined above,
or a salt thereof (hereinafter sometimes to be referred to as compound (Ia) (same in other formulas)), and compound (Ib), which is compound (I) wherein
A is a group represented by

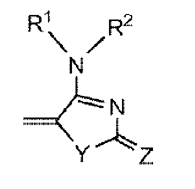

wherein each symbol is as defined above,
can be produced by the following method A or method B, or a method analogous thereto.

### [Method A]

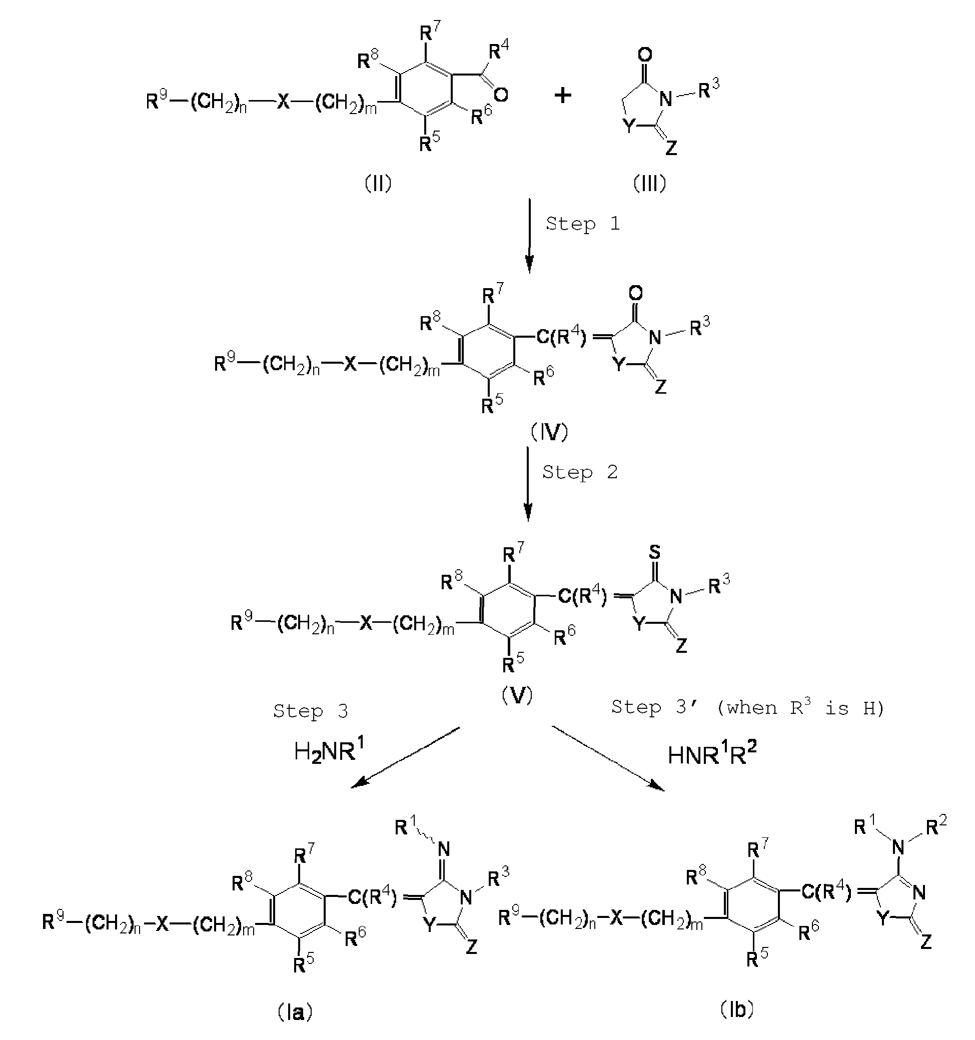

wherein each symbol is as defined above.

### [step 1]

In this step, compound (IV) is produced by subjecting compound (II), which is a benzene derivative substituted by a formyl group or an acyl group, and compound (III) to a condensation reaction under basic conditions.
Compound (II) may be a commercially available product or can be produced according to a method known per se, for example, the method described in "Advanced Organic Chemistry, 4th Ed." (Jerry March), "Comprehensive Organic Transformations, 2nd Ed." (Richard C. Larock) and the like or a method analogous thereto.
This reaction is generally performed in the presence of a base in an inert solvent. Compound (III) may be a commercially available product or can be produced according to a method known per se [e.g., the method described in Journal of the Chemical Society, 1644(1956); Journal of the Chemical Society, 389(1954); Journal of the American Chemical Society, 81, 6498(1959); Journal of the American Chemical Society, 57, 2627(1935) and the like] or a method analogous thereto.
The amount of compound (III) to be used is generally 1 - 10 molar equivalents relative to compound (II).
Examples of the inert solvent include tetrahydrofuran, diethyl ether, 1,2-dimethoxyethane, 1,4-dioxane, toluene, benzene, xylene, N,N-dimethylformamide, dimethyl sulfoxide, methanol, ethanol, acetonitrile and the like. Two or more kinds of these solvents may be mixed in an appropriately ratio and used.
Examples of the base include primary amines, secondary amines such as piperidine and the like, tertiary amines or a salt thereof; and inorganic bases such as potassium fluoride, cesium fluoride, ammonium acetate, sodium hydride, potassium carbonate, cesium carbonate, potassium tert-butoxide and the like.
The amount of the base to be used is generally 0.1 - 10 molar equivalents, preferably 0.5 - 5 molar equivalents, relative to compound (II).
The reaction temperature is generally 0 - 150°C.
While the reaction time is not particularly limited, it is generally 0.1 - 100 hr, preferably 0.5 - 72 hr.

### [step 2]

In this step, compound (IV) is reacted with a Lawesson's reagent or P₂S₅ to produce compound (V).
This reaction is generally performed in an inert solvent.
Examples of the inert solvent include tetrahydrofuran, diethyl ether, 1,2-dimethoxyethane, 1,4-dioxane, toluene, benzene, xylene and the like. Two or more kinds of these solvents may be mixed in an appropriately ratio and used.
The amount of the Lawesson's reagent or P₂S₅ to be used is generally 1 - 10 molar equivalents, preferably 1 - 5 molar equivalents, relative to compound (IV).
The reaction temperature is generally about 0 - 150°C.
While the reaction time is not particularly limited, it is generally 0.1 - 100 hr, preferably 0.5 - 72 hr.

### [step 3]

In this step, compound (V) is reacted with an amine represented by the formula: H₂NR¹ wherein R¹ is as defined above (hereinafter sometimes to be abbreviated as H₂NR¹) or a salt thereof to produce compound (Ia).
H₂NR¹ may be a commercially available product or can be produced according to a method known per se, for example, the method described in "Advanced Organic Chemistry, 4th Ed." (Jerry March), "Comprehensive Organic Transformations, 2nd Ed." (Richard C. Larock) and the like or a method analogous thereto.
This reaction is generally performed in an inert solvent and under microwave irradiation where necessary.
Examples of the inert solvent include tetrahydrofuran, diethyl ether, 1,2-dimethoxyethane, 1,4-dioxane, toluene, benzene, xylene, methanol, ethanol, N,N-dimethylformamide, dimethyl sulfoxide and the like. Two or more kinds of these solvents may be mixed in an appropriately ratio and used.
The amount of H₂NR¹ or a salt thereof to be used is generally 1 - 20 molar equivalents, preferably 1 - 10 molar equivalents, relative to compound (V).
The reaction temperature is generally about 0 - 150°C.
While the reaction time is not particularly limited, it is generally 0.1 - 100 hr, preferably 0.5 - 72 hr.
When a salt of H₂NR¹ is used, the progress of the reaction can be accelerated by adding an inorganic base such as sodium hydride, potassium carbonate, cesium carbonate, potassium tert-butoxide and the like to the reaction system.
The amount of the inorganic base to be used is generally 1 molar equivalent or above relative to a salt of H₂NR¹.

### [step 3']

When R³ of compound (V) is a hydrogen atom, compound (V) can be reacted with an amine represented by the formula: HNR¹R² wherein each symbol is as defined above (hereinafter sometimes to be abbreviated as HNR¹R²) or a salt thereof to produce compound (Ib).
HNR¹R² may be a commercially available product or can be produced according to a method known per se, for example, the method described in "Advanced Organic Chemistry, 4th Ed." (Jerry March), "Comprehensive Organic Transformations, 2nd Ed." (Richard C. Larock) and the like or a method analogous thereto.
This reaction can be performed by a method similar to the aforementioned step 3.

### [method B]

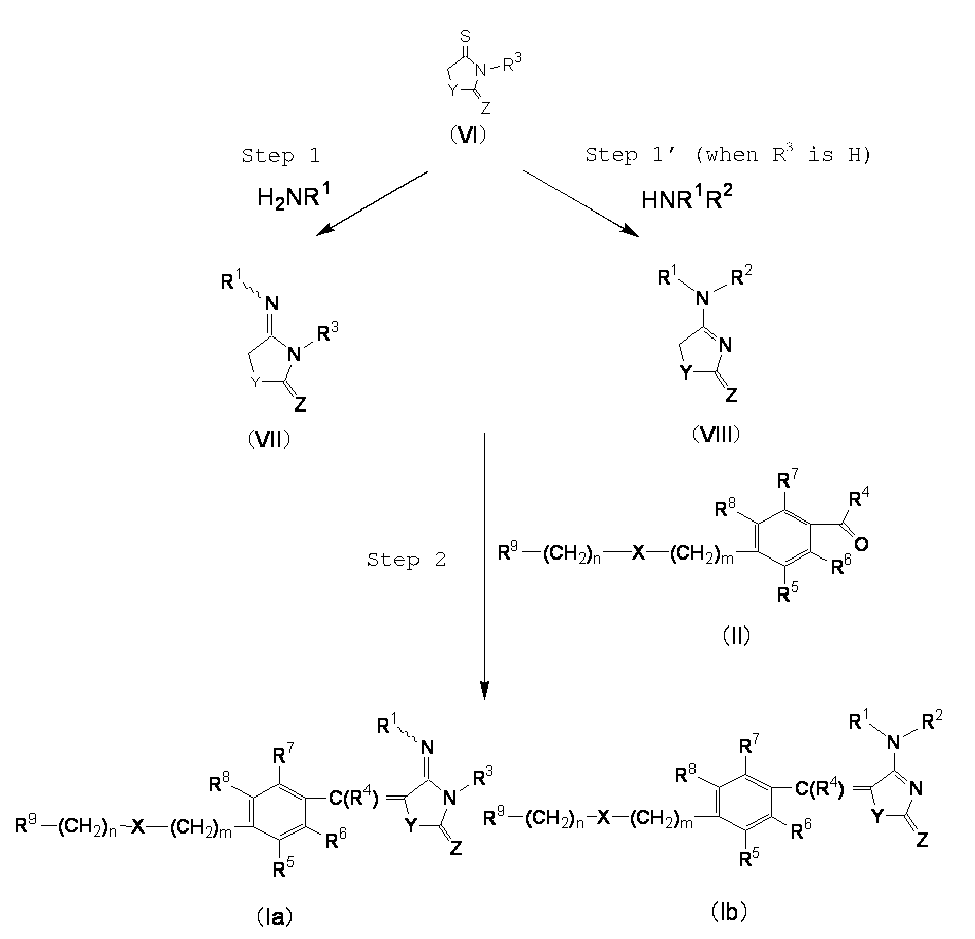

wherein each symbol is as defined above.

### [step 1]

In this step, compound (VI) is reacted with H₂NR¹ or a salt thereof to produce compound (VII).
Compound (VI) may be a commercially available product or can be produced according to a method known per se, for example, the method described in "Advanced Organic Chemistry, 4th Ed." (Jerry March), "Comprehensive Organic Transformations, 2nd Ed." (Richard C. Larock) and the like or a method analogous thereto.
This reaction can be performed by a method similar to the aforementioned method A, step 3.

### [step 1']

When R³ of compound (VI) is a hydrogen atom, compound (VI) can be reacted with HNR¹R² or a salt thereof to produce compound (VIII).
This reaction can be performed by a method similar to the aforementioned method A, step 3'.

### [step 2]

In this step, compound (VII) or compound (VIII) and compound (II) are subjected to a condensation reaction under basic conditions to produce compound (Ia) or compound (Ib), respectively.
This reaction can be performed by a method similar to the aforementioned method A, step 1.

Compound (V) in method A can also be produced by the following method C or a method analogous thereto.

### [method C]

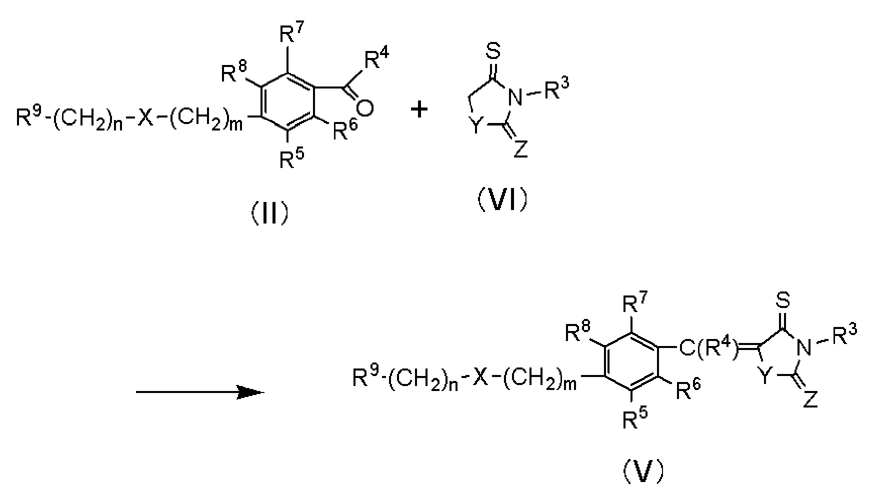

wherein each symbol is as defined above.
In this step, compound (II) and compound (VI) are subjected to a condensation reaction under basic conditions to produce compound (V).
This reaction can be performed by a method similar to the aforementioned method A, step 1.

Compounds (Ic), (Id), (Ie), (If) and (Ig), which are compounds (I) wherein A is a group each represented by

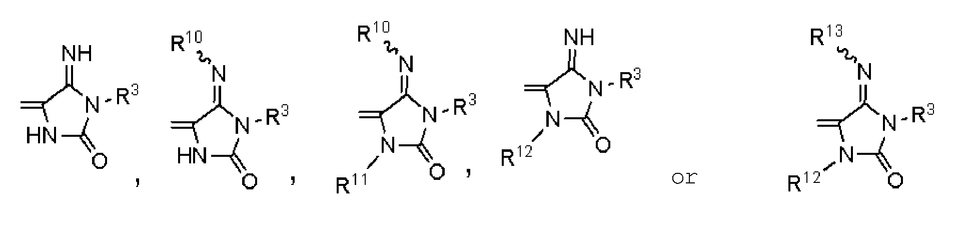

wherein R³ is as defined above, and other symbols are as defined below, can be effectively produced by the following method D or method E, or a method analogous thereto.

### [method D]

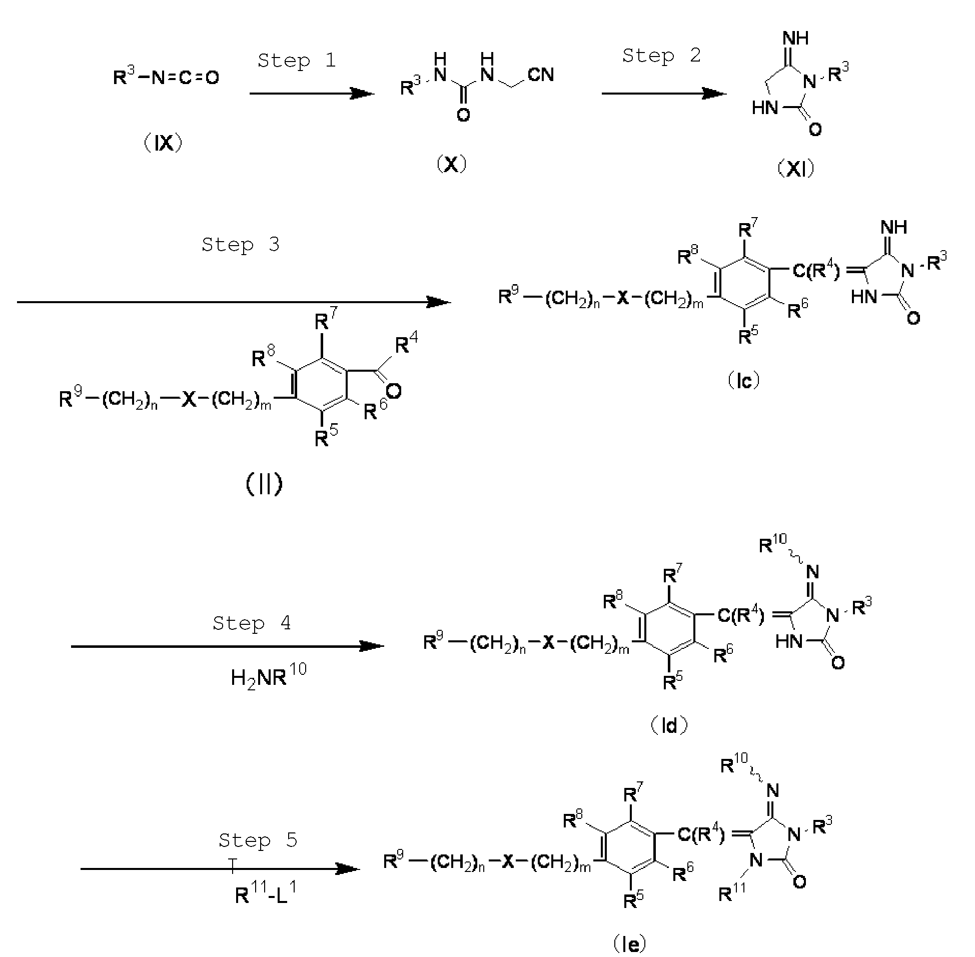

wherein R¹⁰ and R¹¹ are each independently an alkyl group optionally having substituent(s); L¹ is a leaving group; and other symbols are as defined above.

Examples of the "alkyl group" of the "alkyl group optionally having substituent (s)" for R¹⁰ or R¹¹ include a C₁₋₁₀ alkyl group and the like. Examples of the C₁-₁₀ alkyl group include those exemplified as the "hydrocarbon group" of the aforementioned "hydrocarbon group optionally having substituent(s)" for R¹ and the like. The aforementioned C₁₋₁₀ alkyl group optionally has 1 to 3 substituents at substitutable position(s). When the number of substituents is two or more, the substituents may be the same or different.
Examples of the substituent of the C₁₋₁₀ alkyl group include those exemplified as the substituent that the C₁₋₁₀ alkyl group and the like exemplified as the "hydrocarbon group" of the "hydrocarbon group optionally having substituent(s)" for R¹ and the like optionally have.
Examples of the leaving group for L¹ include a halogen atom (e.g., a chlorine atom, a bromine atom, an iodine atom), sulfonic acid ester (e.g., methanesulfonyloxy group, toluenesulfonyloxy group) and the like.

### [step 1]

In this step, compound (IX) is reacted with aminoacetonitrile or a salt thereof to produce compound (X).
Compound (IX) may be a commercially available product or can be produced according to a method known per se, for example, the method described in "Advanced Organic Chemistry, 4th Ed." (Jerry March), "Comprehensive Organic Transformations, 2nd Ed." (Richard C. Larock) and the like or a method analogous thereto.
This reaction is generally performed in an inert solvent.
Examples of the inert solvent include tetrahydrofuran, diethyl ether, 1,2-dimethoxyethane, 1,4-dioxane, toluene, benzene, xylene, N,N-dimethylformamide, dimethyl sulfoxide and the like. Two or more kinds of these solvents may be mixed in an appropriately ratio and used.
The amount of aminoacetonitrile or a salt thereof to be used is generally 1 - 10 molar equivalents, preferably 1 - 5 molar equivalents, relative to isocyanate (IX).
The reaction temperature is generally about 0 - 100°C.
While the reaction time is not particularly limited, it is generally 0.1 - 100 hr, preferably 0.5 - 72 hr.
When a salt of aminoacetonitrile is used, the progress of the reaction can be accelerated by adding an organic base (e.g.: triethylamine, N,N-diisopropylethylamine, 1,8-diazabicyclo[5.4.0]-undec-7-ene) or an inorganic base (e.g.: potassium carbonate, cesium carbonate) to the reaction system.
The amount of the base to be used is generally 0.5 - 10 molar equivalents, preferably 1 - 5 molar equivalents, relative to a salt of aminoacetonitrile.

### [step 2]

In this step, compound (X) is reacted with a base to produce compound (XI).
This reaction is generally performed in an inert solvent.
Examples of the inert solvent include tetrahydrofuran, diethyl ether, 1,2-dimethoxyethane, 1,4-dioxane, toluene, benzene, xylene, N,N-dimethylformamide, dimethyl sulfoxide, ethanol and the like. Two or more kinds of these solvents may be mixed in an appropriately ratio and used.
Examples of the base include triethylamine, 1,8-diazabicyclo[5.4.0]-undec-7-ene, potassium carbonate, cesium carbonate, sodium hydride, potassium tert-butoxide and the like, with preference given to sodium hydride and potassium tert-butoxide.
The amount of the base to be used is generally 1 - 20 molar equivalents, preferably 1 - 10 molar equivalents, relative to compound (X).
The reaction temperature is generally about 0 - 100°C.
While the reaction time is not particularly limited, it is generally 0.1 - 100 hr, preferably 0.5 - 72 hr.

Compound (XI) can also be produced by a one-step reaction from compound (IX) without going through compound (X).
This reaction can be performed by a method similar to the above-mentioned step 1.

### [step 3]

In this step, compound (XI) and compound (II) are subjected to a condensation reaction under basic conditions to produce compound (Ic).
This reaction is generally performed in the presence of a base in an inert solvent.
The amount of compound (XI) to be used is generally 1 - 10 molar equivalents, preferably 1 - 5 molar equivalents, relative to compound (II).
Examples of the inert solvent include tetrahydrofuran, diethyl ether, 1,2-dimethoxyethane, 1,4-dioxane, N,N-dimethylformamide, dimethyl sulfoxide, methanol, ethanol and the like. Two or more kinds of these solvents may be mixed in an appropriately ratio and used.
Examples of the base include primary amines, secondary amines such as piperidine and the like, tertiary amines or a salt thereof; inorganic bases such as potassium fluoride, cesium fluoride, ammonium acetate, sodium hydride, potassium carbonate, cesium carbonate, potassium tert-butoxide and the like, with preference given to potassium tert-butoxide and piperidine.
The amount of the base to be used is generally 1 - 10 molar equivalents, preferably 1 - 5 molar equivalents, relative to compound (II).
The reaction temperature is generally about 0 - 100°C.
While the reaction time is not particularly limited, it is generally 0.1 - 100 hr, preferably 0.5 - 72 hr.

### [step 4]

In this step, compound (Ic) is reacted with a salt of amine represented by the formula: H₂NR¹⁰ wherein R¹⁰ is as defined above (hereinafter sometimes to be abbreviated as H2NR¹⁰) to produce compound (Id).
H₂NR¹⁰ may be a commercially available product or can be produced according to a method known per se, for example, the method described in "Advanced Organic Chemistry, 4th Ed." (Jerry March), "Comprehensive Organic Transformations, 2nd Ed." (Richard C. Larock) and the like or a method analogous thereto.
This reaction is generally performed in an inert solvent.
Examples of the inert solvent include tetrahydrofuran, diethyl ether, 1,2-dimethoxyethane, 1,4-dioxane, N,N-dimethylformamide, dimethyl sulfoxide, methanol, ethanol and the like. Two or more kinds of these solvents may be mixed in an appropriately ratio and used.
As a salt of H₂NR¹⁰ hydrochloride, hydrobromide, sulfate, carbonate, methanesulfonate, tosylate and the like can be used, with preference given to hydrochloride.
The amount of the salt of H₂NR¹⁰ to be used is generally 1 - 20 molar equivalents, preferably 1 - 10 molar equivalents, relative to compound (Ic).
The reaction temperature is generally about 0 - 100°C.
While the reaction time is not particularly limited, it is generally 0.1 - 100 hr, preferably 0.5 - 72 hr.

### [step 5]

In this step, compound (Id) is reacted with a compound represented by the formula: R¹¹-L¹ wherein each symbol is as defined above (hereinafter sometimes to be abbreviated as R¹¹-L¹) to produce compound (Ie).
R¹¹-L¹ may be a commercially available product or can be produced according to a method known per se, for example, the method described in "Advanced Organic Chemistry, 4th Ed." (Jerry March), "Comprehensive Organic Transformations, 2nd Ed." (Richard C. Larock) and the like or a method analogous thereto.
This reaction is generally performed in an inert solvent in the presence of a base.
The amount of R¹¹-L¹ to be used is generally 1 - 20 molar equivalents, preferably 1 - 10 molar equivalents, relative to compound (Id).
Examples of the inert solvent include tetrahydrofuran, diethyl ether, 1,2-dimethoxyethane, 1,4-dioxane, N,N-dimethylformamide, dimethyl sulfoxide and the like. Two or more kinds of these solvents may be mixed in an appropriately ratio and used.
As a base, an inorganic base such as sodium hydride, potassium carbonate, cesium carbonate, potassium tert-butoxide and the like can be used, with preference given to potassium tert-butoxide.
The amount of the base to be used is generally 1 - 10 molar equivalents, preferably 1 - 5 molar equivalents, relative to compound (Id).
The reaction temperature is generally about 0 - 100°C.
While the reaction time is not particularly limited, it is generally 0.1 - 100 hr, preferably 0.5 - 72 hr.

### [method E]

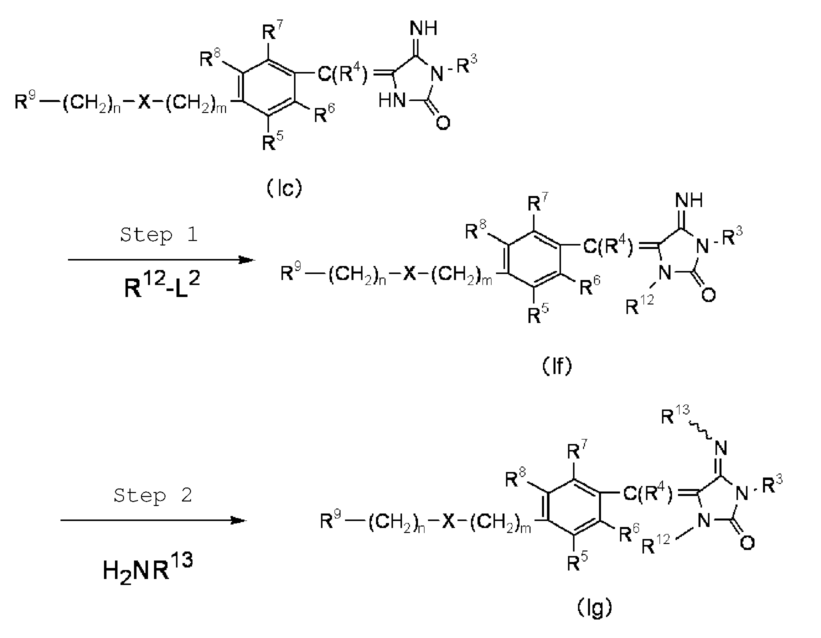

wherein R¹² is an alkyl group optionally having substituent(s); R¹³ is an alkyl group optionally having substituent(s) or an acyl group; L² is a leaving group; and other symbols are as defined above.

Examples of the "alkyl group" of the "alkyl group optionally having substituent(s)" for R¹² or R¹³ include a C₁₋₁₀ alkyl group and the like. Examples of the C₁₋₁₀ alkyl group include those exemplified as the "hydrocarbon group" of the aforementioned "hydrocarbon group optionally having substituent(s)" for R¹ and the like.
The aforementioned C₁₋₁₀ alkyl group optionally has 1 to 3 substituents at substitutable position(s). When the number of substituents is two or more, the substituents may be the same or different.
Here, examples of the substituent of the C₁₋₁₀ alkyl group include those exemplified as the substituent that the C₁₋₁₀ alkyl group and the like exemplified as the "hydrocarbon group" of the "hydrocarbon group optionally having substituent(s)" for R¹ and the like optionally have.
Examples of the "acyl group" for R¹³ include those similar to the aforementioned "acyl group" for R¹ and the like.
Examples of the leaving group for L² include a halogen atom (e.g., a chlorine atom, a bromine atom, an iodine atom), sulfonic acid ester (e.g., methanesulfonyloxy group, toluenesulfonyloxy group) and the like.

### [step 1]

In this step, compound (Ic) is reacted with a compound represented by the formula: R¹²-L² wherein each symbol is as defined above (hereinafter sometimes to be abbreviated as R¹²-L²) to produce compound (If).
R¹²-L² may be a commercially available product or can be produced according to a method known per se, for example, the method described in "Advanced Organic Chemistry, 4th Ed." (Jerry March), "Comprehensive Organic Transformations, 2nd Ed." (Richard C. Larock) and the like or a method analogous thereto.
This reaction is generally performed in an inert solvent in the presence of a base.
The amount of R¹²-L² to be used is generally 1 - 20 molar equivalents, preferably 1 - 10 molar equivalents, relative to compound (Ic).
Examples of the inert solvent include tetrahydrofuran, diethyl ether, 1,2-dimethoxyethane, 1,4-dioxane, N,N-dimethylformamide, dimethyl sulfoxide and the like. Two or more kinds of these solvents may be mixed in an appropriately ratio and used.
Examples of the base include inorganic bases such as sodium hydride, potassium carbonate, cesium carbonate, potassium tert-butoxide and the like, with preference given to potassium tert-butoxide.
The amount of the base to be used is generally 1 - 10 molar equivalents, preferably 1 - 5 molar equivalents, relative to compound (Ic).
The reaction temperature is generally about 0 - 100°C.
While the reaction time is not particularly limited, it is generally 0.1 - 100 hr, preferably 0.5 - 72 hr.

### [step 2]

In this step, when R¹³ is an alkyl group optionally having substituent(s), compound (If) is reacted with a salt of amine represented by the formula: H₂NR¹³ (hereinafter sometimes to be abbreviated as H₂NR¹³), or when R¹³ is an acyl group, compound (If) is reacted with the corresponding acylating agent in the presence of a base to produce compound (Ig).
H₂NR¹³ may be a commercially available product or can be produced according to a method known per se, for example, the method described in "Advanced Organic Chemistry, 4th Ed." (Jerry March), "Comprehensive Organic Transformations, 2nd Ed." (Richard C. Larock) and the like or a method analogous thereto.
The reaction of compound (If) with a salt of H₂NR¹³ can be performed by a method similar to the aforementioned method D, step 4.
The reaction of compound (If) with the corresponding acylating agent is generally performed in an inert solvent.
Examples of the corresponding acylating agent include corresponding acid chloride, corresponding acid anhydride, corresponding active ester and the like.
The amount of the corresponding acylating agent to be used is generally 1 - 10 molar equivalents, preferably 1 - 5 molar equivalents, relative to compound (If).
Examples of the inert solvent include tetrahydrofuran, diethyl ether, 1,2-dimethoxyethane, 1,4-dioxane, N,N-dimethylformamide, dimethyl sulfoxide and the like. Two or more kinds of these solvents may be mixed in an appropriately ratio and used.
Examples of the base include organic bases (e.g., triethylamine, 1,8-diazabicyclo[5.4.0]-undec-7-ene) and inorganic bases (e.g., sodium hydride, potassium carbonate, cesium carbonate, potassium tert-butoxide).
The amount of the base to be used is generally 1 - 10 molar equivalents, preferably 1 - 5 molar equivalents, relative to compound (If).
The reaction temperature is generally about 0 - 100°C.
While the reaction time is not particularly limited, it is generally 0.1 - 100 hr, preferably 0.5 - 72 hr.

Compound (Ih), which is compound (I) wherein A is a group represented by

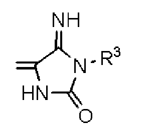

wherein R³ is as defined above, and
X is -O- or a salt thereof can be produced by the following method F, or a method analogous thereto.

### [method F]

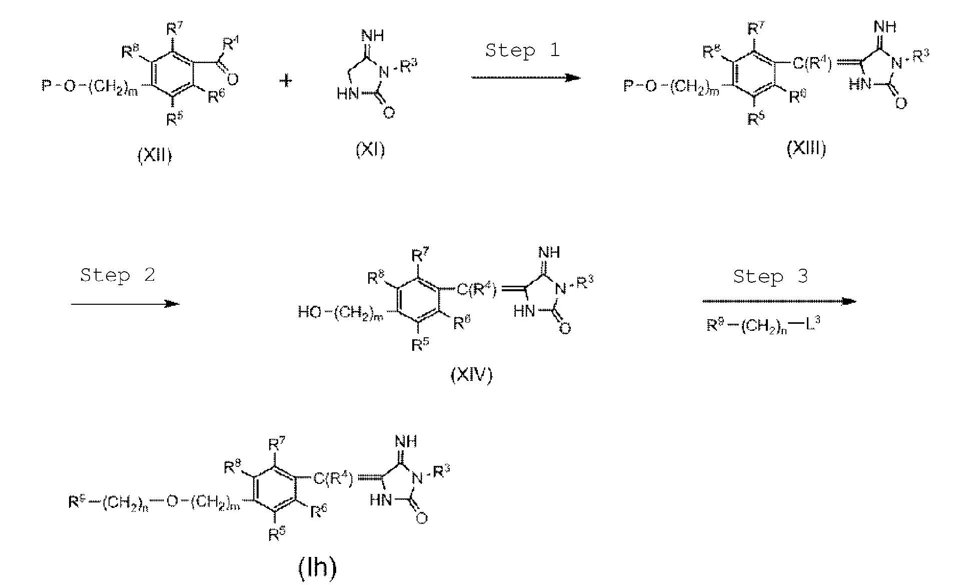

wherein P is a protecting group; L³ is a leaving group, -SO₂-Cl, -CO-Cl or a hydroxyl group; and other symbols are as defined above.
Examples of the protecting group for P include a hydroxyl-protecting group described in Protective Groups in Organic Synthesis, 3rd printing, pages 17 - 292, Theodora W. Greene, Peter G. M. Wuts, and the like, with preference given to 4-methoxybenzyl group and the like.
Examples of the leaving group for L³ include a halogen atom (e.g., a chlorine atom, a fluorine atom, a bromine atom, an iodine atom), sulfonic acid ester (e.g., methanesulfonyloxy group, toluenesulfonyloxy group) and the like.

### [step 1]

In this step, compound (XII) and compound (XI) are condensed under basic conditions to produce compound (XIII).
Compound (XII) may be a commercially available product or can be produced according to a method known per se, for example, the method described in "Advanced Organic Chemistry, 4th Ed." (Jerry March), "Comprehensive Organic Transformations, 2nd Ed." (Richard C. Larock) and the like or a method analogous thereto.
This reaction can be performed by a method similar to method D, step 3.

### [step 2]

In this step, a hydroxyl-protecting group P of compound (XIII) is subjected to a removal reaction thereof to produce compound (XIV).
While the reaction conditions vary depending on the kind of the protecting group, this reaction can be performed appropriately according to the method of removing hydroxyl-protecting group described in Protective Groups in Organic Synthesis, 3rd printing, pages 17 - 292, Theodora W. Greene, Peter G. M. Wuts, or a method analogous thereto.

### [step 3]

In this step, compound (XIV) is reacted with a compound represented by R⁹-(CH₂)ₙ-L³ wherein each symbol is as defined above (hereinafter to be abbreviated as R⁹-(CH₂)ₙ-L³) to produce compound (Ih).
R⁹-(CH₂)ₙ-L³ may be a commercially available product or can be produced according to a method known per se, for example, the method described in "Advanced Organic Chemistry, 4th Ed." (Jerry March), "Comprehensive Organic Transformations, 2nd Ed." (Richard C. Larock) and the like or a method analogous thereto.
For example, when m is 0; n is an integer of 1 - 3; and L³ is a hydroxyl group, compound (Ih) can be produced under the reaction conditions known as the Mitsunobu reaction.
This reaction is generally performed in an inert solvent and in the presence of azodicarboxylate, and triphenylphosphine or trialkylphosphine.
The amount of R⁹-(CH₂)ₙ-OH to be used is generally 1 - 10 molar equivalents, preferably 1 - 5 molar equivalents, relative to compound (XIV).
Examples of the inert solvent include tetrahydrofuran, 1,4-dioxane, N,N-dimethylformamide, dichloromethane, chloroform, benzene, toluene and the like. Two or more kinds of these solvents may be mixed in an appropriately ratio and used.
Preferable examples of azodicarboxylate include diethyl azodicarboxylate and dimethyl azodicarboxylate and the amount thereof to be used is generally 1 - 10 molar equivalents, preferably 1 - 5 molar equivalents, relative to compound (XIV).
The amount of triphenylphosphine or trialkylphosphine to be used is generally 1 - 10 molar equivalents, preferably 1 - 5 molar equivalents, relative to compound (XIV). As trialkylphosphine, tributylphosphine is preferable.
The reaction temperature is generally about 0 - 100°C.
While the reaction time is not particularly limited, it is generally 0.1 - 100 hr, preferably 0.5 - 72 hr.

When L³ is other than a hydroxyl group, this reaction is generally performed in an inert solvent and in the presence of a base where necessary.
The amount of R⁹-(CH₂)ₙ-L³ to be used is generally 1 - 10 molar equivalents, preferably 1 - 5 molar equivalents, relative to compound (XIV).
Examples of the inert solvent include tetrahydrofuran, diethyl ether, 1,2-dimethoxyethane, 1,4-dioxane, N,N-dimethylformamide, dimethyl sulfoxide, pyridine and the like. Two or more kinds of these solvents may be mixed in an appropriately ratio and used.
Examples of the base include inorganic bases such as sodium hydride, potassium carbonate, cesium carbonate, lithium carbonate, potassium tert-butoxide and the like.
The amount of the base to be used is generally 1 - 10 molar equivalents, preferably 1 - 5 molar equivalents, relative to compound (XIV).
The reaction temperature is generally about 0 - 100°C.
While the reaction time is not particularly limited, it is generally 0.1 - 100 hr, preferably 0.5 - 72 hr.

Compound (Ii), which is compound (I) wherein A is a group represented by

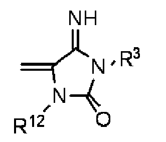

wherein each symbol is as defined above, and X is -O-, or a salt thereof can be produced by the following method G, or a method analogous thereto.

### [method G]

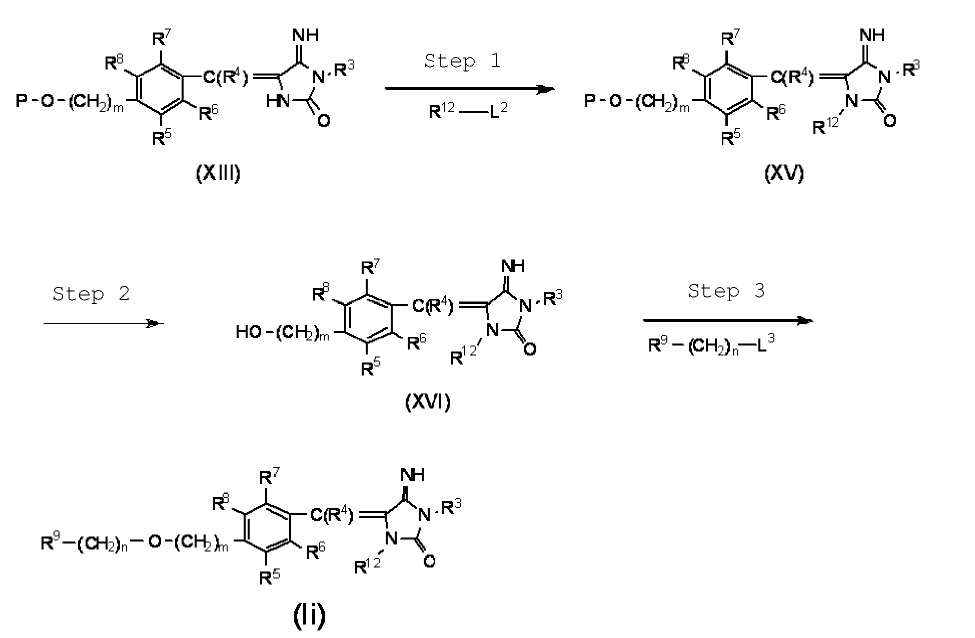

wherein each symbol is as defined above.

### [step 1]

In this step, compound (XIII) is reacted with R¹²-L² to produce compound (XV).
This reaction can be performed by a method similar to method E, step 1.

### [step 2]

In this step, a hydroxyl-protecting group P of compound (XV) is subjected to a removal reaction thereof to produce compound (XVI).
This reaction can be performed by a method similar to method F, step 2.

### [step 3]

In this step, compound (XVI) is reacted with R⁹-(CH₂)ₙ-L³ to produce compound (Ii).
This reaction can be performed by a method similar to method F, step 3.

It is also possible to produce a compound encompassed in the present invention by further applying substituent introduction or functional group conversion to compound (I) (e.g., compounds (Ia) - (Ih) obtained by the above-mentioned methods) by a means known per se. Substituent introduction and functional group conversion are performed by known conventional methods such as conversion to carboxy group by ester hydrolysis, conversion to carbamoyl group by amidation of carboxy group, conversion to hydroxymethyl group by reduction of carboxy group, conversion to alcohol form by reduction or alkylation of carbonyl group, reductive amination of carbonyl group, oximation of carbonyl group, acylation, ureation, sulfonylation or alkylation of amino group, amination of activated halogen with amine, conversion to amino group by reduction of nitro group, and acylation, carbamation, sulfonylation or alkylation of hydroxy group. When a reactive substituent causing an unintended reaction during substituent introduction and functional group conversion is present, a protecting group may be introduced in advance into the reactive substituent as necessary by a means known per se, the object reaction is performed and the protecting group is removed by a means known per se, whereby compounds encompassed in the present invention can be produced.

### Examples

The present invention is explained in detail in the following by referring to Reference Examples, Examples, Experimental Examples and Formulation Examples. However, the examples do not limit the present invention.
The elution by column chromatography in the Reference Examples and Examples was performed under the observation by TLC (Thin Layer Chromatography). In the observation by TLC, Kieselgel 60 F₂₅₄ plate manufactured by Merck was used as a TLC plate, the solvent used as an elution solvent in column chromatography was used as an eluent, and UV detector was used as a detection method. As silica gel for the column, the same Kieselgel 60 F₂₅₄ (70 - 230 mesh) manufactured by Merck or Purif-Pack (SI 60 µm) and Purif-Pack (NH 60 µm) manufactured by MORITEX were used. NMR spectrum shows proton NMR, which was measured by VARIAN Mercury-300 (300 MHz) or JMTC0400/54 (400 MHz) type BRUKER AVANCE II 300, BRUKER AVANCE 400 manufactured by JEOL Ltd., using tetramethylsilane as the internal standard, and δ value is shown in ppm. The melting point was measured by using MPA100 type melting point measuring apparatus Optimelt manufactured by Stanford Research Systems. As a microwave reactor, Initiator Microwave Synthesis Systems manufactured by Biotage was used.
The abbreviations used in Reference Examples and Examples mean the following.
The abbreviations used in Examples and Reference Examples mean as described below.
LC: liquid chromatography
MS: mass spectrometry spectrum
ESI: electrospray method
NMR: nuclear magnetic resonance spectrum
s : singlet
bar : broad
brs: broad singlet
d : doublet
t : triplet
q : quartet
dd : double doublet
ddd: double double doublet
dt : double triplet
m : multiplet
J : coupling constant
Hz : hertz
CDCl₃: deuterated chloroform
DMSO: dimethyl sulfoxide
DMF _{:} N,N-dimethylformamide
MeOH: methanol

### Reference Example 1

3-methoxy-4-(naphthalen-1-ylmethoxy)benzaldehyde

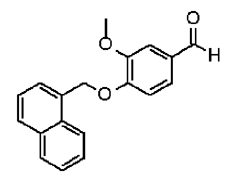

To a solution (50 mL) of vanillin (5 g) in anhydrous DMF were added 1-(chloromethyl)naphthalene (5.4 mL) and potassium carbonate (4.99 g), and the mixture was stirred at room temperature for 2 days. Water was added to the reaction mixture, and the mixture was extracted with ethyl acetate. The organic layer was washed with saturated brine, dried over anhydrous magnesium sulfate, and filtered, and the filtrate was concentrated under reduced pressure. The residue was purified by silica gel column chromatography (eluent: hexane-ethyl acetate=95:5→1:1) to give the title compound as a pale-yellow oil (yield: 8.5 g, 88%).
¹H-NMR (CDCl₃, 400 MHz):δ3.93 (3H, s), 5.68 (2H, s), 7.11 (1H, d, J = 8.3 Hz), 7.40-7. 49 (3H, m), 7.51-7.63 (3H, m), 7.88 (2H, dd, J = 19.9, 8.4 Hz), 7.99-8.08 (1H, m), 9.86 (1H, s).

### Reference Example 2

### 4-{[2,4-bis(trifluoromethyl)benzyl]oxy}-3-methoxybenzaldehyde

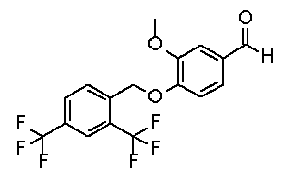

To a solution (25 mL) of vanillin (2.13 g) in anhydrous DMF were added 2,4-bis(trifluoromethyl)benzyl bromide (4.72 g) and potassium carbonate (2.13 g), and the mixture was stirred at room temperature for 2 hr. Water was added to the reaction mixture, and the precipitated solid was collected by filtration. The solid was washed with water, and dried to give the title compound as a white solid (yield: 5.2 g, 98%).
¹H-NMR (DMSO-d₆, 400 MHz):δ3.87 (3H, s), 5.46 (2H, s), 7.28 (1H, d, J = 8.3 Hz), 7.47 (1H, d, J = 1.7 Hz), 7.57 (1H, dd, J = 8.3, 1.7 Hz), 8.04 (1H, d, J = 8.1 Hz), 8.12 (1H, s), 8.19 (1H, d, J = 8.3 Hz), 9.87 (1H, s).

### Reference Example 3

### (5Z)-5-{[3-methoxy-4-(naphthalen-1-ylmethoxy)phenyl]methylidene}-1,3-thiazolidine-2,4-dione

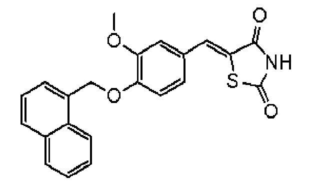

To a solution (11 mL) of 3-methoxy-4-(naphthalen-l-ylmethoxy)benzaldehyde (1.0 g) in ethanol were added 1,3-thiazolidine-2,4-dione (401 mg) and piperidine (68 µL), and the mixture was heated under reflux for 24 hr. The reaction mixture was cooled to room temperature, and the solid was filtered to give the title compound as a yellow solid (yield: 1.05 g, 78%).
¹H-NMR (DMSO-d₆, 400 MHz)3.73-3.81 (3H, m), 5.62 (2H, s), 7.19-7.26 (2H, m), 7.42 (1H, d, J = 8.1 Hz), 7.50-7.63 (3H, m), 7.69 (1H, d, J = 6.8 Hz), 7.78 (1H, s), 7.93-8.03 (2H, m), 8.04-8.13 (1H, m), 12.55 (1H, brs).

### Reference Example 4

### (5Z)-5-[(4-{[2,4-bis(trifluoromethyl)benzyl]oxy}-3-methoxyphenyl)methylidene]-1,3-thiazolidine-2,4-dione

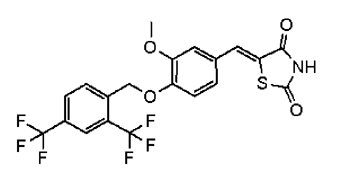

To a solution (5 mL) of 4-{[2,4-bis(trifluoromethyl)benzyl]oxy}-3-methoxybenzaldehyde (567 mg) in ethanol were added 1,3-thiazolidine-2,4-dione (176 mg) and piperidine (30 µL), and the mixture was heated under reflux for 3 days. The reaction mixture was cooled to room temperature, and the solid was filtered to give the title compound as a pale-yellow solid (yield: 576 g, 80%).
¹H-NMR (DMSO-d₆, 400 MHz)3.84 (3H, s), 5.42 (2H, s), 7.16-7.22 (2H, m), 7.28 (1H, s), 7.77 (1H, s), 8.02 (1H, d, J = 8.1 Hz), 8.12 (1H, s), 8.18 (H, d, J = 8.6 Hz), 12.57 (1H, s).

### Reference Example 5

### (5Z)-5-{[3-methoxy-4-(naphthalen-1-ylmethoxy)phenyllmethylidenel-4-thioxo-1,3-thiazolidin-2-one

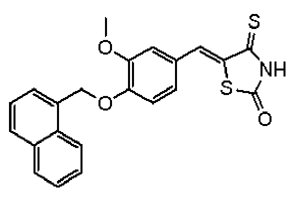

To a solution (4 mL) of (5Z)-5-{[3-methoxy-4-(naphthalen-1-ylmethoxy)phenyl]methylidene}-1,3-thiazolidine-2,4-dione (186 mg) in toluene was added a Lawesson reagent (135 mg), and the mixture was heated under reflux for 10 hr. The reaction mixture was cooled to room temperature, and the solid was filtered to give the title compound as a red solid (yield: 109 mg, 56%).
¹H-NMR (DMSO-d₆, 400 MHz)δ3.76-3.84 (3H, m), 5.65 (2H, s), 7.30-7.37 (2H, m), 7.45 (1H, d, J = 8.6 Hz), 7.51-7.63 (3H, m), 7.69 (1H, d, J = 6.8 Hz), 7.94-8.03 (2H, m), 8.06-8.13 (2H, m), 13.81 (1H, brs).

### Reference Example 6

### (5Z)-5-[(4-{[2,4-bis(trifluoromethyl)benzyl]oxy}-3-methoxyphenyl)methylidene]-4-thioxo-1,3-thiazolidin-2-one

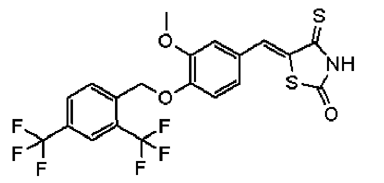

To a solution (20 mL) of (5Z)-5-[(4-{[2,4-bis(trifluoromethyl)benzyl]oxy}-3-methoxyphenyl)methylidene]-1,3-thiazolidine-2,4-dione (1.2 g) in toluene was added a Lawesson reagent (712 mg), and the mixture was heated under reflux for 10 hr. The reaction mixture was cooled to room temperature, and the solid was filtered to give the title compound as a red solid (yield: 1.1 g, 89%).
¹H-NMR (DMSO-d₆, 400 MHz)δ3.83-3.89 (3H, m), 5.44 (2H, s), 7.21-7.27 (1H, m), 7.27-7.34 (1H, m), 7.35 (1H, d, J = 2.0 Hz), 8.02 (1H, d, J = 8.1 Hz), 8.07-8.14 (2H, m), 8.18 (1H, s), 13.83 (1H, brs).

### Reference Example 7

### 4-{[2,4-bis(trifluoromethyl)benzyl]oxy}-3-methylbenzaldehyde

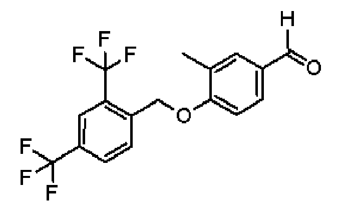

To a solution (7.3 mL) of 4-hydroxy-3-methylbenzaldehyde (1.00 g) and potassium carbonate (1.22 g) in DMF was added 1-(bromomethyl)-2,4-bis(trifluoromethyl)benzene (1.52 mL), and the mixture was stirred at room temperature for 1 hr. The reaction mixture was poured into water, and the mixture was extracted with ethyl acetate. The organic layer was washed with saturated brine, dried over anhydrous magnesium sulfate and concentrated under reduced pressure to give the title compound as a colorless powder (yield: 2.30 g, 85%).
¹H-NMR (CDCl₃, 300 MHz):δ2.41 (3H, s), 5.44 (2H, s), 6.95 (1H, d, J= 8.5 Hz), 7.73 (1H, dd, J = 8.5, 1.7 Hz), 7.76-7.80 (1H, m), 7.86-7.97 (2H, m), 8.00 (1H, s), 9.91 (1H, s).

### Reference Example 8

### 4-{[2,4-bis(trifluoromethyl)benzyl]oxy}-2-methylbenzaldehyde

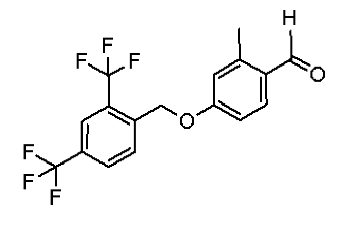

To a solution (7.3 mL) of 4-hydroxy-2-methylbenzaldehyde (1.00 g) and potassium carbonate (1.22 g) in DMF was added 1-(bromomethyl)-2,4-bis(trifluoromethyl)benzene (1.52 mL), and the mixture was stirred at room temperature for 3.5 hr. The reaction mixture was poured into water, and the mixture was extracted with ethyl acetate. The organic layer was washed with saturated brine, dried over anhydrous magnesium sulfate and concentrated under reduced pressure to give the title compound as a pale-yellow powder (yield: 2.50 g, 94%).
¹H-NMR (CDCl₃, 300 MHz):δ2.69 (3H, s), 5.40 (2H, s), 6.85 (1H, d, J= 2.4 Hz), 6.92 (1H, dd, J = 8.7, 2.4 Hz), 7.80 (1H, d, J = 8.7 Hz), 7.84-7.94 (2H, m), 7.99 (1H, s), 10.16 (1H, s).

### Reference Example 9

### 4-{[2,4-bis(trifluoromethyl)benzyl]oxy}-2-chlorobenzaldehyde

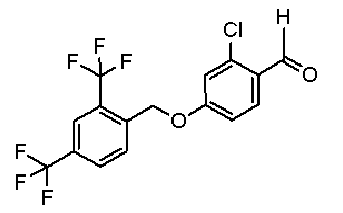

To a solution (6.4 mL) of 2-chloro-4-hydroxybenzaldehyde (1.00 g) and potassium carbonate (1.06 g) in DMF was added 1-(bromomethyl)-2,4-bis(trifluoromethyl)benzene (1.32 mL), and the mixture was stirred at room temperature for 3.5 hr. The reaction mixture was poured into water, and the mixture was extracted with ethyl acetate. The organic layer was washed with saturated brine, dried over anhydrous magnesium sulfate and concentrated under reduced pressure to give the title compound as a pale-yellow powder (yield: 1.97 g, 81%).
¹H-NMR (CDCl₃, 300 MHz)δ5.38 (2H, s), 6.91-7.00 (1H, m), 7.03 (1H, d, J= 2.3 Hz), 7.88 (2H, d, J = 1.1 Hz), 7.93 (1H, d, J = 8.7 Hz), 7.99 (1H, s), 10.36 (1H, d, J = 0.8 Hz).

### Reference Example 10

### 4-{[2,4-bis(trifluoromethyl)benzyl]oxy}-3-chlorobenzaldehyde

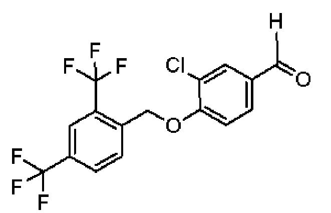

To a solution (6.4 mL) of 3-chloro-4-hydroxybenzaldehyde (1.00 g) and potassium carbonate (1.06 g) in DMF was added 1-(bromomethyl)-2,4-bis(trifluoromethyl)benzene (1.32 mL), and the mixture was stirred at room temperature for 1 hr. The reaction mixture was poured into water, and the mixture was extracted with ethyl acetate. The organic layer was washed with saturated brine, dried over anhydrous magnesium sulfate and concentrated under reduced pressure to give the title compound as a colorless powder (yield: 2.25 g, 92%).
¹H-NMR (CDCl₃, 300 MHz):δ5.48 (2H, s), 7.08 (1H, d, J = 8.5 Hz), 7.79 (1H, dd, J = 8.5, 2.07 Hz), 7.91 (1H, d, J = 8.3 Hz), 7.97-8.00 (2H, m), 8.08 (1H, d, J= 8.3 Hz), 9.89 (1H, s).

### Reference Example 11

### 4-{[2,4-bis(trifluoromethyl)benzyl]oxy}-2-methoxybenzaldehyde

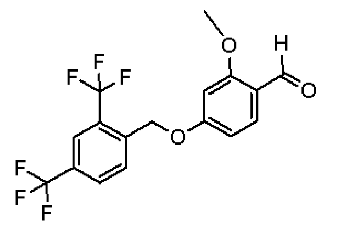

To a solution (6.6 mL) of 4-hydroxy-2-methoxybenzaldehyde (1.00 g) and potassium carbonate (1.09 g) in DMF was added 1-(bromomethyl)-2,4-bis(trifluoromethyl)benzene (1.36 mL), and the mixture was stirred at room temperature for 3.5 hr. The reaction mixture was poured into water, and the mixture was extracted with ethyl acetate. The organic layer was washed with saturated brine, dried over anhydrous magnesium sulfate and concentrated under reduced pressure to give the title compound as a colorless powder (yield: 2.25 g, 91%).
¹H-NMR (CDCl₃, 300 MHz):δ3.90 (3H, s), 5.39 (2H, s), 6.54 (1H, d, J= 2.3 Hz), 6.59 (1H, dd, J = 8.7, 2.3 Hz), 7.83 (1H, d, J = 8.7 Hz), 7.86-7.93 (2H, m), 7.98 (1H, s), 10.31 (1H, s).

### Reference Example 12

### 4-{[2,4-bis(trifluoromethyl)benzyl]oxy}-3-ethoxybenzaldehyde

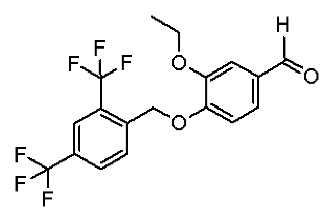

To a solution (3.1 mL) of 3-ethoxy-4-hydroxybenzaldehyde (513.5 mg) and potassium carbonate (512.5 mg) in DMF was added 1-(bromomethyl)-2,4-bis(trifluoromethyl)benzene (0.64 mL), and the mixture was stirred at room temperature for 3.5 hr. Water was added to the reaction mixture, and the precipitate was collected by filtration to give the title compound as a colorless powder (yield: 1.19 g, 98%).
¹H-NMR (CDCl₃, 300 MHz):δ1.50 (3H, t, J = 7.0 Hz), 4.20 (2H, q, J = 7.0 Hz), 5.46 (2H, s), 6.96 (1H, d, J = 8.1 Hz), 7.41 (1H, dd, J = 8.1, 1.9 Hz), 7.46 (1H, d, J = 1.9 Hz), 7.86 (1H, d, J = 8.3 Hz), 7.95 (1H, s), 8.02 (1H, d, J = 8.3 Hz), 9.86 (1H, s) .

### Reference Example 13

### 4-{[2,4-bis(trifluoromethyl)benzyl]oxy}-3-hydroxybenzaldehyde

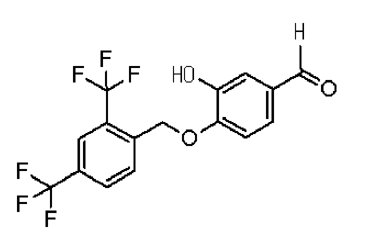

To a solution (20 mL) of 3,4-dihydroxybenzaldehyde (2.00 g) and potassium carbonate (2.40 g) in DMF was added 1-(bromomethyl)-2,4-bis(trifluoromethyl)benzene (2.99 mL), and the mixture was stirred at room temperature overnight. The reaction mixture was poured into aqueous ammonium chloride solution, and the mixture was extracted with ethyl acetate. The organic layer was washed with saturated brine, dried over anhydrous magnesium sulfate and concentrated under reduced pressure. The residue was purified by silica gel column chromatography (eluent: hexane-ethyl acetate=10:0→7:3) to give the title compound as a colorless powder (yield: 2.60 g, 49%). ¹H-NMR (CDCl₃, 300 MHz):δ5.48 (2H, s), 5.72 (1H, s), 6.95 (1H, d, J= 8.3 Hz), 7.41 (1H, dd, J = 8.3, 2.1 Hz), 7.50 (1H, d, J = 2.1 Hz), 7.79 (1H, d, J = 8.5 Hz), 7.88 (1H, d, J = 8.1 Hz), 8.01 (1H, s), 9.86 (1H, s).

### Reference Example 14

### 4-{[2,4-bis(trifluoromethyl)benzyl]oxy}-3-(2-methylpropoxy)benzaldehyde

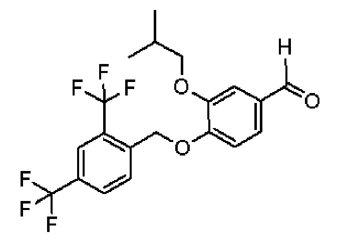

To a solution (6 mL) of 4-{[2,4-bis(trifluoromethyl)benzyl]oxy}-3-hydroxybenzaldehyde (1.09 g) and potassium carbonate (826.5 mg) in DMF was added 1-bromo-2-methylpropane (0.34 mL), and the mixture was stirred at 80°C overnight. The reaction mixture was cooled to room temperature, and poured into water, and the mixture was extracted with ethyl acetate. The organic layer was washed with saturated brine, dried over anhydrous magnesium sulfate and concentrated under reduced pressure. The residue was purified by silica gel column chromatography (eluent: hexane-ethyl acetate=10:0→7:3) to give the title compound as a colorless powder (yield: 688.5 mg, 55%).
¹H-NMR (CDCl₃, 300 MHz):δ1.08 (6H, d, J = 6.8 Hz), 2.12-2.27 (1H, m), 3.88 (2H, d, J = 6.6 Hz), 5.44 (2H, s), 7.00 (1H, d, J= 8.1 Hz), 7.42 (1H, dd, J = 8.1, 1.9 Hz), 7.45 (1H, d, J = 1.9 Hz), 7.86 (1H, d, J = 8.3 Hz), 7.95 (1H, s), 8.08 (1H, d, J= 8.3 Hz), 9.87 (1H, s).

### Reference Example 15

### 3-(benzyloxy)-4-{[2,4-bis(trifluoromethyl)benzyl]oxy}benzaldehyde

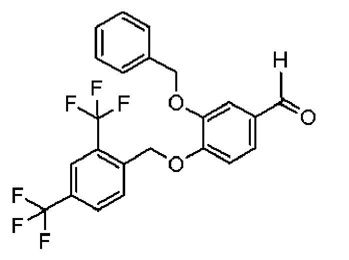

To a solution (5.71 mL) of 4-{[2,4-bis(trifluoromethyl)benzyl]oxy}-3-hydroxybenzaldehyde (1.04 g) and potassium carbonate (789.3 mg) in DMF was added benzyl bromide (0.40 mL), and the mixture was stirred at room temperature overnight. The reaction mixture was poured into water, and the mixture was extracted with ethyl acetate. The organic layer was washed with saturated brine, dried over anhydrous magnesium sulfate and concentrated under reduced pressure to give the title compound as a colorless powder (yield: 1.20 g, 92%).
¹H-NMR (CDCl₃, 300 MHz):δ5.22 (2H, s), 5.46 (2H, s), 7.00 (1H, d, J= 8.3 Hz), 7.33-7.50 (6H, m), 7.55 (1H, d, J = 1.9 Hz), 7.79 (1H, d, J= 8.5 Hz), 7.94 (1H, s), 7.99 (1H, d, J= 8.5 Hz), 9.85 (1H, s).

### Reference Example 16

### 4-{[2,4-bis(trifluoromethyl)benzyl]oxy}-3-propoxybenzaldehyde

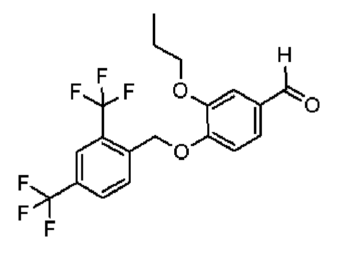

To a solution (7 mL) of 4-{[2,4-bis(trifluoromethyl)benzyl]oxy}-3-hydroxybenzaldehyde (504.3 mg) and potassium carbonate (381.5 mg) in DMF was added 1-bromopropane (0.19 mL), and the mixture was stirred at 80°C overnight. Water was added to the reaction mixture, and the mixture was cooled to room temperature. The precipitate was collected by filtration, and washed with diisopropylether to give the title compound as a pale-yellow powder (yield: 468.5 mg, 84%).
¹H-NMR (CDCl₃, 300 MHz):δ1.09 (3H, t, J = 7.5 Hz), 1.82-1.98 (2H, m), 4.09 (2H, t, J = 6.5 Hz), 5.46 (2H, s), 6.98 (1H, d, J= 8.1 Hz), 7.41 (1H, dd, J = 8.1, 1.5 Hz), 7.46 (1H, d, J = 1.5 Hz), 7.86 (1H, d, J = 8.3 Hz), 7.96 (1H, s), 8.04 (1H, d, J = 8.3 Hz), 9.86 (1H, s).

### Reference Example 17

### 4-{[2,4-bis(trifluoromethyl)benzyl]oxy}-3-butoxybenzaldehyde

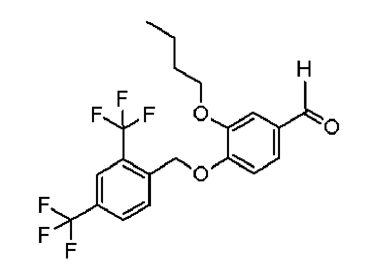

To a solution (7 mL) of 4-{[2,4-bis(trifluoromethyl)benzyl]oxy}-3-hydroxybenzaldehyde (501.3 mg) and potassium carbonate (381.5 mg) in DMF was added 1-bromobutane (0.22 mL), and the mixture was stirred at 80°C overnight. Water was added to the reaction mixture, and the mixture was cooled to room temperature. The precipitate was collected by filtration, and washed with diisopropylether to give the title compound as a pale-yellow powder (yield: 430.9 mg, 74%).
¹H-NMR (CDCl₃, 300 MHz):δ1.01 (3H, t, J = 7.4 Hz), 1.47-1.62 (2H, m), 1.78-1.94 (2H, m), 4.12 (2H, t, J= 6.5 Hz), 5.46 (2H, s), 6.97 (1H, d, J= 8.1 Hz), 7.41 (1H, dd, J = 8.1, 1.5 Hz), 7.46 (1H, d, J = 1.5 Hz), 7.86 (1H, d, J = 8.3 Hz), 7.96 (1H, s), 8.03 (1H, d, J = 8.3 Hz), 9.86 (1H, s).

### Reference Example 18

### 4-{[2,4-bis(trifluoromethyl)benzyl]oxy}-3-(1-methylethoxy)benzaldehyde

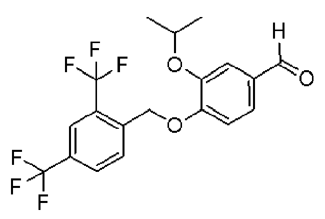

To a solution (7 mL) of 4-{[2,4-bis(trifluoromethyl)benzyl]oxy}-3-hydroxybenzaldehyde (493.3 mg) and potassium carbonate (373.2 mg) in DMF was added 2-bromopropane (0.19 mL), and the mixture was stirred at 80°C for 16 hr. The reaction mixture was poured into water, and the mixture was extracted with ethyl acetate. The organic layer was washed with saturated brine, dried over magnesium sulfate, and concentrated under reduced pressure. The residue was washed with diisopropyl ether to give the title compound as a brown powder (yield: 273.8 mg, 50%).
¹H-NMR (CDCl₃, 300 MHz):δ1.42 (6H, dd, J = 6.0, 0.8 Hz), 4.69 (1H, m), 5.45 (2H, s), 6.98 (1H, d, J = 8.3 Hz), 7.41 (1H, ddd, J = 8.2, 1.7, 0.8 Hz), 7.48 (1H, d, J = 1.7 Hz), 7.87 (1H, d, J = 8.3 Hz), 7.96 (1H, s), 8.03 (1H, d, J= 8.3 Hz), 9.86 (1H, d, J = 0.8 Hz).

### Reference Example 19

### 4-{[2,4-bis(trifluoromethyl)benzyl]oxy}-3-(cyclohexylmethoxy)benzaldehyde

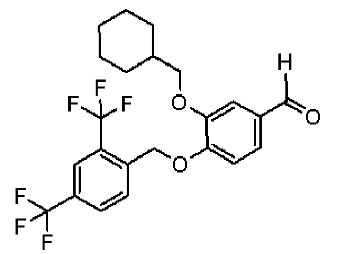

To a solution (7 mL) of 4-{[2,4-bis(trifluoromethyl)benzyl]oxy}-3-hydroxybenzaldehyde (503.3 mg) and potassium carbonate (381.5 mg) in DMF was added (bromomethyl)cyclohexane (0.29 mL), and the mixture was stirred at 80°C overnight. Water was added to the reaction mixture, and the mixture was cooled to room temperature. The precipitate was collected by filtration, and washed with diisopropylether to give the title compound as a brown powder (yield: 273.7 mg, 43%).
¹H-NMR (CDCl₃, 300 MHz):δ1.04-1.42 (5H, m), 1.68-1.98 (6H, m), 3.91 (2H, d, J = 5.9 Hz), 5.45 (2H, s), 6.99 (1H, d, J = 8.1 Hz), 7.41 (1H, dd, J = 8.1, 1.7 Hz), 7.45 (1H, d, J = 1.7 Hz), 7.85 (1H, d, J = 8.3 Hz), 7.96 (1H, s), 8.06 (1H, d, J = 8.3 Hz), 9.86 (1H, s).

### Reference Example 20

### 4-{[2,4-bis(trifluoromethyl)benzyl]oxy}-3-fluorobenzaldehyde

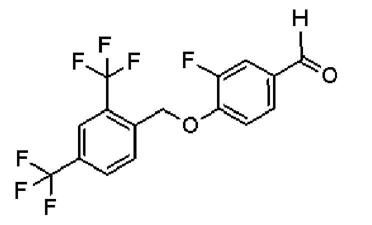

To a solution (35 mL) of 3-fluoro-4-hydroxybenzaldehyde (1.07 g) and potassium carbonate (1.27 g) in DMF was added 1-(bromomethyl)-2,4-bis(trifluoromethyl)benzene (1.56 mL), and the mixture was stirred at room temperature for 3 hr. The reaction mixture was poured into water, and the mixture was extracted with ethyl acetate. The organic layer was washed with saturated brine, dried over anhydrous magnesium sulfate and concentrated under reduced pressure to give the title compound as a colorless powder (yield: 2.78 g, 99%).
¹H-NMR (CDCl₃, 300 MHz):δ5.47 (2H, s), 7.02-7.17 (1H, m), 7.61-7.71 (2H, m), 7.86-7.92 (1H, m), 7.95-8.03 (2H, m), 9.89 (1H, d, J = 2.1 Hz).

### Reference Example 21

### 4-{[2,4-bis(trifluoromethyl)benzyl]oxy}benzaldehyde

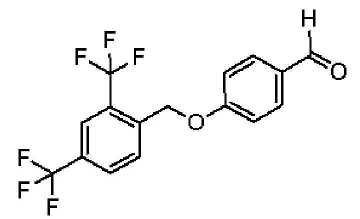

To a solution (7.4 mL) of 4-hydroxybenzaldehyde (902.0 mg) and potassium carbonate (1.23 g) in DMF was added 1-(bromomethyl)-2,4-bis(trifluoromethyl)benzene (1.52 mL), and the mixture was stirred at room temperature for 1.5 hr. The reaction mixture was poured into water, and the mixture was extracted with ethyl acetate. The organic layer was washed successively with aqueous potassium carbonate solution and saturated brine, dried over anhydrous magnesium sulfate and concentrated under reduced pressure. The residue was washed with a small amount of hexane to give the title compound as a colorless powder (yield: 2.08 g, 81%).
¹H-NMR (CDCl₃, 300 MHz):δ5.41 (2H, s), 7.08 (2H, d, J = 8.9 Hz), 7.88 (2H, d, J = 8.9 Hz), 7.86-7.91 (2H, m), 7.98 (1H, s), 9.92 (1H, s).

### Reference Example 22

### (5Z)-5-[(4-{[2,4-bis(trifluoromethyl)benzyl]oxy}-3-methylphenyl)methylidene]-1,3-thiazolidine-2,4-dione

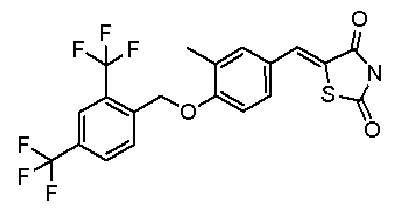

To a solution (60 mL) of 4-{[2,4-bis(trifluoromethyl)benzyl]oxy}-3-methylbenzaldehyde (2.30 g) and 1,3-thiazolidine-2,4-dione (818.2 mg) in ethanol was added piperidine (0.125 mL), and the mixture was stirred with heating under reflux overnight. The reaction mixture was cooled to room temperature, and the precipitate was collected by filtration, and washed with diethyl ether to give the title compound as a colorless powder (yield: 2.43 g, 83%).
¹H-NMR (DMSO-d₆, 300 MHz):δ2.26 (3H, s), 5.45 (2H, s), 7.18 (1H, d, J = 9.4 Hz), 7.43-7.50 (2H, m), 7.71 (1H, s), 8.06 (1H, d, J= 8.1 Hz), 8.13 (1H, s), 8.18 (1H, d, J= 8.1 Hz), 12.53 (1H, brs) .

### Reference Example 23

### (5Z)-5-[(4-{[2,4-bis(trifluoromethyl)benzyl]oxy}-2-methylphenyl)methylidene]-1,3-thiazolidine-2,4-dione

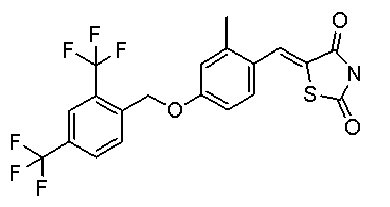

To a solution (57 mL) of 4-{[2,4-bis(trifluoromethyl)benzyl]oxy}-2-methylbenzaldehyde (2.05 g) and 1,3-thiazolidine-2,4-dione (729.1 mg) in ethanol was added piperidine (0.111 mL), and the mixture was stirred with heating under reflux overnight. The reaction mixture was cooled to room temperature, and the precipitate was collected by filtration to give the title compound as a colorless powder (yield: 1.71 g, 83%).
¹H-NMR (DMSO-d₆, 300 MHz):δ2.41 (3H, s), 5.40 (2H, s), 7.03 (1H, dd, J = 8.5, 2.5 Hz), 7.08 (1H, d, J = 2.5 Hz), 7.40 (1H, d, J = 8.5 Hz), 7.84 (1H, s), 8.03 (1H, d, J = 8.1 Hz), 8.11 (1H, s), 8.16 (1H, d, J = 8.1 Hz), 12.56 (1H, brs).

### Reference Example 24

### (5Z)-5-[(4-{[2,4-bis(trifluoromethyl)benzyl]oxy}-2-chlorophenyl)methylidene]-1,3-thiazolidine-2,4-dione

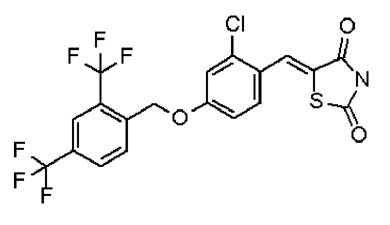

To a solution (41 mL) of 4-{[2,4-bis(trifluoromethyl)benzyl]oxyl-2-chlorobenzaldehyde (1.58 g) and 1,3-thiazolidine-2,4-dione (531.9 mg) in ethanol was added piperidine (0.81.6 mL), and the mixture was stirred with heating under reflux overnight. The reaction mixture was cooled to room temperature, and the precipitate was collected by filtration to give the title compound as a pale-yellow powder (yield: 0.88 g, 44%).
¹H-NMR (DMSO-d₆, 300 MHz):δ5.46 (2H, s), 7.22 (1H, dd, J = 8.8, 2.6 Hz), 7.42 (1H, d, J = 2.6 Hz), 7.55 (1H, d, J = 8.8 Hz), 7.90 (1H, s), 8.05 (1H, d, J = 8.1 Hz), 8.12 (1H, s), 8.17 (1H, d, J = 8.1 Hz), 12.70 (1H, brs).

### Reference Example 25

### (5Z)-5-[(4-{[2,4-bis(trifluoromethyl)benzyl]oxy}-3-chlorophenyl)methylidene]-1,3-thiazolidine-2,4-dione

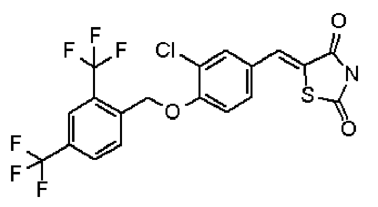

To a solution (60 mL) of 4-{[2,4-bis(trifluoromethyl)benzyl]oxyl-3-chlorobenzaldehyde (2.25 g) and 1,3-thiazolidine-2,4-dione (757.6 mg) in ethanol was added piperidine (0.116 mL), and the mixture was stirred with heating under reflux overnight. The reaction mixture was cooled to room temperature, and the precipitate was collected by filtration, and washed with diethyl ether to give the title compound as a pale-yellow powder (yield: 1.61 g, 57%).
¹H-NMR (DMSO-d₆, 300 MHz):δ5.53 (2H, s), 7.43 (1H, d, J = 8.5 Hz), 7.58 (1H, dd, J = 8.5, 2.1 Hz), 7.75 (1H, s), 7.78 (1H, d, J = 2.1 Hz), 8.08 (1H, d, J = 8.1 Hz), 8.14 (1H, s), 8.21 (1H, d, J = 8.1 Hz), 12.63 (1H, brs).

### Reference Example 26

### (5Z)-5-[(4-{[2,4-bis(trifluoromethyl)benzyl]oxy}-2-methoxyphenyl)methylidene]-1,3-thiazolidine-2,4-dione

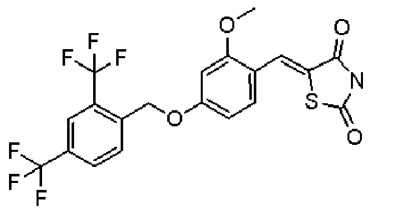

To a solution (48 mL) of 4-{[2,4-bis(trifluoromethyl)benzyl]oxy}-2-methoxybenzaldehyde (1.83 g) and 1,3-thiazolidine-2,4-dione (623.3 mg) in ethanol was added piperidine (0.95.6 mL), and the mixture was stirred with heating under reflux overnight. The reaction mixture was cooled to room temperature, and the precipitate was collected by filtration to give the title compound as a pale-yellow powder (yield: 2.14 g, 93%).
¹H-NMR (DMSO-d₆, 300 MHz):δ3.90 (3H, s), 5.44 (2H, s), 6.76-6.84 (2H, m), 7.38 (1H, d, J = 8.5 Hz), 7.93 (1H, s), 8.05 (1H, d, J= 8.3 Hz), 8.12 (1H, s), 8.17 (1H, d), 12.47 (1H, brs).

### Reference Example 27

### 4-thioxo-1,3-thiazolidin-2-one

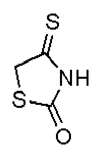

1,3-Thiazolidine-2,4-dione (17.8 g, 0.152 mol) and a Lawesson reagent (36.9 g) were suspended in toluene/tetrahydrofuran (2:1, 510 mL), and the suspension was stirred at 80°C overnight. The reaction mixture was concentrated under reduced pressure. The residue was washed successively with ethyl acetate and ethanol, and cooled to room temperature, and the precipitate was collected by filtration. The obtained solid was washed with ethyl acetate to give the title compound as a pale-yellow powder (yield: 13.8 g, 68%).
¹H-NMR (DMSO-d₆, 300 MHz):δ4.61 (2H, s), 13.52 (1H, brs).

### Reference Example 28

### 4-(methylamino)-1,3-thiazol-2(5H)-one

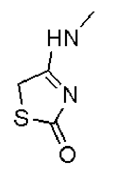

To a solution (520 mL) of 4-thioxo-1,3-thiazolidin-2-one (13.8 g) in methanol was added 40% methylamine-methanol solution (40.4 mL), and the mixture was stirred at room temperature overnight. The reaction mixture was concentrated, and the residue was recrystallized from ethyl acetate (80°C→0°C) to give the title compound as a pale-yellow powder (yield: 11.1 g, 82%).
¹H-NMR (DMSO-d₆, 300 MHz):δ2.88 (3H, s), 4.21 (2H, d, J = 0.8 Hz), 8.96 (1H, brs).

### Reference Example 29

### 5-imino-1-(phenylcarbonyl)imidazolidin-2-one

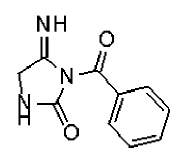

To a solution (40 mL) of aminoacetonitrile (1.24 g) in tetrahydrofuran was added dropwise under ice-cooling a solution (20 mL) of benzoyl isocyanate (3.51 g) in tetrahydrofuran, and the reaction solution was stirred at room temperature for 18 hr. The precipitate was collected by filtration, and washed with ethyl acetate to give the title compound as a white powder (yield: 3.31 g, 74%).
¹H-NMR (DMSO-d₆, 300 MHz):δ4.27 (2H, d, J = 5.9 Hz), 7.48-7.56 (2H, m), 7.60-7.68 (1H, m), 7.97 (2H, d, J = 7.2 Hz), 9.05 (1H, t, J = 5.8 Hz), 11.01 (1H, s).

### Reference Example 30

### 1-(cyanomethyl)-3-ethylurea

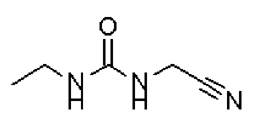

To a solution (600 mL) of aminoacetonitrile sulfate (50.2 g) in tetrahydrofuran were added triethylamine (104 mL) and a solution (50 mL) of ethyl isocyanate (21.3 g) in tetrahydrofuran at room temperature. The reaction solution was stirred at 60°C for 18 hr, saturated aqueous ammonium chloride was added at room temperature, and the mixture was extracted with ethyl acetate (400 mL, 300 mL x 4). The organic layer was washed with saturated brine, dried over anhydrous magnesium sulfate, filtered, and concentrated under reduced pressure. The residue was recrystallized from ethyl acetate/n-heptane, the obtained powder was dissolved in tetrahydrofuran, and the mixture was purified by basic silica gel column chromatography (eluent: ethyl acetate). The residue was washed with diisopropylether to give the title compound as a white powder (yield: 27.1 g, 73%).
¹H-NMR (DMSO-d₆, 300 MHz):δ0.99 (3H, t, J = 7.2 Hz), 2.97-3.07 (2H, m), 4.00 (2H, d, J = 6.0 Hz), 6.29 (1H, t, J = 5.0 Hz), 6.40 (1H, t, J = 6.0 Hz).

### Reference Example 31

### 3-methoxy-4-[(4-methoxybenzyl)oxy]benzaldehyde

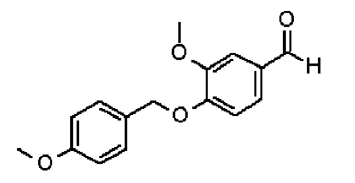

To a solution (200 mL) of 4-hydroxy-3-methoxybenzaldehyde (33.1 g, 213 mmol) in DMF was added potassium carbonate (26.3 g), and the mixture was stirred at room temperature for 1 hr. To the reaction solution was added 1-(chloromethyl)-4-methoxybenzene (30.4 mL), and the mixture was stirred at room temperature for 3 days. Water (400 mL) was added, and the mixture was extracted with ethyl acetate/tetrahydrofuran. The organic layer was washed with water and saturated brine, dried over anhydrous magnesium sulfate, filtered, and concentrated under reduced pressure. The residue was recrystallized from ethyl acetate/n-heptane to give the title compound as colorless crystals (yield: 55.0 g, 95%).
¹H-NMR (DMSO-d₆, 300 MHz):δ3.76 (3H, s), 3.82 (3H, s), 5.13 (2H s), 6.96 (2H, d, J = 8.5 Hz), 7.28 (1H, d, J = 8.3 Hz), 7.36-7.43 (3H, m), 7.54 (1H, dd, J = 8.3, 1.5 Hz), 9.84 (1H, s).

### Reference Example 32

### (4Z)-1-ethyl-4-[(4-hydroxy-3-methoxyphenyl)methylidene]-5-iminoimidazolidin-2-one

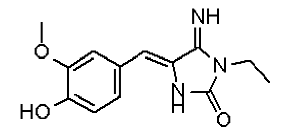

A mixture of (4Z)-1-ethyl-5-imino-4-({3-methoxy-4-[(4-methoxybenzyl)oxy]phenyl}methylidene)imidazolidin-2-one (10.0 g) and trifluoroacetic acid (50 mL) was stirred at room temperature for 1 hr. The reaction solution was concentrated under reduced pressure, ethyl acetate was added, and the mixture was washed with 1 mol/L aqueous sodium hydroxide solution. To the aqueous layer was added 3 mol/L aqueous hydrochloric acid solution to pH=6, and the mixture was extracted with ethyl acetate (150 mL x 7). The organic layer was dried over anhydrous magnesium sulfate, filtered, and concentrated under reduced pressure. The residue was washed with ethyl acetate to give the title compound as a yellow powder (yield 5.92 g, 87%).
¹H-NMR (DMSO-d₆, 300 MHz):δ1.14 (3H, t, J = 7.1 Hz), 3.64 (2H, q, J = 7.1 Hz), 3.85 (3H, s), 6.79-6.87 (2H, m), 6.97-7.06 (2H, m), 9.58 (1H, brs).

### Reference Example 33

### 1-benzyl-3-(cyanomethyl)urea

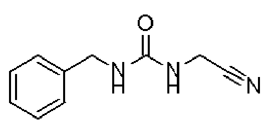

To a suspension (100 mL) of aminoacetonitrile sulfate (6.30 g) in anhydrous tetrahydrofuran were added benzyl isocyanate (3.99 g) and N,N-diisopropylethylamine (3.88 g). The mixture was stirred at room temperature for 5 hr, and concentrated under reduced pressure. The residue was washed with ethyl acetate to give the title compound as a white solid (yield: 4.0 g, 70%).
¹H-NMR (DMSO-d₆, 300 MHz):δ4.05 (2H, d, J = 5.9 Hz), 4.23 (2H, d, J = 6.0 Hz), 6.55 (1H, t, J = 5.9 Hz), 6.86 (1H, t, J = 5.9 Hz), 7.16-7.39 (5H, m)

### Reference Example 34 1-benzyl-5-iminoimidazolidin-2-one

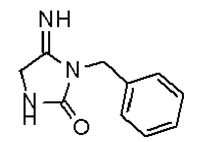

To a solution (20 mL) of 1-benzyl-3-(cyanomethyl)urea (945 mg) in anhydrous tetrahydrofuran was added sodium hydride (60% in oil, 300 mg) at 0°C. After stirring at room temperature for 30 min, the precipitate was collected by filtration, and washed with ethyl acetate to give the title compound as white crystals (370 mg, 39%).
¹H-NMR (DMSO-d₆, 300 MHz):δ3.95 (2H, s), 4.57 (2H, s), 7.15-7.40 (5H, m), 7.55 (1H, s), 7.81 (1H, brs).

### Reference Example 35

### 4-{[2,4-bis(trifluoromethyl)benzyl]oxy}-3-(trifluoromethyl)benzaldehyde

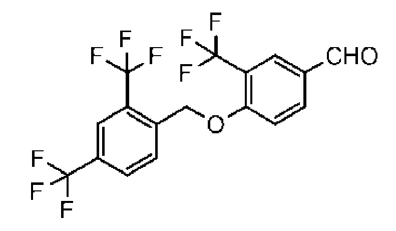

To a solution (2.7 mL) of 4-hydroxy-3-(trifluoromethyl)benzaldehyde (520.9 mg) and potassium carbonate (454.4 mg) in N,N-dimethylformamide was added 1-(bromomethyl)-2,4-bis(trifluoromethyl)benzene (0.565 mL), and the mixture was stirred at room temperature for 3.5 hr. Water was added to the reaction mixture, and the precipitate was collected by filtration, and washed with water and diisopropylether to give the title compound as a colorless powder (yield: 1.12 g, 98%).
¹H-NMR (CDCl₃, 300 MHz):δ5.52 (2H, s), 7.17 (1H, d, J = 8.5 Hz), 7.91 (1H, d, J = 8.7 Hz), 7.97-8.02 (2H, m), 8.07 (1H, dd, J = 8.5, 2.1 Hz), 8.20 (1H, d, J = 1.7 Hz), 9.96 (1H, s).

### Reference Example 36

### 4-(4-formyl-2-methoxyphenoxy)naphthalene-l-carbonitrile

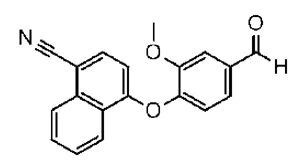

To a solution (700 mL) of 4-hydroxy-3-methoxybenzaldehyde (63.5 g) in dimethyl sulfoxide was added lithium carbonate (45.8 g), and the mixture was stirred at room temperature for 0.5 hr. To the reaction mixture was added 4-fluoronaphthalene-1-carbonitrile (70.0 g), and the mixture was stirred at 100°C for 2 days. Water was added to the reaction mixture, and the precipitate was collected by filtration. The obtained solid was recrystallized from tetrahydrofuran to give the title compound as a white solid (yield: 106 g, 85%).
¹H-NMR (DMSO-d₆, 300 MHz):δ3.84 (3H, s), 6.75 (1H, d, J = 8.1 Hz), 7.46 (1H, d, J = 8.1 Hz), 7.67 (1H, dd, J = 8.1, 1.9 Hz), 7.75 (1H, d, J = 1.9 Hz), 7.77-7.84 (1H, m), 7.87-7.94 (1H, m), 8.06 (1H, d, J = 8.3 Hz), 8.15 (1H, d, J = 8.3 Hz), 8.42 (1H, d, J = 8.3 Hz), 10.03 (1H, s).

### Reference Example 37

### 4-amino-1,3-thiazol-2(5H)-one

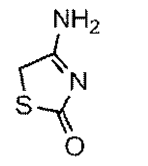

To a solution (75 mL) of 4-thioxo-1,3-thiazolidin-2-one (2.00 g) in methanol was added 8 M ammonia-methanol solution (9.38 mL), and the mixture was stirred at room temperature overnight. The reaction mixture was concentrated, and the residue was recrystallized from methanol/diethylether to give the title compound as a gray white powder (yield: 660.2 mg, 38%).
¹H-NMR (DMSO-d₆, 300 MHz)4.22 (2H, s), 8.51 (2H, brs)

### Reference Example 38

### 4-[2-chloro-4-(trifluoromethyl)phenoxy]-3-methoxybenzaldehyde

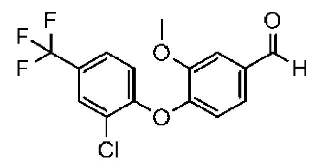

To a solution (900 mL) of 4-hydroxy-3-methoxybenzaldehyde (55.2 g) in dimethyl sulfoxide was added potassium carbonate (98.7 g), and the mixture was stirred at room temperature for 0.5 hr. To the reaction mixture was added a solution (100 mL) of 2-chloro-1-fluoro-4-(trifluoromethyl)benzene (80.0 g) in dimethyl sulfoxide, and the mixture was stirred at 100°C for 24 hr. Water was added to the reaction mixture, and the mixture was extracted with ethyl acetate. The organic layer was washed with water and saturated brine, dried over anhydrous magnesium sulfate, and filtered, and the filtrate was concentrated under reduced pressure. The residue was purified by silica gel column chromatography (eluent: hexane-ethyl acetate=100:0→70:30) to give the title compound as a pale-yellow oil (yield: 74.5 g, 63%).
¹H-NMR (DMSO-d₆, 300 MHz):δ3.86 (3H, s), 7.00 (1H, d, J = 8.7 Hz), 7.31 (1H, d, J = 8.1 Hz), 7.63 (1H, dd, J = 8.1, 1.9 Hz), 7.65-7.72 (2H, m), 8.04 (1H, d, J = 1.9 Hz), 10.00 (1H, s).

### Reference Example 39

### 4-{[2,4-bis(trifluoromethyl)benzyl]oxy}-3-nitrobenzaldehyde

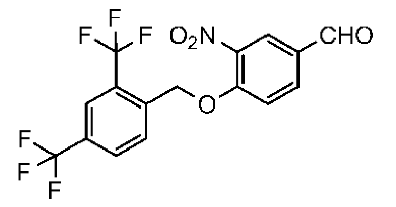

To a solution (20 mL) of 4-hydroxy-3-nitrobenzaldehyde (5 g) and potassium carbonate (5 g) in N,N-dimethylformamide was added 1-(bromomethyl)-2,4-bis(trifluoromethyl)benzene (6.07 mL), and the mixture was stirred at room temperature for 2 hr. The reaction mixture was poured into ice water, and the precipitate was collected by filtration. The obtained solid was dissolved in ethyl acetate, and the mixture was dried over anhydrous sodium sulfate, and concentrated under reduced pressure to give the title compound as a pale-brown powder (yield: 7.5 g, 64%).
¹H-NMR (DMSO-d₆,400 MHz):δ5.69 (2H, s), 7.73 (1H, d, J = 8.8 Hz), 8.10-8.15 (2H, m), 8.25 (2H, dd, J = 8.8, 2.0 Hz), 8.51 (1H, d, J = 2.0 Hz), 10.00 (1H, s).

### Reference Example 40

### methyl 2-{[2,4-bis(trifluoromethyl)benzyl]oxy}-5-formylbenzoate

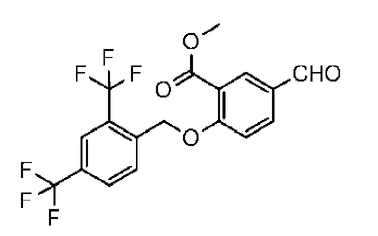

To a solution (20 mL) of methyl 5-formyl-2-hydroxybenzoate (15 g) and potassium carbonate (13.8 g) in N,N-dimethylformamide was added 1-(bromomethyl)-2,4-bis(trifluoromethyl)benzene (16 mL), and the mixture was stirred at room temperature for 2 hr. The reaction mixture was poured into ice water, and the precipitate was collected by filtration. The obtained solid was dissolved in ethyl acetate, and the mixture was dried over anhydrous sodium sulfate, and concentrated under reduced pressure to give the title compound as a pale-brown powder (yield: 32.4 g, 96%).
¹H-NMR (400 MHz, CDCl₃): δ 3.97 (3H, s), 5.50 (2H, s), 7.15 (1H, d, J = 8.8 Hz), 7.93-7.96 (2H, m), 8.05 (1H, dd, J = 8.8, 2.0 Hz), 8.31 (1H, d, J = 8.0 Hz), 8.44 (1H, d, J = 2.0 Hz), 9.95 (1H, s).

### Reference Example 41

### 3-methoxy-4-{[4-(trifluoromethyl)benzyl]oxy}benzaldehyde

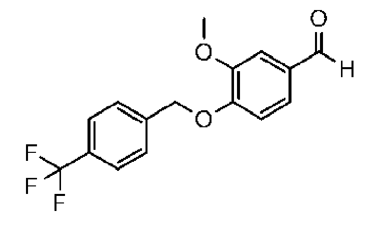

To a solution (15 mL) of 4-hydroxy-3-methoxybenzaldehyde (1.00 g) in dimethyl sulfoxide were added potassium carbonate (1.35 g) and 1-(bromomethyl)-4-(trifluoromethyl)benzene (1.89 g), and the mixture was stirred at room temperature for 16 hr. Water was added to the reaction mixture, and the mixture was extracted with ethyl acetate. The organic layer was washed with water and saturated brine, dried over anhydrous magnesium sulfate, and filtered, and the filtrate was concentrated under reduced pressure. The residue was purified by silica gel column chromatography (eluent: hexane-ethyl acetate=100:0→80:20) to give the title compound as a pale-yellow oil (yield: 2.03 g).
¹H-NMR (DMSO-d₆, 300 MHz):δ3.87 (3H, s), 5.35 (2H, s), 7.26 (1H, d, J = 8.3 Hz), 7.44 (1H, d, J = 1.9 Hz), 7.55 (1H, dd, J = 8.3, 1.9 Hz), 7.69 (2H, d, J = 8.3 Hz), 7.79 (2H, d, J = 8.3 Hz), 9.85 (1H, s).

### Reference Example 42

### 3-chloro-4-(4-formyl-2-methoxyphenoxy)benzonitrile

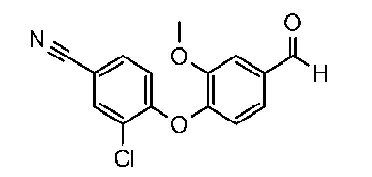

To a solution (15 mL) of 4-hydroxy-3-methoxybenzaldehyde (1.00 g) in dimethyl sulfoxide were added lithium carbonate (721 mg) and 3-chloro-4-fluorobenzonitrile (1.21 g), and the mixture was stirred at 100°C for 16 hr. Water was added to the reaction mixture, and the mixture was extracted with ethyl acetate. The organic layer was washed with water and saturated brine, dried over anhydrous magnesium sulfate, and filtered, and the filtrate was concentrated under reduced pressure. The residue was purified by silica gel column chromatography (eluent: hexane-ethyl acetate=100:0→70:30) to give the title compound as a colorless oil (yield: 1.48 g, 80%).
¹H-NMR (DMSO-d₆, 300 MHz):δ3.85 (3H, s), 6.92 (1H, d, J = 8.7 Hz), 7.35 (1H, d, J = 8.1 Hz), 7.63 (1H, dd, J = 8.1, 1.7 Hz), 7.70 (1H, d, J = 1.7 Hz), 7.76 (1H, dd, J = 8.7, 1.9 Hz), 8.22 (1H, d, J = 1.9 Hz), 10.00 (1H, s).

### Reference Example 43

### 2-chloro-4-(4-formyl-2-methoxyphenoxy)benzonitrile

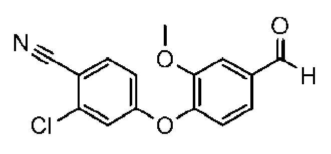

To a solution (15 mL) of 4-hydroxy-3-methoxybenzaldehyde (1.00 g) in dimethyl sulfoxide were added lithium carbonate (721 mg) and 2-chloro-4-fluorobenzonitrile (1.21 g), and the mixture was stirred at 100°C for 16 hr. Water was added to the reaction mixture, and the mixture was extracted with ethyl acetate. The organic layer was washed with water and saturated brine, dried over anhydrous magnesium sulfate, and filtered, and the filtrate was concentrated under reduced pressure. The residue was recrystallized from ethyl acetate/n-heptane to give the title compound as a colorless solid (yield: 1.62 g, 88%).
¹H-NMR (DMSO-d₆, 300 MHz):δ3.84 (3H, s), 7.01 (1H, dd, J = 8.7, 2.3 Hz), 7.32 (1H, d, J = 2.3 Hz), 7.44 (1H, d, J = 8.1 Hz), 7.65 (1H, dd, J = 8.1, 1.7 Hz), 7.70 (1H, d, J = 1.7 Hz), 7.94 (1H, d, J = 8.7 Hz), 10.01 (1H, s).

### Reference Example 44

### 4-(4-formyl-2-methoxyphenoxy)-2-(trifluoromethyl)benzonitrile

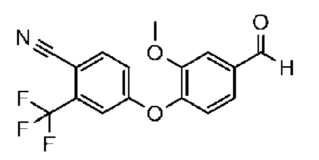

To a solution (15 mL) of 4-hydroxy-3-methoxybenzaldehyde (1.00 g) in dimethyl sulfoxide were added lithium carbonate (721 mg) and 4-fluoro-2-(trifluoromethyl)benzonitrile (1.49 g), and the mixture was stirred at 100°C for 2 days. Water was added to the reaction mixture, and the mixture was extracted with ethyl acetate. The organic layer was washed with water and saturated brine, dried over anhydrous magnesium sulfate, and filtered, and the filtrate was concentrated under reduced pressure. The residue was recrystallized from ethyl acetate/n-heptane to give the title compound as a colorless solid (yield: 1.91 g, 92%).
¹H-NMR (DMSO-d₆, 300 MHz):δ3.84 (3H, s), 7.26 (1H, dd, J = 8.7, 2.5 Hz), 7.48 (1H, d, J = 7.9 Hz), 7.57 (1H, d, J = 2.5 Hz), 7.67 (1H, dd, J = 7.9, 1.7 Hz), 7.72 (1H, d, J = 1.7 Hz), 8.12 (1H, d, J = 8.7 Hz), 10.02 (1H, s).

### Reference Example 45

### 4-(4-formyl-2-methoxyphenoxy)benzonitrile

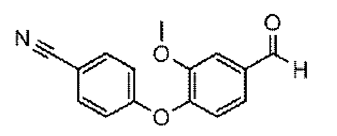

To a solution (15 mL) of 4-hydroxy-3-methoxybenzaldehyde (1.00 g) in dimethyl sulfoxide were added lithium carbonate (721 mg) and 4-fluorobenzonitrile (955 mg), and the mixture was stirred at 100°C for 3 days. Water was added to the reaction mixture, and the mixture was extracted with ethyl acetate. The organic layer was washed with water and saturated brine, dried over anhydrous magnesium sulfate, and filtered, and the filtrate was concentrated under reduced pressure. The residue was purified by silica gel column chromatography (eluent: hexane-ethyl acetate=100:0→70:30) to give the title compound as a pale-yellow oil (yield: 150 mg, 9%).
¹H-NMR (DMSO-d₆, 300 MHz):δ3.83 (3H, s), 7.03-7.10 (2H, m), 7.37 7 (1H, d, J = 7.9 Hz), 7.64 (1H, dd, J = 7.9, 1.5 Hz), 7.69 (1H, d, J = 1.5 Hz), 7.79-7.87 (2H, m), 10.00 (1H, s).

### Reference Example 46

### 3-methoxy-4-{[5-(trifluoromethyl)pyridin-2-yl]oxy}benzaldehyde

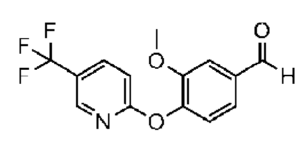

To a solution (15 mL) of 4-hydroxy-3-methoxybenzaldehyde (1.00 g) in dimethyl sulfoxide were added lithium carbonate (721 mg) and 2-chloro-5-(trifluoromethyl)pyridine (1.45 g), and the mixture was stirred at 100°C for 2 days. Water was added to the reaction mixture, and the mixture was extracted with ethyl acetate. The organic layer was washed with water and saturated brine, dried over anhydrous magnesium sulfate, and filtered, and the filtrate was concentrated under reduced pressure. The residue was purified by silica gel column chromatography (eluent: hexane-ethyl acetate=100:0→70:30) to give the title compound as a colorless oil (yield: 524 mg, 27%).
¹H-NMR (DMSO-d₆, 300 MHz):δ3.79 (3H, s), 7.32 (1H, d, J = 8.7 Hz), 7.46 (1H, d, J = 7.7 Hz), 7.60-7.68 (2H, m), 8.25 (1H, dd, J = 8.7, 2.6 Hz), 8.53 (1H, d, J = 0.8 Hz), 10.00 (1H, s).

### Reference Example 47

### 2-(4-formyl-2-methoxyphenoxy)-5-(trifluoromethyl)benzonitrile

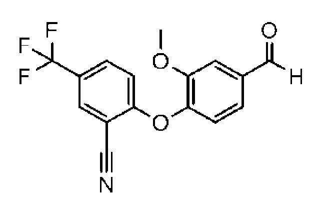

To a solution (15 mL) of 4-hydroxy-3-methoxybenzaldehyde (805 mg) in dimethyl sulfoxide were added lithium carbonate (580 mg) and 2-fluoro-5-(trifluoromethyl)benzonitrile (1.00 g), and the mixture was stirred at 100°C for 1 day. Water was added to the reaction mixture, and the mixture was extracted with ethyl acetate. The organic layer was washed with water and saturated brine, dried over anhydrous magnesium sulfate, and filtered, and the filtrate was concentrated under reduced pressure. The residue was purified by silica gel column chromatography (eluent: hexane-ethyl acetate=100:0→70:30) to give the title compound as a colorless oil (yield: 1.70 g). ¹H-NMR (DMSO-d₆, 300 MHz):δ3.86 (3H, s), 6.95 (1H, d, J = 8.9 Hz), 7.56 (1H, d, J = 8.1 Hz), 7.69 (1H, dd, J = 8.1, 1.5 Hz), 7.75 (1H, d, J = 1.5 Hz), 7.96 (1H, dd, J = 8.9, 2.1 Hz), 8.44 (1H, d, J = 2.1 Hz), 10.04 (1H, s).

### Reference Example 48

### 4-{[3-chloro-5-(trifluoromethyl)pyridin-2-yl]oxy}-3-methoxybenzaldehyde

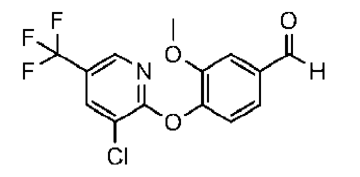

To a solution (100 mL) of 4-hydroxy-3-methoxybenzaldehyde (5.86 g) in dimethyl sulfoxide were added lithium carbonate (4.22 g) and 2,3-dichloro-5-(trifluoromethyl)pyridine (8.15 g), and the mixture was stirred at 100°C for 1 day. Water was added to the reaction mixture, and the mixture was extracted with ethyl acetate. The organic layer was washed with water and saturated brine, dried over anhydrous magnesium sulfate, and filtered, and the filtrate was concentrated under reduced pressure. The residue was purified by silica gel column chromatography (eluent: hexane-ethyl acetate=100:0→70:30) to give the title compound as a colorless oil (yield: 11.6 g, 93% .
¹H-NMR (DMSO-d₆, 300 MHz):δ3.79 (3H, s), 7.49-7.54 (1H, m), 7.62-7.68 (2H, m), 8.47-8.50 (1H, m), 8.60 (1H, d, J = 2.3 Hz), 10.01 (1H, s).

### Reference Example 49

### 3-methoxy-4-[2-nitro-4-(trifluoromethyl)phenoxy]benzaldehyde

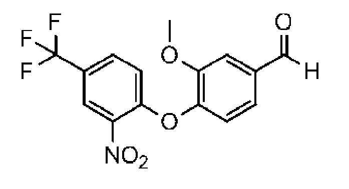

To a solution (15 mL) of 4-hydroxy-3-methoxybenzaldehyde (1.00 g) in dimethyl sulfoxide were added lithium carbonate (721 mg) and a solution (5 mL) of 1-fluoro-2-nitro-4-(trifluoromethyl)benzene (1.74 g) in dimethyl sulfoxide, and the mixture was stirred at 100°C for 1 day. Water was added to the reaction mixture, and the mixture was extracted with ethyl acetate. The organic layer was washed with water and saturated brine, dried over anhydrous magnesium sulfate, and filtered, and the filtrate was concentrated under reduced pressure. The residue was purified by silica gel column chromatography (eluent: hexane-ethyl acetate=100:0→70:30) to give the title compound as a yellow oil (yield: 1.97 g, 90%).
¹H-NMR (DMSO-d₆, 300 MHz):δ3.86 (3H, s), 7.14 (1H, d, J = 8.9 Hz), 7.49 (1H, d, J = 8.1 Hz), 7.67 (1H, dd, J = 8.1, 1.7 Hz), 7.73 (1H, d, J = 1.7 Hz), 7.99 (1H, dd, J = 8.9, 2.1 Hz), 8.49 (1H, d, J = 2.1 Hz), 10.03 (1H, s).

### Reference Example 50

### 4-{[2,4-bis(trifluoromethyl)benzyl]oxy}-2-chloro-5-methoxybenzaldehyde

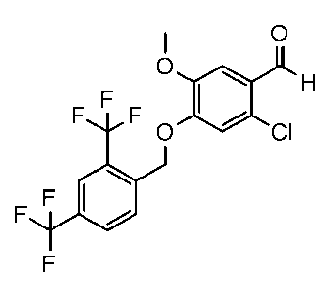

To a solution (3 mL) of 2-chloro-4-hydroxy-5-methoxybenzaldehyde (300 mg) in N,N-dimethylformamide were added 1-(bromomethyl)-2,4-bis(trifluoromethyl)benzene (544 mg) and potassium carbonate (267 mg), and the mixture was stirred at 80°C for 8 hr. Water was added to the reaction mixture, and the precipitate was collected by filtration, and washed with water to give the title compound as a white solid (yield: 650 mg, 98%).
¹H-NMR (DMSO-d₆, 300 MHz):δ3.33 (3H, s), 5.48 (2H, s), 7.39 (2H, s), 8.04 (1H, d, J = 8.1 Hz), 8.13 (1H, s), 8.20 (1H, d, J = 8.1 Hz), 10.22 (1H, s).

### Reference Example 51

### 3-{[2,4-bis(trifluoromethyl)benzyl]oxy}-4-methoxybenzaldehyde

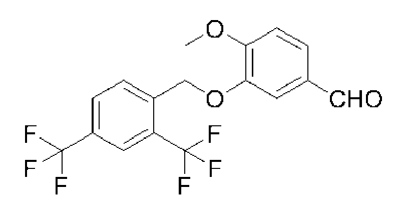

1-(Bromomethyl)-2,4-bis(trifluoromethyl)benzene (3.1 g) and 3-hydroxy-4-methoxybenzaldehyde (1.5 g) were dissolved in N,N-dimethylformamide (50 mL), potassium carbonate (2.76 g) was added, and the mixture was stirred at 80°C for 6 hr. To the reaction mixture were added water and ethyl acetate, and the mixture was extracted with ethyl acetate. The organic layer was washed with saturated brine, dried over anhydrous magnesium sulfate, and filtered, and the filtrate was concentrated under reduced pressure. The residue was purified by silica gel column chromatography to give the title compound as a white solid (yield: 3.8 g, 100%).
¹H-NMR (DMSO-d₆, 300 MHz):δ3.91 (3H, s), 5.41 (2H, s), 7.26 (1H, d, J = 8.3 Hz), 7.49 (1H, d, J = 1.9 Hz), 7.64 (1H, dd, J = 8.3, 1.7 Hz), 8.04 (1H, d, J = 8.1 Hz), 8.11 (1H, s), 8.18 (1H, d, J = 8.1 Hz), 9.84 (1H, s).

### Reference Example 52

### 3-hydroxy-2-methoxybenzaldehyde

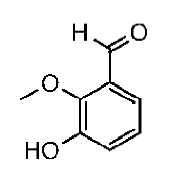

To a solution (80 mL) of 2,3-dihydroxybenzaldehyde (5.00 g) in N,N-dimethylformamide were added potassium carbonate (4.93 g) and a solution (20 mL) of iodomethane (6.89 g) in N,N-dimethylformamide, and the mixture was stirred at room temperature for 20 hr. Water was added to the reaction mixture, and the mixture was extracted with ethyl acetate. The organic layer was washed with saturated brine, dried over anhydrous magnesium sulfate, and filtered, and the filtrate was concentrated under reduced pressure. The residue was purified by silica gel column chromatography (eluent: hexane-ethyl acetate=100:0→60:40) to give the title compound as a white solid (yield: 2.16 g, 40%).
¹H-NMR (CDCl₃, 300 MHz):δ3.98 (3H, s), 5.83 (1H, s), 7.15 (1H, dd, J = 8 . 1, 7.7 Hz), 7 . 24 (1H, dd, J = 8.1, 1.8 Hz), 7.38 (1H, dd, J = 7.6, 1.8 Hz), 10.27 (1H, s).

### Reference Example 53

### 3-{[2,4-bis(trifluoromethyl)benzyl]oxy}-2-methoxybenzaldehyde

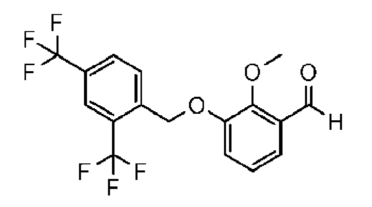

To a solution (16 mL) of 3-hydroxy-2-methoxybenzaldehyde (1.00 g) in N,N-dimethylformamide was added potassium carbonate (1.37 g), and the mixture was stirred at room temperature for 0.5 hr. To the reaction mixture was added a solution (2 mL) of 1-(bromomethyl)-2,4-bis(trifluoromethyl)benzene (2.29 g) in N,N-dimethylformamide, and the mixture was stirred at room temperature for 3 days. Water was added to the reaction mixture, and the mixture was extracted with ethyl acetate. The organic layer was washed with water and saturated brine, dried over anhydrous magnesium sulfate, and filtered, and the filtrate was concentrated under reduced pressure. The residue was purified by silica gel column chromatography (eluent: hexane-ethyl acetate=100:0→90:10) to give the title compound as a pale-yellow oil (yield: 2.27 g, 91%).
¹H-NMR (DMSO-d₆, 300 MHz):δ3.92 (3H, s), 5.44 (2H, s), 7.20-7.29 (1H, m), 7.36 (1H, dd, J = 7.9, 1.6 Hz), 7.49 (1H, dd, J = 8.0, 1.6 Hz), 8.10 (1H, d, J = 8.3 Hz), 8.14 (1H, s), 8.20 (1H, d, J = 8.3 Hz), 10.32 (1H, s).

### Reference Example 54

### 4-(3-formyl-2-methoxyphenoxy)-3-(trifluoromethyl)benzonitrile

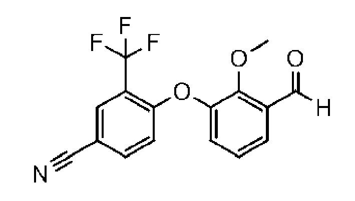

To a solution (20 mL) of 3-hydroxy-2-methoxybenzaldehyde (1.00 g) in dimethyl sulfoxide were added lithium carbonate (743 mg) and 4-fluoro-3-(trifluoromethyl)benzonitrile (1.54 g), and the mixture was stirred at 100°C for 4 hr. Water was added to the reaction mixture, and the mixture was extracted with ethyl acetate. The organic layer was washed with water and saturated brine, dried over anhydrous magnesium sulfate, and filtered, and the filtrate was concentrated under reduced pressure. The residue was washed with diisopropylether to give the title compound as a pale-yellow solid (yield: 1.50 g, 71%).
¹H-NMR (DMSO-d₆, 300 MHz):δ3.86 (3H, s), 7.01 (1H, d, J = 8.7 Hz), 7.40 (1H, dd, J = 8.1, 7.1 Hz), 7.62 (1H, dd, J = 8.1, 1.7 Hz), 7.74 (1H, dd, J = 7. 7, 1.7 Hz), 8.07 (1H, dd, J = 8.7, 2.1 Hz), 8.40 (1H, d, J = 2.1 Hz), 10.31 (1H, s).

### Reference Example 55

### 2-bromo-4-hydroxy-5-methoxybenzaldehyde

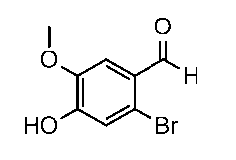

To a solution (120 mL) of potassium bromide (10.3 g) in water were added 4-formyl-2-methoxyphenyl acetate (5.00 g) and bromine (1.42 mL), and the mixture was stirred at room temperature for 8 hr. The precipitate was collected by filtration, and washed with water. To the obtained solid was added 6 N hydrochloric acid (120 mL), and the mixture was stirred at 90°C for 16 hr. The precipitate was collected by filtration, and washed with saturated aqueous sodium hydrogen carbonate and water. The obtained solid was dissolved in ethyl acetate, and the mixture was filtered through silica gel to give the title compound as a pale-yellow solid (yield: 4.88 g). The obtained solid was recrystallized from ethyl acetate/n-heptane to give the title compound as a pale-yellow solid (yield: 3.98 g, 68%).
¹H-NMR (DMSO-d₆, 300 MHz):δ3.83 (3H, s), 7.11 (1H, s), 7.34 (1H, s), 10.01 (1H, s), 10.86 (1H, brs).

### Reference Example 56

### 4-{[2,4-bis(trifluoromethyl)benzyl]oxy}-2-bromo-5-methoxybenzaldehyde

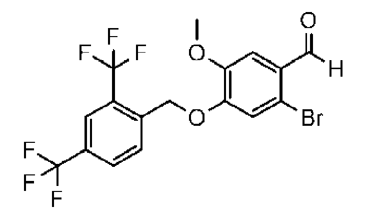

To a solution (60 mL) of 2-bromo-4-hydroxy-5-methoxybenzaldehyde (4.85 g) in N,N-dimethylformamide were added potassium carbonate (5.83 g) and a solution (20 mL) of 1-(bromomethyl)-2,4-bis(trifluoromethyl)benzene (7.31 g) in N,N-dimethylformamide, and the mixture was stirred at 60°C for 3 hr. Water was added to the reaction mixture, and the mixture was extracted with ethyl acetate. The organic layer was washed with water and saturated brine, dried over anhydrous magnesium sulfate, and filtered, and the filtrate was concentrated under reduced pressure. The residue was recrystallized from ethyl acetate/n-heptane to give the title compound as a colorless solid (yield: 6.96 g, 73%).
¹H-NMR (DMSO-d₆,300 MHz):δ3.84 (3H, s), 5.48 (2H, s), 7.40 (1H, s), 7.52 (1H, s), 8.04 (1H, d, J = 8.1 Hz), 8.12 (1H, s), 8.20 (1H, d, J = 8.1 Hz), 10.09 (1H, s).

### Reference Example 57

### (5Z)-4-amino-5-[(4-hydroxy-3-methoxyphenyl)methylidene]-1,5-dihydro-2H-imidazol-2-one

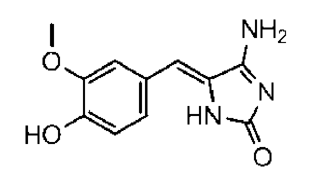

A mixed solution of (5Z)-4-amino-5-({3-methoxy-4-[(4-methoxybenzyl)oxy]phenyl}methylidene)-1,5-dihydro-2H-imidazol-2-one (150 mg) and trifluoroacetic acid (2 mL) was stirred at room temperature for 1 hr. To the reaction mixture was added 1 N aqueous sodium hydroxide solution, and the mixture was extracted with ethyl acetate. The organic layer was dried over anhydrous magnesium sulfate, and filtered, and the filtrate was concentrated under reduced pressure. The residue was purified by silica gel column chromatography (eluent: ethyl acetate-methanol=100:0→90:10) to give the title compound as a yellow solid (yield: 65 mg, 76%).
¹H-NMR (DMSO-d₆, 300 MHz):δ3.84 (3H, s), 6.44 (1H, s), 6.78 (1H, d, J = 8.1 Hz), 6.92 (1H, dd, J = 8.1, 1.7 Hz), 6.97 (1H, d, J = 1.7 Hz), 7.94 (2H, brs), 9.33 (1H, brs), 9.68 (1H, s).

### Reference Example 58

### 4-imino-1-methyl-3-(phenylcarbonyl)imidazolidin-2-one

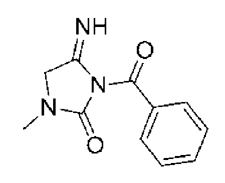

(Methylamino)acetonitrile (3.50 g) was dissolved in anhydrous tetrahydrofuran (200 mL), and benzoyl isocyanate (7.35 g) was added under ice-cooling. The mixture was reacted at room temperature overnight, and the solvent was removed under reduced pressure. The residue was purified by silica gel column chromatography (eluent: hexane-ethyl acetate=10:90→50:50) to give the title compound as a yellow oil (yield 10.7 g, 99%).
¹H-NMR (DMSO-d₆, 300 MHz):δ3.03 (3H, s), 4.49 (2H, s), 7.47-7.55 (2H, m), 7.58-7.66 (1H, m), 7.87 (2H, d, J = 7.7 Hz), 10.42 (1H, brs).

### Reference Example 59

### (4E)-1-ethyl-4-[(4-hydroxy-3-methoxyphenyl)methylidene]-5-imino-3-methylimidazolidin-2-one

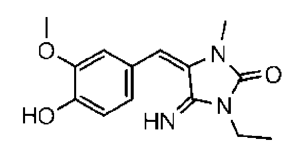

A mixed solution of (4Z)-1-ethyl-5-imino-4-({3-methoxy-4-[(4-methoxybenzyl)oxy]phenyl}methylidene)-3-methylimidazolidin-2-one (26.6 g) and trifluoroacetic acid (150 mL) was stirred at room temperature for 2.5 hr. The reaction mixture was concentrated under reduced pressure, and the mixture was extracted with ethyl acetate and 2 N aqueous sodium hydroxide solution. To the aqueous layer was added 3 N hydrochloric acid to pH=6, and the mixture was extracted with ethyl acetate. The organic layer was dried over anhydrous magnesium sulfate, and filtered, and the filtrate was concentrated under reduced pressure. The residue was recrystallized from ethyl acetate/n-heptane to give the title compound as a yellow solid (yield: 15.0 g, 81%).
¹H-NMR (CDCl₃, 300 MHz):δ1.22 (3H, t, J = 7.2 Hz), 3.68 (2H, q, J = 7.2 Hz), 3.88 (3H, s), 6.04 (1H, brs), 6.08 (1H, s), 6.81-6.99 (3H, m), 7.95 (1H, brs).

### Reference Example 60

### 4-[2,4-bis(trifluoromethyl)phenoxy]-3-methoxybenzaldehyde

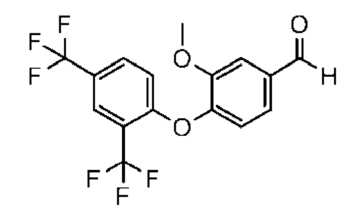

To a solution (6 mL) of 4-hydroxy-3-methoxybenzaldehyde (500 mg) in dimethyl sulfoxide were added lithium carbonate (361 mg) and a solution (2 mL) of 1-fluoro-2,4-bis(trifluoromethyl)benzene (944 mg) in dimethyl sulfoxide, and the mixture was stirred at 80-100°C for 2 days. To the reaction mixture was added 1 N aqueous sodium hydroxide solution, and the mixture was extracted with ethyl acetate. The organic layer was washed with water and saturated brine, dried over anhydrous magnesium sulfate, and filtered, and the filtrate was concentrated under reduced pressure. The residue was purified by silica gel column chromatography (eluent: hexane-ethyl acetate=100:0→80: 20) to give the title compound as a colorless oil (yield: 1.06 g, 81%).
¹H-NMR (DMSO-d₆, 300 MHz):δ3.85 (3H, s), 7.01 (1H, d, J = 8.7 Hz), 7.44 (1H, d, J = 8.1 Hz), 7.67 (1H, dd, J = 8.1, 1.3 Hz), 7.73 (1H, d, J = 1.3 Hz), 7.97 (1H, d, J = 8.7 Hz), 8.09 (1H, s), 10.03 (1H, s).

### Reference Example 61

### (5Z)-5-[(3-{[2,4-bis(trifluoromethyl)benzyl]oxy}-4-methoxyphenyl)methylidene]-4-(methylamino)-1,3-thiazol-2(5H)-one

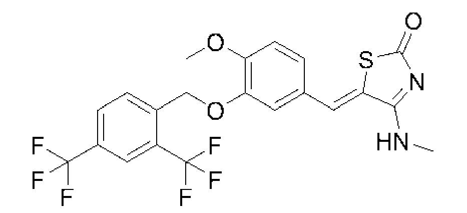

3-{[2,4-bis(Trifluoromethyl)benzyl]oxy}-4-methoxybenzaldehyde (378 mg) and 4-(methylamino)-1,3-thiazol-2(5H)-one (156 mg) were dissolved in ethanol (5 mL), and potassium tert-butoxide (134 mg) was added. After stirring under refluxing conditions for 3 hr, to the reaction mixture were added saturated aqueous ammonium chloride solution and ethyl acetate, and the mixture was extracted with ethyl acetate. The organic layer was washed with saturated brine, dried over anhydrous magnesium sulfate, and filtered, and the filtrate was concentrated under reduced pressure. The residue was recrystallized (tetrahydrofuran and ethyl acetate) to give the title compound (yield: 120 mg, 28%).
¹H-NMR (DMSO-d₆, 300 MHz):δ3.03 (3H, s), 3.87 (3H, s), 5.41 (2H, s), 7.06 (1H, s), 7.14-7.28 (2H, m), 7.68 (1H, s), 8.02 (1H, d, J = 7.9 Hz), 8.12 (1H, s), 8.18 (1H, d, J = 8.3 Hz).

### Reference Example 62

### (5Z)-5-[(3-{[2,4-bis(trifluoromethyl)benzyl]oxy}-4-methoxyphenyl)methylidene]-4-{[2-(diethylamino)ethyl]amino}-1,3-thiazol-2(5H)-one

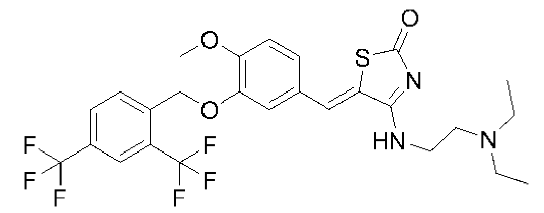

4-Thioxo-1,3-thiazolidin-2-one (266 mg) and N,N-diethylethane-1,2-diamine (232 mg) were dissolved in ethanol (10 mL), and the mixture was stirred at room temperature for 3 hr. To the reaction mixture were added 3-{[2,4-bis(trifluoromethyl)benzyl]oxy}-4-methoxybenzaldehyde (757 mg) and potassium tert-butoxide (224 mg), and the mixture was stirred under refluxing conditions for 3 hr. To the reaction mixture were added saturated aqueous ammonium chloride solution and ethyl acetate, and the mixture was extracted with ethyl acetate. The organic layer was washed with saturated brine, dried over anhydrous magnesium sulfate, and filtered, and the filtrate was concentrated under reduced pressure. The residue was purified by silica gel column chromatography (eluent: ethyl acetate-methanol=50:50→0:100) and recrystallized (ethyl acetate and hexane) to give the title compound as a yellow solid (yield: 540 mg, 47%).
¹H-NMR (DMSO-d₆, 300 MHz):δ0.97 (6H, t, J = 7.1 Hz), 2.52-2.58 (4H, m), 2.65 (2H, brs), 3.53 (2H, t, J = 6.2 Hz), 3.88 (3H, s), 5.41 (2H, s), 7.08 (1H, s), 7.22 (2H, s), 7.71 (1H, s), 8.02 (1H, d, J = 8.1 Hz), 8.12 (1H, s), 8.18 (1H, d, J = 8.3 Hz), 9.28 (1H, brs).

### Reference Example 63

### 2-{[2,4-bis(trifluoromethyl)benzyl]oxy}-5-{(Z)-[4-(methylamino)-2-oxo-1,3-thiazol-5(2H)-ylidene]methyl}benzoic acid

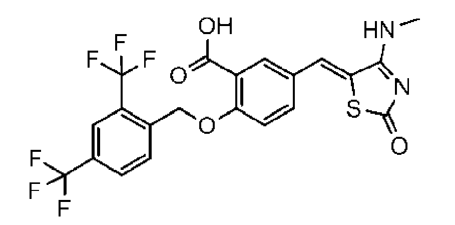

Methyl 2-{[2,4-bis(trifluoromethyl)benzyl]oxy}-5-{(Z)-[4-(methylamino)-2-oxo-1,3-thiazol-5(2H)-ylidene]methyl}benzoate (2.07 g) was suspended in methanol (20 mL), 2 mol/L aqueous sodium hydroxide solution (1 mL) was added, the mixture was stirred at 50°C overnight, and methanol was evaporated under reduced pressure. To the obtained aqueous solution was added 1 mol/L hydrochloric acid, and the precipitate was collected by filtration, and washed with water and diethyl ether to give the title compound as a pale-yellow powder (yield: 1.29 g, 64%).
¹H-NMR (DMSO-d₆, 300 MHz):δ3.06 (3H, d, J = 4.1 Hz), 5.51 (2H, s), 7.36 (1H, d, J = 8.7 Hz), 7.67 (1H, dd, J = 8.7, 2.5 Hz), 7.79 (1H, s), 7.91 (1H, d, J = 2.4 Hz), 8.10 (1H, s), 8.17 (1H, d, J = 8.3 Hz), 8.27 (1H, d, J = 8.3 Hz), 9.43 (1H, d, J = 4.1 Hz), 13.13 (1H, brs).

### Reference Example 64

### (5Z)-5-[(3-{[2,4-bis(trifluoromethyl)benzyl]oxy}-2-methoxyphenyl)methylidene]-4-(methylamino)-1,3-thiazol-2(5H)-one

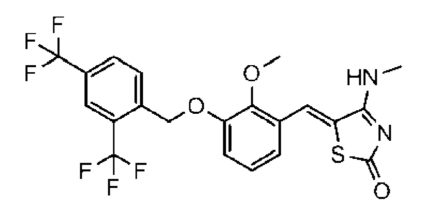

To a solution (4 mL) of 4-(methylamino)-1,3-thiazol-2(5H)-one (124 mg) in ethanol were added potassium tert-butoxide (126 mg) and 3-{[2,4-bis(trifluoromethyl)benzyl]oxy}-2-methoxybenzaldehyde (300 mg) and the mixture was heated under reflux for 1 hr under a nitrogen atmosphere. To the reaction mixture was added saturated aqueous ammonium chloride solution, and the mixture was extracted with ethyl acetate. The organic layer was washed with water and saturated brine, dried over anhydrous magnesium sulfate, and filtered, and the filtrate was concentrated under reduced pressure. The residue was purified by basic silica gel column chromatography (eluent: hexane-ethyl acetate=50:50→0:100) to give the title compound as a white solid (yield: 329 mg, 85%). The obtained solid was recrystallized from ethyl acetate/n-heptane to give the title compound as a white solid (yield: 263 mg, 68%). ¹H-NMR (CDCl₃, 300 MHz):δ3.31 (3H, d, J = 4.9 Hz), 3.94 (3H, s), 5.39 (2H, s), 6.30 (1H, brs), 6.95-7.00 (1H, m), 7.12-7.19 (1H, m), 7.30-7.36 (1H, m), 7.46 (1H, s), 7.88 (1H, d, J = 7.9 Hz), 7.97 (1H, d, J = 7.9 Hz), 7.98 (1H, s).

### Reference Example 65

### (5Z)-5-[(3-{[2,4-bis(trifluoromethyl)benzyl]oxy}-2-methoxyphenyl)methylidene]-4-{[2-(diethylamino)ethyl]amino}-1,3-thiazol-2(5H)-one

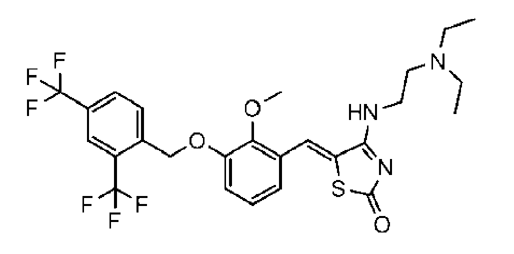

To a solution (4 mL) of N,N-diethylethane-1,2-diamine (113 mg) in ethanol was added 4-thioxo-1,3-thiazolidin-2-one (124 mg) and the mixture was stirred at room temperature for 3 hr under a nitrogen atmosphere. To the reaction mixture were added 3-{[2,4-bis(trifluoromethyl)benzyl]oxy}-2-methoxybenzaldehyde (300 mg) and potassium tert-butoxide (126 mg), and the mixture was heated under reflux for 1 hr. To the reaction mixture was added saturated aqueous ammonium chloride solution, and the mixture was extracted with ethyl acetate. The organic layer was washed with water and saturated brine, dried over anhydrous magnesium sulfate, and filtered, and the filtrate was concentrated under reduced pressure. The residue was purified by basic silica gel column chromatography (eluent: hexane-ethyl acetate=50:50→0:100) to give the title compound as a yellow solid (yield: 272 mg, 60%). The obtained solid was recrystallized from ethyl acetate/n-heptane to give the title compound as a yellow solid (yield: 200 mg, 44%). ¹H-NMR (DMSO-d₆, 300 MHz):δ0.98 (6H, t, J = 7.1 Hz), 2.52 (4H, q, J = 7.1 Hz), 2.67 (2H, t, J = 6.9 Hz), 3.56 (2H, t, J = 6.9 Hz), 3.81 (3H, s), 5.42 (2H, s), 7.19-7.27 (3H, m), 7.90 (1H, s), 8.09 (1H, d, J = 8.3 Hz), 8.13 (1H, s), 8.19 (1H, d, J = 8.1 Hz), 9.57 (1H, brs).

### Reference Example 66

### 4-(2-methoxy-3-{(Z)-[4-(methylamino)-2-oxo-1,3-thiazol-5(2H)-ylidene]methyl}phenoxy)-3-(trifluoromethyl)benzonitrile

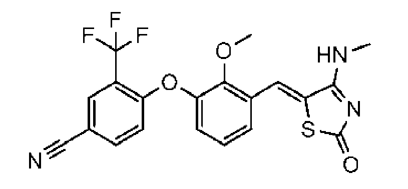

To a solution (4 mL) of 4-(methylamino)-1,3-thiazol-2(5H)-one (146 mg) in ethanol were added potassium tert-butoxide (148 mg) and 4-(3-formyl-2-methoxyphenoxy)-3-(trifluoromethyl)benzonitrile (300 mg) and the mixture was heated under reflux for 0.5 hr under a nitrogen atmosphere. To the reaction mixture was added saturated aqueous ammonium chloride solution, and the mixture was extracted with ethyl acetate. The organic layer was washed with water and saturated brine, dried over anhydrous magnesium sulfate, and filtered, and the filtrate was concentrated under reduced pressure. The residue was purified by basic silica gel column chromatography (eluent: hexane-ethyl acetate=50:50→0:100) to give the title compound as a pale-yellow solid (yield: 247 mg, 61%). The obtained solid was recrystallized from ethyl acetate/n-heptane to give the title compound as a pale-yellow solid (yield: 209 mg, 52%).
¹H-NMR (DMSO-d₆, 300 MHz):δ3.06 (3H, s), 3.74 (3H, s), 6.96 (1H, d, J = 8.7 Hz), 7.32-7.44 (2H, m), 7.58 (1H, dd, J = 7.4, 1.9 Hz), 7.86 (1H, s), 8.05 (1H, dd, J = 8.7, 1.9 Hz), 8.38 (1H, d, J = 1.9 Hz), 9.68 (1H, brs).

### Reference Example 67

### 4-(3-{(Z)-[4-{[2-(diethylamino)ethyl]amino}-2-oxo-1,3-thiazol-5(2H)-ylidene]methyl}-2-methoxyphenoxy)-3-(trifluoromethyl)benzonitrile

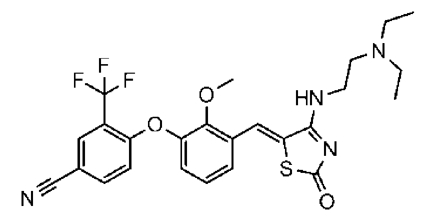

To a solution (4 mL) of N,N-diethylethane-1,2-diamine (133 mg) in ethanol was added 4-thioxo-1,3-thiazolidin-2-one (149 mg) and the mixture was stirred at room temperature for 5 hr under a nitrogen atmosphere. To the reaction mixture were added 4-(3-formyl-2-methoxyphenoxy)-3-(trifluoromethyl)benzonitrile (300 mg) and potassium tert-butoxide (148 mg), and the mixture was heated under reflux for 0.5 hr. To the reaction mixture was added saturated aqueous ammonium chloride solution, and the mixture was extracted with ethyl acetate. The organic layer was washed with water and saturated brine, dried over anhydrous magnesium sulfate, and filtered, and the filtrate was concentrated under reduced pressure. The residue was purified by basic silica gel column chromatography (eluent: hexane-ethyl acetate=50:50→0:100) to give the title compound as a yellow oil (yield: 277 mg, 57%).
¹H-NMR (DMSO-d₆, 300 MHz):δ0.97 (6H, t, J = 7.1 Hz), 2.52 (4H, q, J = 7.1 Hz), 2.66 (2H, t, J = 6.9 Hz), 3.56 (2H, t, J = 6.9 Hz), 3.72 (3H, s), 6.96 (1H, d, J = 8.9 Hz), 7.33-7.45 (2H, m), 7.58 (1H, dd, J = 7.4, 2.1 Hz), 7.88 (1H, s), 8.05 (1H, dd, J = 8.9, 2.1 Hz), 8.38 (1H, d, J = 1.7 Hz), 9.60 (1H, brs).

### Example 1

### (5Z)-4-amino-5-[(4-{[2,4-bis(trifluoromethyl)benzyl]oxy}-3-methoxyphenyl)methylidene]-1,3-thiazol-2(5H)-one

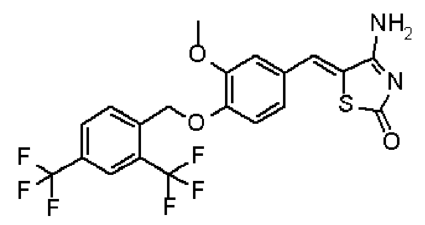

To a solution (1 mL) of (5Z)-5-[(4-{[2,4-bis(trifluoromethyl)benzyl]oxy}-3-methoxyphenyl)methylidene]-4-thioxo-1,3-thiazolidin-2-one (62 mg) in methanol was added ammonia-methanol solution (2 mol/L solution, 376 µL) and the mixture was stirred at 100°C for 2 hr under microwave irradiation. The reaction mixture was cooled to room temperature, and filtered to give the title compound as a pale-yellow solid (yield: 37.5 mg, 62%).
¹H-NMR (DMSO-d₆, 400 MHz):δ3.85 (3H, s), 5.41 (2H, s), 7.10-7.25 (2H, m), 7.17-7.25 (1H, m), 7.82 (1H, s), 8.03 (1H, d, J = 7.8 Hz), 8.12 (1H, s), 8.19 (1H, d, J = 8.1 Hz).

### Example 2

### (5Z)-4-amino-5-{[3-methoxy-4-(naphthalen-1-ylmethoxy)phenyl]methylidene}-1,3-thiazol-2(5H)-one

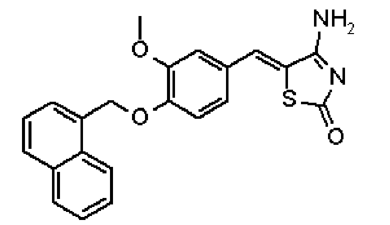

To a solution (2 mL) of (5Z)-5-{[3-methoxy-4-(naphthalen-1-ylmethoxy)phenyl]methylidenel-4-thioxo-1,3-thiazolidin-2-one (100 mg) in methanol was added ammonia-methanol solution (2 mol/L solution, 613 µL) and the mixture was stirred at 100°C for 4 hr under microwave irradiation. The reaction mixture was cooled to room temperature, and filtered to give the title compound as a pale-brown solid (yield: 33 mg, 34%).
¹H NMR (DMSO-d₆, 400 MHz):δ3.79 (3H, s), 5.61 (2H, s), 7.11 (1H, d, J = 2.0 Hz), 7.17 (1H, dd, J = 8.3, 2.0 Hz), 7.42 (1H, d, J = 8.6 Hz), 7.50-7.63 (3H, m), 7.69 (1H, d, J = 6.6 Hz), 7.83 (1H, s), 7.93-8.05 (2H, m), 8.05-8.13 (1H, m).

### Example 3

### (5Z)-5-[(4-{[2,4-bis(trifluoromethyl)benzyl]oxy}-3-methoxyphenyl)methylidene]-4-(methylamino)-1,3-thiazol-2(5H)-one

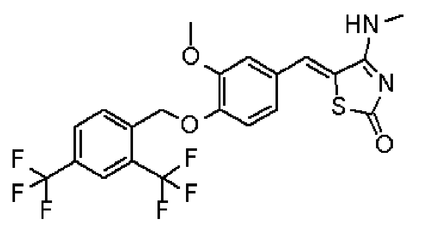

To a solution (3 mL) of (5Z)-5-[(4-{[2,4-bis(trifluoromethyl)benzyl]oxy}-3-methoxyphenyl)methylidene]-4-thioxo-1,3-thiazolidin-2-one (150 mg) in methanol was added methylamine-tetrahydrofuran solution (2 mol/L solution, 760 µL) and the mixture was stirred at 100°C for 2 hr under microwave irradiation. The reaction mixture was cooled to room temperature, 1 mol/L hydrochloric acid was added and the precipitate was collected by filtration to give the title compound as a pale-yellow solid (yield: 9 mg, 6%) .
¹H-NMR (DMSO-d₆, 400 MHz):δ3.04 (3H, d, J = 4.4 Hz), 3.85 (3H, s), 5.41 (2H, s), 7.09-7.16 (2H, m), 7.16-7.24 (1H, m), 7.73 (1H, s), 8.02 (1H, d, J = 8.1 Hz), 8.11 (1H, s), 8.18 (1H, s), 9.34-9.42 (1H, m).

### Example 4

### (5Z)-4-(benzylamino)-5-[(4-{[2,4-bis(trifluoromethyl)benzyl]oxy}-3-methoxyphenyl)methylidene]-1,3-thiazol-2(5H)-one

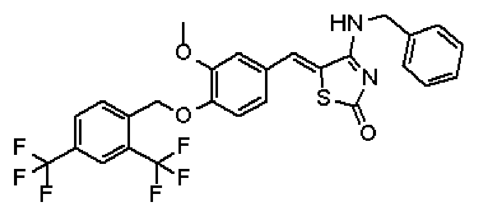

To a solution (5 mL) of (5Z)-5-[(4-{[2,4-bis(trifluoromethyl)benzyl]oxy}-3-methoxyphenyl)methylidene]-4-thioxo-1,3-thiazolidin-2-one (150 mg) in methanol was added benzylamine (100 µL) and the mixture was stirred at 100°C for 2 hr under microwave irradiation. The reaction mixture was cooled to room temperature, and the precipitate was collected by filtration, and washed with 2-propanol to give the title compound as a yellow solid (yield: 46 mg, 27%).
¹H-NMR (DMSO-d₆, 400 MHz):δ3.85 (3H, s), 4.72 (2H, s), 5.41 (2H s), 7.12-7.18 (2H, m), 7.18-7.24 (1H, m), 7.28-7.35 (1H, m), 7.35-7.42 (4H, m), 7.87 (1H, s), 8.03 (1H, d, J = 8.3 Hz), 8.12 (1H, s), 8.15-8.22 (1H, m), 9.83 (1H, brs).

### Example 5

### (5Z)-5-{[3-methoxy-4-(naphthalen-1-ylmethoxy)phenyl]methylidene}-4-(methylamino)-1,3-thiazol-2(5H)-one

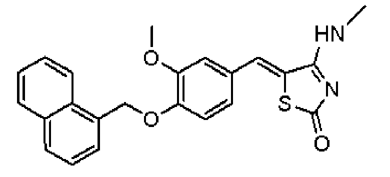

A mixed solution of (5Z)-5-{[3-methoxy-4-(naphthalen-1-ylmethoxy)phenyllmethylidenel-4-thioxo-1,3-thiazolidin-2-one (122 mg), methylamine-tetrahydrofuran solution (2 mol/L solution, 0.45 mL) and methanol (2.5 mL) was stirred at 100°C for 2 hr under microwave irradiation. The reaction mixture was allowed to cool to room temperature, and the precipitate was collected by filtration and washed with diisopropylether to give the title compound as a pale-brown powder (yield: 53.7 mg, 44.3%).
¹H-NMR (DMSO-d₆, 400 MHz):δ3.05 (3H, s), 3.79 (3H, s), 5.61 (2H, s), 7.09 (1H, d, J = 1.9 Hz), 7.16 (1H, dd, J = 1.6, 8.4 Hz), 7.57 (1H, d, J = 8.3 Hz), 7.50-7.60 (3H, m), 7.68 (1H, d, J = 6.6 Hz), 7.74 (1H, s), 7.95-8.05 (2H, m), 8.05-8.10 (1H, m), 9.30-9.40 (1H, br).

### Example 6

### 4-(2-methoxy-4-{(Z)-[4-(methylamino)-2-oxo-1,3-thiazol-5(2H)-ylidene]methyl}phenoxy)-3-(trifluoromethyl)benzonitrile

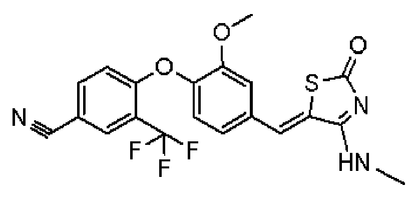

To a solution (8 mL) of 4-{4-[(Z)-(2,4-dioxo-1,3-thiazolidin-5-ylidene)methyl]-2-methoxyphenoxy}-3-(trifluoromethyl)benzonitrile (990 mg) in toluene was added a Lawesson reagent (667 mg). The reaction mixture was stirred at 120°C for 4.5 hr, and allowed to cool to room temperature, and the precipitate was collected by filtration, and washed with diisopropylether. The precipitate was dissolved in ethyl acetate, and the mixture was washed successively with water and brine, and dried over anhydrous magnesium sulfate. After concentration under reduced pressure, the residue was purified by silica gel column chromatography (eluent: hexane-ethyl acetate=3:1→1:1), and recrystallized from ethyl acetate and hexane to give 4-{2-methoxy-4-[(Z)-(2-oxo-4-thioxo-1,3-thiazolidin-5-ylidene)methyl]phenoxyl-3-(trifluoromethyl)benzonitrile as a pale-brown powder (yield: 476.3 mg, 46.2%).
A mixed solution of 4-{2-methoxy-4-[(Z)-(2-oxo-4-thioxo-1,3-thiazolidin-5-ylidene)methyllphenoxyl-3-(trifluoromethyl)benzonitrile (135 mg), methylamine-tetrahydrofuran solution (2 mol/L solution, 0.45 mL), methanol (1.0 mL) and tetrahydrofuran (1.0 mL) was stirred at 80°C for 1 hr. The reaction mixture was allowed to cool to room temperature, and concentrated under reduced pressure. The residue was purified by silica gel column chromatography (eluent: hexane-ethyl acetate=1:4→ethyl acetate), and recrystallized from ethyl acetate and diisopropylether to give the title compound as a pale-yellow powder (yield: 19.4 mg, 14.4%).
¹H-NMR (DMSO-d₆, 400 MHz):δ3.07 (3H, s), 3.80 (3H, s), 6.93 (1H, d, J = 8.5 Hz), 7.23 (1H, dd, J = 1.5, 7.9 Hz), 7.29 (1H, d, J = 1.7 Hz), 7.38 (1H, d, J = 8.3 Hz), 7.80 (1H, s), 8.02 (1H, dd, J = 1.6, 8.8 Hz), 8.34 (1H, s), 9.52 (1H, brs).

### Example 7

### (5Z)-5-[(4-{[2,4-bis(trifluoromethyl)benzyl]oxy}-3-methylphenyl)methylidene]-4-(methylamino)-1,3-thiazol-2(5H)-one

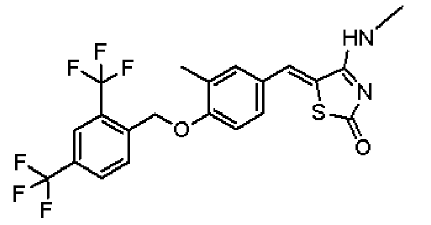

(5Z)-5-[(4-{[2,4-bis(Trifluoromethyl)benzyl]oxy}-3-methylphenyl)methylidene]-1,3-thiazolidine-2,4-dione (524.2 mg) and a Lawesson reagent (276.7 mg) were suspended in toluene/tetrahydrofuran (5:1, 6.6 mL), and the suspension was stirred at 80°C for 6 hr. The reaction mixture was cooled to room temperature, and the precipitate was collected by filtration. The obtained solid was washed with ethyl acetate to give (5Z)-5-[(4-{[2,4-bis(trifluoromethyl)benzyl]oxy}-3-methylphenyl)methylidene]-4-thioxo-1,3-thiazolidin-2-one (312.5 mg) as a red orange powder. To a suspension (2.63 mL) of the present compound (251.3 mg) in methanol was added 40% methylamine-methanol solution (0.20 mL), and the mixture was stirred at room temperature for 4 hr. The precipitate was collected by filtration, and washed with ethyl acetate to give the title compound as a pale-yellow powder (yield: 105.9 mg, 24% (2 steps)).
¹H-NMR (DMSO-d₆, 300 MHz):δ2.27 (3H, s), 3.05 (3H, s), 5.43 (2H, s), 7.17 (1H, d, J = 8.3 Hz), 7.35-7.45 (2H, m), 7.74 (1H, s), 8.06 (1H, d, J = 8.3 Hz), 8.12 (1H, s), 8.17 (1H, d, J = 8.3 Hz).

### Example 8

### (5Z)-5-[(4-{[2,4-bis(trifluoromethyl)benzyl]oxy}-2-methylphenyl)methylidene]-4-(methylamino)-1,3-thiazol-2(5H)-one

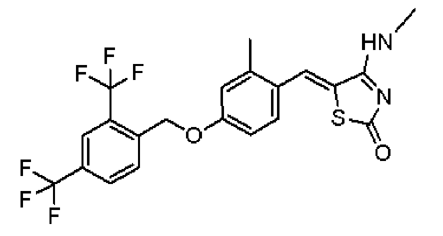

(5Z)-5-[(4-{[2,4-bis(Trifluoromethyl)benzyl]oxy}-2-methylphenyl)methylidene]-1,3-thiazolidine-2,4-dione (513.3 mg) and a Lawesson reagent (270.0 mg) were suspended in toluene/tetrahydrofuran (5:1, 6.6 mL), and the suspension was stirred at 80°C overnight. The reaction mixture was cooled to room temperature, and the precipitate was collected by filtration. The obtained solid was washed with ethyl acetate to give (5Z)-5-[(4-{[2,4-bis(trifluoromethyl)benzyl]oxy}-2-methylphenyl)methylidene]-4-thioxo-1,3-thiazolidin-2-one (251.0 mg) as a red orange powder. To a suspension (1.83 mL) of the present compound (175.1 mg) in methanol was added 40% methylamine-methanol solution (0.14 mL, 1.83 mmol), and the mixture was stirred at room temperature for 1.5 hr. The precipitate was collected by filtration, and washed with ethyl acetate to give the title compound as a pale-yellow powder (yield: 44.9 mg, 12%).
¹H-NMR (DMSO-d₆, 300 MHz):δ2.40 (3H, s), 3.05 (3H, d, J = 2.8 Hz), 5.39 (2H, s), 6.97-7.04 (2H, m), 7.50 (1H, d, J = 9.4 Hz), 7.77 (1H, s), 8.02 (1H, d, J= 8.5 Hz), 8.10 (1H, s), 8.15 (1H, d, J= 8.5 Hz), 9.41 (1H, brs).

### Example 9

### (5Z)-5-[(4-{[2,4-bis(trifluoromethyl)benzyl]oxy}-2-chlorophenyl)methylidene]-4-(methylamino)-1,3-thiazol-2(5H)-one

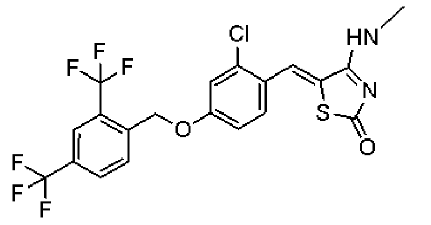

(5Z)-5-[(4-{[2,4-bis(Trifluoromethyl)benzyl]oxy}-2-chlorophenyl)methylidene]-1,3-thiazolidine-2,4-dione (392.9 mg) and a Lawesson reagent (197.9 mg) were suspended in toluene/tetrahydrofuran (5:1, 4.92 mL), and the suspension was stirred at 80°C overnight. The reaction solution was purified by silica gel column chromatography (eluent: hexane-ethyl acetate=10:0→7:3) to give (5Z)-5-[(4-{[2,4-bis(trifluoromethyl)benzyl]oxy}-2-chlorophenyl)methylidene]-4-thioxo-1,3-thiazolidin-2-one (175.1 mg). To a suspension (1.83 mL) of the present compound (175.1 mg) in methanol was added 40% methylamine-methanol solution (0.14 mL), and the mixture was stirred at room temperature for 1.5 hr. The precipitate was collected by filtration, and washed successively with ethyl acetate and methanol to give the title compound as a pale-yellow powder (yield: 83.2 mg, 15%).
¹H-NMR (DMSO-d₆, 300 MHz):δ3.06 (3H, s), 5.44 (2H, s), 7.19 (1H, dd, J = 8.9, 2.5 Hz), 7.34 (1H, d, J = 2.6 Hz), 7.64 (1H, d, J = 8.9 Hz), 7.82 (1H, s), 8.05 (1H, d, J = 8.1 Hz), 8.12 (1H, s), 8.17 (1H, d, J = 8.1 Hz).

### Example 10

### (5Z)-5-[(4-{[2,4-bis(trifluoromethyl)benzyl]oxy}-3-chlorophenyl)methylidene]-4-(methylamino)-1,3-thiazol-2(5H)-one

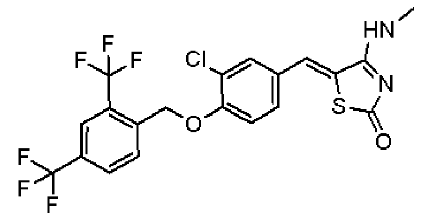

(5Z)-5-[(4-{[2,4-bis(Trifluoromethyl)benzyl]oxy}-3-chlorophenyl)methylidene]-1,3-thiazolidine-2,4-dione (440.5 mg) and a Lawesson reagent (279.3 mg) were suspended in toluene/tetrahydrofuran (5:1, 7 mL), and the suspension was stirred at 80°C overnight. The reaction mixture was cooled to room temperature, and the precipitate was collected by filtration. The obtained solid was washed with ethyl acetate to give (5Z)-5-[(4-{[2,4-bis(trifluoromethyl)benzyl]oxy}-3-chlorophenyl)methylidene]-4-thioxo-1,3-thiazolidin-2-one (262.0 mg) as a red orange powder. To a suspension (2.4 mL) of the present compound (239.6 mg) in methanol was added 40% methylamine-methanol solution (0.19 mL), and the mixture was stirred at room temperature for 1.5 hr. The precipitate was collected by filtration to give the title compound as a yellow powder (yield: 111.4 mg, 21%).
¹H-NMR (DMSO-d₆, 300 MHz):δ3.06 (3H, s), 5.52 (2H, s), 7.42 (1H, d, J = 8.7 Hz), 7.52 (1H, dd, J = 8.7, 2.1 Hz), 7.59 (1H, d, J = 2.1 Hz), 7.70 (1H, s), 8.08 (1H, d, J = 8.3 Hz), 8.12 (1H, s), 8.20 (1H, m, J = 8.3 Hz).

### Example 11

### (5Z)-5-[(4-{[2,4-bis(trifluoromethyl)benzyl]oxy}-2-methoxyphenyl)methylidene]-4-(methylamino)-1,3-thiazol-2(5H)-one

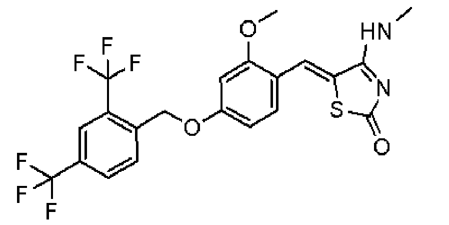

(5Z)-5-[(4-{[2,4-bis(Trifluoromethyl)benzyl]oxy}-2-methoxyphenyl)methylidene]-1,3-thiazolidine-2,4-dione (653.3 mg) and a Lawesson reagent (332.1 mg) were suspended in toluene/tetrahydrofuran (5:1, 8.22 mL), and the suspension was stirred at 80°C overnight. The reaction mixture was cooled to room temperature, and the precipitate was collected by filtration. The obtained solid was washed with ethyl acetate to give a mixture (311.2 mg) of (5Z)-5-[(4-{[2,4-bis(trifluoromethyl)benzyl]oxy}-2-methoxyphenyl)methylidene]-4-thioxo-1,3-thiazolidin-2-one and a small amount of (5Z)-5-[(4-{[2,4-bis(trifluoromethyl)benzyl]oxy}-2-methoxyphenyl)methylidene]-1,3-thiazolidine-2,4-dione. To a suspension (2.68 mL) of the mixture (264.8 mg) in methanol was added 40% methylamine-methanol solution (0.21 mL), and the mixture was stirred at room temperature for 1.5 hr. The precipitate was collected by filtration, washed with ethyl acetate and purified by silica gel column chromatography (eluent: hexane-ethyl acetate=1:1→0:10) to give the title compound as a yellow powder (yield: 11.2 mg, 2%).
¹H-NMR (DMSO-d₆, 300 MHz):→3.02 (3H, d, J = 4.5 Hz), 3.88 (3H, s), 5.42 (2H, s), 6.75-6.80 (2H, m), 7.47 (1H, d, J = 9.2 Hz), 7.83 (1H, s), 8.05 (1H, d, J = 8.1 Hz), 8.11 (1H, s), 8.16 (1H, d, J = 8.1 Hz).

### Example 12

### (5Z)-5-[(4-{[2,4-bis(trifluoromethyl)benzyl]oxy}-3-ethoxyphenyl)methylidene]-4-(methylamino)-1,3-thiazol-2(5H)-one

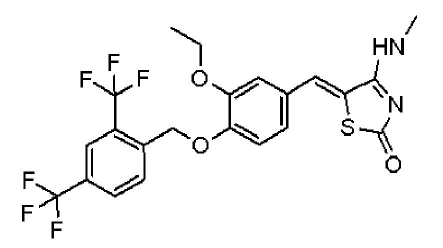

4-{[2,4-bis(Trifluoromethyl)benzyl]oxy}-3-ethoxybenzaldehyde (102.4 mg) and 4-(methylamino)-1,3-thiazol-2(5H)-one (37.4 mg) were dissolved in ethanol (1.3 mL), and potassium tert-butoxide (35.1 mg) was added. The reaction mixture was stirred under heating under reflux for 3 hr, and concentrated under reduced pressure. The residue was dissolved in ethyl acetate, and the mixture was washed successively with water and saturated brine, dried over anhydrous magnesium sulfate and concentrated under reduced pressure. The residue was purified by silica gel column chromatography (eluent: hexane-ethyl acetate=7:3→1:9) to give the title compound as a yellow powder (yield: 74.5 mg, 57%).
¹H-NMR (DMSO-d₆, 300 MHz):δ1.37 (3H, t, J = 6.8 Hz), 3.04 (3H, s), 4.11 (2H, q, J = 6.8 Hz), 5.42 (2H, s), 7.05-7.21 (3H, m), 7.70 (1H, s), 8.03 (1H, d, J = 7.0 Hz), 8.09 (1H, s), 8.17 (1H, d, J = 7.0 Hz), 9.32 (1H, brs).

### Example 13

### (5Z)-5-{[4-{[2,4-bis(trifluoromethyl)benzyl]oxy}-3-(2-methylpropoxy)phenyl]methylidene}-4-(methylamino)-1,3-thiazol-2(5H)-one

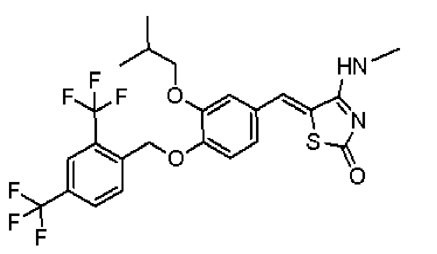

4-{[2,4-bis(Trifluoromethyl)benzyl]oxy}-3-(2-methylpropoxy)benzaldehyde (116.3 mg) and 4-(methylamino)-1,3-thiazol-2(5H)-one (39.6 mg) were dissolved in ethanol (1.4 mL), and potassium tert-butoxide (34.1 mg) was added. The reaction mixture was stirred under heating under reflux overnight, and concentrated under reduced pressure. The residue was dissolved in ethyl acetate, and the mixture was washed successively with water and saturated brine, dried over anhydrous magnesium sulfate and concentrated under reduced pressure. The residue was recrystallized from ethyl acetate (80°C→0°C) to give the title compound as a yellow powder (yield: 87.2 mg, 59%).
¹H-NMR (DMSO-d₆, 300 MHz):δ0.99 (6H, d, J = 6.8 Hz), 2.00-2.14 (1H, m), 3.04 (3H, s), 3.82 (2H, d, J = 6.6 Hz), 5.43 (2H, s), 7.08-7.15 (2H, m), 7.21 (1H, d, J = 8.3 Hz), 7.73 (1H, s), 8.04-8.11 (3H, m), 8.14-8.21 (1H, m).

### Example 14

### (5Z)-5-{[3-(benzyloxy)-4-{[2,4-bis(trifluoromethyl)benzyl]oxy}phenyl]methylidene}-4-(methylamino)-1,3-thiazol-2(5H)-one

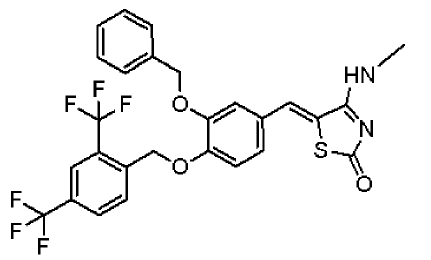

3-(Benzyloxy)-4-{[2,4-bis(trifluoromethyl)benzyl]oxy}benzaldehyde (104.4 mg) and 4-(methylamino)-1,3-thiazol-2(5H)-one (32.9 mg) were dissolved in ethanol (1.2 mL), and potassium tert-butoxide (28.4 mg) was added. The reaction mixture was stirred under heating under reflux overnight, and concentrated under reduced pressure. The residue was dissolved in ethyl acetate, and the mixture was washed successively with water and saturated brine, dried over anhydrous magnesium sulfate and concentrated under reduced pressure. The residue was recrystallized from ethyl acetate (80°C→0°C) to give the title compound as a yellow powder (yield: 50.0 mg, 38%).
¹H-NMR (DMSO-d₆, 300 MHz):δ3.03 (3H, s), 5.21 (2H, s), 5.45 (2H, s), 7.12 (1H, dd, J = 8.5, 1.9 Hz), 7.18 (1H, d, J = 1.9 Hz), 7.22 (1H, d, J = 8.5 Hz), 7.28-7.49 (5H, m), 7.66 (1H, s), 8.04 (1H, d, J = 8.5 Hz), 8.09 (1H, s), 8.13 (1H, d, J = 8.5 Hz), 9.31 (1H, brs).

### Example 15

### (5Z)-5-[(4-{[2,4-bis(trifluoromethyl)benzyl]oxy}-3-propoxyphenyl)methylidene]-4-(methylamino)-1,3-thiazol-2(5H)-one

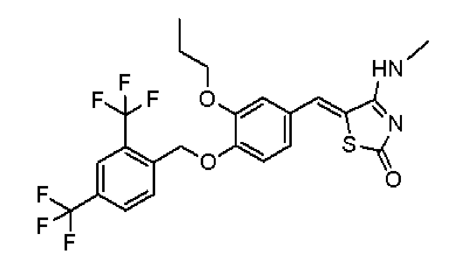

4-{[2,4-bis(Trifluoromethyl)benzyl]oxy}-3-propoxybenzaldehyde (142.7 mg) and 4-(methylamino)-1,3-thiazol-2(5H)-one (50.3 mg) were dissolved in ethanol (1.8 mL), and potassium tert-butoxide (47.3 mg) was added. The reaction mixture was stirred with heating under reflux for 4.5 hr, and poured into water, and the mixture was extracted with ethyl acetate. The organic layer was washed with saturated brine, dried over anhydrous magnesium sulfate and concentrated under reduced pressure. The residue was purified by silica gel column chromatography (eluent: hexane-ethyl acetate=6:4→1:9), and washed with diethyl ether to give the title compound as a yellow powder (yield: 22.6 mg, 15%).
¹H-NMR (DMSO-d₆, 300 MHz):δ0.99 (3H, t, J = 7.4 Hz), 1.70-1.83 (2H, m), 3.04 (3H, s), 4.01 (2H, t, J = 6.5 Hz), 5.43 (2H, s), 7.07-7.15 (2H, m), 7.20 (1H, m, J = 8.3 Hz), 7.70 (1H, s), 8.05 (1H, d, J= 8.1 Hz), 8.10 (1H, s), 8.17 (1H, m), 9.32 (1H, brs).

### Example 16

### (5Z)-5-[(4-{[2,4-bis(trifluoromethyl)benzyl]oxy}-3-butoxyphenyl)methylidene]-4-(methylamino)-1,3-thiazol-2(5H)-one

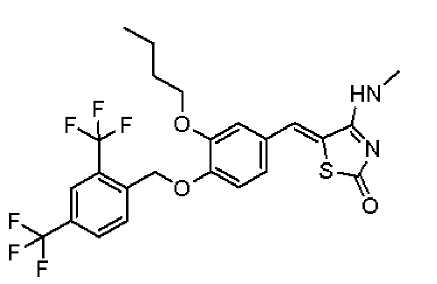

4-{[2,4-bis(Trifluoromethyl)benzyl]oxy}-3-butoxy benzaldehyde (152.3 mg) and 4-(methylamino)-1,3-thiazol-2(5H)-one (51.9 mg) were dissolved in ethanol (1.8 mL), and potassium tert-butoxide (48.7 mg) was added. The reaction mixture was stirred with heating under reflux for 4.5 hr, and poured into water, and the mixture was extracted with ethyl acetate. The organic layer was washed with saturated brine, dried over anhydrous magnesium sulfate and concentrated under reduced pressure. The residue was purified by silica gel column chromatography (eluent: hexane-ethyl acetate=6:4→1:9), and washed with diethyl ether to give the title compound as a yellow powder (yield: 32.2 mg, 17%).
¹H-NMR (DMSO-d₆, 300 MHz):δ0.91 (3H, m), 1.36-1.51 (2H, m), 1.65-1.79 (2H, m), 3.04 (3H, s), 4.05 (2H, t, J = 6.4 Hz), 5.42 (2H, s), 7.07-7.14 (2H, m), 7.20 (1H, d, J = 8.5 Hz), 7.71 (1H, s), 8.05 (1H, d, J = 8.1 Hz), 8.09 (1H, s), 8.17 (1H, d, J = 8.1 Hz), 9.32 (1H, brs).

### Example 17

### (5Z)-5-{[4-{[2,4-bis(trifluoromethyl)benzyl)oxy}-3-(1-methylethoxy)phenyl]methylidene}-4-(methylamino)-1,3-thiazol-2(5H)-one

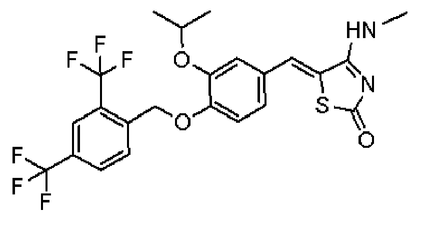

4-{[2,4-bis(Trifluoromethyl)benzyl)oxy}-3-(1-methylethoxy)benzaldehyde (104.4 mg) and 4-(methylamino)-1,3-thiazol-2(5H)-one (36.8 mg) were dissolved in ethanol (1.3 mL), and potassium tert-butoxide (34.6 mg) was added. The reaction mixture was stirred with heating under reflux for 4.5 hr, and poured into water, and the mixture was extracted with ethyl acetate. The organic layer was washed with saturated brine, dried over anhydrous magnesium sulfate and concentrated under reduced pressure. The residue was purified by silica gel column chromatography (eluent: hexane-ethyl acetate=6:4→1:9), and washed with diethyl ether to give the title compound as a yellow powder (yield: 11.8 mg, 9%).
¹H-NMR (DMSO-d₆, 300 MHz):δ1.31 (6H, d, J = 5.9 Hz), 3.04 (3H, s), 4.57 (1H, m), 5.42 (2H, s), 7.07-7.16 (2H, m), 7.20 (1H, d, J = 8.5 Hz), 7.70 (1H, s), 8.04 (1H, d, J= 8.1 Hz), 8.11 (1H, s), 8.19 (1H, d, J= 8.1 Hz), 9.35 (1H, brs).

### Example 18

### (5Z)-5-{[4-{[2,4-bis(trifluoromethyl)benzyl]oxy}-3-(cyclohexylmethoxy)phenyl]methylidene}-4-(methylamino)-1,3-thiazol-2(5H)-one

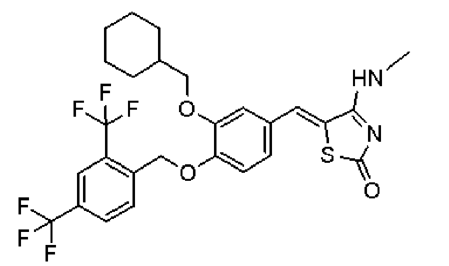

4-{[2,4-bis(Trifluoromethyl)benzyl)oxy}-3-(cyclohexylmethoxy)benzaldehyde (107.7 mg) and 4-(methylamino)-1,3-thiazol-2(5H)-one (33.5 mg) were dissolved in ethanol (1.2 mL), and potassium tert-butoxide (31.5 mg) was added. The reaction mixture was stirred with heating under reflux for 4.5 hr, and poured into water, and the mixture was extracted with ethyl acetate. The organic layer was washed with saturated brine, dried over anhydrous magnesium sulfate and concentrated under reduced pressure. The residue was purified by silica gel column chromatography (eluent: hexane-ethyl acetate=6:4→1:9), and washed with diethyl ether to give the title compound as a yellow powder (yield: 35.9 mg, 27%). ¹H-NMR (DMSO-d₆, 300 MHz):δ0.93-1.31 (5H, m), 1.55-1.85 (6H, m), 3.04 (3H, s), 3.85 (2H, d, J = 5.9 Hz), 5.43 (2H, s), 7.07-7.13 (2H, m), 7.20 (1H, d, J = 8.5 Hz), 7.71 (1H, s), 8.05 (1H, d, J = 8.3 Hz), 8.11 (1H, s), 8.16 (1H, d, J = 8.3 Hz), 9.36 (1H, brs).

### Example 19

### (5Z)-5-[(4-{[2,4-bis(trifluoromethyl)benzyl]oxy}-3-fluorophenyl)methylidene]-4-(methylamino)-1,3-thiazol-2(5H)-one

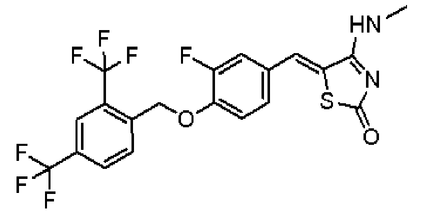

4-{[2,4-bis(Trifluoromethyl)benzyl]oxy}-3-fluorobenzaldehyde (540.8 mg) and 4-(methylamino)-1,3-thiazol-2(5H)-one (211.4 mg) were dissolved in ethanol (7.4 mL), and potassium tert-butoxide (199.3 mg) was added. The reaction mixture was stirred with heating under reflux for 3.5 hr. Water was added to the reaction mixture, and the mixture was cooled to room temperature. The precipitate was collected by filtration, and washed successively with ethyl acetate and ethanol to give the title compound as a yellow powder (yield: 481.2 mg, 68%).
¹H-NMR (DMSO-d₆, 300 MHz):δ3.05 (3H, s), 5.50 (2H, s), 7.30-7.48 (3H, m), 7.68 (1H, s), 8.05 (1H, d, J= 8.1 Hz), 8.12 (1H, s), 8.18 (1H, d, J= 8.1 Hz), 9.34 (1H, brs).

### Example 20

### (5Z)-5-[(4-{[2,4-bis(trifluoromethyl)benzyl]oxy}phenyl)methylidene]-4-(methylamino)-1,3-thiazol-2(5H)-one

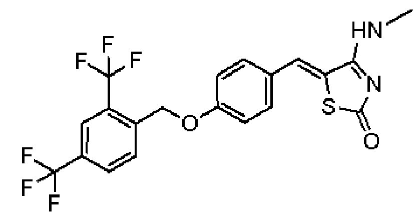

4-{[2,4-bis(Trifluoromethyl)benzyl]oxy}benzaldehyde (194.2 mg) and 4-(methylamino)-1,3-thiazol-2(5H)-one (79.8 mg) were dissolved in ethanol (2.8 mL), and potassium tert-butoxide (75.0 mg) was added. The reaction mixture was stirred with heating under reflux for 3.5 hr. Water was added to the reaction mixture, and the mixture was cooled to room temperature. The precipitate was collected by filtration, and washed successively with ethyl acetate and ethanol to give the title compound as a yellow powder (yield: 161.5 mg, 63%). ¹H-NMR (DMSO-d₆, 300 MHz):δ 3.05 (3H, s), 5.42 (2H, s), 7.20 (2H, d, J = 8.9 Hz), 7.52 (2H, d, J = 8.9 Hz), 7.72 (1H, s), 8.03 (1H, d, J = 8.1 Hz), 8.11 (1H, s), 8.15 (1H, d, J = 8.1 Hz), 9.33 (1H, brs).

### Example 21

### (4Z)-1-ethyl-5-imino-4-({3-methoxy-4-[(4-methoxybenzyl)oxy]phenyl}methylidene)imidazolidin-2-one

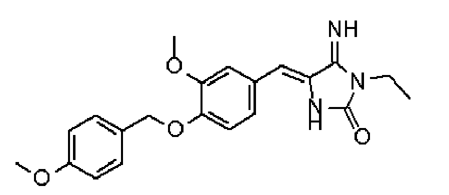

To a solution (200 mL) of 1-(cyanomethyl)-3-ethylurea (10.0 g) in ethanol was added potassium tert-butoxide (10.4 g), and the mixture was stirred at room temperature for 0.5 hr. To the reaction solution were added 3-methoxy-4-[(4-methoxybenzyl)oxy]benzaldehyde (21.4 g) and potassium tert-butoxide (10.4 g), and the mixture was heated under reflux for 4 hr, and water (200 mL) and saturated aqueous ammonium chloride (200 mL) were added at room temperature. The precipitate was collected by filtration, washed successively with water and ethyl acetate and recrystallized from tetrahydrofuran/n-heptane to give the title compound as a yellow powder (yield: 18.1 g, 60%).
¹H-NMR (DMSO-d₆, 300 MHz):δ1.12 (3H, t, J = 7.0 Hz), 3.55 (2H, q, J = 7.0 Hz), 3.76 (3H, s), 3.82 (3H, s), 5.02 (2H, s), 6.53 (1H, s), 6.92-6.98 (2H, m), 6.98-7.07 (3H, m), 7.37 (2H, d, J = 8.5 Hz), 8.69 (1H, brs), 10.17 (1H, brs).

### Example 22

### (4Z)-1-ethyl-5-imino-4-[(3-methoxy-4-{[2-(trifluoromethyl)benzyl]oxylphenyl)methylidenelimidazolidin-2-one

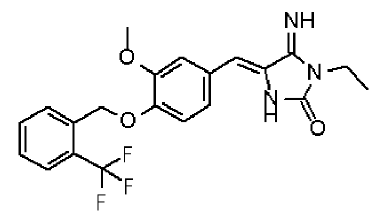

To a solution (5 mL) of (4Z)-1-ethyl-4-[(4-hydroxy-3-methoxyphenyl)methylidene]-5-iminoimidazolidin-2-one (200 mg) in tetrahydrofuran were added [2-(trifluoromethyl)phenyl]methanol (278 mg), diethyl azodicarboxylate (700 µL) and triphenylphosphine (414 mg), and the mixture was stirred at room temperature for 16 hr. Water (30 mL) was added and the mixture was extracted with ethyl acetate (50 mL). The organic layer was washed with saturated brine, dried over anhydrous magnesium sulfate, filtered, and concentrated under reduced pressure. The residue was purified by basic silica gel column chromatography (eluent: hexane/ethyl acetate=10 - 60%) to give the title compound as a pale-yellow powder (yield: 46.3 mg, 14%), which was recrystallized from ethyl acetate/n-heptane to give the title compound as colorless crystals (yield: 12.6 mg, 4%).
¹H-NMR (DMSO-d₆, 300 MHz):δ1.11 (3H, t, J = 7.6 Hz), 3.56 (2H, d, J = 7.6 Hz), 3.84 (3H, d, J = 1.5 Hz), 5.24 (2H, s), 6.55 (1H, s), 6.96-7.11 (3H, m), 7.55-7.64 (1H, m), 7.69-7.86 (3H, m), 8.73 (1H, brs), 10.19 (1H, brs).

### Example 23

### (4Z)-1-ethyl-5-imino-4-[(3-methoxy-4-{[4-(trifluoromethyl)benzyl]oxylphenyl)methylidene]-3-[4-(trifluoromethyl)benzyl]imidazolidin-2-one

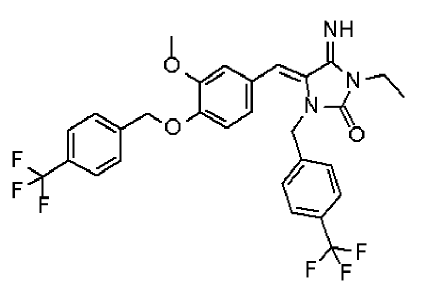

To a solution (5 mL) of (4Z)-1-ethyl-4-[(4-hydroxy-3-methoxyphenyl)methylidene]-5-iminoimidazolidin-2-one (200 mg) in tetrahydrofuran were added [4-(trifluoromethyl)phenyl]methanol (278 mg), diethyl azodicarboxylate (700 µL) and triphenylphosphine (414 mg), and the mixture was stirred at room temperature for 16 hr. Water (30 mL) was added and the mixture was extracted with ethyl acetate (50 mL). The organic layer was washed with saturated brine, dried over anhydrous magnesium sulfate, filtered, and concentrated under reduced pressure. The residue was purified by silica gel column chromatography (eluent: hexane/ethyl acetate=30 - 60%) and basic silica gel column chromatography (eluent: hexane/ethyl acetate=10 - 100%) to give the title compound as a pale-yellow powder (yield: 136 mg, 31%), which was recrystallized from ethyl acetate/n-heptane to give the title compound as a white powder (yield: 86.7 mg, 20%).
¹H-NMR (DMSO-d₆, 300 MHz):δ1.18 (3H, t, J = 6.9 Hz), 3.55 (3H, s), 3.66 (2H, q, J = 6.9 Hz), 4.69 (2H, brs), 5.25 (2H, s), 6.48-6.63 (2H, m), 6.66-6.81 (3H, m), 6.89 (1H, d, J = 8.3 Hz), 7.39 (2H, d, J = 8.1 Hz), 7.68 (2H, d, J = 8.3 Hz), 7.79 (2H, d, J = 8.3 Hz), 9.03 (1H, brs).

### Example 24

### 4-[(Z)-(1-ethyl-5-imino-2-oxoimidazolidin-4-ylidene)methyl]-2-methoxyphenyl 4-methylbenzenesulfonate

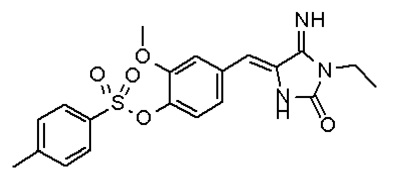

To a solution (4 mL) of (4Z)-1-ethyl-4-[(4-hydroxy-3-methoxyphenyl)methylidene]-5-iminoimidazolidin-2-one (300 mg) in pyridine was added 4-methylbenzenesulfonyl chloride (271 mg), and the mixture was stirred at room temperature for 16 hr. Saturated aqueous ammonium chloride (30 mL) was added and the mixture was extracted with ethyl acetate (50 mL). The organic layer was washed with water and saturated brine, dried over anhydrous magnesium sulfate, filtered, and concentrated under reduced pressure. The residue was washed with ethyl acetate to give the title compound as a pale-yellow powder (yield: 129 mg, 27%).
¹H-NMR (DMSO-d₆, 300 MHz):δ1.10 (3H, t, J = 7.1 Hz), 2.43 (3H, s), 3.51-3.61 (2H, m), 3.57 (3H, s), 6.54 (1H, s), 6.98-7.10 (3H, m), 7.47 (2H, d, J = 8.1 Hz), 7.71 (2H, d, J = 8.1 Hz), 8.86 (1H, brs), 10.36 (1H, brs).

### Example 25

### 4-{4-[(Z)-(1-ethyl-5-imino-2-oxoimidazolidin-4-ylidene)methyl]-2-methoxyphenoxy}-3-(trifluoromethyl)benzonitrile

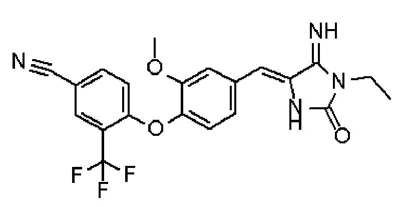

To a solution (3 mL) of (4Z)-1-ethyl-4-[(4-hydroxy-3-methoxyphenyl)methylidene]-5-iminoimidazolidin-2-one (200 mg) in DMF were added 4-fluoro-3-(trifluoromethyl)benzonitrile (149 mg) and cesium carbonate (788 mg), and the mixture was stirred at 80°C for 20 hr. Water (30 mL) was added at room temperature, and the mixture was extracted with ethyl acetate (50 mL). The organic layer was washed with water and saturated brine, dried over anhydrous magnesium sulfate, filtered, and concentrated under reduced pressure. The residue was purified by basic silica gel column chromatography (eluent: hexane/ethyl acetate=30 - 50%) to give the title compound as a yellow powder (yield: 85.7 mg, 26%), which was recrystallized from ethyl acetate/n-heptane to give the title compound as a yellow powder (yield: 62.4 mg, 19%).
¹H-NMR (DMSO-d₆, 300 MHz):δ1.13 (3H, t, J = 6.9 Hz), 3.57 (2H, q, J = 6.9 Hz), 3.80 (3H, s), 6.65 (1H, brs), 6.84 (1H, d, J = 8.7 Hz), 7.09-7.35 (3H, m), 8.03 (1H, dd, J = 8.7, 2.0 Hz), 8.31 (1H, d, J = 2.0 Hz), 8.88 (1H, brs), 10.38 (1H, brs).

### Example 26

### 4-{4-[(Z)-(1-ethyl-5-imino-2-oxoimidazolidin-4-ylidene)methyl]-2-methoxyphenoxy}naphthalene-1-carbonitrile

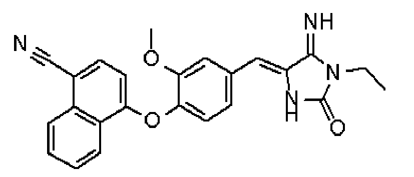

To a solution (3 mL) of (4Z)-1-ethyl-4-[(4-hydroxy-3-methoxyphenyl)methylidene]-5-iminoimidazolidin-2-one (200 mg) in DMSO were added 4-fluoronaphthalene-1-carbonitrile (131 mg) and lithium carbonate (114 mg), and the mixture was stirred at 80°C for 1 day. Water (30 mL) was added at room temperature, and the mixture was extracted with ethyl acetate (50 mL). The organic layer was washed with 1 mol/L aqueous sodium hydroxide solution, water and saturated brine, dried over anhydrous magnesium sulfate, filtered, and concentrated under reduced pressure. The residue was purified by silica gel column chromatography (eluent: hexane/ethyl acetate=30 - 60%) to give the title compound as a white powder (yield: 26.9 mg, 9%), which was recrystallized from ethyl acetate/n-heptane to give the title compound as colorless crystals (yield: 13.5 mg, 4%). ¹H-NMR (DMSO-d₆, 300 MHz):δ1.14 (3H, brs), 3.58 (2H, q, J = 6.4 Hz), 3.79 (3H, s), 6.58-6.72 (2H, m), 7.13-7.39 (3H, m), 7.75-7.84 (1H, m), 7.85-7.95 (1H, m), 8.04 (1H, dd, J = 8.0, 1.8 Hz), 8.13 (1H, d, J = 8.3 Hz), 8.47 (1H, d, J = 8.3 Hz), 8.89 (1H, brs), 10.39 (1H, brs).

### Example 27

### (4Z)-1-benzyl-4-[(4-{[2,4-bis(trifluoromethyl)benzyl]oxy}-3-methoxyphenyl)methylidene]-5-iminoimidazolidin-2-one

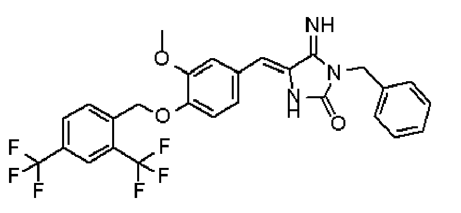

To a solution (10 mL) of 4-{[2,4-bis(trifluoromethyl)benzyl]oxy}-3-methoxybenzaldehyde (680 mg) in ethanol were added 1-benzyl-5-iminoimidazolidin-2-one (284 mg) and piperidine (64 mg), and the mixture was reacted overnight under refluxing conditions. After cooling to room temperature, the precipitate was collected by filtration, and washed with ethanol to give the title compound as white crystals (yield: 630 mg, 64%).
¹H-NMR (DMSO-d₆, 300 MHz):δ3.86 (3H, s), 4.72 (2H, s), 5.36 (2H, s), 6.60 (1H, s), 6.92-7.19 (3H, m), 7.20-7.44 (5H, m), 8.03 (1H, d, J = 8.1 Hz), 8.10 (1H, s), 8.17 (1H, d, J = 8.1 Hz), 8.85 (1H, brs), 10.34 (1H, brs).

### Example 28

### (4Z)-4-[(4-{[2,4-bis(trifluoromethyl)benzyl]oxy}-3-methoxyphenyl)methylidene]-5-iminoimidazolidin-2-one

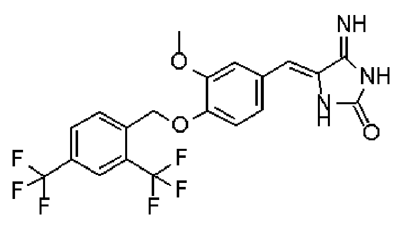

To a solution (10 mL) of 4-{[2,4-bis(trifluoromethyl)benzyl]oxy}-3-methoxybenzaldehyde (757 mg) in ethanol were added 5-imino-1-(phenylcarbonyl)imidazolidin-2-one (406 mg) and piperidine (85 mg), and the mixture was stirred under refluxing conditions for 5 hr. The reaction solution was concentrated under reduced pressure, and the residue was purified by silica gel column chromatography (eluent: ethyl acetate-methanol=100:0→90:10) to give two fractions. The fraction eluted later was concentrated under reduced pressure to give the title compound as a white solid (yield: 80 mg, 9%). The fraction eluted earlier was concentrated under reduced pressure to give (4Z)-4-[(4-{[2,4-bis(trifluoromethyl)benzyl]oxy}-3-methoxyphenyl)methylidene]-5-imino-1-(phenylcarbonyl)imidazolidin-2-one (yield: 30 mg, 3%).
¹H-NMR (DMSO-d₆, 300 MHz):δ3.87 (3H, s), 5.36 (2H, s), 6.47 (1H, s), 6.93-7.15 (3H, m), 7.85-8.26 (5H, m), 9.79 (1H, s).

### Example 29

### (4Z)-4-[(4-{[2,4-bis(trifluoromethyl)benzyl]oxy}-3-methoxyphenyl)methylidene]-5-imino-1-(phenylcarbonyl)imidazolidin-2-one

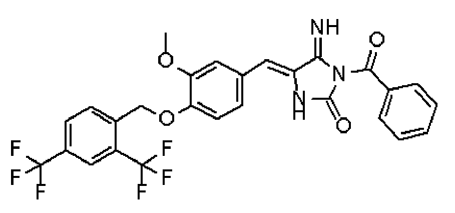

To a solution (10 mL) of 4-{[2,4-bis(trifluoromethyl)benzyl]oxy}-3-methoxybenzaldehyde (757 mg) in ethanol were added 5-imino-1-(phenylcarbonyl)imidazolidin-2-one (406 mg) and piperidine (85 mg), and the mixture was stirred under refluxing conditions for 5 hr. The reaction solution was concentrated under reduced pressure, and the residue was purified by silica gel column chromatography (eluent: ethyl acetate-methanol=100:0→90:10) to give two fractions. The fraction eluted earlier was concentrated under reduced pressure to give the title compound as a white solid (yield: 30 mg, 3%). The fraction eluted later was concentrated under reduced pressure to give (4Z)-4-[(4-{[2,4-bis(trifluoromethyl)benzyl]oxy}-3-methoxyphenyl)methylidene]-5-iminoimidazolidin-2-one (yield: 80 mg, 9%).
¹H-NMR (DMSO-d₆, 300 MHz):δ3.90 (3H, s), 5.40 (2H, s), 7.02 (1H, s), 7.07 (1H, d, J = 8.9 Hz), 7.31 (2H, dd, J = 4.2, 2.5 Hz), 7.48-7.58 (2H, m), 7.58-7.70 (1H, m), 8.04 (1H, d, J = 7.9 Hz), 8.11 (1H, s), 8.15-8.28 (3H, m).

### Example 30

### (4Z)-4-[(4-{[2,4-bis(trifluoromethyl)benzyl]oxy}-3-methoxyphenyl)methylidene]-1-ethyl-5-iminoimidazolidin-2-one

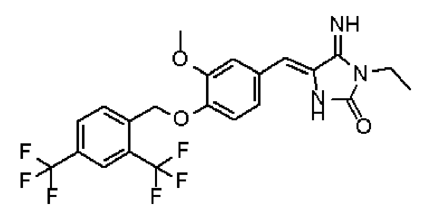

To a solution (10 mL) of 1-(cyanomethyl)-3-ethylurea (373 mg) in ethanol was added potassium tert-butoxide (330 mg), and the mixture was stirred at room temperature for 30 min. 4-{[2,4-bis(Trifluoromethyl)benzyl]oxy}-3-methoxybenzaldehyde (1.13 g) and potassium tert-butoxide (330 mg) were added, and the mixture was stirred under refluxing conditions for 5 hr. After cooling to room temperature, the mixture was concentrated under reduced pressure. The residue was dissolved in ethyl acetate, saturated aqueous ammonium chloride solution was added and the mixture was stirred. The mixture was extracted with ethyl acetate, and the organic layer was washed with saturated brine, dried over anhydrous magnesium sulfate and concentrated under reduced pressure. The residue was purified by silica gel column chromatography (eluent: hexane-ethyl acetate=50:50→100:0) to give the title compound as white crystals (yield: 1.02 g, 70%).
¹H-NMR (DMSO-d₆, 300 MHz):δ1.12 (3H, t, J = 6.5 Hz), 3.56 (2H, q, J = 7.1 Hz), 3.86 (3H, s), 5.35 (2H, s), 6.55 (1H, s), 6.93-7.20 (3H, m), 8.03 (1H, d, J = 8.3 Hz), 8.10 (1H, s), 8.17 (1H, d, J = 8.1 Hz), 8.74 (1H, brs), 10.20 (1H, brs).

### Example 31

### (4Z)-4-[(4-{[2,4-bis(trifluoromethyl)benzyl]oxy}-2-methoxyphenyl)methylidene]-5-iminoimidazolidin-2-one

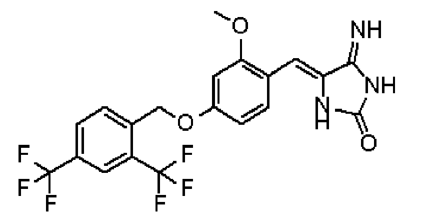

To a solution (5 mL) of 4-{[2,4-bis(trifluoromethyl)benzyl]oxy}-2-methoxybenzaldehyde (38 mg) in ethanol were added 5-imino-1-(phenylcarbonyl)imidazolidin-2-one (20 mg) and potassium tert-butoxide (112 mg), and the mixture was stirred overnight under refluxing conditions. Ethyl acetate and saturated aqueous ammonium chloride solution were added, and the mixture was stirred and extracted with ethyl acetate. The organic layer was washed with saturated brine, dried over anhydrous magnesium sulfate, and concentrated under reduced pressure, and the residue was purified by silica gel column chromatography (eluent: ethyl acetate-methanol=100:0→90:10) to give the title compound as white crystals (yield: 30 mg, 65%).
¹H-NMR (DMSO-d₆, 300 MHz):δ3.83 (3H, s), 5.39 (2H, s), 6.53-6.65 (2H, m), 6.71 (1H, d, J = 2.3 Hz), 7.49 (1H, d, J = 8.5 Hz), 7.90-8.08 (3H, m), 8.08-8.24 (2H, m), 9.47 (1H, s).

### Example 32

### (4Z)-4-[(4-{[2,4-bis(trifluoromethyl)benzyl]oxy}-3-methoxyphenyl)methylidene]-1-ethyl-5-imino-3-methylimidazolidin-2-one

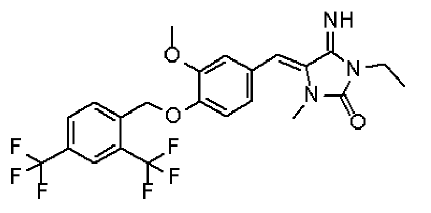

To a solution (3 mL) of (4Z)-4-[(4-{[2,4-bis(trifluoromethyl)benzyl]oxy}-3-methoxyphenyl)methylidene]-1-ethyl-5-iminoimidazolidin-2-one (97 mg) in anhydrous tetrahydrofuran was added potassium tert-butoxide (22 mg) at 0°C. After stirring at room temperature for 30 min, methyl iodide (28 mg) was added, and the mixture was stirred at room temperature for 5 hr. Ethyl acetate and saturated aqueous ammonium chloride solution were added, and the mixture was stirred and extracted with ethyl acetate. The organic layer was washed with saturated brine, dried over anhydrous magnesium sulfate, and concentrated under reduced pressure, and the residue was purified by basic silica gel column chromatography (eluent: hexane-ethyl acetate=50:50→80:20), and recrystallized from heptane to give the title compound as white crystals (yield: 40 mg, 40%).
¹H-NMR (DMSO-d₆, 300 MHz):δ1.11 (3H, t, J = 7.0 Hz), 2.79 (3H, s), 3.58 (2H, q, J = 7.0 Hz), 3.81 (3H, s), 5.34 (2H, s), 6.78 (1H, s), 6.85 (1H, dd, J = 8.2, 1.4 Hz), 6.96 (1H, s), 7.03 (1H, d, J = 8.3 Hz), 8.05 (1H, d, J = 8.1 Hz), 8.10 (1H, s), 8.18 (1H, d, J = 8.1 Hz), 8.90 (1H, brs).

### Example 33

### (4Z,5E)-4-[(4-{[2,4-bis(trifluoromethyl)benzyl]oxy}-3-methoxyphenyl)methylidene]-1-ethyl-5-(methylimino)imidazolidin-2-one

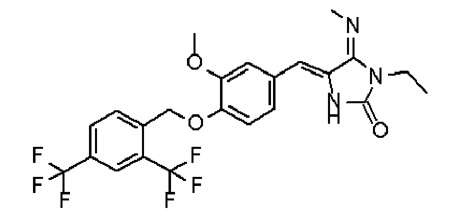

To a solution (5 mL) of (4Z)-4-[(4-{[2,4-bis(trifluoromethyl)benzyl]oxy}-3-methoxyphenyl)methylidene]-1-ethyl-5-iminoimidazolidin-2-one (97 mg) in ethanol was added methylamine hydrochloride (200 mg), and the mixture was stirred under refluxing conditions for 2 hr, and concentrated under reduced pressure. Ethyl acetate and saturated sodium hydrogen carbonate solution were added, and the mixture was stirred and extracted with ethyl acetate. The organic layer was washed with saturated brine, dried over anhydrous magnesium sulfate, and concentrated under reduced pressure, and the residue was purified by silica gel column chromatography to give the title compound as a white solid (yield: 60 mg, 60%).
¹H-NMR (DMSO-d₆, 300 MHz):δ1.06 (3H, t, J = 7.1 Hz), 3.42 (3H, s), 3.50 (2H, q, J = 7.0 Hz), 3.85 (3H, s), 5.36 (2H, s), 6.48 (1H, s), 6.98-7.04 (1H, m), 7.04-7.12 (1H, m), 7.15 (1H, d, J = 1.7 Hz), 8.03 (1H, d, J = 7.9 Hz), 8.10 (1H, s), 8.17 (1H, d, J = 7.9 Hz), 10.02 (1H, s).

### Example 34

### (4Z,5E)-5-(benzylimino)-4-[(4-{[2,4-bis(trifluoromethyl)benzyl]oxy}-3-methoxyphenyl)methylidene]-1-ethylimidazolidin-2-one

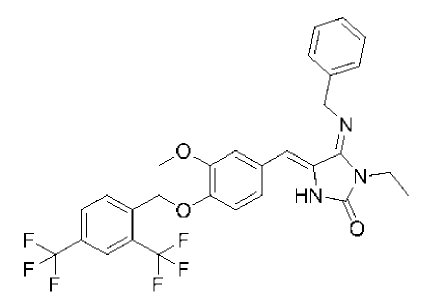

To a solution (3 mL) of (4Z)-4-[(4-{[2,4-bis(trifluoromethyl)benzyl]oxy}-3-methoxyphenyl)methylidene]-1-ethyl-5-iminoimidazolidin-2-one (97 mg) in ethanol was added benzylamine hydrochloride (287 mg), and the mixture was stirred overnight under refluxing conditions, and concentrated under reduced pressure. Ethyl acetate and saturated aqueous sodium hydrogen carbonate solution were added, and the mixture was stirred and extracted with ethyl acetate. The organic layer was washed with saturated brine, dried over anhydrous magnesium sulfate, and concentrated under reduced pressure, and the residue was purified by silica gel column chromatography (eluent: hexane-ethyl acetate=50:50→80:20) to give the title compound as a white solid (yield: 51 mg, 44%). ¹H-NMR (DMSO-d₆, 300 MHz):δ1.14 (3H, t, J = 7.0 Hz), 3.52-3.69 (2H, m), 3.86 (3H, s), 4.89 (2H, s), 5.37 (2H, s), 6.56 (1H, s), 6.99-7.07 (1H, m), 7.06-7.15 (1H, m), 7.17 (1H, s), 7.21-7.31 (1H, m), 7.37 (2H, t, J = 7.6 Hz), 7.44-7.59 (2H, m), 8.04 (1H, d, J = 8.3 Hz), 8.11 (1H, s), 8.17 (1H, s), 10.10 (1H, brs).

### Example 35

### (5Z)-1-benzyl-5-[(4-{[2,4-bis(trifluoromethyl)benzyl]oxy}-3-methoxyphenyl)methylidene]-3-ethyl-4-iminoimidazolidin-2-one

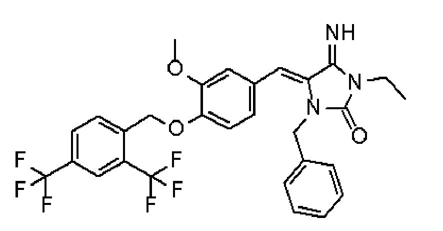

To a solution (3 mL) of (4Z)-4-[(4-{[2,4-bis(trifluoromethyl)benzyl]oxy}-3-methoxyphenyl)methylidene]-1-ethyl-5-iminoimidazolidin-2-one (146 mg) in anhydrous tetrahydrofuran were added benzyl bromide (77 mg) and potassium tert-butoxide (40 mg), and the mixture was stirred at room temperature overnight. Ethyl acetate and saturated aqueous ammonium chloride solution were added, and the mixture was stirred and extracted with ethyl acetate. The organic layer was washed with saturated brine, dried over anhydrous magnesium sulfate, and concentrated under reduced pressure, and the residue was purified by basic silica gel column chromatography (eluent: hexane-ethyl acetate=50:50→80:20) to give the title compound as a white solid (yield: 130 mg, 75%). ¹H-NMR (DMSO-d₆, 300 MHz):δ1.16 (3H, s), 3.57 (3H, s), 3.59-3.75 (2H, m), 4.62 (2H, s), 5.35 (2H, s), 6.42-6.69 (4H, m), 6.75 (1H, s), 6.92 (1H, d, J = 8.3 Hz), 6.99-7.24 (3H, m), 8.05 (1H, d, J = 8.3 Hz), 8.10 (1H, s), 8.13-8.25 (1H, m), 8.98 (1H, s).

The LC-MS analysis in the following Examples 36 - 81 was performed based on the measurement of the following 4ch-LC/MS (MUX).

### 4ch-LC/MS (MUX)

measurement device: Waters LC-MS system
HPLC moiety: Waters 1525 binary gradient pump, 2488 muitichannel absorbance detector, 2777 sample manager
MS moiety: Micromass ZQ2000, Waters MUX interface

### HPLC conditions

column: CAPCELL PAK C18UG120, S-3 µm, 1.5 x 35 mm (Shiseido Co., Ltd.)
solvent: SOLUTION A; 5 mM ammonium acetate-containing 2% aqueous acetonitrile, SOLUTION B; 5 mM ammonium acetate-containing 95% aqueous acetonitrile
gradient cycle: 0.00 min (SOLUTION A /SOLUTION B=100/0), 4.00 min (SOLUTION A/SOLUTION B=0/100), 5.00 min (SOLUTION A/SOLUTION B=0/100), 5.01 min (SOLUTION A/SOLUTION B=100/0), 5.30 min (SOLUTION A/SOLUTION B=100/0)
injection volume: 2 µL, flow rate: 0.5 mL/min, detection method: UV 220 nm

### MS conditions

ionization method: ESI

The purification by preparative HPLC in the following Examples 36 - 81 was performed under the following conditions.
device: Gilson Inc. High-throughput purification system
column: CombiPrep Pro C18 RS S-5 µm, 20 x 50 mm (YMC)
solvent: SOLUTION A; 0.1% trifluoroacetic acid-containing water, SOLUTION B; 0.1% trifluoroacetic acid-containing acetonitrile
gradient cycle: 0.00 min (SOLUTION A/SOLUTION B=95/5), 1.00 min (SOLUTION A/SOLUTION B=95/5), 5.20 min (SOLUTION A/SOLUTION B=0/100), 6.40 min (SOLUTION A/SOLUTION B=0/100), 6.50 min (SOLUTION A/SOLUTION B=95/5), 6.60 min (SOLUTION A/SOLUTION B=95/5)
flow rate: 20 mL/min, detection method: UV 220 nm

### Example 36

### (5Z)-5-[(4-{[2,4-bis(trifluoromethyl)benzyl]oxy}-3-methoxyphenyl)methylidene]-4-(butylamino)-1,3-thiazol-2(5H)-one

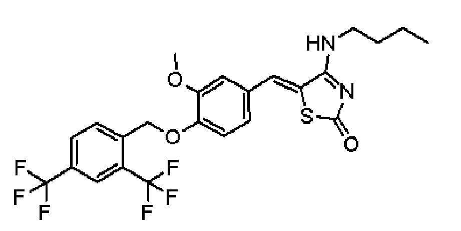

A 0.05 mol/L solution (1.6 mL, 80 µmol) of (5Z)-5-[(4-{[2,4-bis(trifluoromethyl)benzyl]oxy}-3-methoxyphenyl)methylidene]-4-thioxo-1,3-thiazolidin-2-one in methanol and n-butylamine (36 mg, 500 µmol) were mixed and the mixture was stirred at 100°C for 1.5 hr under microwave irradiation. The reaction mixture was extracted with ethyl acetate (3.5 mL) and 2% aqueous sodium hydrogen carbonate solution (1 mL), and the organic layer was separated by upper layer Phase Septube (manufactured by Wako Pure Chemical Industries, Ltd.). The solvent was evaporated under reduced pressure, the residue was dissolved in DMSO-MeOH (1:1) (1 mL), and the mixture was purified by preparative HPLC to give the title compound.
yield: 1.4 mg
molecular ion peak: 533
LC-MS analysis: purity 100%
retention time: 3.99 min

### Examples 37 - 81

Using 0.05 mol/L solution (1.6 mL, 80 µmol) of (5Z)-5-[(4-{[2,4-bis(trifluoromethyl)benzyl]oxy}-3-methoxyphenyl)methylidene]-4-thioxo-1,3-thiazolidin-2-one in methanol and various corresponding amines (500 µmol), an operation similar to that in Example 36 was performed to synthesize the compounds of Example Nos. 37 - 81 shown in the following Table 1.

**[Table 1-1]**

| 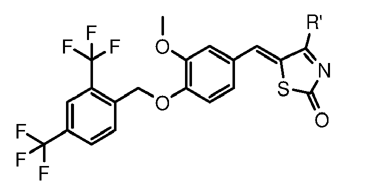 | | | | | |
|---|---|---|---|---|---|
| Ex. No. | R' | yield (mg) | molecular ion peak | purity (%) | retention time (min) |
| 37 | 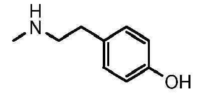 | 17.0 | 597 | 100% | 3.82 |
| 38 | 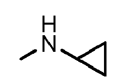 | 16.9 | 517 | 95% | 3.74 |
| 39 | 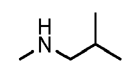 | 16.3 | 533 | 97% | 3.98 |
| 40 | 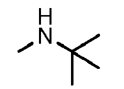 | 9.0 | 533 | 70% | 3.99 |
| 41 | 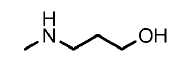 | 15.7 | 535 | 100% | 3.62 |
| 42 | 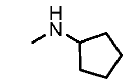 | 14.7 | 545 | 91% | 3.99 |
| 43 | 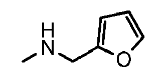 | 16.8 | 557 | 97% | 3.93 |
| 44 | 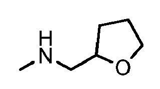 | 20.2 | 561 | 92% | 3.85 |
| 45 | 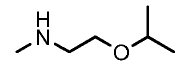 | 21.6 | 563 | 100% | 3.93 |
| 46 | 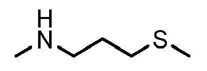 | 16.9 | 565 | 95% | 3.96 |
| 47 | 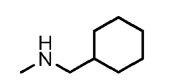 | 14.6 | 573 | 94% | 4.16 |

**[Table 1-2]**

| Ex. No. | R' | yield (mg) | molecular ion peak | purity (%) | retention time (min) |
|---|---|---|---|---|---|
| 48 | 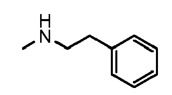 | 15.1 | 581 | 94% | 4.02 |
| 49 | 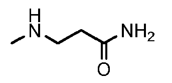 | 9.2 | 548 | 100% | 3.57 |
| 50 | 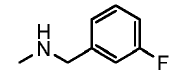 | 14.9 | 585 | 100% | 4.02 |
| 51 | 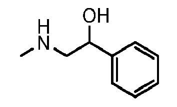 | 8.6 | 597 | 100% | 3.84 |
| 52 | 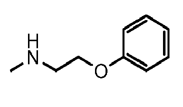 | 10.5 | 597 | 100% | 4.03 |
| 53 | 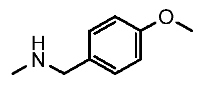 | 8.1 | 597 | 100% | 3.99 |
| 54 | 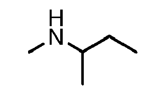 | 11.3 | 533 | 97% | 3.93 |
| 55 | 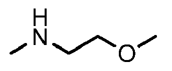 | 17.9 | 535 | 100% | 3.79 |
| 56 | 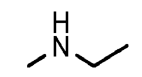 | 1.1 | 505 | 100% | 3.79 |
| 57 | 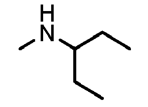 | 7.5 | 547 | 88% | 3.97 |
| 58 | 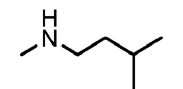 | 17.8 | 547 | 100% | 4.06 |
| 59 | 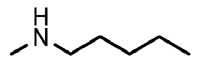 | 17.6 | 547 | 100% | 4.09 |

**[Table 1-3]**

| Ex. No. | R' | yield (mg) | molecular ion peak | purity (%) | retention time (min) |
|---|---|---|---|---|---|
| 60 | 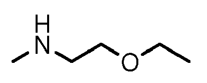 | 17.1 | 549 | 100% | 3.87 |
| 61 | 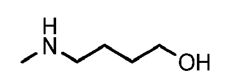 | 16.1 | 549 | 100% | 3.63 |
| 62 | 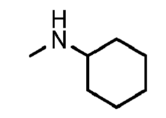 | 13.6 | 559 | 88% | 4.06 |
| 63 | 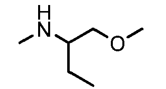 | 12.7 | 563 | 89% | 3.92 |
| 64 | 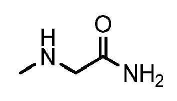 | 1.4 | 534 | 100% | 3.56 |
| 65 | 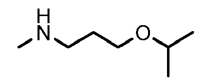 | 18.8 | 577 | 96% | 3.99 |
| 66 | 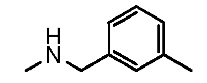 | 14.2 | 581 | 100% | 4.07 |
| 67 | 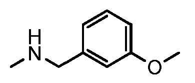 | 17.0 | 597 | 100% | 3.98 |
| 68 | 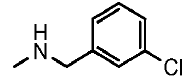 | 18.2 | 601 | 96% | 4.05 |
| 69 | 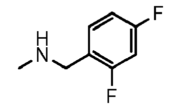 | 12.2 | 603 | 100% | 4.01 |
| 70 | 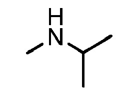 | 12.9 | 519 | 100% | 3.92 |
| 71 | 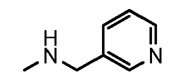 | 3.8 | 568 | 100% | 3.73 |

**[Table 1-4]**

| Ex. No. | R' | yield (mg) | molecular ion peak | purity (%) | retention time (min) |
|---|---|---|---|---|---|
| 72 | 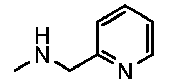 | 3.8 | 568 | 93% | 3.79 |
| 73 | 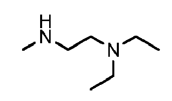 | 3.7 | 576 | 90% | 3.56 |
| 74 | 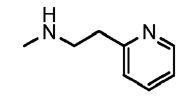 | 20.6 | 582 | 100% | 3.81 |
| 75 | 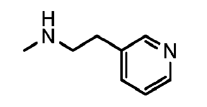 | 21.2 | 582 | 100% | 3.77 |
| 76 | 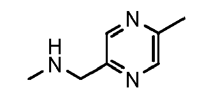 | 16.0 | 583 | 100% | 3.75 |
| 77 | 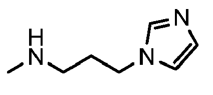 | 5.9 | 585 | 92% | 3.62 |
| 78 | 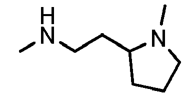 | 2.9 | 588 | 100% | 3.54 |
| 79 | 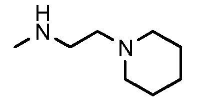 | 3.1 | 588 | 90% | 3.64 |
| 80 | 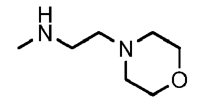 | 3.8 | 590 | 100% | 3.69 |
| 81 | 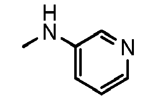 | 2.7 | 554 | 100% | 3.81 |

### Example 82

### (5Z)-5-[(4-{[2,4-bis(trifluoromethyl)benzyl]oxy}-3-methoxyphenyl)methylidene]-4-(dimethylamino)-1,3-thiazol-2(5H)-one

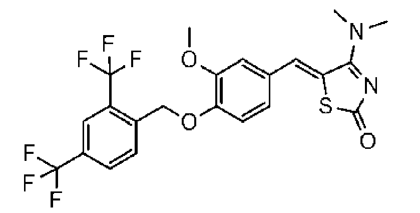

To a solution (1.5 mL) of (5Z)-5-[(4-{[2,4-bis(trifluoromethyl)benzyl]oxy}-3-methoxyphenyl)methylidene]-4-(methylamino)-1,3-thiazol-2(5H)-one (147.5 mg) and potassium carbonate (62.4 mg) in N,N-dimethylformamide was added methyl iodide (0.0624 mL), and the mixture was stirred at room temperature for 3 hr. Methyl iodide (0.0624 mL) was added and the mixture was stirred for 2 hr. Methyl iodide (0.0624 mL) was added, and the mixture was stirred at 60°C for 2 hr. The reaction mixture was poured into water, and the mixture was extracted with ethyl acetate. The organic layer was washed with water and saturated brine, dried over magnesium sulfate, and concentrated under reduced pressure. The residue was purified by silica gel column chromatography (eluent: hexane-ethyl acetate=1:0→1:9) to give two kinds of compounds. A highly-polar compound was recrystallized from ethyl acetate/hexane to give the title compound as pale-yellow crystals (yield: 41.1 mg, 27%). In addition, a less polar compound was recrystallized from diethylether/hexane to give (4E,5Z)-5-[(4-{[2,4-bis(trifluoromethyl)benzyl]oxy}-3-methoxyphenyl)methylidene]-3-methyl-4-(methylimino)-1,3-thiazolidin-2-one as pale-yellow crystals (yield: 18.7 mg, 12%).
¹H-NMR (CDCl₃,300 MHz):δ3.49 (6H, s), 3.97 (3H, s), 5.43 (2H, s), 6.88 (1H, d, J = 8.5 Hz), 7.03 (1H, dd, J = 8.5, 2.3 Hz), 7.15 (1H, d, J = 2.3 Hz), 7.29 (1H, s), 7.85 (1H, d, J = 8.3 Hz), 7.95 (1H, s), 7.98 (1H, d, J = 8.3 Hz).

### Example 83

### (5Z)-5-{[4-{[2,4-bis(trifluoromethyl)benzyl]oxy}-3-(trifluoromethyl)phenyl]methylidene}-4-(methylamino)-1,3-thiazol-2(5H)-one

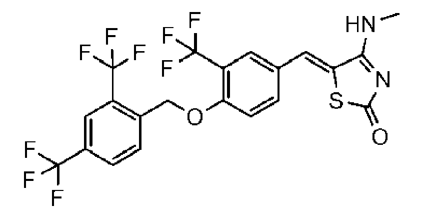

4-{[2,4-bis(Trifluoromethyl)benzyl]oxy}-3-(trifluoromethyl)benzaldehyde (259.5 mg) and 4-(methylamino)-1,3-thiazol-2(5H)-one (89.3 mg) were dissolved in ethanol (3.1 mL), potassium tert-butoxide (83.9 mg) was added, and the mixture was stirred with heating under reflux for 5 hr. The reaction mixture was poured into water, and the mixture was extracted with ethyl acetate. The organic layer was washed with saturated brine, dried over magnesium sulfate, and concentrated under reduced pressure. The residue was purified by silica gel column chromatography (eluent: hexane-ethyl acetate=1:0→1:4) and recrystallized from acetone/water to give the title compound as a pale-yellow powder (yield: 19.3 mg, 6%).
¹H-NMR (DMSO-d₆, 300 MHz):δ3.06 (3H, d, J = 4.5 Hz), 5.57 (2H, s), 7.57 (1H, d, J = 8.7 Hz), 7.75-7.85 (3H, m), 8.02 (1H, d, J = 8.1 Hz), 8.14 (1H, s), 8.23 (1H, d, J = 8.1 Hz), 9.42 (1H, d, J = 4.5 Hz).

### Example 84

### 4-(2-methoxy-4-{(Z)-[4-(methylamino)-2-oxo-1,3-thiazol-5(2H)-ylidene]methyl}phenoxy)naphthalene-1-carbonitrile

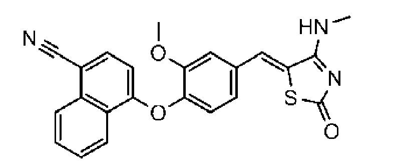

To a solution (4 mL) of 4-(methylamino)-1,3-thiazol-2(5H)-one (179 mg) in anhydrous N,N-dimethylformamide was added a solution (2 mL) of potassium tert-butoxide (399 mg) and 4-(4-formyl-2-methoxyphenoxy)naphthalene-1-carbonitrile (500 mg) in anhydrous N,N-dimethylformamide and the mixture was stirred at room temperature for 2 hr under a nitrogen atmosphere. To the reaction mixture was added saturated aqueous ammonium chloride solution, and the mixture was extracted with ethyl acetate. The organic layer was washed with saturated brine, dried over anhydrous magnesium sulfate, and filtered, and the filtrate was concentrated under reduced pressure. The residue was washed with ethyl acetate to give 4-(4-{hydroxy[4-(methylamino)-2-oxo-2,5-dihydro-1,3-thiazol-5-yl]methyl}-2-methoxyphenoxy)naphthalene-1-carbonitrile as a yellow solid (yield: 209 mg, 35%).
¹H-NMR (DMSO-d₆, 300 MHz):δ2.98 (3H, d, J = 4.5 Hz), 3.73 (3H, s), 5.05 (1H, s), 5.43 (1H, dd, J = 5.8, 2.0 Hz), 5.96 (1H, d,
J = 5.9 Hz), 6.52 (1H, d, J = 8.1 Hz), 7.10 (1H, d, J = 8.3 Hz), 7.24-7.29 (2H, m), 7.75-7.83 (1H, m), 7.85-7.93 (1H, m), 8.03 (1H, d, J = 8.3 Hz), 8.12 (1H, d, J = 8.3 Hz), 8.47 (1H, d, J = 8.3 Hz), 9.07 (1H, d, J = 4.5 Hz).

A mixed solution of 4-(4-{hydroxy[4-(methylamino)-2-oxo-2,5-dihydro-1,3-thiazol-5-yl]methyl}-2-methoxyphenoxy)naphthalene-1-carbonitrile (150 mg) and 4 M hydrogen chloride/ethyl acetate solution (4 mL) was heated under reflux for 2 hr. The reaction mixture was concentrated, saturated aqueous sodium hydrogen carbonate solution was added, and the mixture was extracted with ethyl acetate/tetrahydrofuran. The organic layer was washed with saturated brine, dried over anhydrous magnesium sulfate, and filtered, and the filtrate was concentrated under reduced pressure. The residue was recrystallized from tetrahydrofuran/n-heptane to give the title compound as a yellow solid (yield: 93 mg, 65%).
¹H-NMR (DMSO-d₆, 300 MHz):δ3.08 (3H, d, J = 3.6 Hz), 3.80 (3H, s), 6.70 (1H, d, J = 8.1 Hz), 7.25 (1H, d, J = 8.3 Hz), 7.32 (1H, s), 7.42 (1H, d, J = 8.3 Hz), 7.75-7.86 (2H, m), 7.85-7.96 (1H, m), 8.03 (1H, d, J = 8.1 Hz), 8.14 (1H, d, J = 8.1 Hz), 8.44 (1H, d, J = 8.3 Hz), 9.50 (1H, d, J = 3.6 Hz).

### Example 85

### 4-{4-[(Z)-(4-amino-2-oxo-1,3-thiazol-5(2H)-ylidene)methyl]-2-methoxyphenoxy}naphthalene-1-carbonitrile

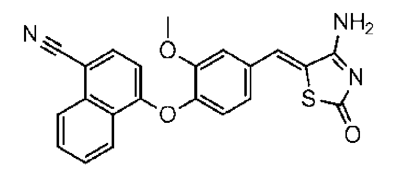

To a solution (3 mL) of 4-amino-1,3-thiazol-2(5H)-one (63.8 mg) in anhydrous N,N-dimethylformamide was added potassium tert-butoxide (218 mg) and the mixture was stirred at room temperature for 0.5 hr under a nitrogen atmosphere. 4-(4-Formyl-2-methoxyphenoxy)naphthalene-1-carbonitrile (200 mg) was added, and the mixture was stirred at room temperature for 7 hr. To the reaction mixture was added saturated aqueous ammonium chloride solution, and the mixture was extracted with ethyl acetate/tetrahydrofuran. The organic layer was washed with water and saturated brine, dried over anhydrous magnesium sulfate, and filtered, and the filtrate was concentrated under reduced pressure. To the residue was added 4 M hydrogen chloride/ethyl acetate solution (4 mL), and the mixture was heated under reflux for 3 hr. To the reaction mixture was added 1 N aqueous sodium hydroxide solution, and the mixture was extracted with ethyl acetate. The organic layer was washed with water and saturated brine, dried over anhydrous magnesium sulfate, and filtered, and the filtrate was concentrated under reduced pressure. The residue was recrystallized from tetrahydrofuran/n-heptane to give the title compound as a brown solid (yield: 59 mg, 27%).
¹H-NMR (DMSO-d₆, 300 MHz):δ3.80 (3H, s), 6.70 (1H, d, J = 8.3 Hz), 7.26 (1H, dd, J = 8.3, 1.7 Hz), 7.34 (1H, d, J = 1.7 Hz), 7.43 (1H, d, J = 8.3 Hz), 7.76-7.84 (1H, m), 7.86-7.95 (2H, m), 8.04 (1H, d, J = 8.1 Hz), 8.14 (1H, d, J = 8.3 Hz), 8.45 (1H, d, J = 8.3 Hz), 9.03 (1H, brs), 9.25 (1H, brs).

### Example 86

### (5Z)-5-[(4-{[2,4-bis(trifluoromethyl)benzyl]oxy}-3-methoxyphenyl)methylidene]-4-[(2-{piperidin-1-yl}ethyl)amino]-1,3-thiazol-2(5H)-one

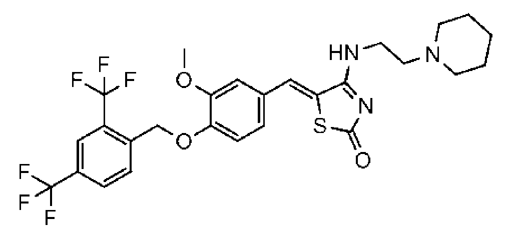

To a solution (2 mL) of (5Z)-5-[(4-{[2,4-bis(trifluoromethyl)benzyl]oxy}-3-methoxyphenyl)methylidene]-4-thioxo-1,3-thiazolidin-2-one (200 mg) in methanol was added a solution (2 mL) of 2-(piperidin-1-yl)ethanamine (265 mg) in methanol, and the mixture was stirred at room temperature for 16 hr. The reaction mixture was concentrated under reduced pressure, and the residue was purified by basic silica gel column chromatography (eluent: hexane-ethyl acetate=50:50→0:100) to give the title compound as a yellow solid (yield: 134 mg, 56%). The obtained solid was recrystallized from ethyl acetate/n-heptane to give the title compound as a pale-yellow solid (yield: 108 mg, 46%).
¹H-NMR (DMSO-d₆, 300 MHz):δ1.33-1.43 (2H, m), 1.44-1.55 (4H, m), 2.34-2.44 (4H, m), 2.47-2.57 (2H, m), 3.60 (2H, t, J = 6.9 Hz), 3.85 (3H, s), 5.41 (2H, s), 7.10-7.16 (2H, m), 7.18-7.24 (1H, m), 7.78 (1H, s), 8.03 (1H, d, J = 8.1 Hz), 8.11 (1H, s), 8.18 (1H, d, J = 8.1 Hz), 9.29 (1H, brs).

### Example 87

### (5Z)-5-[(4-{[2,4-bis(trifluoromethyl)benzyl]oxy}-3-methoxyphenyl)methylidene]-4-{[2-(1-methylpyrrolidin-2-yl)ethyl]aminol-1,3-thiazol-2(5H)-one

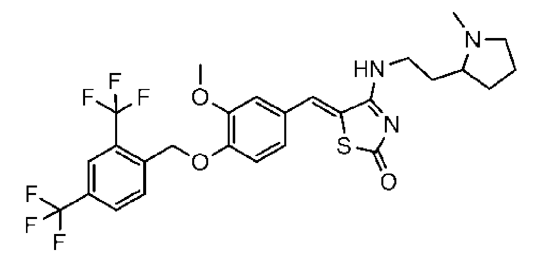

To a solution (3.1 mL) of 4-thioxo-1,3-thiazolidin-2-one (91.4 mg) in ethanol was added 2-(1-methylpyrrolidin-2-yl)ethanamine (0.109 mL), and the mixture was stirred at room temperature for 3 hr. To the reaction mixture were added 4-{[2,4-bis(trifluoromethyl)benzyl]oxy}-2-methoxybenzaldehyde (235.9 mg) and potassium tert-butoxide (84.0 mg), and the mixture was stirred with heating under reflux for 4 hr. The reaction mixture was concentrated, and the residue was purified by basic silica gel column chromatography (eluent: hexane-ethyl acetate=3:7→0:1) and recrystallized from acetone/diethylether to give the title compound as a pale-yellow powder (yield: 100.5 mg, 27%).
¹H-NMR (DMSO-d₆,300 MHz):δ1.36-1.76 (4H, m), 1.86-2.01 (2H, m), 2.02-2.18 (2H, m), 2.24 (3H, s), 2.91-3.00 (1H, m), 3.52 (2H, t, J = 7.4 Hz), 3.86 (2H, s), 5.41 (3H, s), 7.10-7.24 (3H, m), 7.73 (1H, s), 8.04 (1H, d, J = 8.5 Hz), 8.11 (1H, s), 8.18 (1H, d, J = 8.5 Hz), 9.35 (1H, brs).

### Example 88

### (5Z)-5-[(4-{[2,4-bis(trifluoromethyl)benzyl]oxy}-3-methoxyphenyl)methylidene]-4-{[3-(1H-imidazol-1-yl)propyl]amino}-1,3-thiazol-2(5H)-one

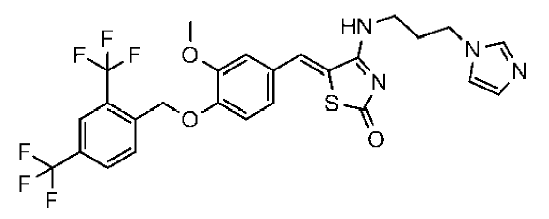

To a solution (2 mL) of (5Z)-5-[(4-{[2,4-bis(trifluoromethyl)benzyl]oxy}-3-methoxyphenyl)methylidene]-4-thioxo-1,3-thiazolidin-2-one (200 mg) in methanol was added a solution (2 mL) of 3-(1H-imidazol-1-yl)propan-1-amine (256 mg) in methanol, and the mixture was stirred at room temperature for 15 hr. The reaction mixture was concentrated under reduced pressure, and the residue was purified by basic silica gel column chromatography (eluent: ethyl acetate-methanol=100:0→90:10) to give the title compound as a yellow solid (yield: 213 mg, 90%). The obtained solid was recrystallized from ethyl acetate to give the title compound as a pale-yellow solid (yield: 26 mg, 11%).
¹H-NMR (DMSO-d₆, 300 MHz):δ2.08 (2H, tt, J = 6.9, 6.8 Hz), 3.46 (2H, t, J = 6.8 Hz), 3.85 (3H, s), 4.07 (2H, t, J = 6.9 Hz), 5.41 (2H, s), 6.90 (1H, s), 7.12-7.17 (2H, m), 7.19-7.25 (2H, m), 7.68 (1H, s), 7.75 (1H, s), 8.03 (1H, d, J = 8.1 Hz), 8.11 (1H, s), 8.18 (1H, d, J = 8.1 Hz), 9.32 (1H, brs).

### Example 89

### (5Z)-5-[(4-{[2,4-bis(trifluoromethyl)benzyl]oxy}-3-methoxyphenyl)methylidene]-4-{[2-(dimethylamino)ethyl]amino}-1,3-thiazol-2(5H)-one

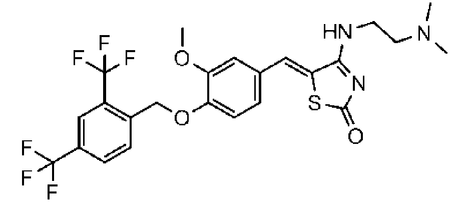

To a solution (2 mL) of (5Z)-5-[(4-{[2,4-bis(trifluoromethyl)benzyl]oxy}-3-methoxyphenyl)methylidene]-4-thioxo-1,3-thiazolidin-2-one (200 mg) in methanol was added a solution (2 mL) of N,N-dimethylethane-1,2-diamine (184 mg) in methanol, and the mixture was stirred at room temperature for 24 hr. The reaction mixture was concentrated under reduced pressure, and the residue was purified by basic silica gel column chromatography (eluent: hexane-ethyl acetate=50:50→0:100) to give the title compound as a yellow solid (yield: 122 mg, 55%). The obtained solid was recrystallized from ethyl acetate/n-heptane to give the title compound as a pale-yellow solid (yield: 89 mg, 40%).
¹H-NMR (DMSO-d₆, 300 MHz):δ2.20 (6H, s), 2.47-2.54 (2H, m), 3.59 (2H, t, J = 6.3 Hz), 3.85 (3H, s), 5.41 (2H, s), 7.11-7.17 (2H, m), 7.18-7.25 (1H, m), 7.79 (1H, s), 8.03 (1H, d, J = 8.1 Hz), 8.11 (1H, s), 8.18 (1H, d, J = 8.1 Hz), 9.26 (1H, brs).

### Example 90

### 4-{4-[(Z)-(4-amino-2-oxo-1,3-thiazol-5(2H)-ylidene)methyl]-2-methoxyphenoxy}-3-(trifluoromethyl)benzonitrile

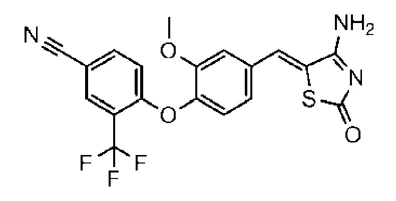

To a solution (8 mL) of 4-amino-1,3-thiazol-2(5H)-one (181 mg) in anhydrous N,N-dimethylformamide was added potassium tert-butoxide (616 mg) and the mixture was stirred at room temperature for 0.5 hr under a nitrogen atmosphere. To the reaction mixture was added 4-(4-formyl-2-methoxyphenoxy)-3-(trifluoromethyl)benzonitrile (500 mg), and the mixture was stirred at room temperature for 6 hr. To the reaction mixture was added saturated aqueous ammonium chloride solution, and the mixture was extracted with ethyl acetate. The organic layer was washed with saturated brine, dried over anhydrous magnesium sulfate, and filtered, and the filtrate was concentrated under reduced pressure. To the residue was added 4 M hydrogen chloride/ethyl acetate solution (5 mL), and the mixture was heated under reflux for 2 hr. The reaction solution was concentrated under reduced pressure, saturated aqueous sodium hydrogen carbonate solution was added, and the mixture was extracted with ethyl acetate. The organic layer was washed with saturated brine, dried over anhydrous magnesium sulfate, and filtered, and the filtrate was concentrated under reduced pressure. The residue was purified by basic silica gel column chromatography (eluent: ethyl acetate-methanol=100:0→90:10) to give the title compound as a pale-yellow solid (yield: 242 mg, 37%). The obtained solid was recrystallized from ethyl acetate/n-heptane to give the title compound as a pale-yellow solid (yield: 45 mg, 7%).
¹H-NMR (DMSO-d₆, 300 MHz):δ3.85 (3H, s), 5.41 (2H, s), 7.11-7.17 (2H, m), 7.18-7.24 (1H, m), 7.81 (1H, s), 8.03 (1H, d, J = 8.1 Hz), 8.11 (1H, s), 8.18 (1H, d, J = 8.1 Hz).

### Example 91

### (5Z)-5-[(4-{[2,4-bis(trifluoromethyl)benzyl]oxy}-3-methoxyphenyl)methylidene]-4-{[2-(diethylamino)ethyl]amino}-1,3-thiazol-2(5H)-one

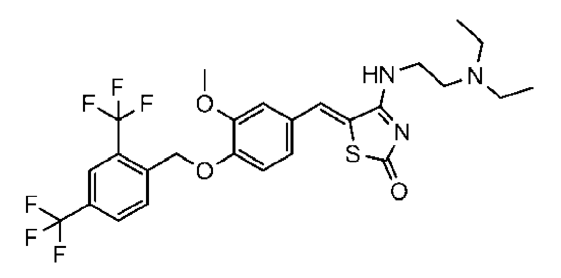

To a solution (2 mL) of (5Z)-5-[(4-{[2,4-bis(trifluoromethyl)benzyl]oxy}-3-methoxyphenyl)methylidene]-4-thioxo-1,3-thiazolidin-2-one (180 mg) in methanol was added a solution (2 mL) of N,N-diethylethane-1,2-diamine (216 mg) in methanol, and the mixture was stirred at room temperature for 20 hr. The reaction mixture was concentrated, and the residue was purified by basic silica gel column chromatography (eluent: ethyl acetate) to give the title compound as a yellow solid (yield: 120 mg, 57%). The obtained solid was recrystallized from ethyl acetate/n-heptane to give the title compound as a yellow solid (yield: 81 mg, 39%).
¹H-NMR (DMSO-d₆, 300 MHz):δ0.97 (6H, t, J = 7.1 Hz), 2.46-2.59 (4H, m), 2.66 (2H, t, J = 6.8 Hz), 3.54 (2H, t, J = 6.8 Hz), 3.85 (3H, s), 5.41 (2H, s), 7.08-7.17 (2H, m), 7.17-7.25 (1H, m), 7.76 (1H, s), 8.03 (1H, d, J = 8.1 Hz), 8.11 (1H, s), 8.18 (1H, d, J = 8.1 Hz), 9.30 (1H, brs).

### Example 92

### 4-(4-{(Z)-[4-{[2-(diethylamino)ethyl]amino}-2-oxo-1,3-thiazol-5(2H)-ylidene]methyl}-2-methoxyphenoxy)-3-(trifluoromethyl)benzonitrile

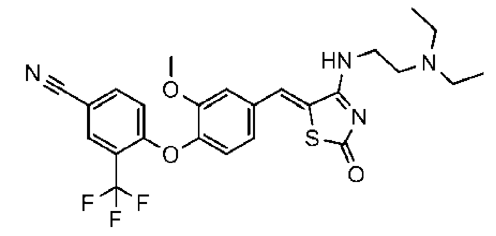

To a solution (150 mL) of N,N-diethylethane-1,2-diamine (6.64 g) in ethanol was added 4-thioxo-1,3-thiazolidin-2-one (7.46 g) and the mixture was stirred at room temperature for 2 hr under a nitrogen atmosphere. To the reaction mixture were added 4-(4-formyl-2-methoxyphenoxy)-3-(trifluoromethyl)benzonitrile (15.0 g) and potassium tert-butoxide (7.40 g), and the mixture was heated under reflux for 1 hr. To the reaction mixture was added saturated aqueous ammonium chloride solution, and the mixture was concentrated under reduced pressure. To the residue was added water, and the mixture was extracted with ethyl acetate. The organic layer was dried over anhydrous magnesium sulfate, and filtered, and the filtrate was concentrated under reduced pressure. The residue was purified by basic silica gel column chromatography (eluent: hexane-ethyl acetate=30:70→0:100), and washed with diethyl ether to give the title compound as a yellow solid (yield: 6.50 g, 27%). The obtained solid was recrystallized from ethyl acetate/n-heptane to give the title compound as a pale-yellow solid (yield: 6.17 g, 26%).
¹H-NMR (DMSO-d₆, 300 MHz):δ0.97 (6H, t, J = 7.1 Hz), 2.53 (4H, q, J = 7.1 Hz), 2.67 (2H, t, J = 6.9 Hz), 3.56 (2H, t, J = 6.9 Hz), 3.80 (3H, s), 6.93 (1H, d, J = 8.7 Hz), 7.24 (1H, dd, J = 8.3, 1.7 Hz), 7.30 (1H, d, J = 1.7 Hz), 7.38 (1H, d, J = 8.3 Hz), 7.84 (1H, s), 8.02 (1H, dd, J = 8.7, 1.7 Hz), 8.33 (1H, d, J = 1.7 Hz), 9.42 (1H, brs).

### Example 93

### 4-(2-methoxy-4-{(Z)-[4-{[2-(1-methylpyrrolidin-2-yl)ethyl]aminol-2-oxo-1,3-thiazol-5(2H)-ylidene]methyl}phenoxy)-3-(trifluoromethyl)benzonitrile

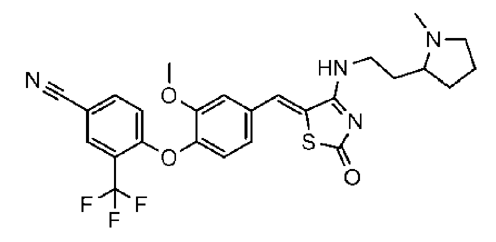

To a solution (90 mL) of 2-(1-methylpyrrolidin-2-yl)ethanamine (4.12 g) in ethanol was added 4-thioxo-1,3-thiazolidin-2-one (4.15 g) and the mixture was stirred at room temperature for 3 hr under a nitrogen atmosphere. To the reaction mixture were added 4-(4-formyl-2-methoxyphenoxy)-3-(trifluoromethyl)benzonitrile (8.85 g) and potassium tert-butoxide (4.11 g), and the mixture was heated under reflux for 1 hr. To the reaction mixture was added saturated aqueous ammonium chloride solution, and the mixture was concentrated under reduced pressure. To the residue was added water, and the mixture was extracted with ethyl acetate. The organic layer was washed with saturated brine, dried over anhydrous magnesium sulfate, and filtered, and the filtrate was concentrated under reduced pressure. The residue was purified by basic silica gel column chromatography (eluent: ethyl acetate-methanol=100:0→95:5) to give the title compound as a pale-yellow solid (yield: 3.65 g, 24%). The obtained solid was washed with diethyl ether, and recrystallized from ethyl acetate/n-heptane to give the title compound as a pale-yellow solid (yield: 2.62 g, 17%).
¹H-NMR (DMSO-d₆, 300 MHz):δ1.37-1.72 (4H, m), 1.86-2.18 (4H, m), 2.24 (3H, s), 2.90-3.02 (1H, m), 3.54 (2H, t, J = 6.9 Hz), 3.80 (3H, s), 6.93 (1H, d, J = 8.9 Hz), 7.24 (1H, dd, J = 8.3, 1.7 Hz), 7.30 (1H, d, J = 1.9 Hz), 7.38 (1H, d, J = 8.3 Hz), 7.82 (1H, s), 8.03 (1H, dd, J = 8. 9, 1.9 Hz), 8.33 (1H, d, J = 1.7 Hz), 9.50 (1H, brs).

### Example 94

### (5Z)-5-({4-[2-chloro-4-(trifluoromethyl)phenoxy]-3-methoxyphenyl}methylidene)-4-{[2-(diethylamino)ethyl]amino}-1,3-thiazol-2(5H)-one

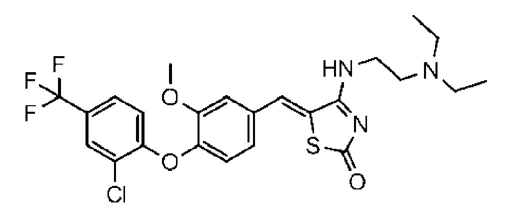

To a solution (700 mL) of 4-thioxo-1,3-thiazolidin-2-one (34.9 g) in ethanol was added a solution (100 mL) of N,N-diethylethane-1,2-diamine (31.1 g) in ethanol and the mixture was stirred at room temperature for 3 hr under a nitrogen atmosphere. To the reaction mixture were added a solution (100 mL) of 4-[2-chloro-4-(trifluoromethyl)phenoxy]-3-methoxybenzaldehyde (72.2 g) in ethanol and potassium tert-butoxide (34.6 g), and the mixture was heated under reflux for 0.5 hr. To the reaction mixture was added saturated aqueous ammonium chloride solution, and the mixture was concentrated under reduced pressure. To the residue was added water, and the mixture was extracted with ethyl acetate. The organic layer was washed with water and saturated brine, dried over anhydrous magnesium sulfate, and filtered, and the filtrate was concentrated under reduced pressure. The residue was purified by basic silica gel column chromatography (eluent: hexane-ethyl acetate=50:50→0:100), and washed with diethyl ether. The obtained pale-yellow solid was recrystallized from ethyl acetate/n-heptane to give the title compound as a pale-yellow solid (yield: 19.6 g, 17%).
¹H-NMR (DMSO-d₆,300 MHz):δ0.98 (6H, t, J = 7.1 Hz), 2.53 (4H, q, J = 7.1 Hz), 2.67 (2H, t, J = 6.9 Hz), 3.56 (2H, t, J = 6.9 Hz), 3.83 (3H, s), 6.93 (1H, d, J = 8.7 Hz), 7.18-7.26 (1H, m), 7.26-7.33 (2H, m), 7.64 (1H, dd, J = 9.1, 1.9 Hz), 7.84 (1H, s), 8.01 (1H, d, J = 1.9 Hz), 9.41 (1H, brs).

### Example 95

### 4-(4-{(Z)-[4-{[2-(diethylamino)ethyl]amino}-2-oxo-1,3-thiazol-5(2H)-ylidene]methyl}-2-methoxyphenoxy)naphthalene-1-carbonitrile

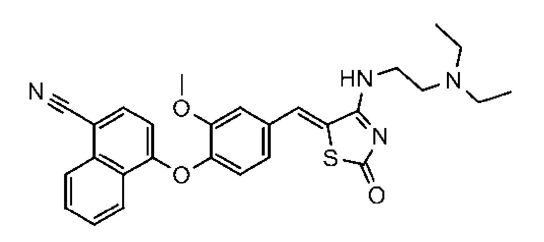

To a solution (4 mL) of N,N-diethylethane-1,2-diamine (141 mg) in ethanol was added 4-thioxo-1,3-thiazolidin-2-one (158 mg) and the mixture was stirred at room temperature for 2 hr under a nitrogen atmosphere. To the reaction mixture were added 4-(4-formyl-2-methoxyphenoxy)naphthalene-1-carbonitrile (300 mg) and potassium tert-butoxide (157 mg), and the mixture was heated under reflux for 0.5 hr. To the reaction mixture was added saturated aqueous ammonium chloride solution, and the mixture was concentrated under reduced pressure. To the residue was added water, and the mixture was extracted with ethyl acetate. The organic layer was washed with saturated brine, dried over anhydrous magnesium sulfate, and filtered, and the filtrate was concentrated under reduced pressure. The residue was purified by basic silica gel column chromatography (eluent: ethyl acetate-methanol=100:0→90:10) to give the title compound as a yellow solid (yield: 187 mg, 32%). The obtained solid was recrystallized from ethyl acetate/n-heptane to give the title compound as a yellow solid (yield: 124 mg, 21%). ¹H-NMR (DMSO-d₆, 300 MHz):δ0.98 (6H, t, J = 7.1 Hz), 2.54 (4H, q, J = 7.1 Hz), 2.68 (2H, t, J = 6.9 Hz), 3.57 (2H, t, J = 6.9 Hz), 3.80 (3H, s), 6.70 (1H, d, J = 8.1 Hz), 7.26 (1H, dd, J = 8.3, 1.7 Hz), 7.33 (1H, d, J = 1.7 Hz), 7.42 (1H, d, J = 8.3 Hz), 7.76-7.84 (1H, m), 7.86 (1H, s), 7.87-7.94 (1H, m), 8.04 (1H, d, J = 8.1 Hz), 8.14 (1H, d, J = 8.3 Hz), 8.44 (1H, d, J = 8.3 Hz), 9.42 (1H, brs).

### Example 96

### 4-(2-methoxy-4-{(Z)-[4-{[2-(1-methylpyrrolidin-2-yl)ethyl]aminol-2-oxo-1,3-thiazol-5(2H)-ylidene]methyl}phenoxy)naphthalene-1-carbonitrile

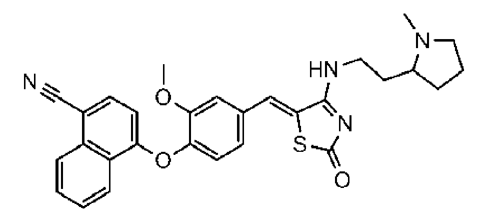

To a solution (650 mL) of 4-thioxo-1,3-thiazolidin-2-one (46.0 g) in ethanol was added a solution (100 mL) of 2-(1-methylpyrrolidin-2-yl)ethanamine (45.7 g) in ethanol and the mixture was stirred at room temperature for 3 hr under a nitrogen atmosphere. To the reaction mixture were added 4-(4-formyl-2-methoxyphenoxy)naphthalene-1-carbonitrile (87.3 g) and potassium tert-butoxide (45.6 g), and the mixture was heated under reflux for 0.5 hr. To the reaction mixture was added saturated aqueous ammonium chloride solution, and the mixture was concentrated under reduced pressure. To the residue was added water, and the mixture was extracted with ethyl acetate. The organic layer was dried over anhydrous magnesium sulfate, and filtered by basic silica gel column chromatography, and the filtrate was concentrated under reduced pressure. The residue was purified by basic silica gel column chromatography (eluent: ethyl acetate-methanol=100:0→95:5), and washed with diethyl ether. The obtained yellow solid was recrystallized from tetrahydrofuran/n-heptane to give the title compound as a yellow solid (yield: 11.9 g, 8%).
¹H-NMR (DMSO-d₆, 300 MHz):δ1.40-1.72 (4H, m), 1.86-2.17 (4H, m), 2.24 (3H, s), 2.91-3.00 (1H, m), 3.47-3.63 (2H, m), 3.80 (3H, s), 6.70 (1H, d, J = 8.3 Hz), 7.23-7.30 (1H, m), 7.33 (1H, d, J = 1.9 Hz), 7.42 (1H, d, J = 8.3 Hz), 7.75-7.95 (3H, m), 8.04 (1H, d, J = 8.3 Hz), 8.14 (1H, d, J = 8.3 Hz), 8.45 (1H, d, J = 8.1 Hz), 9.52 (1H, brs).

### Example 97

### (5Z)-5-{[4-{[2,4-bis(trifluoromethyl)benzyl]oxy}-3-(propylamino)phenyl]methylidene}-4-(methylamino)-1,3-thiazol-2(5H)-one

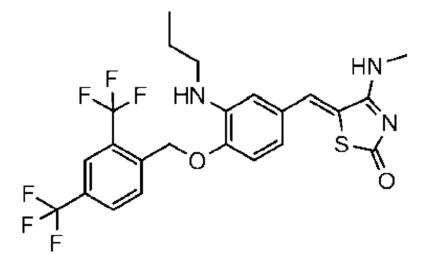

(5Z)-5-[(3-Amino-4-{[2,4-bis(trifluoromethyl)benzyl]oxy}phenyl)methylidene]-4-(methylamino)-1,3-thiazol-2(5H)-one (0.20 g) was dissolved in tetrahydrofuran/acetonitrile (2/1, 12 mL), and propionaldehyde (0.031 mL), sodium cyanoborohydride (0.079 g) and acetic acid (5 drops) were added, and the mixture was stirred at room temperature overnight. The solvent was removed under reduced pressure, water was added, and the mixture was extracted with ethyl acetate (15 mL x 3). The organic layers were combined, dried over anhydrous sodium sulfate, and concentrated under reduced pressure. The residue was purified by silica gel column chromatography (eluent: petroleum ether-ethyl acetate=1:1) to give the title compound as yellow crystals (yield: 50 mg, 23%).
¹H-NMR (DMSO-d₆, 400 MHz): δ 0.95 (3H, t, J = 7.6 Hz), 1.60-1.65 (2H, m), 3.04 (3H, s), 3.09-3.14 (2H, m), 5.23 (1H, t, J = 5.6 Hz), 5.43 (2H, s), 6.74-6.78 (2H, m), 7.00 (1H, d, J = 8.0 Hz), 7.67 (1H, s), 8.07-8.18 (3H, m), 9.28 (1H, brs).

### Example 98

### methyl 2-{[2,4-bis(trifluoromethyl)benzyl]oxy}-5-{(Z)-[4-(methylamino)-2-oxo-1,3-thiazol-5(2H)-ylidene]methyl}benzoate

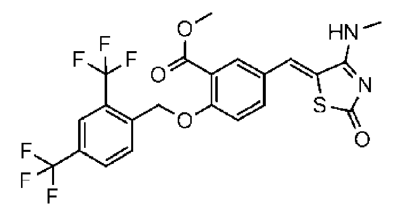

Methyl 2-{[2,4-bis(trifluoromethyl)benzyl]oxy}-5-formylbenzoate (17.15 g) and 4-(methylamino)-1,3-thiazol-2(5H)-one (6.0 g) were dissolved in anhydrous methanol (150 mL), potassium tert-butoxide (14.11 g) was added, and the mixture was stirred at 60°C for 2 hr. The reaction mixture was cooled to room temperature, and the precipitate was collected by filtration to give the title compound as a yellow powder (yield: 15.94 g, 73%).
¹H-NMR (DMSO-d₆, 400 MHz): δ 3.05 (3H, s), 3.87 (3H, s), 5.51 (2H, s), 7.36 (1H, d, J = 8.8 Hz) 7.54 (1H, s), 7.71 (1H, dd, J = 8.8, 2.4 Hz), 7.95 (1H, d, J = 2.4 Hz), 8.11 (1H, s), 8.25 (2H, s).

### Example 99

### (5Z)-5-{[4-{[2,4-bis(trifluoromethyl)benzyl]oxy}-3-(methylamino)phenyllmethylidenel-4-(methylamino)-1,3-thiazol-2(5H)-one

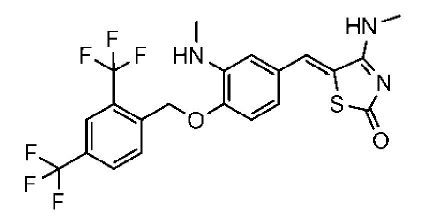

(5Z)-5-[(3-Amino-4-{[2,4-bis(trifluoromethyl)benzyl]oxy}phenyl)methylidene]-4-(methylamino)-1,3-thiazol-2(5H)-one (0.30 g) was dissolved in tetrahydrofuran/acetonitrile (2/1, 15 mL), and formalin (0.07 mL), sodium cyanoborohydride (0.13 g) and acetic acid (5 drops) were added, and the mixture was stirred at room temperature overnight. The solvent was removed under reduced pressure, water was added, and the mixture was extracted with ethyl acetate (15 mL x 3). The organic layers were combined, dried over anhydrous sodium sulfate, and concentrated under reduced pressure. The residue was purified by preparative HPLC to give the title compound as a yellow powder (yield: 50 mg, 17%).
¹H-NMR (DMSO-d₆,400 MHz): δ 2.79 (3H, d, J = 4.8 Hz), 3.05 (3H, d, J = 3.6 Hz), 5.41-5.44 (3H, m), 6.68 (1H, t, J = 1.6 Hz), 6.79 (1H, dd, J = 8.4, 2.0 Hz), 7.01 (1H, d, J = 8.0 Hz), 7.69 (1H, s), 8.12-8.15 (3H, m), 9.29 (1H, brs).

### Example 100

### (5Z)-5-{[4-{[2,4-bis(trifluoromethyl)benzyl]oxy}-3-(ethylamino)phenyl]methylidene}-4-(methylamino)-1,3-thiazol-2(5H)-one

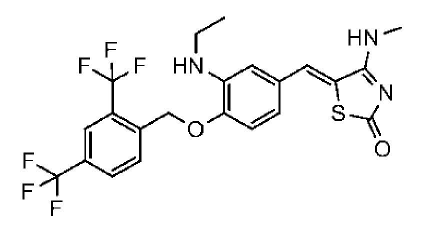

(5Z)-5-[(3-Amino-4-{[2,4-bis(trifluoromethyl)benzyl]oxy}phenyl)methylidene]-4-(methylamino)-1,3-thiazol-2(5H)-one (0.31 g) was dissolved in tetrahydrofuran/acetonitrile (2/1, 15 mL), and acetaldehyde (40%, 0.084 mL), sodium cyanoborohydride (0.13 g) and acetic acid (5 droops) were added, and the mixture was stirred at room temperature overnight. The solvent was removed under reduced pressure, water was added, and the mixture was extracted with ethyl acetate (15 mL x 3). The organic layers were combined, dried over anhydrous sodium sulfate, and concentrated under reduced pressure. The residue was purified by silica gel column chromatography (eluent: petroleum ether-ethyl acetate=1:1) to give the title compound as yellow crystals (yield: 68 mg, 20%).
¹H-NMR (DMSO-d₆, 400 MHz): δ 1.22 (3H, t, J = 7.2 Hz), 3.04 (3H, d, J = 4.4 Hz), 3.16-3.22 (2H, m), 5.18 (1H, t, J = 5.6 Hz), 5.43 (2H, s), 6.74-6.79 (2H, m), 7.00 (1H, d, J = 8.4 Hz), 7.68 (1H, s), 8.07-8.18 (3H, m), 9.29 (1H, brs).

### Example 101

### (5Z)-5-[(4-{[2,4-bis(trifluoromethyl)benzyl]oxy}-3-[(1-methylethyl)amino]phenyl)methylidene]-4-(methylamino)-1,3-thiazol-2(5H)-one

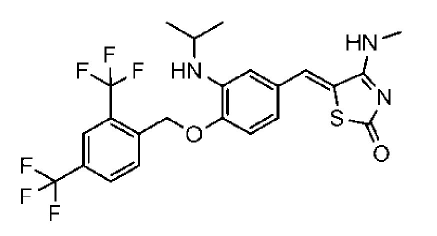

(5Z)-5-[(3-Amino-4-{[2,4-bis(trifluoromethyl)benzyl]oxy}phenyl)methylidene]-4-(methylamino)-1,3-thiazol-2(5H)-one (0.35 g) was dissolved in tetrahydrofuran/acetonitrile (2/1, 15 mL), and acetone (0.27 mL), sodium cyanoborohydride (0.14 g) and acetic acid (5 drops) were added, and the mixture was stirred at room temperature overnight. The solvent was removed under reduced pressure, water was added, and the mixture was extracted with ethyl acetate (15 mL x 3). The organic layers were combined, dried over anhydrous sodium sulfate, and concentrated under reduced pressure. The residue was purified by silica gel column chromatography (eluent: petroleum ether-ethyl acetate=1:1) to give the title compound as yellow crystals (yield: 51 mg, 13%).
¹H-NMR (DMSO-d₆, 400 MHz): δ 1.22 (6H, d, J = 6.4 Hz), 3.05 (3H, d, J = 4.4 Hz), 3.58-3.63 (1H, m), 4.67 (1H, d, J = 8.0 Hz), 5.45 (2H, s), 6.76-6.78 (2H, m), 7.01 (1H, d, J = 8.4 Hz), 7.67 (1H, s), 8.02 (1H, d, J = 8.4 Hz), 8.14 (1H, s), 8.18 (1H, d, J = 8.0 Hz), 9.29 (1H, brs).

### Example 102

### (5Z)-5-[(4-{[2,4-bis(trifluoromethyl)benzyl]oxy}-3-[(2-methylpropyl)amino]phenyl)methylidene]-4-(methylamino)-1,3-thiazol-2(5H)-one

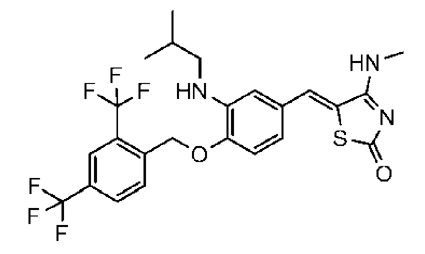

(5Z)-5-[(3-Amino-4-{[2,4-bis(trifluoromethyl)benzyl]oxy}phenyl)methylidene]-4-(methylamino)-1,3-thiazol-2(5H)-one (0.35 g) was dissolved in tetrahydrofuran/acetonitrile (2/1, 15 mL), and isobutylaldehyde (0.14 g), sodium cyanoborohydride (0.14 g) and acetic acid (5 drops) were added, and the mixture was stirred at room temperature overnight. The solvent was removed under reduced pressure, water was added, and the mixture was extracted with ethyl acetate (15 mL x 3). The organic layers were combined, dried over anhydrous sodium sulfate, and concentrated under reduced pressure. The residue was purified by silica gel column chromatography (eluent: petroleum ether-ethyl acetate=1:1) to give the title compound as yellow crystals (yield: 75 mg, 19%).
¹H-NMR (DMSO-d₆, 400 MHz): δ 0.94 (6H, d, J = 6.4 Hz), 1.93-2.02 (1H, m), 2.96 (2H, t, J = 6.4 Hz), 3.05 (3H, s), 5.27 (1H, t, J = 6.0 Hz), 5.44 (2H, s), 6.73-6.76 (2H, m), 7.01 (1H, d, J = 8.0 Hz), 7.66 (1H, s), 8.06-8.18 (3H, m), 9.29 (1H, brs).

### Example 103

### (5Z)-5-{[3-(benzylamino)-4-{[2,4-bis(trifluoromethyl)benzyl]oxy}phenyl]methylidene}-4-(methylamino)-1,3-thiazol-2(5H)-one

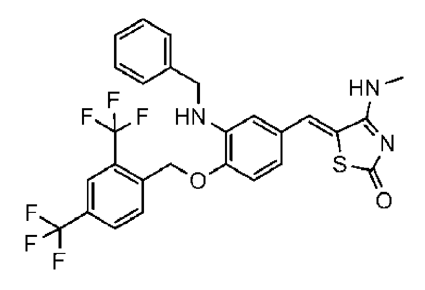

(5Z)-5-[(3-Amino-4-{[2,4-bis(trifluoromethyl)benzyl]oxy}phenyl)methylidene]-4-(methylamino)-1,3-thiazol-2(5H)-one (0.23 g) was dissolved in tetrahydrofuran/acetonitrile (2/1, 15 mL), and benzaldehyde (77 mg), sodium cyanoborohydride (0.089 g) and acetic acid (5 drops) were added, and the mixture was stirred at room temperature overnight. The solvent was removed under reduced pressure, water was added, and the mixture was extracted with ethyl acetate (15 mL x 3). The organic layers were combined, dried over anhydrous sodium sulfate, and concentrated under reduced pressure. The residue was purified by silica gel column chromatography (eluent: petroleum ether-ethyl acetate=1:1) to give the title compound as yellow crystals (yield: 53 mg, 20%).
¹H-NMR (DMSO-d₆, 400 MHz): δ 3.00 (3H, d, J = 4.8 Hz), 4.43 (2H, d, J = 6.4 Hz), 5.47 (2H, s), 6.08 (1H, t, J = 6.0 Hz), 6.56 (1H, d, J = 2.0 Hz), 6.75 (1H, dd, J = 8.4, 2.0 Hz), 7.03 (1H, d, J = 8.4 Hz), 7.23 (1H, t, J = 6.8 Hz), 7.31-7.38 (4H, m), 7.53 (1H, s), 8.14 (1H, s), 8.18 (2H, s), 9.22 (1H, brs).

### Example 104

### (5Z)-5-[(3-amino-4-{[2,4-bis(trifluoromethyl)benzyl]oxy}phenyl)methylidene]-4-(methylamino)-1,3-thiazol-2(5H)-one

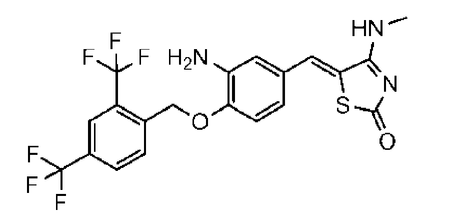

(5Z)-5-[(4-{[2,4-bis(Trifluoromethyl)benzyl]oxy}-3-nitrophenyl)methylidene]-4-(methylamino)-1,3-thiazol-2(5H)-one (13.2 g) was dissolved in tetrahydrofuran/ethanol/water (3/1/1, 280 mL), and reduced iron powder (7.3 g) and acetic acid were added, and the mixture was stirred with heating under reflux for 3 hr. The reaction mixture was filtered, and mother liquor was concentrated. The residue was dissolved in ethyl acetate, and the precipitate was collected by filtration to give the title compound as a yellow powder (yield: 7.5 g, 77%).
¹H-NMR (DMSO-d₆, 400 MHz): δ 3.04 (3H, d, J = 3.6 Hz), 5.20 (2H, s), 5.41 (2H, s), 6.75-6.77 (1H, m), 6.94-7.01 (2H, m), 7.62 (1H, s), 8.11-8.14 (3H, m), 9.31 (1H, brs).

### Example 105

### (5Z)-4-{[2-(diethylamino)ethyl]amino}-5-[(3-methoxy-4-{[4-(trifluoromethyl)benzyl]oxy}phenyl)methylidene]-1,3-thiazol-2(5H)-one

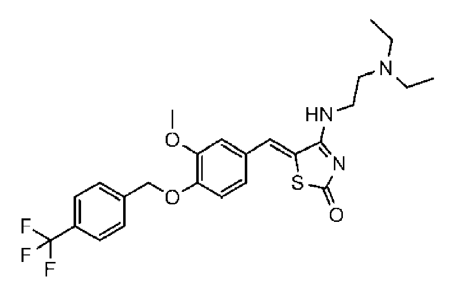

To a solution (2 mL) of N,N-diethylethane-1,2-diamine (138 mg) in ethanol was added 4-thioxo-1,3-thiazolidin-2-one (155 mg) and the mixture was stirred at room temperature for 2 hr under a nitrogen atmosphere. To the reaction mixture were added a solution (2 mL) of 3-methoxy-4-{[4-(trifluoromethyl)benzyl]oxylbenzaldehyde (300 mg) in ethanol and potassium tert-butoxide (153 mg), and the mixture was heated under reflux for 2 hr. To the reaction mixture was added saturated aqueous ammonium chloride solution, and the mixture was extracted with ethyl acetate. The organic layer was washed with water and saturated brine, dried over anhydrous magnesium sulfate, and filtered, and the filtrate was concentrated under reduced pressure. The residue was purified by basic silica gel column chromatography (eluent: ethyl acetate-methanol=100:0→95:5) to give the title compound as a yellow solid (yield: 197 mg, 40%). The obtained solid was recrystallized from ethyl acetate/n-heptane to give the title compound as a yellow solid (yield: 163 mg, 33%).
¹H-NMR (DMSO-d₆, 300 MHz):δ0.90-1.06 (6H, m), 2.46-2.58 (4H, m), 2.66 (2H, t, J = 5.5 Hz), 3.54 (2H, t, J = 5.5 Hz), 3.85 (3H, s), 5.30 (2H, s), 7.07-7.16 (2H, m), 7.16-7.27 (1H, m), 7.61-7.73 (2H, m), 7.73-7.88 (3H, m), 9.28 (1H, brs).

### Example 106

### 3-chloro-4-(4-{(Z)-[4-{[2-(diethylamino)ethyl]amino}-2-oxo-1,3-thiazol-5(2H)-ylidene]methyl}-2-methoxyphenoxy)benzonitrile

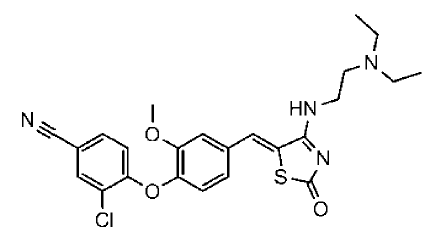

To a solution (4 mL) of N,N-diethylethane-1,2-diamine (148 mg) in ethanol was added 4-thioxo-1,3-thiazolidin-2-one (167 mg) and the mixture was stirred at room temperature for 2 hr under a nitrogen atmosphere. To the reaction mixture were added 3-chloro-4-(4-formyl-2-methoxyphenoxy)benzonitrile (300 mg) and potassium tert-butoxide (165 mg), and the mixture was heated under reflux for 0.5 hr. To the reaction mixture was added saturated aqueous ammonium chloride solution, and the mixture was extracted with ethyl acetate. The organic layer was washed with water and saturated brine, dried over anhydrous magnesium sulfate, and filtered, and the filtrate was concentrated under reduced pressure. The residue was purified by basic silica gel column chromatography (eluent: ethyl acetate-methanol=100:0→95:5) to give the title compound as a yellow solid (yield: 199 mg, 40%). The obtained solid was recrystallized from ethyl acetate/n-heptane to give the title compound as a yellow solid (yield: 174 mg, 35%).
¹H-NMR (DMSO-d₆, 300 MHz):δ0.97 (6H, t, J = 7.1 Hz), 2.47-2.58 (4H, m), 2.67 (2H, t, J = 6.7 Hz), 3.56 (2H, t, J = 6.7 Hz), 3.81 (3H, s), 6.85 (1H, d, J = 8.7 Hz), 7.22 (1H, d, J = 8.3 Hz), 7.29 (1H, s), 7.33 (1H, d, J = 8.3 Hz), 7.73 (1H, dd, J = 8.7, 1.7 Hz), 7.84 (1H, s), 8.19 (1H, d, J = 1.7 Hz), 9.38 (1H, brs).

### Example 107

### 2-chloro-4-(4-{(Z)-[4-{[2-(diethylamino)ethyl]amino}-2-oxo-1,3-thiazol-5(2H)-ylidene]methyl}-2-methoxyphenoxy)benzonitrile

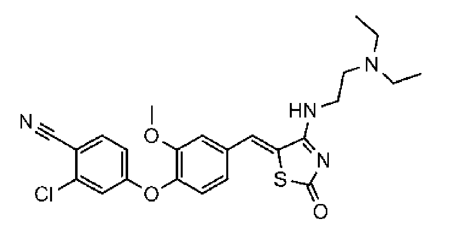

To a solution (4 mL) of N,N-diethylethane-1,2-diamine (148 mg) in ethanol was added 4-thioxo-1,3-thiazolidin-2-one (167 mg) and the mixture was stirred at room temperature for 2 hr under a nitrogen atmosphere. To the reaction mixture were added 2-chloro-4-(4-formyl-2-methoxyphenoxy)benzonitrile (300 mg) and potassium tert-butoxide (165 mg), and the mixture was heated under reflux for 0.5 hr. To the reaction mixture was added saturated aqueous ammonium chloride solution, and the mixture was extracted with ethyl acetate. The organic layer was washed with water and saturated brine, dried over anhydrous magnesium sulfate, and filtered, and the filtrate was concentrated under reduced pressure. The residue was purified by basic silica gel column chromatography (eluent: ethyl acetate-methanol=100:0→95:5) to give the title compound as a yellow solid (yield: 212 mg, 42%). The obtained solid was recrystallized from ethyl acetate/n-heptane to give the title compound as a brown solid (yield: 189 mg, 37%).
¹H-NMR (DMSO-d₆, 300 MHz):δ0.97 (6H, t, J = 7.1 Hz), 2.48-2.58 (4H, m), 2.67 (2H, t, J = 6.8 Hz), 3.57 (2H, t, J = 6.8 Hz), 3.81 (3H, s), 6.95-7.02 (1H, m), 7.23 (1H, d, J = 8.3 Hz), 7.26-7.32 (2H, m), 7.37 (1H, d, J = 8.3 Hz), 7.84 (1H, s), 7.92 (1H, d, J = 8.7 Hz), 9.44 (1H, brs).

### Example 108

### 4-(4-{(Z)-[4-{[2-(diethylamino)ethyl]amino}-2-oxo-1,3-thiazol-5(2H)-ylidene]methyl}-2-methoxyphenoxy)-2-(trifluoromethyl)benzonitrile

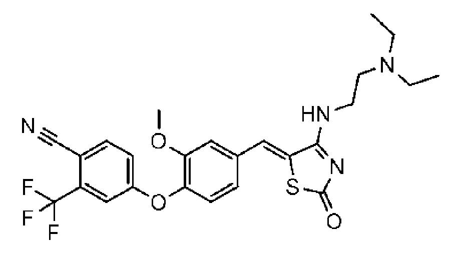

To a solution (4 mL) of N,N-diethylethane-1,2-diamine (133 mg) in ethanol was added 4-thioxo-1,3-thiazolidin-2-one (149 mg) and the mixture was stirred at room temperature for 2 hr under a nitrogen atmosphere. To the reaction mixture were added 4-(4-formyl-2-methoxyphenoxy)-2-(trifluoromethyl)benzonitrile (300 mg) and potassium tert-butoxide (148 mg), and the mixture was heated under reflux for 0.5 hr. To the reaction mixture was added saturated aqueous ammonium chloride solution, and the mixture was extracted with ethyl acetate. The organic layer was washed with water and saturated brine, dried over anhydrous magnesium sulfate, and filtered, and the filtrate was concentrated under reduced pressure. The residue was purified by basic silica gel column chromatography (eluent: ethyl acetate-methanol=100:0→95:5) to give the title compound as a yellow solid (yield: 161 mg, 32%). The obtained solid was recrystallized from ethyl acetate/n-heptane to give the title compound as a brown solid (yield: 116 mg, 23%).
¹H-NMR (DMSO-d₆, 300 MHz):δ0.98 (6H, t, J = 7.1 Hz), 2.53 (4H, q, J = 7.1 Hz), 2.68 (2H, t, J = 6.8 Hz), 3.57 (2H, t, J = 6.8 Hz), 3.81 (3H, s), 7.18-7.28 (2H, m), 7.30 (1H, s), 7.41 (1H, d, J = 8.1 Hz), 7.55 (1H, d, J = 2.1 Hz), 7.85 (1H, s), 8.11 (1H, d, J = 8.7 Hz), 9.44 (1H, brs).

### Example 109

### 4-(4-{(Z)-[4-{[2-(diethylamino)ethyl]amino}-2-oxo-1,3-thiazol-5(2H)-ylidene]methyl}-2-methoxyphenoxy)benzonitrile

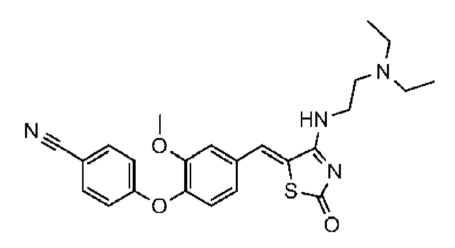

To a solution (2 mL) of N,N-diethylethane-1,2-diamine (79 mg) in ethanol was added 4-thioxo-1,3-thiazolidin-2-one (88 mg) and the mixture was stirred at room temperature for 6 hr under a nitrogen atmosphere. To the reaction mixture were added a solution (2 mL) of 4-(4-formyl-2-methoxyphenoxy)benzonitrile (140 mg) in ethanol and potassium tert-butoxide (88 mg), and the mixture was heated under reflux for 0.5 hr. To the reaction mixture was added saturated aqueous ammonium chloride solution, and the mixture was extracted with ethyl acetate. The organic layer was washed with saturated brine, dried over anhydrous magnesium sulfate, and filtered, and the filtrate was concentrated under reduced pressure. The residue was purified by basic silica gel column chromatography (eluent: ethyl acetate-methanol=100:0→95:5) to give the title compound as a white solid (yield: 37 mg, 15%). The obtained solid was recrystallized from ethyl acetate/n-heptane to give the title compound as a white solid (yield: 14 mg, 6%).
¹H-NMR (DMSO-d₆, 300 MHz):δ0.97 (6H, t, J = 7.1 Hz), 2.51-2.58 (4H, m), 2.67 (2H, t, J = 6.9 Hz), 3.56 (2H, t, J = 6.9 Hz), 3.80 (3H, s), 6.99-7.07 (2H, m), 7.22 (1H, dd, J = 8.1, 1.7 Hz), 7.28 (1H, d, J = 1.7 Hz), 7.33 (1H, d, J = 8.1 Hz), 7.78-7.83 (2H, m), 7.84 (1H, s), 9.42 (1H, brs).

### Example 110

### (5Z)-4-{[2-(diethylamino)ethyl]amino}-5-[(3-methoxy-4-{[5-(trifluoromethyl)pyridin-2-yl]oxy}phenyl)methylidene]-1,3-thiazol-2(5H)-one

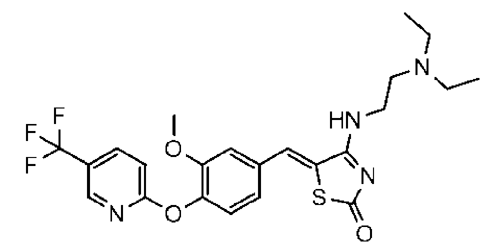

To a solution (2 mL) of N,N-diethylethane-1,2-diamine (144 mg) in ethanol was added 4-thioxo-1,3-thiazolidin-2-one (161 mg) and the mixture was stirred at room temperature for 6 hr under a nitrogen atmosphere. To the reaction mixture were added a solution (2 mL) of 3-methoxy-4-{[5-(trifluoromethyl)pyridin-2-ylloxylbenzaldehyde (300 mg) in ethanol and potassium tert-butoxide (160 mg), and the mixture was heated under reflux for 0.5 hr. To the reaction mixture was added saturated aqueous ammonium chloride solution, and the mixture was concentrated under reduced pressure. To the residue was added water, and the mixture was extracted with ethyl acetate. The organic layer was washed with saturated brine, dried over anhydrous magnesium sulfate, and filtered, and the filtrate was concentrated under reduced pressure. The residue was purified by basic silica gel column chromatography (eluent: ethyl acetate-methanol=100:0→95:5) to give the title compound as a yellow solid (yield: 192 mg, 39%). The obtained solid was recrystallized from ethyl acetate/n-heptane to give the title compound as a pale-yellow solid (yield: 164 mg, 33%). ¹H-NMR (DMSO-d₆, 300 MHz):δ0.98 (6H, t, J = 7.1 Hz), 2.53 (4H, q, J = 7.1 Hz), 2.68 (2H, t, J = 6.9 Hz), 3.57 (2H, t, J = 6.9 Hz), 3.76 (3H, s), 7.21 (1H, d, J = 8.1 Hz), 7.24 (1H, s), 7.29 (1H, d, J = 8.7 Hz), 7.37 (1H, d, J = 8.1 Hz), 7.83 (1H, s), 8.23 (1H, dd, J = 8.7, 2.3 Hz), 8.53 (1H, d, J = 0.6 Hz), 9.42 (1H, brs).

### Example 111

### 2-(4-{(Z)-[4-{[2-(diethylamino)ethyl]amino}-2-oxo-1,3-thiazol-5(2H)-ylidene]methyl}-2-methoxyphenoxy)-5-(trifluoromethyl)benzonitrile

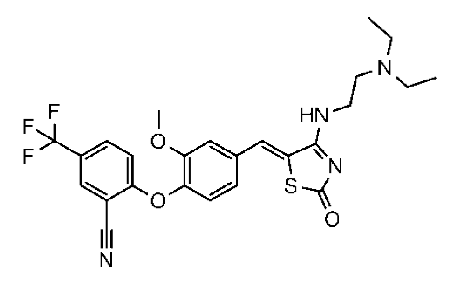

To a solution (2 mL) of N,N-diethylethane-1,2-diamine (133 mg) in ethanol was added 4-thioxo-1,3-thiazolidin-2-one (149 mg) and the mixture was stirred at room temperature for 2 hr under a nitrogen atmosphere. To the reaction mixture were added a solution (2 mL) of 2-(4-formyl-2-methoxyphenoxy)-5-(trifluoromethyl)benzonitrile (300 mg) in ethanol and potassium tert-butoxide (148 mg), and the mixture was heated under reflux for 0.5 hr. To the reaction mixture was added saturated aqueous ammonium chloride solution, and the mixture was concentrated under reduced pressure. To the residue was added water, and the mixture was extracted with ethyl acetate. The organic layer was washed with saturated brine, dried over anhydrous magnesium sulfate, and filtered, and the filtrate was concentrated under reduced pressure. The residue was purified by basic silica gel column chromatography (eluent: ethyl acetate-methanol=100:0→95:5) to give the title compound as a pale-yellow solid (yield: 127 mg, 26%). The obtained solid was recrystallized from ethyl acetate/n-heptane to give the title compound as a white solid (yield: 87 mg, 18%).
¹H-NMR (DMSO-d₆, 300 MHz):δ0.98 (6H, t, J = 7.1 Hz), 2.53 (4H, q, J = 7.1 Hz), 2.68 (2H, t, J = 6.8 Hz), 3.57 (2H, t, J = 6.8 Hz), 3.82 (3H, s), 6.93 (1H, d, J = 8.9 Hz), 7.26 (1H, dd, J = 8.1, 1.7 Hz), 7.31 (1H, d, J = 1.7 Hz), 7.48 (1H, d, J = 8.1 Hz), 7.86 (1H, s), 7.94 (1H, dd, J = 8.9, 1.9 Hz), 8.42 (1H, d, J = 1.9 Hz), 9.43 (1H, brs).

### Example 112

### (5Z)-5-[(4-{[3-chloro-5-(trifluoromethyl)pyridin-2-yl]oxy}-3-methoxyphenyl)methylidene]-4-{[2-(diethylamino)ethyl]amino}-1,3-thiazol-2(5H)-one

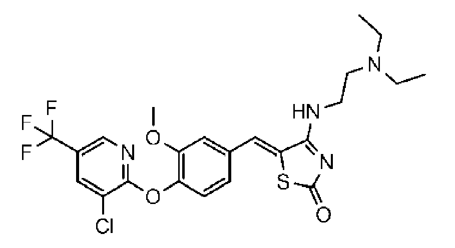

To a solution (70 mL) of N,N-diethylethane-1,2-diamine (4.98 g) in ethanol was added 4-thioxo-1,3-thiazolidin-2-one (5.60 g) and the mixture was stirred at room temperature for 3 hr under a nitrogen atmosphere. To the reaction mixture were added a solution (20 mL) of 4-{[3-chloro-5-(trifluoromethyl)pyridin-2-ylloxyl-3-methoxybenzaldehyde (11.6 g) in ethanol and potassium tert-butoxide (5.55 g), and the mixture was heated under reflux for 2 hr. To the reaction mixture was added saturated aqueous ammonium chloride solution, and the mixture was concentrated under reduced pressure. To the residue was added water, and the mixture was extracted with ethyl acetate. The organic layer was dried over anhydrous magnesium sulfate, and filtered, and the filtrate was concentrated under reduced pressure. The residue was purified by basic silica gel column chromatography (eluent: hexane-ethyl acetate=50:50→0:100) to give the title compound as a yellow solid (yield: 4.30 g, 23%). The obtained solid was washed with diethyl ether and recrystallized from ethyl acetate/n-heptane to give the title compound as a pale-yellow solid (yield: 3.36 g, 18%).
¹H-NMR (DMSO-d₆, 300 MHz):δ0.98 (6H, t, J = 7.1 Hz), 2.53 (4H, q, J = 7.1 Hz), 2.67 (2H, t, J = 6.9 Hz), 3.56 (2H, t, J = 6.9 Hz), 3.77 (3H, s), 7.17-7.28 (2H, m), 7.42 (1H, d, J = 8.1 Hz), 7.83 (1H, s), 8.49 (1H, s), 8.58 (1H, d, J = 2.1 Hz), 9.41 (1H, brs).

### Example 113

### (5Z)-4-{[2-(diethylamino)ethyl]amino}-5-({3-methoxy-4-[2-nitro-4-(trifluoromethyl)phenoxy]phenyl}methylidene)-1,3-thiazol-2(5H)-one

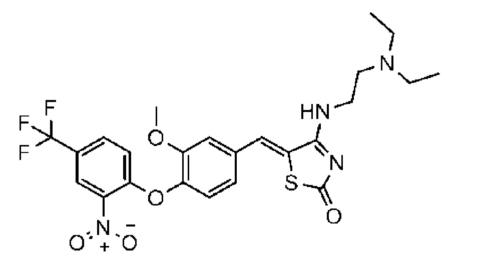

To a solution (2 mL) of N,N-diethylethane-1,2-diamine (125 mg) in ethanol was added 4-thioxo-1,3-thiazolidin-2-one (141 mg) and the mixture was stirred at room temperature for 2 hr under a nitrogen atmosphere. To the reaction mixture were added a solution (2 mL) of 3-methoxy-4-[2-nitro-4-(trifluoromethyl)phenoxy]benzaldehyde (300 mg) in ethanol and potassium tert-butoxide (139 mg), and the mixture was heated under reflux for 0.5 hr. To the reaction mixture was added saturated aqueous ammonium chloride solution, and the mixture was concentrated under reduced pressure. To the residue was added water, and the mixture was extracted with ethyl acetate. The organic layer was washed with saturated brine, dried over anhydrous magnesium sulfate, and filtered, and the filtrate was concentrated under reduced pressure. The residue was purified by basic silica gel column chromatography (eluent: ethyl acetate-methanol=100:0→95:5) to give the title compound as a pale-yellow solid (yield: 125 mg, 26%). The obtained solid was recrystallized from ethyl acetate/n-heptane to give the title compound as a white solid (yield: 82 mg, 17%).
¹H-NMR (DMSO-d₆, 300 MHz):δ0.98 (6H, t, J = 7.1 Hz), 2.53 (4H, q, J = 7.1 Hz), 2.67 (2H, t, J = 6.8 Hz), 3.57 (2H, t, J = 6.8 Hz), 3.82 (3H, s), 7.09 (1H, d, J = 8.9 Hz), 7.24 (1H, dd, J = 8.3, 1.7 Hz), 7.30 (1H, d, J = 1.7 Hz), 7.43 (1H, d, J = 8.3 Hz), 7.84 (1H, s), 7.96 (1H, dd, J = 8.9, 2.0 Hz), 8.47 (1H, d, J = 2.0 Hz), 9.44 (1H, brs).

### Example 114

### (5Z)-5-[(4-{[2,4-bis(trifluoromethyl)benzyl]oxy}-2-chloro-5-methoxyphenyl)methylidene]-4-{[2-(1-methylpyrrolidin-2-yl)ethyl]aminol-1,3-thiazol-2(5H)-one

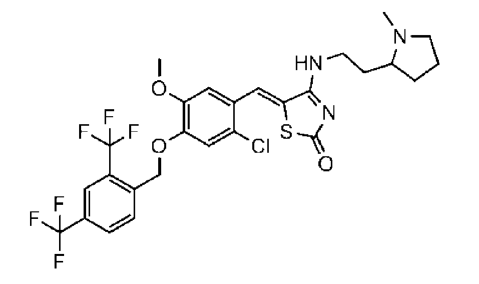

To a solution (2 mL) of 4-thioxo-1,3-thiazolidin-2-one (107 mg) in ethanol was added 2-(1-methylpyrrolidin-2-yl)ethanamine (0.126 mL), and the mixture was stirred at room temperature for 3 hr. To the reaction mixture were added 4-{[2,4-bis(trifluoromethyl)benzyl]oxy}-2-chloro-5-methoxybenzaldehyde (300 mg) and potassium tert-butoxide (98 mg) and the mixture was stirred with heating under reflux for 3 hr. Water was added to the reaction mixture, and the mixture was extracted with ethyl acetate. The organic layer was washed with saturated brine, dried over anhydrous magnesium sulfate, and filtered, and the filtrate was concentrated under reduced pressure. The residue was purified by silica gel column chromatography (eluent: methanol-ethyl acetate=0:100→5:95) and recrystallized from ethyl acetate/hexane to give the title compound as a pale-yellow powder (yield: 72 mg, 16%).
¹H-NMR (DMSO-d₆, 300 MHz):δ1.29 - 2.18 (8H, m), 2.24 (3H, s), 2.96 (1H, dt, J = 9.3, 4.7 Hz), 3.54 (2H, t, J = 7.5 Hz), 3.82 (3H, s), 5.42 (2H, s), 7.23 (1H, s), 7.35 (1H, s), 7.80 (1H, s), 8.04 (1H, d, J = 8.3 Hz), 8.12 (1H, s), 8.19 (1H, d, J = 8.7 Hz), 9.74 (1H, brs).

### Example 115

### (5Z)-5-[(4-{[2,4-bis(trifluoromethyl)benzyl]oxy}-2-chloro-5-methoxyphenyl)methylidene]-4-(methylamino)-1,3-thiazol-2(5H)-one

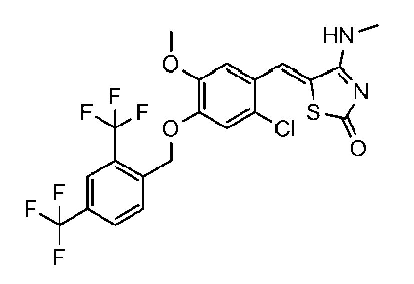

4-{[2,4-bis(Trifluoromethyl)benzyl]oxy}-2-chloro-5-methoxybenzaldehyde (200 mg) and 4-(methylamino)-1,3-thiazol-2(5H)-one (76 mg) were dissolved in ethanol (2 mL), potassium tert-butoxide (65 mg) was added, and the mixture was stirred at 80°C for 3 hr. The reaction mixture was concentrated, water was added, and the mixture was extracted with ethyl acetate. The organic layer was washed with saturated brine, dried over anhydrous magnesium sulfate, and filtered, and the filtrate was concentrated under reduced pressure. The residue was purified by silica gel column chromatography (NH)(eluent: hexane-ethyl acetate=30:70→0:100) and recrystallized from ethyl acetate to give the title compound as a pale-yellow powder (yield: 59 mg, 23%).
¹H-NMR (DMSO-d₆, 300 MHz):δ3.06 (3H, s), 3.82 (3H, s), 5.42 (2H, s), 7.23 (1H, s), 7.34 (1H, s), 7.82 (1H, s), 8.04 (1H, d, J = 7.9 Hz), 8.12 (1H, s), 8.19 (1H, d, J = 8.5 Hz), 9.65 (1H, brs.)

### Example 116

### 4-{4-[(Z)-{4-[(2-hydroxy-2-methylpropyl)amino]-2-oxo-1,3-thiazol-5(2H)-ylidene}methyl]-2-methoxyphenoxy}-3-(trifluoromethyl)benzonitrile

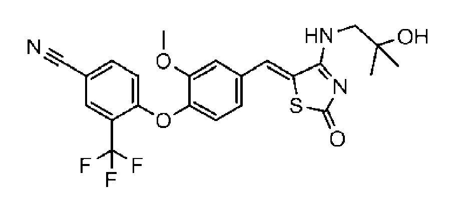

To a solution (410 mL) of 1-amino-2-methylpropan-2-ol (17.7 g) in ethanol was added 4-thioxo-1,3-thiazolidin-2-one (26.5 g) and the mixture was stirred at room temperature for 3 hr under a nitrogen atmosphere. To the reaction mixture were added 4-(4-formyl-2-methoxyphenoxy)-3-(trifluoromethyl)benzonitrile (53.3 g) and potassium tert-butoxide (26.3 g), and the mixture was heated under reflux for 0.5 hr. To the reaction mixture was added saturated aqueous ammonium chloride solution, and the mixture was concentrated under reduced pressure. To the residue was added water, and the mixture was extracted with ethyl acetate. The organic layer was dried over anhydrous magnesium sulfate, and filtered, and the filtrate was concentrated under reduced pressure. The residue was purified by basic silica gel column chromatography (eluent: hexane-ethyl acetate=50:50→0:100) to give the title compound as a yellow solid (yield: 12.6 g, 13%). The obtained solid was recrystallized from ethyl acetate/n-heptane to give the title compound as a pale-yellow solid (yield: 6.04 g, 7%). ¹H-NMR (DMSO-d₆, 300 MHz):δ1.18 (6H, s), 3.54 (2H, s), 3.81 (3H, s), 4.76 (1H, s), 6.93 (1H, d, J = 8.9 Hz), 7.27 (1H, dd, J = 8.3, 1.7 Hz), 7.33 (1H, d, J = 1.7 Hz), 7.38 (1H, d, J = 8.3 Hz), 8.03 (1H, dd, J = 8.9, 1.7 Hz), 8.06 (1H, s), 8.33 (1H, d, J = 1.7 Hz), 9.22 (1H, brs).

### Example 117

### (5Z)-5-[(4-{[2,4-bis(trifluoromethyl)benzyl]oxy}-3-nitrophenyl)methylidene]-4-(methylamino)-1,3-thiazol-2(5H)-one

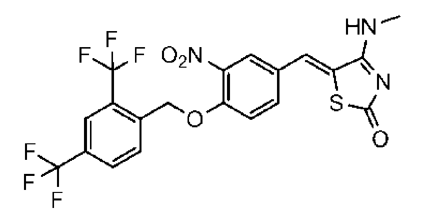

4-{[2,4-bis(Trifluoromethyl)benzyl]oxy}-3-nitrobenzaldehyde (6.1 g) and 4-(methylamino)-1,3-thiazol-2(5H)-one (2.3 g) were dissolved in ethanol (50 mL), and potassium tert-butoxide (5.2 g) was added, and the mixture was stirred at 70°C for 2 hr. The reaction mixture was cooled to room temperature, and the precipitate was collected by filtration to give the title compound as a yellow powder (yield: 5.5 g, 70%).
¹H-NMR (DMSO-d₆, 400MHz):δ3.07 (3H, s), 5.62 (2H, s), 7.64 (1H, d, J = 8.8 Hz), 7.77 (1H, s), 7.84 (1H, dd, J = 8.8 Hz, 2.0 Hz), 8.06-8.13 (3H, m), 8.24 (1H, d, J = 8.4 Hz), 9.50 (1H, br s).

### Example 118

### (5Z)-4-amino-5-({3-methoxy-4-[(4-methoxybenzyl)oxy]phenyl}methylidene)-1,5-dihydro-2H-imidazol-2-one

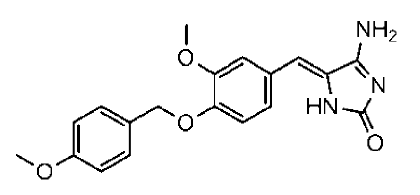

To a solution (8 mL) of 5-imino-1-(phenylcarbonyl)imidazolidin-2-one (402 mg) in ethanol were added potassium tert-butoxide (97.4 mg) and 3-methoxy-4-[(4-methoxybenzyl)oxy]benzaldehyde (300 mg) and the mixture was heated under reflux for 16 hr under a nitrogen atmosphere. Water was added to the reaction mixture, and the mixture was extracted with ethyl acetate/tetrahydrofuran. The organic layer was washed with saturated brine, dried over anhydrous magnesium sulfate, and filtered, and the filtrate was concentrated under reduced pressure. The residue was washed with ethyl acetate to give the title compound as a red solid (yield: 170 mg, 28%).
¹H-NMR (DMSO-d₆, 300 MHz):δ3.76 (3H, s), 3.83 (3H, s), 5.03 (2H, s), 6.46 (1H, s), 6.90-7.09 (5H, m), 7.37 (2H, d, J = 8.5 Hz), 8.00 (2H, brs), 9.76 (1H, s).

### Example 119

### N-(2-{[2,4-bis(trifluoromethyl)benzyl]oxy}-5-{(Z)-[4-(methylamino)-2-oxo-1,3-thiazol-5(2H)-ylidene]methyl}phenyl)acetamide

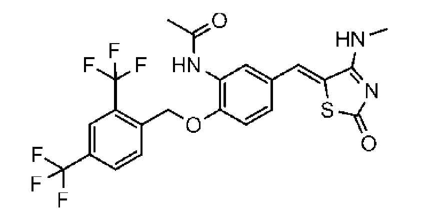

(5Z)-5-[(3-Amino-4-{[2,4-bis(trifluoromethyl)benzyl]oxy}phenyl)methylidene]-4-(methylamino)-1,3-thiazol-2(5H)-one (203.3 mg) was dissolved in pyridine (1.4 mL) and acetic anhydride (0.7 mL), and the mixture was stirred at room temperature for 15 min. The precipitate was collected by filtration, washed with ethyl acetate and recrystallized from dimethyl sulfoxide/water to give the title compound as yellow crystals (yield: 152.2 mg, 69%).
¹H-NMR (DMSO-d₆, 300 MHz):δ2.13 (3H, s), 3.04 (3H, s), 5.51 (2H, s), 7.18 (1H, d, J = 8.5 Hz), 7.22-7.31 (1H, m, J = 8.5 Hz), 7.72 (1H, s), 8.01-8.22 (3H, m), 8.29 (1H, brs), 9.27-9.45 (2H, m).

### Example 120

### N-(2-{[2,4-bis(trifluoromethyl)benzyl]oxy}-5-{(Z)-[4-(methylamino)-2-oxo-1,3-thiazol-5(2H)-ylidenelmethyllphenyl)methanesulfonamide

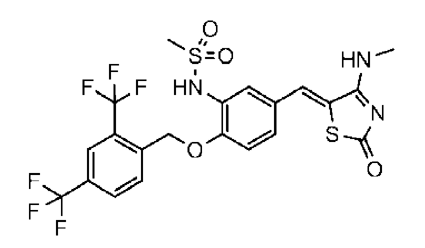

(5Z)-5-[(3-Amino-4-{[2,4-bis(trifluoromethyl)benzyl]oxy}phenyl)methylidene]-4-(methylamino)-1,3-thiazol-2(5H)-one (202.9 mg) was dissolved in tetrahydrofuran (2 mL), and triethylamine (0.0714 mL) and methanesulfonyl chloride (0.0361 mL) were added, and the mixture was stirred at 50°C for 1 hr. The reaction mixture was poured into aqueous ammonium chloride solution, and the mixture was extracted with ethyl acetate. The organic layer was washed with saturated brine, dried over magnesium sulfate, and concentrated under reduced pressure. The residue was recrystallized from methanol/water, purified by silica gel column chromatography (eluent: hexane-ethyl acetate=3:7→1:19), further purified by preparative HPLC and recrystallized from methanol/water to give the title compound as pale-yellow crystals (yield: 35.8 mg, 15%).
¹H-NMR (DMSO-d₆, 300 MHz):δ2.95 (3H, s), 3.04 (3H, d, J = 1.7 Hz), 5.48 (2H, s), 7.22 (1H, d, J = 8.7 Hz), 7.32 (1H, dd, J = 8.7, 2.3 Hz), 7.56 (1H, d, J = 2.3 Hz), 8.12 (1H, s), 8.17-8.21 (2H, m), 9.40 (2H, brs).

### Example 121

### 2-{[2,4-bis(trifluoromethyl)benzyl]oxy}-5-{(Z)-[4-(methylamino)-2-oxo-1,3-thiazol-5(2H)-ylidene]methyl}benzamide

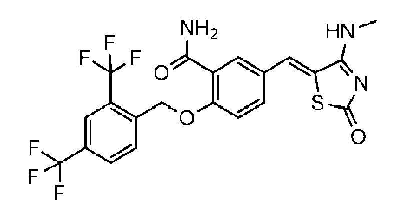

To a solution (7.3 mL) of 2-{[2,4-bis(trifluoromethyl)benzyl]oxy}-5-{(Z)-[4-(methylamino)-2-oxo-1,3-thiazol-5(2H)-ylidene]methyllbenzoic acid (732.0 mg) and 1-hydroxybenzotriazole ammonium salt (331.7 mg) in N,N-dimethylformamide was added 1-ethyl-3-(3-dimethylaminopropyl)carbodiimide hydrochloride (417.3 mg), and the mixture was stirred at room temperature for 2 hr. To the reaction mixture was added dropwise water, and the precipitate was collected by filtration, washed with 1 mol/L hydrochloric acid, saturated aqueous sodium hydrogen carbonate and diisopropylether and recrystallized from ethyl acetate/hexane to give the title compound as brown crystals (yield: 427.1 mg, 59%).
¹H-NMR (DMSO-d₆, 300 MHz):δ3.05 (3H, d, J = 4.1 Hz), 5.56 (2H, s), 7.31 (1H, d, J = 8.9 Hz), 7.62 (1H, dd, J = 8.9, 2.3 Hz), 7.67 (2H, d, J = 4.1 Hz), 7.78 (1H, s), 7.91 (1H, d, J = 2.3 Hz), 8.07 (1H, d, J = 7.9 Hz), 8.11-8.21 (2H, m), 9.38 (1H, d, J = 4.1 Hz).

### Example 122

### 2-{[2,4-bis(trifluoromethyl)benzyl]oxy}-N-methyl-5-{(Z)-[4-(methylamino)-2-oxo-1,3-thiazol-5(2H)-ylidene]methyl}benzamide

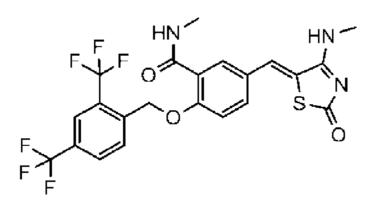

To a solution (2 mL) of 2-{[2,4-bis(trifluoromethyl)benzyl]oxy}-5-{(Z)-[4-(methylamino)-2-oxo-1,3-thiazol-5(2H)-ylidene]methyllbenzoic acid (200.0 mg), 2 mol/L methylamine-tetrahydrofuran solution (0.298 mL) and 1-hydroxybenzotriazole (80.4 mg) in N,N-dimethylformamide was added 1-ethyl-3-(3-dimethylaminopropyl)carbodiimide hydrochloride (114.0 mg), and the mixture was stirred at room temperature for 2 hr. To the reaction mixture was added dropwise water, and the precipitate was collected by filtration, washed with 1 mol/L hydrochloric acid, saturated aqueous sodium hydrogen carbonate and diisopropylether, and recrystallized from ethyl acetate/hexane to give the title compound as brown crystals (yield: 142.8 mg, 70%).
¹H-NMR (DMSO-d₆, 300 MHz):δ2.81 (3H, d, J = 4.7 Hz), 3.05 (3H, d, J = 4.5 Hz), 5.56 (2H, s), 7.28 (1H, d, J = 8.9 Hz), 7.60 (1H, dd, J = 8.9, 2.4 Hz), 7.77 (1H, s), 7.83 (1H, d, J = 2.4 Hz), 8.02 (1H, d, J = 8.5 Hz), 8.13 (1H, s), 8.19 (1H, d, J = 8.5 Hz), 8.27 (1H, d, J = 4.7 Hz), 9.38 (1H, d, J = 4.5 Hz).

### Example 123

### (4Z)-1-ethyl-5-imino-4-({3-methoxy-4-[(4-methoxybenzyl)oxy]phenyl}methylidene)-3-methylimidazolidin-2-one

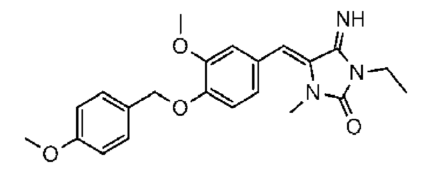

To a solution (150 mL) of (4Z)-1-ethyl-5-imino-4-({3-methoxy-4-[(4-methoxybenzyl)oxy]phenyl}methylidene)imidazolidin-2-one (31.4 g) in N,N-dimethylformamide was added a solution (50 mL) of potassium carbonate (17.2 g) and iodomethane (13.5 g) in N,N-dimethylformamide, and the mixture was stirred at 60°C for 3 hr. Water was added to the reaction mixture, and the mixture was extracted with ethyl acetate. The organic layer was washed with water and saturated brine, dried over anhydrous magnesium sulfate, and filtered, and the filtrate was concentrated under reduced pressure. The residue was purified by silica gel column chromatography (eluent: hexane-ethyl acetate=70:30→30:70) to give the title compound as a yellow solid (yield: 28.3 g, 87%). The obtained solid was recrystallized from ethyl acetate/n-heptane to give the title compound as a pale-yellow solid (yield: 26.6 g, 82%).
¹H-NMR (DMSO-d₆, 300 MHz):δ1.11 (3H, t, J = 7.1 Hz), 2.79 (3H, s), 3.58 (2H, q, J = 7.1 Hz), 3.76 (3H, s), 3.77 (3H, s), 5.00 (2H, s), 6.77 (1H, s), 6.82 (1H, dd, J = 8.3, 1.5 Hz), 6.90 (1H, d, J = 1.5 Hz), 6.92-6.98 (2H, m), 7.04 (1H, d, J = 8.3 Hz), 7.33-7.41 (2H, m), 8.85 (1H, brs).

### Example 124

### (4Z,5E)-4-[(4-{[2,4-bis(trifluoromethyl)benzyl]oxy}-3-methoxyphenyl)methylidene]-1-ethyl-3-methyl-5-(methylimino)imidazolidin-2-one

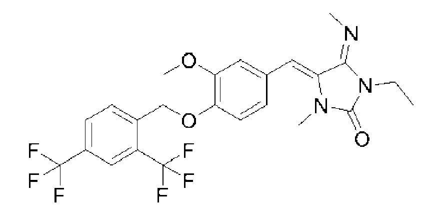

To a solution of (4Z,5E)-4-[(4-{[2,4-bis(trifluoromethyl)benzyl]oxy}-3-methoxyphenyl)methylidene]-1-ethyl-5-(methylimino)imidazolidin-2-one (200 mg) and potassium carbonate (110 mg) in N,N-dimethylformamide (5 mL) was added iodomethane (114 mg), and the mixture was stirred at 60°C overnight. To the reaction mixture were added ethyl acetate and water, and the mixture was extracted with ethyl acetate. The organic layer was washed with saturated brine, dried over anhydrous magnesium sulfate, and filtered, and the filtrate was concentrated under reduced pressure. The residue was purified by silica gel column chromatography (eluent: hexane-ethyl acetate=60:40→40:60) to give the title compound as a white solid (yield 150 mg, 73%).
¹H-NMR (DMSO-d₆, 300 MHz):δ1.07 (3H, t, J = 7.0 Hz), 2.73 (3H, s), 3.42 (3H, s), 3.54 (2H, q, J = 6.9 Hz), 3.81 (3H, s), 5.34 (2H, s), 6.72 (1H, s), 6.86 (1H, dd, J = 8.3, 1.5 Hz), 6.99-7.09 (2H, m), 8.06 (1H, s), 8.09 (1H, s), 8.13-8.22 (1H, m).

### Example 125

### (4Z)-4-[(4-{[2,4-bis(trifluoromethyl)benzyl]oxy}-3-ethoxyphenyl)methylidene]-1-ethyl-5-iminoimidazolidin-2-one

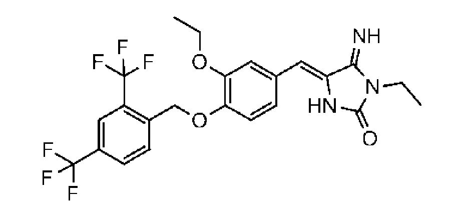

To a solution (6.4 mL) of 1-(cyanomethyl)-3-ethylurea (178.3 mg) in ethanol was added potassium tert-butoxide (171.0 mg) and the mixture was stirred at 0°C for 1 hr. To the reaction mixture were added 4-{[2,4-bis(trifluoromethyl)benzyl]oxy}-3-ethoxybenzaldehyde (500.0 mg) and potassium tert-butoxide (171.0 mg) and the mixture was heated under reflux for 4.5 hr. The reaction mixture was concentrated under reduced pressure, and the residue was suspended in ethyl acetate/water. The precipitate was collected by filtration, and washed with ethyl acetate to give the title compound as a pale-yellow powder (yield: 482.0 mg, 76%).
¹H-NMR (CDCl₃, 300 MHz):δ1.26 (3H, t, J = 7.2 Hz), 1.48 (3H, t, J = 7.0 Hz), 3.71 (2H, q, J = 7.2 Hz), 4.14 (2H, q, J = 7.0 Hz), 5.40 (2H, s), 6.30 (1H, s), 6.88-6.92 (3H, m), 7.44 (1H, brs), 7.85 (1H, d, J = 8.3 Hz), 7.94 (1H, s), 8.04 (1H, d, J = 8.3 Hz).

### Example 126

### (4Z,5E)-1-ethyl-4-[(3-methoxy-4-{[2-(trifluoromethyl)benzyl]oxylphenyl)methylidenel-3-methyl-5-(methylimino)imidazolidin-2-one

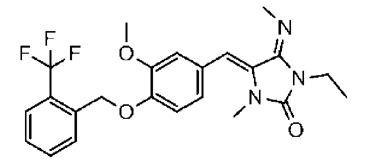

To a solution (3 mL) of (4Z)-1-ethyl-5-imino-4-[(3-methoxy-4-{[2-(trifluoromethyl)benzyl]oxy}phenyl)methylidene]-3-methylimidazolidin-2-one (300 mg) in N,N-dimethylformamide was added a solution (1 mL) of potassium carbonate (199 mg) and iodomethane (160 mg) in N,N-dimethylformamide, and the mixture was stirred at 60°C for 16 hr. Water was added to the reaction mixture, and the mixture was extracted with ethyl acetate. The organic layer was washed with saturated aqueous sodium thiosulfate solution, water and saturated brine, dried over anhydrous magnesium sulfate, and filtered, and the filtrate was concentrated under reduced pressure. The residue was purified by silica gel column chromatography (eluent: hexane-ethyl acetate=70:30→20:80) and the obtained solid was recrystallized from ethyl acetate/n-heptane to give the title compound as a white solid (yield: 45 mg, 14%).
¹H-NMR (CDCl₃, 300 MHz):δ1.20 (3H, t, J = 7.1 Hz), 2.82 (3H, s), 3.49 (3H, s), 3.68 (2H, q, J = 7.1 Hz), 3.92 (3H, s), 5.37 (2H, s), 6.64 (1H, s), 6.71-6.87 (3H, m), 7.37-7.46 (1H, m), 7.53-7.62 (1H, m), 7.69 (1H, d, J = 7.7 Hz), 7.80 (1H, d, J = 7.7 Hz).

### Example 127

### (4Z)-4-({4-[2-chloro-4-(trifluoromethyl)phenoxy]-3-methoxyphenyl}methylidene)-1-ethyl-5-imino-3-methylimidazolidin-2-one

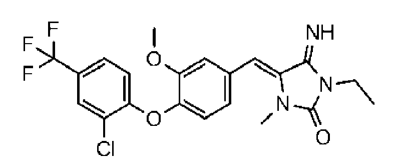

To a solution (1 mL) of (4Z)-4-({4-[2-chloro-4-(trifluoromethyl)phenoxy]-3-methoxyphenyl}methylidene)-1-ethyl-5-iminoimidazolidin-2-one (70 mg) in N,N-dimethylformamide was added a solution (1 mL) of potassium carbonate (44 mg) and iodomethane (29 mg) in N,N-dimethylformamide, and the mixture was stirred at room temperature for 2 hr. Water was added to the reaction mixture, and the mixture was extracted with ethyl acetate. The organic layer was washed with water and saturated brine, dried over anhydrous magnesium sulfate, and filtered, and the filtrate was concentrated under reduced pressure. The residue was purified by silica gel column chromatography (eluent: hexane-ethyl acetate=70:30→40:60) to give the title compound as a pale-yellow solid (yield: 47 mg, 64%). The obtained solid was recrystallized from ethyl acetate/n-heptane to give the title compound as a white solid (yield: 30 mg, 42%).
¹H-NMR (CDCl₃, 300 MHz):δ1.28 (3H, t, J = 7.1 Hz), 2.92 (3H, s), 3.67-3.79 (2H, m), 3.81 (3H, s), 6.42 (1H, brs), 6.77 (1H, d, J = 8.7 Hz), 6.84-6.90 (1H, m), 6.91 (1H, s), 7.02 (1H, d, J = 8.1 Hz), 7.37-7.44 (1H, m), 7.74 (1H, brs), 7.72 (1H, d, J = 1.7 Hz).

### Example 128

### (5Z)-5-[(4-{[2,4-bis(trifluoromethyl)benzyl]oxy}-2-bromo-5-methoxyphenyl)methylidene]-4-(methylamino)-1,3-thiazol-2(5H)-one

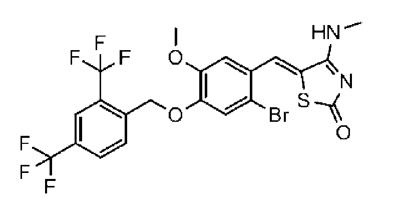

To a solution (10 mL) of 4-(methylamino)-1,3-thiazol-2(5H)-one (342 mg) in ethanol were added potassium tert-butoxide (347 mg) and 4-{[2,4-bis(trifluoromethyl)benzyl]oxy}-2-bromo-5-methoxybenzaldehyde (1.00 g) and the mixture was heated under reflux for 3 hr under a nitrogen atmosphere. To the reaction mixture was added saturated aqueous ammonium chloride solution, and the mixture was extracted with ethyl acetate. The organic layer was washed with saturated brine, dried over anhydrous magnesium sulfate, and filtered, and the filtrate was concentrated under reduced pressure. The residue was purified by basic silica gel column chromatography (eluent: hexane-ethyl acetate=50:50→0:100) to give the title compound as a yellow solid (yield: 1.13 g, 90%). The obtained solid was recrystallized from ethyl acetate/n-heptane to give the title compound as a yellow solid (yield: 468 mg, 38%). ¹H-NMR (300 MHz, DMSO-d₆):δ3.06 (3H, s), 3.82 (3H, s), 5.42 (2H, s), 7.22 (1H, s), 7.47 (1H, s), 7.73 (1H, s), 8.04 (1H, d, J = 8.3 Hz), 8.11 (1H, s), 8.19 (1H, d, J = 8.3 Hz), 9.65 (1H, s).

### Example 129

### (4E,5Z)-5-[(4-{[2,4-bis(trifluoromethyl)benzyl]oxy}-3-fluorophenyl)methylidene]-3-methyl-4-(methylimino)-1,3-thiazolidin-2-one

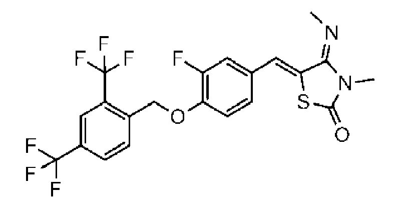

To a solution (1.4 mL) of (5Z)-5-[(4-{[2,4-bis(trifluoromethyl)benzyl]oxy}-3-fluorophenyl)methylidene]-4-(methylamino)-1,3-thiazol-2(5H)-one (134.8 mg) and potassium carbonate (58.5 mg) in N,N-dimethylformamide was added methyl iodide (0.0263 mL), and the mixture was stirred at 60°C for 2 hr. Methyl iodide (0.0526 mL) was added and the mixture was stirred for 2 hr. The reaction mixture was poured into water, and the mixture was extracted with ethyl acetate. The organic layer was washed with saturated brine, dried over magnesium sulfate, and concentrated under reduced pressure. The residue was purified by silica gel column chromatography (eluent: hexane-ethyl acetate=2:3→1:9) and recrystallized from ethyl acetate/hexane to give the title compound as colorless crystals (yield: 20.2 mg, 15%).
¹H-NMR (DMSO-d₆, 300 MHz):δ3.06 (3H, s), 3.61 (3H, s), 5.50 (2H, s), 7.36-7.55 (3H, m), 7.69 (1H, s), 8.06 (1H, d, J = 8.1 Hz), 8.12 (1H, s), 8.19 (1H, d, J = 8.1 Hz).

### Example 130

### (4E,5Z)-5-[(4-{[2,4-bis(trifluoromethyl)benzyl]oxy}-3-methoxyphenyl)methylidene]-3-methyl-4-(methylimino)-1,3-thiazolidin-2-one

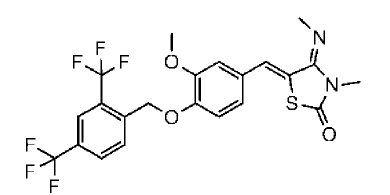

To a solution (1.5 mL) of (5Z)-5-[(4-{[2,4-bis(trifluoromethyl)benzyl]oxy}-3-methoxyphenyl)methylidene]-4-(methylamino)-1,3-thiazol-2(5H)-one (147.5 mg) and potassium carbonate (62.4 mg) in N,N-dimethylformamide was added methyl iodide (0.0624 mL), and the mixture was stirred at room temperature for 3 hr. Methyl iodide (0.0624 mL) was added, and the mixture was stirred for 2 hr. Methyl iodide (0.0624 mL) was added, and the mixture was stirred at 60°C for 2 hr. The reaction mixture was poured into water, and the mixture was extracted with ethyl acetate. The organic layer was washed with water and saturated brine, dried over magnesium sulfate, and concentrated under reduced pressure. The residue was purified by silica gel column chromatography (eluent: hexane-ethyl acetate=1:0→1:9) to give two kinds of compounds. A less polar compound was recrystallized from diethylether/hexane to give the title compound as pale-yellow crystals (yield: 18.7 mg, 12%). A highly-polar compound was recrystallized from ethyl acetate/hexane to give (5Z)-5-[(4-{[2,4-bis(trifluoromethyl)benzyl]oxy}-3-methoxyphenyl)methylidene]-4-(dimethylamino)-1,3-thiazol-2(5H)-one as pale-yellow crystals (yield: 41.1 mg, 27%).
¹H-NMR (CDCl₃, 300 MHz):δ3.17 (3H, s), 3.66 (3H, s), 3.96 (3H, s), 5.43 (2H, s), 6.88 (1H, d, J = 8.5 Hz), 6.99 (1H, dd, J = 8.5, 1.9 Hz), 7.04 (1H, d, J = 1.9 Hz), 7.47 (1H, s), 7.85 (1H, d, J = 8.5 Hz), 7.95 (1H, s), 7.99 (1H, d, J = 8.5 Hz).

### Example 131

### (4E,5Z)-5-[(4-{[2,4-bis(trifluoromethyl)benzyl]oxy}-3-methoxyphenyl)methylidene]-3-ethyl-4-(methylimino)-1,3-thiazolidin-2-one

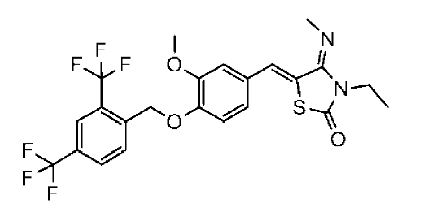

To a solution (1.6 mL) of (5Z)-5-[(4-{[2,4-bis(trifluoromethyl)benzyl]oxy}-3-methoxyphenyl)methylidene]-4-(methylamino)-1,3-thiazol-2(5H)-one (152.4 mg) and potassium carbonate (64.5 mg) in N,N-dimethylformamide were added ethyl bromide (0.116 mL) and n-tetrabutylammonium iodide (11.5 mg), and the mixture was stirred at room temperature for 3 hr. Ethyl bromide (0.116 mL) was added, and the mixture was stirred for 3 hr. Ethyl bromide (0.116 mL) was added, and the mixture was stirred at 60°C for 2 hr. The reaction mixture was poured into water, and the mixture was extracted with ethyl acetate. The organic layer was washed with water and saturated brine, dried over magnesium sulfate, and concentrated under reduced pressure. The residue was purified by silica gel column chromatography (eluent: hexane-ethyl acetate=1:0→1:9) to give two kinds of compounds. A less polar compound was recrystallized from diethylether/hexane to give the title compound as pale-yellow crystals (yield: 12.4 mg, 8%).
¹H-NMR (CDCl₃, 300 MHz):δ1.19 (3H, t, J = 7.2 Hz), 3.66 (3H, d, J = 0.6 Hz), 3.81 (2H, q, J = 7.2 Hz), 3.96 (3H, s), 5.43 (2H, s), 6.88 (1H, d, J = 8.3 Hz), 6.98 (1H, dd, J = 8.3, 2.1 Hz), 7.04 (1H, d, J = 2.1 Hz), 7.46 (1H, s), 7.85 (1H, d, J = 8.3 Hz), 7.95 (1H, s), 7.99 (1H, d, J = 8.3 Hz).

### Example 132

### (5Z)-4-amino-5-{[4-(benzyloxy)-3-methoxyphenyl]methylidene}-1,5-dihydro-2H-imidazol-2-one

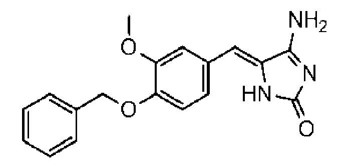

To a solution (4 mL) of (5Z)-4-amino-5-[(4-hydroxy-3-methoxyphenyl)methylidene]-1,5-dihydro-2H-imidazol-2-one (60.0 mg) in tetrahydrofuran were added a solution (2 mL) of benzyl alcohol (84.3 mg) in tetrahydrofuran, a 40% solution (0.35 mL) of diethyl azodicarboxylate in toluene and triphenylphosphine (209 mg), and the mixture was stirred at room temperature for 16 hr and heated under reflux for 4 hr. To the reaction mixture was added 1 N aqueous sodium hydroxide solution, and the mixture was extracted with ethyl acetate. The organic layer was washed with saturated brine, dried over anhydrous magnesium sulfate, and filtered, and the filtrate was concentrated under reduced pressure. The residue was purified by silica gel column chromatography (eluent: ethyl acetate-methanol=100:0→90:10) to give the title compound as a pale-yellow solid (yield: 22 mg, 26%). The obtained solid was recrystallized from isopropanol to give the title compound as a pale-yellow solid (yield: 5 mg, 6%).
¹H-NMR (DMSO-d₆, 300 MHz):δ3.85 (3H, s), 5.12 (2H, s), 6.46 (1H, s), 6.98-7.09 (3H, m), 7.30-7.51 (5H, m), 8.00 (2H, brs), 9.77 (1H, s).

### Example 133

### (5Z)-5-[(4-{[2,4-bis(trifluoromethyl)benzyl]oxy}-3-methoxyphenyl)methylidene]-4-{[2-(1-methylpyrrolidin-2-yl)ethyl]amino}-1,5-dihydro-2H-imidazol-2-one

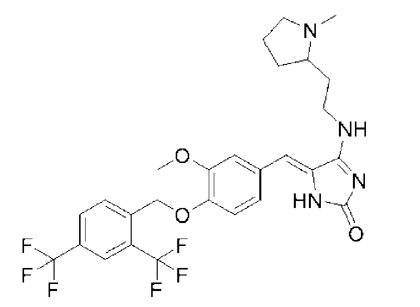

A suspension of (5Z)-4-amino-5-[(4-{[2,4-bis(trifluoromethyl)benzyl]oxy}-3-methoxyphenyl)methylidene]-1,5-dihydro-2H-imidazol-2-one (230 mg) and 2-(1-methylpyrrolidin-2-yl)ethanamine (77 mg) in methanol (5 mL) was stirred under microwave irradiation at 140°C for 1 hr. The reaction mixture was cooled, and concentrated under reduced pressure, and the residue was purified by silica gel column chromatography (eluent: ethyl acetate-methanol=50:50→0:100) and recrystallized (ethyl acetate) to give the title compound as a white solid (yield: 160 mg, 56%).
¹H-NMR (DMSO-d₆, 300 MHz):δ1.32 - 1.73 (4H, m), 1.82-2.14 (4H, m), 2.22 (3H, s), 2.87-2.99 (1H, m), 3.34-3.44 (2H, m), 3.87 (3H, s), 5.36 (2H, s), 6.42 (1H, s), 6.97-7.09 (3H, m), 8.03 (1H, d, J = 8.1 Hz), 8.10 (1H, s), 8.17 (1H, d, J = 8.1 Hz), 8.48 (1H, brs), 9.84 (1H, s).

### Example 134

### (5Z)-5-[(4-{[2,4-bis(trifluoromethyl)benzyl]oxy}-3-methoxyphenyl)methylidene]-4-{[3-(1H-imidazol-1-yl)propyl]amino}-1,5-dihydro-2H-imidazol-2-one

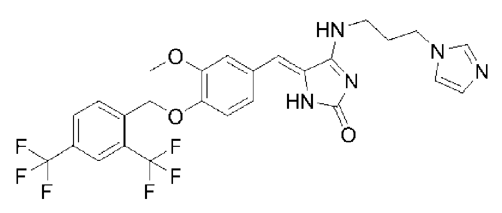

A suspension of (5Z)-4-amino-5-[(4-{[2,4-bis(trifluoromethyl)benzyl]oxy}-3-methoxyphenyl)methylidene]-1,5-dihydro-2H-imidazol-2-one (230 mg) and 3-(1H-imidazol-1-yl)propan-1-amine (125 mg) in methanol (5 mL) was stirred under microwave irradiation at 140°C for 1 hr. The reaction mixture was cooled, and concentrated under reduced pressure, and the residue was purified by silica gel column chromatography (eluent: ethyl acetate-methanol=90:10→50:50) to give the title compound as a white solid (yield: 200 mg, 70%).
¹H-NMR (DMSO-d₆, 300 MHz):δ1.97-2.10 (2H, m), 3.34 (2H, brs), 3.88 (3H, s), 4.05 (2H, t, J = 6.9 Hz), 5.36 (2H, s), 6.44 (1H, s), 6.90 (1H, s), 6.98-7.13 (3H, m), 7.23 (1H, s), 7.68 (1H, s), 8.03 (1H, d, J = 8.1 Hz), 8.10 (1H, s), 8.16 (1H, s), 8.48 (1H, t, J = 5.4 Hz), 9.88 (1H, s).

### Example 135

### (5Z)-4-amino-5-[(4-{[2,4-bis(trifluoromethyl)benzyl]oxy}-3-methoxyphenyl)methylidene]-1-methyl-1,5-dihydro-2H-imidazol-2-one

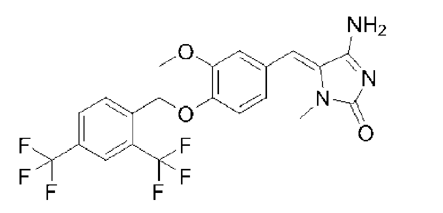

4-{[2,4-bis(Trifluoromethyl)benzyl]oxy}-3-methoxybenzaldehyde (3.78 g) and 4-imino-1-methyl-3-(phenylcarbonyl)imidazolidin-2-one (2.39 g) were dissolved in ethanol (100 mL), potassium tert-butoxide (1.23 g) was added, and the mixture was stirred under refluxing conditions for 4 hr. Ethanol was removed under reduced pressure, the residue was diluted with ethyl acetate and water, and the mixture was extracted with ethyl acetate. The organic layer was washed with saturated brine, dried over anhydrous magnesium sulfate, and filtered, and the filtrate was concentrated under reduced pressure. The residue was purified by silica gel column chromatography and recrystallized (ethyl acetate) to give the title compound as a yellow solid (yield 846 mg, 18%).
¹H-NMR (DMSO-d₆, 300 MHz):δ2.76 (3H, s), 3.81 (3H, s), 5.35 (2H, s), 6.71 (1H, s), 6.86 (1H, dd, J = 8.3, 1.7 Hz), 6.96 (1H, d, J = 1.7 Hz), 7.05 (1H, d, J = 8.3 Hz), 7.93-8.38 (5H, m).

### Example 136

### (5Z)-5-[(4-{[2,4-bis(trifluoromethyl)benzyl]oxy}-3-methoxyphenyl)methylidene]-1-methyl-4-{[2-(1-methylpyrrolidin-2-yl)ethyl]amino}-1,5-dihydro-2H-imidazol-2-one

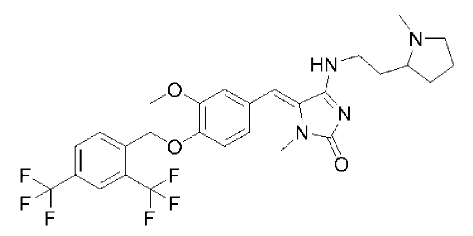

A suspension of (5Z)-4-amino-5-[(4-{[2,4-bis(trifluoromethyl)benzyl]oxy}-3-methoxyphenyl)methylidene]-1-methyl-1,5-dihydro-2H-imidazol-2-one (95 mg) and 2-(1-methylpyrrolidin-2-yl)ethanamine (30 mg) in methanol (5 mL) was stirred under microwave irradiation at 140°C for 1 hr. The reaction mixture was cooled, and concentrated under reduced pressure, and the residue was purified by silica gel column chromatography (eluent: ethyl acetate-methanol=50:50→0:100) and recrystallized (ethyl acetate and hexane) to give the title compound as a white solid (yield: 80 mg, 70%).
¹H-NMR (DMSO-d₆, 300 MHz)):δ1.33-1.75 (4H, m), 1.92 (2H, m), 2.08 (2H, m), 2.23 (3H, s), 2.76 (3H, s), 2.95 (1H, brs), 3.34-3.49 (2H, m), 3.81 (3H, s), 5.34 (2H, s), 6.66 (1H, s), 6.86 (1H, dd, J = 8.1, 1.5 Hz), 6.96 (1H, d, J = 1.7 Hz), 7.05 (1H, d, J = 8.3 Hz), 8.05 (1H, d, J = 8.3 Hz), 8.10 (1H, s), 8.14-8.21 (1H, d, J=8.7 Hz), 8.63 (1H, brs).

### Example 137

### 4-{4-[(Z)-(5-amino-2-oxo-2,3-dihydro-4H-imidazol-4-ylidene)methyl]-2-methoxyphenoxy}-3-(trifluoromethyl)benzonitrile

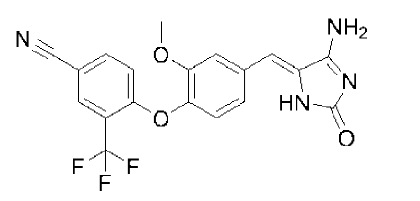

To a suspension of 4-(4-formyl-2-methoxyphenoxy)-3-(trifluoromethyl)benzonitrile (1.25 g) and 5-imino-1-(phenylcarbonyl)imidazolidin-2-one (948 mg) in ethanol (20 mL) was added potassium tert-butoxide (524 mg), and the mixture was stirred at 90°C for 30 min. To the reaction mixture was added saturated aqueous ammonium chloride solution, and ethanol was removed under reduced pressure. The mixture was diluted with ethyl acetate and water, and the mixture was extracted with ethyl acetate. The organic layer was washed with saturated brine, dried over anhydrous magnesium sulfate, and filtered, and the filtrate was concentrated under reduced pressure. The residue was purified by silica gel column chromatography (eluent: ethyl acetate-methanol=100:0→90:10) and recrystallized (tetrahydrofuran) to give the title compound as a white solid (yield 320 mg, 20%).
¹H-NMR (DMSO-d₆, 300 MHz):δ3.81 (3H, s), 6.55 (1H, s), 6.85 (1H, d, J = 8.7 Hz), 7.11-7.18 (1H, m), 7.18-7.26 (2H, m), 8.03 (1H, dd, J = 8.7, 1.9 Hz), 8.06-8.29 (2H, brs), 8.31 (1H, d, J = 1.7 Hz), 9.97 (1H, s).

### Example 138

### 4-{2-methoxy-4-[(Z)-(5-{[2-(1-methylpyrrolidin-2-yl)ethyl]aminol-2-oxo-2,3-dihydro-4H-imidazol-4-ylidene)methyl]phenoxy}-3-(trifluoromethyl)benzonitrile

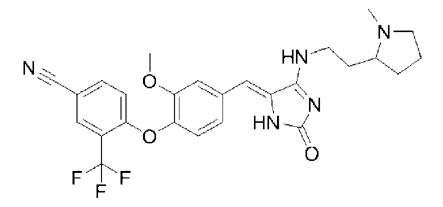

A suspension of 4-{4-[(Z)-(5-amino-2-oxo-2,3-dihydro-4H-imidazol-4-ylidene)methyl]-2-methoxyphenoxy}-3-(trifluoromethyl)benzonitrile (130 mg) and 2-(1-methylpyrrolidin-2-yl)ethanamine (49 mg) in methanol (5 mL) was stirred under microwave irradiation at 140°C for 1 hr. The reaction mixture was cooled, and concentrated under reduced pressure, and the residue was purified by silica gel column chromatography (eluent: ethyl acetate-methanol=50:50→0:100) and the obtained yellow solid was washed with diethylether to give the title compound as a white solid (yield: 70 mg, 43%).
¹H-NMR (DMSO-d₆, 300 MHz):δ1.36-1.75 (4H, m), 1.83-2.15 (4H, m), 2.23 (3H, s), 2.95 (1H, brs), 3.38-3.46 (2H, m), 3.81 (3H, s), 6.49 (1H, s), 6.85 (1H, d, J = 8.9 Hz), 7.09-7.31 (3H, m), 8.03 (1H, dd, J = 8.8, 2.0 Hz), 8.31 (1H, d, J = 1.3 Hz).

### Example 139

### 4-{4-[(Z)-(5-{[2-(diethylamino)ethyl]amino}-2-oxo-2,3-dihydro-4H-imidazol-4-ylidene)methyl]-2-methoxyphenoxy}-3-(trifluoromethyl)benzonitrile

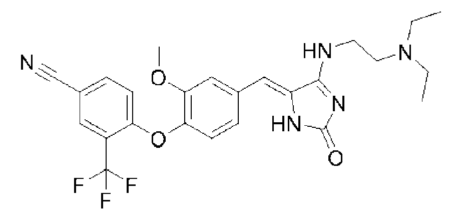

A suspension of 4-{4-[(Z)-(5-amino-2-oxo-2,3-dihydro-4H-imidazol-4-ylidene)methyl]-2-methoxyphenoxy}-3-(trifluoromethyl)benzonitrile (130 mg) and N,N-diethylethane-1,2-diamine (45 mg) in methanol (5 mL) was stirred under microwave irradiation at 140°C for 1 hr. The reaction mixture was cooled, and concentrated under reduced pressure, and the residue was purified by silica gel column chromatography (eluent: ethyl acetate-methanol=50:50-0:100) . The obtained yellow solid was washed with diethylether to give the title compound as a white solid (yield: 85 mg, 53%).
¹H-NMR (DMSO-d₆, 300 MHz):δ0.98 (6H, t, J = 7.1 Hz), 2.52-2.57 (4H, m), 2.63 (2H, t, J = 7.0 Hz), 3.35-3.51 (2H, m), 3.81 (3H, s), 6.51 (1H, s), 6.85 (1H, d, J = 8.9 Hz), 7.06-7.31 (3H, m), 8.02 (1H, dd, J = 8.7, 2.1 Hz), 8.31 (1H, d, J = 1.7 Hz), 10.02 (1H, brs).

### Example 140

### methyl N-{(5Z)-5-[(4-{[2,4-bis(trifluoromethyl)benzyl]oxy}-3-methoxyphenyl)methylidene]-2-oxo-2,5-dihydro-1H-imidazol-4-yl}-β-alaninate

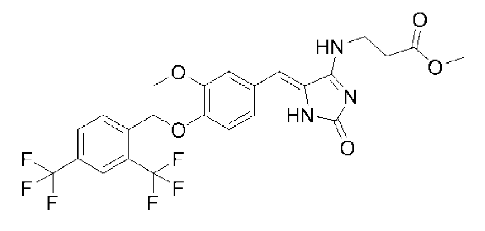

A suspension of (5Z)-4-amino-5-[(4-{[2,4-bis(trifluoromethyl)benzyl]oxy}-3-methoxyphenyl)methylidene]-1,5-dihydro-2H-imidazol-2-one (92 mg) and methyl prop-2-enoate (86 mg) in ethanol (5 mL) was reacted under refluxing conditions overnight, and the reaction mixture was concentrated under reduced pressure, and the residue was purified by silica gel column chromatography (eluent: ethyl acetate-methanol=100:0→97:3) to give the title compound as a yellow solid (yield: 60 mg, 55%).
¹H-NMR (DMSO-d₆, 300 MHz):δ2.65 (2H, t, J = 7.0 Hz), 3.59 (3H, s), 3.77 (2H, t, J = 7.2 Hz), 3.86 (3H, s), 5.35 (2H, s), 6.56 (1H, s), 7.02 (3H, s), 8.03 (1H, d, J = 8.1 Hz), 8.10 (1H, s), 8.13-8.24 (1H, m), 8.79 (1H, s), 10.26 (1H, brs).

### Example 141

### (4Z)-4-[(4-{[2,4-bis(trifluoromethyl)benzyl]oxy}-3-methoxyphenyl)methylidene]-1,3-diethyl-5-iminoimidazolidin-2-one

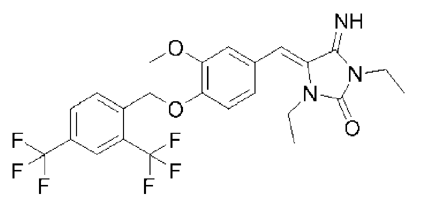

To a solution of (4Z)-4-[(4-{[2,4-bis(trifluoromethyl)benzyl]oxy}-3-methoxyphenyl)methylidene]-1-ethyl-5-iminoimidazolidin-2-one (195 mg) and bromoethane (65 mg) in ethanol (5 mL) was added potassium carbonate (67 mg), and the mixture was stirred at 80°C for 3 hr. The reaction mixture was diluted with ethyl acetate and water, and the mixture was extracted with ethyl acetate. The organic layer was washed with saturated brine, dried over anhydrous magnesium sulfate, and filtered, and the filtrate was concentrated under reduced pressure. The residue was purified by silica gel column chromatography (eluent: hexane-ethyl acetate=50:50→20:80) to give the title compound as a white solid (yield 180 mg, 88%).
¹H-NMR (DMSO-d₆, 300 MHz):δ0.61-0.68 (3H, m), 1.10-1.15 (3H, m), 3.37-3.52 (2H, m), 3.52-3.67 (2H, m), 3.80 (3H, s), 5.35 (2H, s), 6.71-6.91 (2H, m), 6.92-7.10 (2H, m), 7.94-8.25 (3H, m), 8.93 (1H, s).

### Example 142

### (4E)-1-ethyl-5-imino-4-[(3-methoxy-4-{[2-(trifluoromethyl)benzyl]oxylphenyl)methylidenel-3-methylimidazolidin-2-one

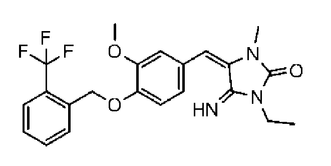

To a solution (3 mL) of (4E)-1-ethyl-4-[(4-hydroxy-3-methoxyphenyl)methylidene]-5-imino-3-methylimidazolidin-2-one (200 mg) in N,N-dimethylformamide was added a solution (2 mL) of potassium carbonate (202 mg) and 1-(bromomethyl)-2-(trifluoromethyl)benzene (217 mg) in N,N-dimethylformamide, and the mixture was stirred at room temperature for 18 hr. To the reaction mixture was added 1 N aqueous sodium hydroxide solution, and the mixture was extracted with ethyl acetate. The organic layer was washed with water and saturated brine, dried over anhydrous magnesium sulfate, and filtered, and the filtrate was concentrated under reduced pressure. The residue was purified by silica gel column chromatography (eluent: hexane-ethyl acetate=60:40→40:60) to give the title compound as a white powder (yield: 191 mg, 61%). The obtained solid was recrystallized from ethyl acetate/n-heptane to give the title compound as a colorless solid (yield: 148 mg, 47%).
¹H-NMR (CDCl₃, 300 MHz):δ1.21 (3H, t, J = 7.1 Hz), 3.12 (3H, s), 3.68 (2H, q, J = 7.1 Hz), 3.91 (3H, s), 5.38 (2H, s), 6.06 (1H, s), 6.79-6.97 (3H, m), 7.36-7.46 (1H, m), 7.52-7.62 (1H, m), 7.69 (1H, d, J = 7.6 Hz), 7.78 (1H, d, J = 7.9 Hz), 7.94 (1H, s).

### Example 143

### 4-{4-[(E)-(1-ethyl-5-imino-3-methyl-2-oxoimidazolidin-4-ylidene)methyl]-2-methoxyphenoxy}-3-(trifluoromethyl)benzonitrile

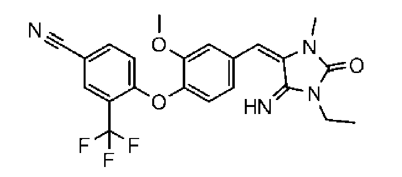

To a solution (70 mL) of (4E)-1-ethyl-4-[(4-hydroxy-3-methoxyphenyl)methylidene]-5-imino-3-methylimidazolidin-2-one (7.00 g) in dimethyl sulfoxide were added lithium carbonate (3.80 g) and 4-fluoro-3-(trifluoromethyl)benzonitrile (5.45 g), and the mixture was stirred at 80°C for 20 hr. Water was added to the reaction mixture, and the mixture was extracted with ethyl acetate. The organic layer was washed with water and saturated brine, dried over anhydrous magnesium sulfate, and filtered, and the filtrate was concentrated under reduced pressure. The residue was purified by silica gel column chromatography (eluent: hexane-ethyl acetate=70:30→100:0) to give the title compound as a white powder (yield: 7.24 g, 64%). The obtained solid was recrystallized from ethyl acetate/n-heptane to give the title compound as a white solid (yield 5.51 g, 49%).
¹H-NMR (CDCl₃, 300 MHz):δ1.23 (3H, t, J = 7.1 Hz), 3.15 (3H, s), 3.69 (2H, q, J = 7.1 Hz), 3.77 (3H, s), 6.09 (1H, s), 6.77 (1H, d, J = 8.7 Hz), 6.98-7.13 (2H, m), 7.18 (1H, d, J = 7.7 Hz), 7.66 (1H, dd, J = 8.7, 1.7 Hz), 7.86 (1H, s), 7.96 (1H, d, J = 1.7 Hz).

### Example 144

### (4Z)-1-ethyl-5-imino-4-[(3-methoxy-4-{[2-(trifluoromethyl)benzyl]oxy}phenyl)methylidene]-3-methylimidazolidin-2-one

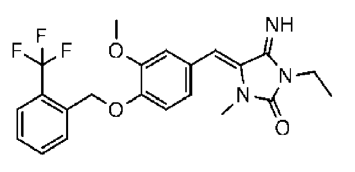

To a solution (3 mL) of (4E)-1-ethyl-4-[(4-hydroxy-3-methoxyphenyl)methylidene]-5-imino-3-methylimidazolidin-2-one (200 mg) in N,N-dimethylformamide was added a solution (2 mL) of potassium carbonate (202 mg) and 1-(bromomethyl)-2-(trifluoromethyl)benzene (217 mg) in N,N-dimethylformamide, and the mixture was stirred at room temperature for 18 hr. To the reaction mixture was added 1 N aqueous sodium hydroxide solution, and the mixture was extracted with ethyl acetate. The organic layer was washed with water and saturated brine, dried over anhydrous magnesium sulfate, and filtered, and the filtrate was concentrated under reduced pressure. The residue was purified by silica gel column chromatography (eluent: hexane-ethyl acetate=60:40→40:60) to give the title compound as a white powder (yield: 98 mg, 31%). The obtained solid was recrystallized from ethyl acetate/n-heptane to give the title compound as a white solid (yield: 68 mg, 22%).
¹H-NMR (CDCl₃, 300 MHz):δ1.27 (3H, t, J = 7.2 Hz), 2.90 (3H, s), 3.63-3.81 (2H, m), 3.91 (3H, s), 5.36 (2H, s), 6.37 (1H, brs), 6.76 (1H, d), 6.83 (1H, d, J = 8.1 Hz), 6.81 (1H, s), 7.36-7.47 (1H, m), 7.53-7.62 (1H, m), 7.69 (1H, d, J = 7.7 Hz), 7.64-7.73 (1H, m), 7.80 (1H, d, J = 7.7 Hz).

### Example 145

### 4-{4-[(Z)-(1-ethyl-5-imino-3-methyl-2-oxoimidazolidin-4-ylidene)methyl]-2-methoxyphenoxy}-3-(trifluoromethyl)benzonitrile

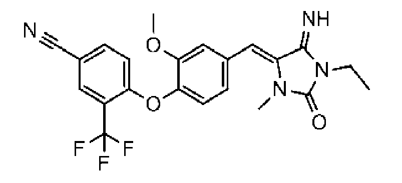

To a solution (25 mL) of 4-{4-[(Z)-(1-ethyl-5-imino-2-oxoimidazolidin-4-ylidene)methyl]-2-methoxyphenoxy}-3-(trifluoromethyl)benzonitrile (4.65 g) in N,N-dimethylformamide was added a solution (3 mL) of potassium carbonate (2.25 g) and iodomethane (1.94 g) in N,N-dimethylformamide, and the mixture was stirred at 60°C for 2 hr. Water was added to the reaction mixture, and the mixture was extracted with ethyl acetate. The organic layer was washed with water and saturated brine, dried over anhydrous magnesium sulfate, and filtered, and the filtrate was concentrated under reduced pressure. The residue was purified by silica gel column chromatography (eluent: hexane-ethyl acetate=50:50→20:80) to give the title compound as a white solid (yield: 4.28 g, 89%). The obtained solid was recrystallized from ethyl acetate/n-heptane to give the title compound as a white solid (yield: 3.96 g, 83%).
¹H-NMR (CDCl₃, 300 MHz):δ1.28 (3H, t, J = 7.2 Hz), 2.93 (3H, s), 3.65-3.84 (2H, m), 3.77 (3H, s), 6.36 (1H, brs), 6.76 (1H, d, J = 8.7 Hz), 6.86-6.97 (2H, m), 7.13 (1H, d, J = 8.7 Hz), 7.67 (1H, dd, J = 8.7, 1.9 Hz), 7.78 (1H, brs), 7.96 (1H, d, J = 1.7 Hz).

### Example 146

### 4-{4-[(Z)-(1-ethyl-5-imino-2-oxoimidazolidin-4-ylidene)methyl]-2-methoxyphenoxy}-3-(trifluoromethyl)benzamide

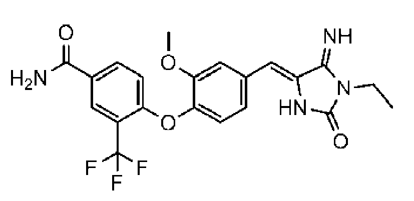

To a solution (10 mL) of 1-(cyanomethyl)-3-ethylurea (380 mg) in ethanol was added potassium tert-butoxide (394 mg) and the mixture was stirred at room temperature for 0.5 hr under a nitrogen atmosphere. To the reaction mixture were added 4-(4-formyl-2-methoxyphenoxy)-3-(trifluoromethyl)benzonitrile (960 mg) and potassium tert-butoxide (394 mg), and the mixture was heated under reflux for 18 hr. To the reaction mixture was added saturated aqueous ammonium chloride solution, and the mixture was concentrated under reduced pressure. To the residue was added water, and the mixture was extracted with ethyl acetate. The organic layer was washed with saturated brine, dried over anhydrous magnesium sulfate, and filtered, and the filtrate was concentrated under reduced pressure. The residue was purified by basic silica gel column chromatography (eluent: hexane-ethyl acetate=70:30→0:100) to give the title compound as a pale-yellow solid (yield: 399 mg, 31%). The obtained solid was recrystallized from tetrahydrofuran/n-heptane to give the title compound as a white solid (yield: 270 mg, 21%).
¹H-NMR (DMSO-d₆, 300 MHz):δ1.13 (3H, t, J = 7.0 Hz), 3.58 (2H, q, J = 7.0 Hz), 3.80 (3H, s), 6.64 (1H, brs), 6.77 (1H, d, J = 8.7 Hz), 7.06-7.25 (3H, m), 7.47 (1H, brs), 8.05 (1H, d, J = 8.9 Hz), 8.12 (1H, brs), 8.24 (1H, s), 8.87 (1H, brs), 10.37 (1H, brs).

### Example 147

### (4E,5Z)-5-[(4-{[2,4-bis(trifluoromethyl)benzyl]oxy}-3-methoxyphenyl)methylidene]-3-ethylimidazolidine-2,4-dione 4-(O-methyloxime)

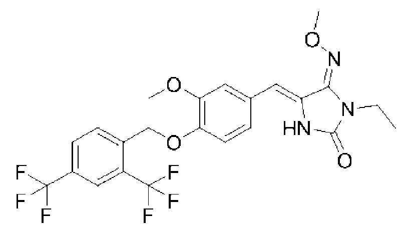

A solution of (4Z)-4-[(4-{[2,4-bis(trifluoromethyl)benzyl]oxy}-3-methoxyphenyl)methylidene]-1-ethyl-5-iminoimidazolidin-2-one (244 mg) and O-methylhydroxylamine hydrochloride (418 mg) in methanol (5 mL) was stirred under refluxing conditions for 5 hr, and the mixture was concentrated under reduced pressure. The residue was diluted with ethyl acetate and saturated aqueous sodium hydrogen carbonate solution, and the mixture was extracted with ethyl acetate. The organic layer was washed with saturated brine, dried over anhydrous magnesium sulfate, and filtered, and the filtrate was concentrated under reduced pressure. The residue was purified by silica gel column chromatography (eluent: hexane-ethyl acetate=70:30→40:60) to give the title compound as a white solid (yield 120 mg, 46%). ¹H-NMR (DMSO-d₆, 300 MHz):δ1.13 (3H, t, J = 7.1 Hz), 3.48 (2H, q, J = 7.0 Hz), 3.86 (3H, s), 3.88 (3H, s), 5.36 (2H, s), 7.01 (2H, s), 7.07 (1H, s), 7.12 (1H, s), 8.04 (1H, d, J = 8.1 Hz), 8.09 (1H, s), 8.17 (1H, d, J = 7.7 Hz), 10.06 (1H, brs).

### Example 148

### (4Z)-4-({4-[2,4-bis(trifluoromethyl)phenoxy]-3-methoxyphenyl}methylidene)-1-ethyl-5-iminoimidazolidin-2-one

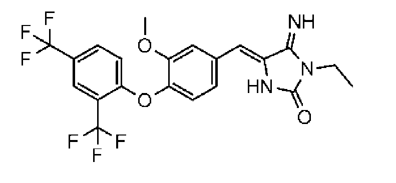

To a solution (10 mL) of 1-(cyanomethyl)-3-ethylurea (349 mg) in ethanol was added potassium tert-butoxide (362 mg) and the mixture was stirred at room temperature for 0.5 hr under a nitrogen atmosphere. To the reaction mixture were added a solution (6 mL) of 4-[2,4-bis(trifluoromethyl)phenoxy]-3-methoxybenzaldehyde (1.00 g) in ethanol and potassium tert-butoxide (362 mg), and the mixture was heated under reflux for 16 hr. To the reaction mixture was added saturated aqueous ammonium chloride solution, and the mixture was concentrated under reduced pressure. To the residue was added water, and the mixture was extracted with ethyl acetate. The organic layer was washed with saturated brine, dried over anhydrous magnesium sulfate, and filtered, and the filtrate was concentrated under reduced pressure. The residue was purified by basic silica gel column chromatography (eluent: hexane-ethyl acetate=100:0→0:100) to give the title compound as a pale-yellow solid (yield: 579 mg, 45%). The obtained solid was recrystallized from ethyl acetate/n-heptane to give the title compound as a white solid (yield: 314 mg, 24%).
¹H-NMR (DMSO-d₆, 300 MHz):δ1.13 (3H, t, J = 7.2 Hz), 3.58 (2H, q, J = 7.2 Hz), 3.80 (3H, s), 6.63 (1H, brs), 6.90 (1H, d, J = 8.9 Hz), 7.10-7.29 (3H, m), 7.94 (1H, dd, J = 8.9, 2.0 Hz), 8.03 (1H, s), 8.86 (1H, brs), 10.35 (1H, brs).

### Example 149

### (4Z)-4-({4-[2,4-bis(trifluoromethyl)phenoxy]-3-methoxyphenyl}methylidene)-1-ethyl-5-imino-3-methylimidazolidin-2-one

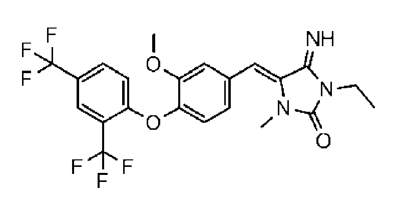

To a solution (1 mL) of (4Z)-4-({4-[2,4-bis(trifluoromethyl)phenoxy]-3-methoxyphenyl}methylidene)-1-ethyl-5-iminoimidazolidin-2-one (200 mg) in N,N-dimethylformamide was added a solution (1 mL) of potassium carbonate (117 mg) and iodomethane (76 mg) in N,N-dimethylformamide, and the mixture was stirred at room temperature for 16 hr. Water was added to the reaction mixture, and the mixture was extracted with ethyl acetate. The organic layer was washed with water and saturated brine, dried over anhydrous magnesium sulfate, and filtered, and the filtrate was concentrated under reduced pressure. The residue was purified by silica gel column chromatography (eluent: hexane-ethyl acetate=90:10→40:60) to give the title compound as a white solid (yield: 210 mg). The obtained solid was recrystallized from ethyl acetate/n-heptane to give the title compound as a white solid (yield: 144 mg, 70%).
¹H-NMR (CDCl₃, 300 MHz):δ1.30 (3H, t, J = 7.2 Hz), 2.95 (3H, s), 3.76 (2H, q, J = 7.2 Hz), 3.80 (3H, s), 6.56 (1H, brs), 6.80 (1H, d, J = 8.7 Hz), 6.90-6.96 (1H, m), 6.94 (1H, s), 7.12 (1H, d, J = 7.9 Hz), 7.66 (1H, dd, J = 8.7, 2.3 Hz), 7.94 (1H, d, J = 1.7 Hz).

### Example 150

### (4Z)-4-({4-[2-chloro-4-(trifluoromethyl)phenoxy]-3-methoxyphenyl}methylidene)-1-ethyl-5-iminoimidazolidin-2-one

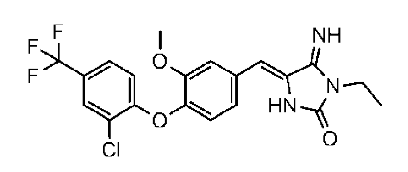

To a solution (2 mL) of 1-(cyanomethyl)-3-ethylurea (88 mg) in ethanol was added potassium tert-butoxide (92 mg) and the mixture was stirred at room temperature for 0.5 hr under a nitrogen atmosphere. To the reaction mixture were added a solution (2 mL) of 4-[2-chloro-4-(trifluoromethyl)phenoxy]-3-methoxybenzaldehyde (230 mg) in ethanol and potassium tert-butoxide (92 mg), and the mixture was heated under reflux for 16 hr. To the reaction mixture was added saturated aqueous ammonium chloride solution, and the mixture was concentrated under reduced pressure. To the residue was added water, and the mixture was extracted with ethyl acetate. The organic layer was washed with saturated brine, dried over anhydrous magnesium sulfate, and filtered, and the filtrate was concentrated under reduced pressure. The residue was purified by silica gel column chromatography (eluent: hexane-ethyl acetate=80:30→20:80) to give the title compound as a pale-yellow solid (yield: 102 mg, 33%). The obtained solid was recrystallized from ethyl acetate/n-heptane to give the title compound as a white solid (yield: 77 mg, 25%).
¹H-NMR (DMSO-d₆, 300 MHz):δ1.13 (3H, t, J = 7.0 Hz), 3.58 (2H, q, J = 7.0 Hz), 3.82 (3H, s), 6.63 (1H, s), 6.81 (1H, d, J = 8.7 Hz), 7.12-7.20 (2H, m), 7.24 (1H, s), 7.62 (1H, dd, J = 8.7, 2.1 Hz), 7.97 (1H, d, J = 2.1 Hz), 8.86 (1H, brs), 10.37 (1H, brs).

The LC-MS analysis in the following Examples 151 - 187 was performed based on the measurement of the following 4ch-LC/MS (MUX).

### 4ch-LC/MS (MUX)

measurement device: Waters LC-MS system
HPLC moiety: Waters 1525 binary gradient pump, 2488 multichannel absorbance detector, 2777 sample manager MS moiety: Micromass ZQ2000, Waters MUX interface

### HPLC conditions

column: CAPCELL PAK C18UG120, S-3 µm, 1.5 x 35 mm (Shiseido Co., Ltd.)
solvent: SOLUTION A; 5 mM ammonium acetate-containing 2% aqueous acetonitrile, SOLUTION B; 5 mM ammonium acetate-containing 95% aqueous acetonitrile
gradient cycle: 0.00 min (SOLUTION A/SOLUTION B=100/0), 4.00 min (SOLUTION A/SOLUTION B=0/100), 5.00 min (SOLUTION A/SOLUTION B=0/100), 5.01 min (SOLUTION A/SOLUTION B=100/0), 5.30 min (SOLUTION A/SOLUTION B=100/0)
injection volume: 2 µL, flow rate: 0.5 mL/min, detection method: UV 220 nm

### MS conditions

ionization method: ESI

The purification by preparative HPLC in the following Examples 151 - 187 was performed under the following conditions.
device: Gilson Inc. High-throughput purification system
column: CombiPrep Pro C18 RS S-5 µm, 20 x 50 mm (YMC)
solvent: SOLUTION A; 0.1% trifluoroacetic acid-containing water, SOLUTION B; 0.1% trifluoroacetic acid-containing acetonitrile
gradient cycle: 0.00 min (SOLUTION A/SOLUTION B=95/5), 1.00 min (SOLUTION A/SOLUTION B=95/5), 5.20 min (SOLUTION A/SOLUTION B=0/100), 6.40 min (SOLUTION A/SOLUTION B=0/100), 6.50 min (SOLUTION A/SOLUTION B=95/5), 7.00 min (SOLUTION A/SOLUTION B=95/5)
flow rate: 25 mL/min, detection method: UV 220 nm, 254 nm

### Example 151

### (4Z)-4-[(4-{[2,4-bis(trifluoromethyl)benzyl]oxy}-3-methoxyphenyl)methylidene]-3-(cyclopropylmethyl)-1-ethyl-5-iminoimidazolidin-2-one

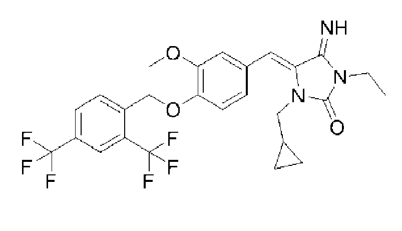

A 0.00016 mmol/L solution (0.5 mL, 0.0080 mmol) of (4Z)-4-[(4-{[2,4-bis(trifluoromethyl)benzyl]oxy}-3-methoxyphenyl)methylidene]-1-ethyl-5-iminoimidazolidin-2-one in N,N-dimethylformamide and a 0.00024 mmol/L solution (0.5 mL, 0.120 mmol) of (bromomethyl)cyclopropane in N,N-dimethylformamide were mixed, potassium carbonate (16.5 mg, 0.120 mmol) was added, and the mixture was stirred at 80°C for 4 hr. The reaction mixture was extracted with ethyl acetate (3.0 mL) and water (1 mL), and the organic layer was separated by upper layer Phase Septube (manufactured by Wako Pure Chemical Industries, Ltd.). The solvent was evaporated under reduced pressure, the residue was dissolved in acetonitrile-water (1:1) (1 mL), and the mixture was purified by preparative HPLC to give the title compound.
yield: 30.1 mg
molecular ion peak: 542
LC-MS analysis: purity 100%
retention time: 3.86 min

### Examples 152 - 187

Using 0.00016 mmol/L solution (0.5 mL, 0.080 mmol) of (4Z)-4-[(4-{[2,4-bis(trifluoromethyl)benzyl]oxy}-3-methoxyphenyl)methylidene]-1-ethyl-5-iminoimidazolidin-2-one in N,N-dimethylformamide and various corresponding halides (0.120 mmol), an operation similar to that in Example 151 was performed to synthesize the compounds of Example Nos. 152 - 187 shown in the following Table 2.

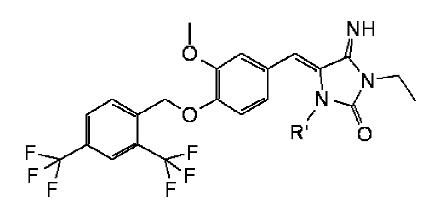

**[Table 2-1]**

| Ex. No. | R' | yield (mg) | molecular ion peak | purity (%) | retention time (min) |
|---|---|---|---|---|---|
| 152 | 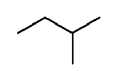 | 21.3 | 544.21 | 100 | 3.94 |
| 153 | 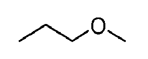 | 27.4 | 546.17 | 100 | 3.74 |
| 154 | 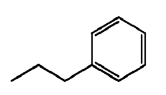 | 17.4 | 592.22 | 100 | 3.92 |
| 155 | 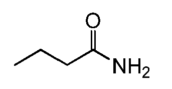 | 4.5 | 559.19 | 100 | 3.48 |
| 156 | 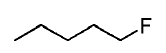 | 30.2 | 562.23 | 85.67 | 3.84 |
| 157 | 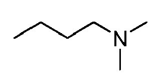 | 4.2 | 573.27 | 83.03 | 3.43 |
| 158 | 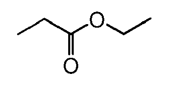 | 28.6 | 574.18 | 100 | 3.77 |
| 159 | 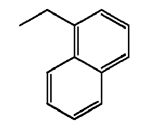 | 30.8 | 628.3 | 92.5 | 4 |
| 160 | 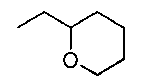 | 19.3 | 586.28 | 100 | 3.86 |
| 161 | 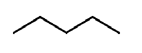 | 28.5 | 544.21 | 90.67 | 3.88 |

**[Table 2-2]**

| Ex. No. | R' | yield (mg) | molecular ion peak | purity (%) | retention time (min) |
|---|---|---|---|---|---|
| 162 | 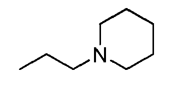 | 15.3 | 599.24 | 88.54 | 3.6 |
| 163 | 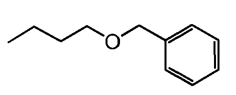 | 26.5 | 636.3 | 80.51 | 4.02 |
| 164 | 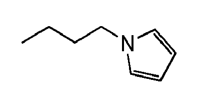 | 30.5 | 595.24 | 100 | 3.89 |
| 165 | 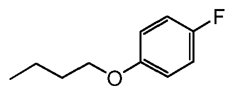 | 29.5 | 640.3 | 100 | 3.93 |
| 166 | 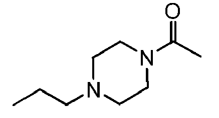 | 36.5 | 642.3 | 100 | 3.58 |
| 167 | 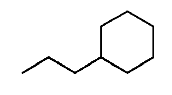 | 31.3 | 598.25 | 84.93 | 4.19 |
| 168 | 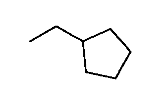 | 14 | 556.21 | 100 | 4 |
| 169 | 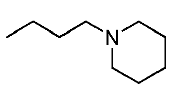 | 30.2 | 613.33 | 94.41 | 3.3 |
| 170 | 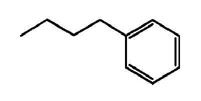 | 35.7 | 606.26 | 100 | 3.99 |
| 171 | 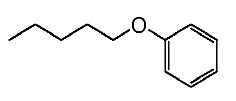 | 34.8 | 636.3 | 100 | 4.04 |

**[Table 2-3]**

| Ex. No. | R' | yield (mg) | molecular ion peak | purity (%) | retention time (min) |
|---|---|---|---|---|---|
| 172 | 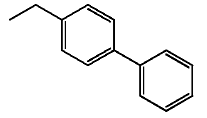 | 38.6 | 654.32 | 100 | 4.09 |
| 173 | 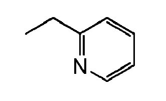 | 10.4 | 579.23 | 100 | 3.68 |
| 174 | 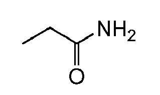 | 12.1 | 545.13 | 100 | 3.43 |
| 175 | 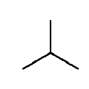 | 10 | 530.17 | 100 | 3.88 |
| 176 | 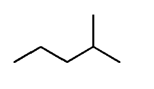 | 27.9 | 558.25 | 100 | 4.01 |
| 177 | 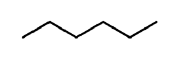 | 32.8 | 558.25 | 100 | 4.01 |
| 178 | 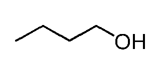 | 23 | 546.17 | 100 | 3.54 |
| 179 | 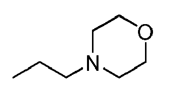 | 29.4 | 601.27 | 100 | 3.68 |
| 180 | 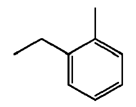 | 35.2 | 592.25 | 100 | 3.99 |
| 181 | 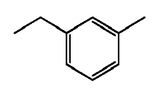 | 35.1 | 592.22 | 100 | 3.99 |

**[Table 2-4]**

| Ex. No. | R' | yield (mg) | molecular ion peak | purity (%) | retention time (min) |
|---|---|---|---|---|---|
| 182 | 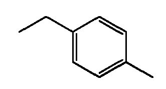 | 35.5 | 592.25 | 100 | 3.93 |
| 183 | 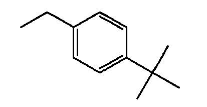 | 36.1 | 634.32 | 100 | 4.16 |
| 184 | 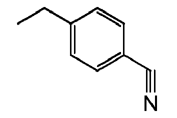 | 36.2 | 603.25 | 100 | 3.84 |
| 185 | 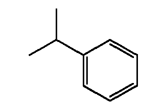 | 25.3 | 592.22 | 100 | 3.99 |
| 186 | 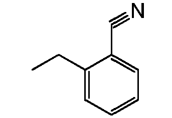 | 34.5 | 603.21 | 100 | 3.77 |
| 187 | 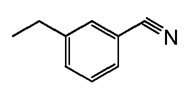 | 35.4 | 603.24 | 100 | 3.81 |

The LC-MS analysis in the following Examples 188 - 218 was performed based on the measurement of the following 4ch-LC/MS (MUX).

### 4ch-LC/MS (MUX)

measurement device: Waters LC-MS system
HPLC moiety: Waters 1525 binary gradient pump, 2488 multichannel absorbance detector, 2777 sample manager MS moiety: Micromass ZQ2000, Waters MUX interface

### HPLC conditions

column: CAPCELL PAK C18UG120, S-3 µm, 1.5 x 35 mm (Shiseido Co., Ltd.)
solvent: SOLUTION A; 5 mM ammonium acetate-containing 2% aqueous acetonitrile, SOLUTION B; 5 mM ammonium acetate-containing 95% aqueous acetonitrile
gradient cycle: 0.00 min (SOLUTION A/SOLUTION B=100/0), 4.00 min (SOLUTION A/SOLUTION B=0/100), 5.00 min (SOLUTION A/SOLUTION B=0/100), 5.01 min (SOLUTION A/SOLUTION B=100/0), 5.30 min (SOLUTION A/SOLUTION B=100/0)
injection volume: 2 µL, flow rate: 0.5 mL/min, detection method: UV 220 nm

### MS conditions

ionization method: ESI

The purification by preparative HPLC in the following Examples 188 - 218 was performed under the following conditions.
device: Gilson Inc. High-throughput purification system
column: CombiPrep Pro C18 RS S-5 µm, 20 x 50 mm (YMC)
solvent: SOLUTION A; 0.1% trifluoroacetic acid-containing water, SOLUTION B; 0.1% trifluoroacetic acid-containing acetonitrile
gradient cycle: 0.00 min (SOLUTION A/SOLUTION B=95/5), 1.00 min (SOLUTION A/SOLUTION B=95/5), 5.20 min (SOLUTION A/SOLUTION B=0/100), 6.40 min (SOLUTION A/SOLUTION B=0/100), 6.50 min (SOLUTION A/SOLUTION B=95/5), 6.60 min (SOLUTION A/SOLUTION B=95/5)
flow rate: 20 mL/min, detection method: UV 220 nm

### Example 188

### (4Z)-1-ethyl-5-imino-4-({3-methoxy-4-[(2-methylbenzyl)oxy]phenyl}methylidene)imidazolidin-2-one

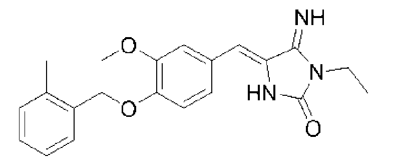

To 2-methylbenzyl chloride (33.7 mg, 0.240 mmol) was added a 0.24 M solution (1 mL, 0.240 mmol) of vanillin in dimethylformamide, a potassium carbonate powder (50 mg, 0.360 mmol) was added and the mixture was stirred at 40°C for 16 hr. The reaction mixture was extracted with ethyl acetate (3.5 mL) and 2% aqueous sodium hydrogen carbonate solution (1 mL), and the organic layer was separated by upper layer Phase Septube (manufactured by Wako Pure Chemical Industries, Ltd.). The solvent was completely evaporated under reduced pressure, and the residue was dissolved in ethanol (0.500 mL) (SOLUTION A). In a separate flask, 1-(cyanomethyl)-3-ethylurea (25.4 mg, 0.200 mmol) and potassium tert-butoxide (22.4 mg, 0.200 mmol) were dissolved in ethanol (0.500 mL), and the mixture was stirred at 25°C for 1 hr (SOLUTION B). To the above-mentioned SOLUTION A was added potassium tert-butoxide (22.4 mg, 0.200 mmol), the mixture was mixed with SOLUTION B, and the mixture was stirred at 50°C for 16 hr. The reaction mixture was extracted with ethyl acetate (3.5 mL) and 2% aqueous sodium hydrogen carbonate solution (1 mL), and the organic layer was separated by upper layer Phase Septube (manufactured by Wako Pure Chemical Industries, Ltd.). The solvent was evaporated under reduced pressure, the residue was dissolved in DMSO-MeOH (1:1) (1 mL), and the mixture was purified by preparative HPLC to give the title compound.
yield: 32.6 mg
molecular ion peak: 366
LC-MS analysis: purity 84%
retention time: 3.58 min

### Examples 189 - 218

An operation similar to that in Example 188 was performed to synthesize the compounds of Example Nos. 189 - 218 shown in the following Table 3.

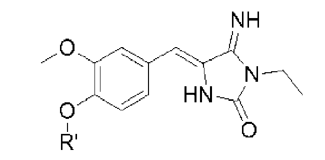

**[Table 3-1]**

| Ex. No. | R' | yield (mg) | molecular ion peak | purity (%) | retention time (min) |
|---|---|---|---|---|---|
| 189 | 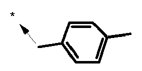 | 12.3 | 366 | 100 | 3.32 |
| 190 | 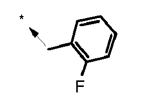 | 14.6 | 370 | 100 | 3.29 |
| 191 | 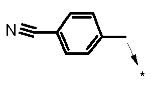 | 6.2 | 377 | 100 | 3.21 |
| 192 | 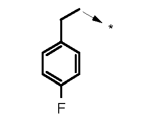 | 5.5 | 384 | 100 | 3.31 |
| 193 | 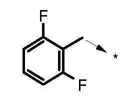 | 11.4 | 388 | 100 | 3.29 |
| 194 | 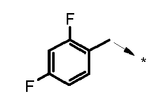 | 17.6 | 388 | 100 | 3.32 |
| 195 | 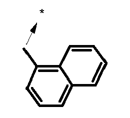 | 8.9 | 402 | 100 | 3.45 |
| 196 | 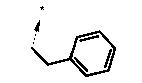 | 4.4 | 366 | 100 | 3.32 |
| 197 | 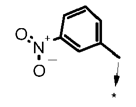 | 13.0 | 397 | 100 | 3.40 |
| 198 | 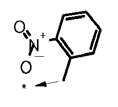 | 2.4 | 397 | 89 | 3.22 |

**[Table 3-2]**

| Ex. No. | R' | yield (mg) | molecular ion peak | purity (%) | retention time (min) |
|---|---|---|---|---|---|
| 199 | 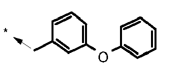 | 7.7 | 444 | 100 | 3.54 |
| 200 | 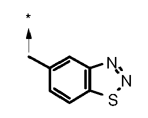 | 5.5 | 410 | 100 | 3.21 |
| 201 | 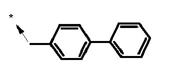 | 6.9 | 428 | 100 | 3.51 |
| 202 | 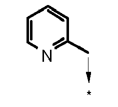 | 12.8 | 353 | 100 | 3.02 |
| 203 | 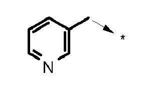 | 2.0 | 353 | 100 | 2.93 |
| 204 | 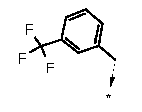 | 13.7 | 420 | 100 | 3.43 |
| 205 | 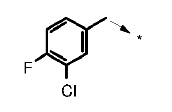 | 11.2 | 404 | 100 | 3.40 |
| 206 | 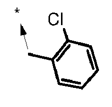 | 10.0 | 386 | 89 | 3.34 |
| 207 | 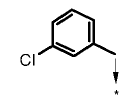 | 9.5 | 386 | 89 | 3.38 |
| 208 | 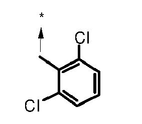 | 8.4 | 420 | 82 | 3.43 |

**[Table 3-3]**

| Ex. No. | R' | yield (mg) | molecular ion peak | purity (%) | retention time (min) |
|---|---|---|---|---|---|
| 209 | 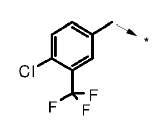 | 11.0 | 454 | 77 | 3.54 |
| 210 | 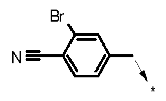 | 5.2 | 455 | 100 | 3.29 |
| 211 | 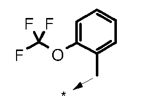 | 12.1 | 436 | 93 | 3.46 |
| 212 | 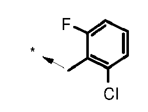 | 9.7 | 404 | 100 | 3.36 |
| 213 | 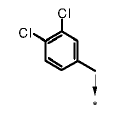 | 4.1 | 420 | 100 | 3.49 |
| 214 | 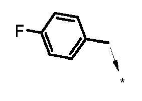 | 6.9 | 370 | 100 | 3.24 |
| 215 | 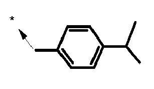 | 9.9 | 394 | 100 | 3.54 |
| 216 | 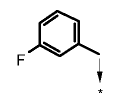 | 8.9 | 370 | 100 | 3.31 |
| 217 | 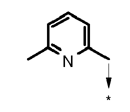 | 4.3 | 367 | 100 | 3.04 |
| 218 | 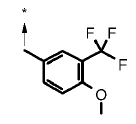 | 13.0 | 450 | 100 | 3.42 |

### Example 219

### 4-{4-[(Z)-(1-ethyl-5-imino-3-methyl-2-oxoimidazolidin-4-ylidene)methyl]-2-methoxyphenoxy}naphthalene-1-carbonitrile

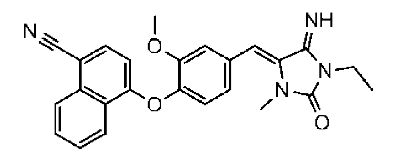

To a solution (3 mL) of (4E)-1-ethyl-4-[(4-hydroxy-3-methoxyphenyl)methylidene]-5-imino-3-methylimidazolidin-2-one (170 mg) in dimethyl sulfoxide were added lithium carbonate (92 mg) and 4-fluoronaphthalene-1-carbonitrile (106 mg), and the mixture was stirred at 100°C for 16 hr. To the reaction mixture was added 1 N aqueous sodium hydroxide solution, and the mixture was extracted with ethyl acetate. The organic layer was washed with water and saturated brine, dried over anhydrous magnesium sulfate, and filtered, and the filtrate was concentrated under reduced pressure. The residue was purified by silica gel column chromatography (eluent: hexane-ethyl acetate=60:40→30:70) to give the title compound as a white powder (yield: 87 mg, 33%). The obtained solid was recrystallized from ethyl acetate/n-heptane to give the title compound as a white solid (yield 59 mg, 23%).
¹H-NMR (CDCl₃, 300 MHz):δ1.28 (3H, t, J = 7.2 Hz), 2.96 (3H, s), 3.67-3.88 (5H, m), 6.40 (1H, brs), 6.58 (1H, d, J = 8.1 Hz), 6.89-6.99 (2H, m), 7.13 (1H, d, J = 8.1 Hz), 7.63-7.71 (1H, m), 7.71-7.87 (3H, m), 8.24 (1H, d, J 8.5 Hz), 8.50 (1H, d, J = 8.3 Hz).

### Experimental Example 1: Compound evaluation in reporter assay system using 293T cells

293T cells were seeded in a 150 cm² flask by 6,000,000 cells, and cultured in a medium (DMEM medium containing 10% inactivated Fetal Bovine Serum sterile filtered (FBS), 50 µg/ml Gentamicin, 10 mM HEPES buffer (pH 7.4)) at 37°C in the presence of 5% CO₂ for 1 day.
Then, vector DNA having a gene encoding a fusion protein containing GAL4-DNA-binding domain and ERR full-length and a vector DNA wherein luciferase gene is fused at the downstream of GAL4-binding sequence were cotransfected by a lipofection method, and the cells were cultured at 37°C in the presence of 5% CO₂ for 1 day. The cells were recovered, sown in a 384 well plate by 15,000 cells, and cultured at 37°C in the presence of 5% CO₂ for 3 hr. Furthermore, 50 µM XCT-790 (final concentration 10 µM) or 15 µM compound (final concentration 3 µM) was added, and the cells were cultured at 37°C in the presence of 5% CO₂ for 1 day. The luciferase activity was measured by using a multiplate reader Envision (Perkin Elmer Inc.). The inverse agonist activity of the compound was calculated in percentage with the luciferase activity inhibited by no addition of compound as 0%, and with addition of final concentration 10 µM of XCT-790 (N-[5-(trifluoromethyl)-1,3,4-thiadiazol-2-yl]-2-cyano-3-[3-methoxy-4-[2,4-di(trifluoromethyl)benzyloxy]phenyl]propenamide) as 100%.

**[Table 4]**

| Example No. | inhibition rate (%) at 3 µM |
|---|---|
| 25 | 89 |
| 32 | 91 |
| 93 | 88 |
| 94 | 90 |
| 96 | 87 |
| 116 | 93 |
| 143 | 94 |
| 145 | 95 |

### Experimental Example 2: Compound evaluation in HTRF assay system using ERRα ligand-binding domain

An assay buffer (25 mM HEPES, 100 mM NaCl, 1 mM DTT, 0.1% BSA) containing 5 nM GST-ERRα ligand-binding domain (final concentration 1.25 nM) was added to a 384 well plate, then 40 µm XCT-790 (final concentration 10 µM), or 12 µM compound (final concentration 3 µM) was added. Furthermore, an assay buffer containing XL665 conjugated Streptavidine, Eu³⁺ criptate conjugated GST antibody and 200 nM biotinylated SRC-1 (676-700) (final concentration 100 nM) was added, and the mixture was reacted at room temperature for 2 hr. Using Multiplate reader Envision (Perkin Elmer Inc.), after excitation at 320 nm, fluorescence values at 615 nm and 665 nm were measured. The inverse agonist activity of the compound was calculated in percentage with the 615/665 ratio value inhibited by no addition of compound as 0%, and with addition of final concentration 10 µM of XCT-790 (N-[5-(trifluoromethyl)-1,3,4-thiadiazol-2-yl]-2-cyano-3-[3-methoxy-4-[2,4-di(trifluoromethyl)benzyloxy]phenyl]propenamide) as 100%.

**[Table 5]**

| Example No. | inhibition rate (%) at 3 µM |
|---|---|
| 25 | 98 |
| 32 | 103 |
| 93 | 105 |
| 94 | 107 |
| 96 | 114 |
| 116 | 105 |
| 143 | 92 |
| 145 | 93 |

As is clear from the results of Tables 4 and 5, the compound of the present invention was shown to have a superior ERR-α inverse agonist activity. Therefore, it is considered to be useful as a drug for the treatment or prophylaxis of ERR-α associated diseases such as cancer and the like.

### Experimental Example 3: Cell proliferation inhibitory test

ES-2 ovarian cancer cells were seeded in a 96 well plate at 1x10³ cells/well, and incubated at 37°C in the presence of 5% CO₂ for 24 hr. Then, the compound was added at a final concentration of 10 µM, and the cells were cultured at 37°C in the presence of 5% CO₂ for 72 hr. For activity measurement of viable cells, a CCK-8 reagent (Cell Counting Kit-8, DOJINDO) was added, the cells were incubated at 37°C for 1.5 hr, and the absorbance at 450 nm was measured by a microplate reader. Assuming the dehydrogenase activity in the DMSO alone addition group as 0%, and that in a cell-free well containing only a medium as 100%, the dehydrogenase activity of the compound addition group was calculated in percentage.

**[Table 6]**

| Example No. | inhibition rate (%) at 10 µM |
|---|---|
| 25 | 61 |
| 32 | 98 |
| 93 | 101 |
| 94 | 100 |
| 96 | 102 |
| 116 | 101 |
| 143 | 88 |
| 145 | 98 |

As is clear from the results of Table 6, the compound of the present invention was shown to have a superior cell proliferation inhibitory action on cancer cells.

### Formulation Example 1

A medicament containing the compound of the present invention as an active ingredient can be produced, for example, according to the following formulation.

### 1. capsule

| | | |
|---|---|---|
| (1) | compound obtained in Example 1 | 10 mg |
| (2) | lactose | 90 mg |
| (3) | microcrystalline cellulose | 70 mg |
| (4) | magnesium stearate | 10 mg |
| | 1 capsule | 180 mg |

The total amount of the above-mentioned (1), (2) and (3) and 5 mg of (4) are blended, and the mixture is granulated. Thereto is added the remaining 5 mg of (4), and the whole is sealed in a gelatin capsule.

### 2. tablet

| | | |
|---|---|---|
| (1) | compound obtained in Example 1 | 10 mg |
| (2) | lactose | 35 mg |
| (3) | cornstarch | 150 mg |
| (4) | microcrystalline cellulose | 30 mg |
| (5) | magnesium stearate | 5 mg |
| | 1 tablet | 230 mg |

The total amount of the above-mentioned (1), (2) and (3), 20 mg of (4) and 2.5 mg of (5) are blended, and the mixture is granulated. Thereto are added the remaining 10 mg of (4) and 2.5 mg of (5), and the mixture is compression-molded to give a tablet.

### Formulation Example 2

The compound (50 mg) obtained in Example 2 is dissolved in the Japanese Pharmacopoeia distilled water (50 mL) for injection, and the Japanese Pharmacopoeia distilled water for injection is added to 100 mL. This solution is filtered under sterilization conditions, this solution (1 mL) is measured and, under sterilization conditions, filled in a vial for injection, and the vial is freeze-dried and tightly sealed.

### Industrial Applicability

The compound of the present invention has a superior activity as an ERR-α modulator (particularly, inverse agonist), is free of non-specific conjugation with glutathione, and has superior properties in terms of in vivo kinetics and the like. Therefore, it is useful as, for example, a prophylactic or therapeutic drug for ERR-α associated diseases such as cancers (e.g., lung cancer, prostate cancer, ovarian cancer, endometrial carcinoma, breast cancer etc.) and the like.

This application is based on patent application No. 2009-181936 filed in Japan, the contents of which are encompassed in full herein.

## Claims

1. A compound represented by the formula 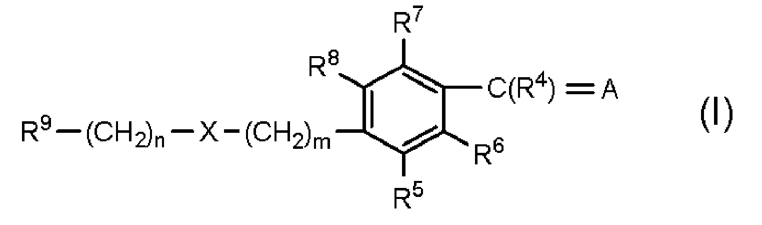 wherein,
A is a group represented by 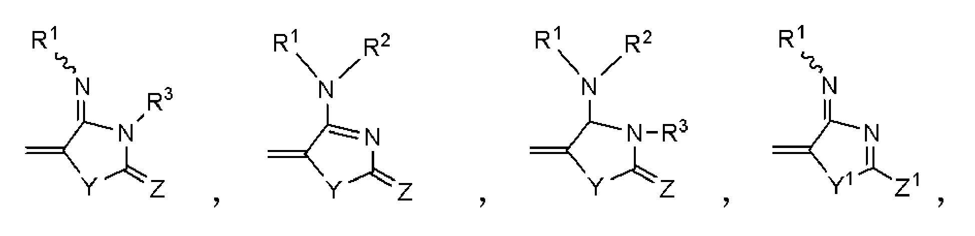 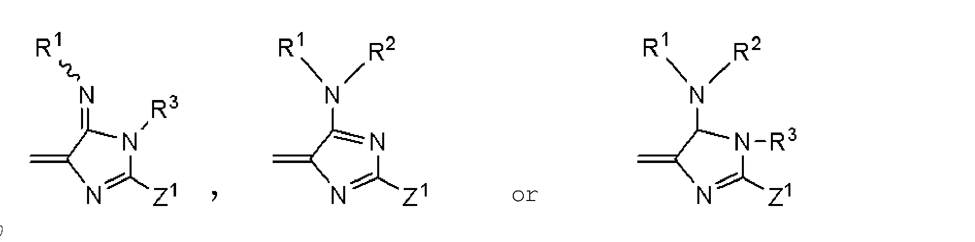 wherein
R¹, R² and R³ are each independently a hydrogen atom, a hydrocarbon group optionally having substituent(s), a heterocyclic group optionally having substituent(s), an acyl group or a hydroxyl group optionally substituted by an alkyl group optionally having substituent(s);
Y is -O-, -S- or -NR^{a}- wherein R^{a} is a hydrogen atom, a hydrocarbon group optionally having substituent(s), a heterocyclic group optionally having substituent(s), an acyl ) group or a hydroxyl group optionally substituted by an alkyl group optionally having substituent(s);
Z is =O, =S or =NR^{b} wherein R^{b} is a hydrogen atom, a hydrocarbon group optionally having substituent(s), a heterocyclic group optionally having substituent(s), an acyl group or a hydroxyl group optionally substituted by an alkyl group optionally having substituent(s);
Y¹ is -O-, -S- or -NR^{c}- wherein R^{c} is a hydrogen atom, a hydrocarbon group optionally having substituent(s), a heterocyclic group optionally having substituent(s) or an acyl group; and
Z¹ is -OR^{d}, -SR^{d} or -NHR^{d} wherein R^{d} are each independently a hydrogen atom, a hydrocarbon group optionally having substituent(s), a heterocyclic group optionally having substituent(s) or an acyl group;
R⁴ is a hydrogen atom or an alkyl group optionally having substituent(s);
R⁵, R⁶, R⁷ and R⁸ are each independently a hydrogen atom, a hydrocarbon group optionally having substituent(s), an amino group optionally having substituent(s), a hydroxyl group optionally having a substituent, a thiol group optionally having a substituent, a carbamoyl group optionally having substituent(s), an alkyloxycarbonyl group optionally having substituent(s), a halogen atom, a cyano group or a nitro group;
m and n are each independently an integer of 0 - 3;
X is -O-, -CO-, -O-CO-, -CO-O-, -SO₂-, -SO-, -S-, -SO₂-O-, - NR^{c1}-, -CO-NR^{c1}-, -NR^{c1}-CO-, -NR^{c1}-CO-NR^{c2}-, -O-CO-NR^{c1}-, -NR^{c1}-COO-, -SO₂-NR^{c1}-, -NR^{c1}-SO₂- or -NR^{c1}-SO₂-NR^{c2}-
wherein R^{c1} and R^{c2} are each independently a hydrogen atom, a hydrocarbon group optionally having substituent(s), a heterocyclic group optionally having substituent(s), an acyl group or a hydroxyl group optionally substituted by an alkyl group optionally having substituent(s); and
R⁹ is an aromatic ring group optionally having substituent(s), excluding the following two compounds: 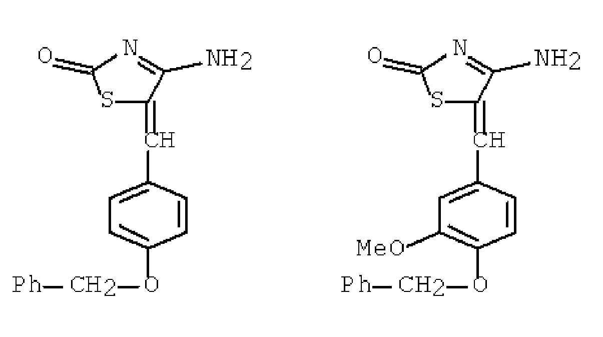 or a salt thereof.

2. The compound according to claim 1, wherein
A is 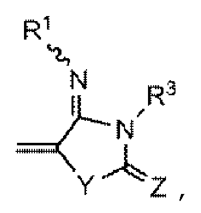 R¹, Y and Z are as defined in claim 1, and
R³ is a hydrocarbon group optionally having substituent(s), a heterocyclic group optionally having substituent(s), an acyl group or a hydroxyl group optionally substituted by an alkyl group optionally having substituent(s),
or a salt thereof.

3. The compound according to claim 1,
wherein
A is 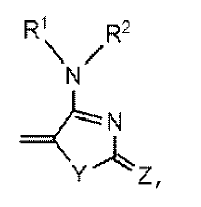 R¹ is a hydrocarbon group optionally having substituent(s) or a heterocyclic group optionally having substituent(s),
R² is a hydrogen atom, and
Y and Z are as defined in claim 1,
or a salt thereof.

4. The compound according to claim 1, wherein R⁵, R⁶, and R⁷ are each a hydrogen atom, and R⁸ is a hydroxyl group substituted by a C₁₋₁₀ alkyl group optionally having substituent(s), or a salt thereof.

5. The compound according to claim 1, wherein X is -O-, or a salt thereof.

6. The compound according to claim 1, wherein R⁹ is C₆₋₁₀ aryl optionally having substituent(s), or a salt thereof.

7. The compound according to claim 1, wherein R⁴ is a hydrogen atom, or a salt thereof.

8. (5Z)-5-({4-[2-Chloro-4-(trifluoromethyl)phenoxy]-3-methoxyphenyl}methylidene)-4-{[2-(diethylamino)ethyl]amino}-1,3-thiazol-2(5H)-one or a salt thereof.

9. 4-(2-Methoxy-4-{(Z)-[4-{[2-(1-methylpyrrolidin-2-yl)ethyl]aminol-2-oxo-1,3-thiazol-5(2H)-ylideneJmethyl}phenoxy)naphthalene-1-carbonitrile or a salt thereof.

10. 4-{4-[(Z)-{4-[(2-Hydroxy-2-methylpropyl)amino]-2-oxo-1,3-thiazol-5(2H)-ylidene}methyl]-2-methoxyphenoxy}-3-(trifluoromethyl)benzonitrile or a salt thereof.

11. 4-{4-[(Z)-(1-Ethyl-5-imino-3-methyl-2-oxoimidazolidin-4-ylidene)methyl]-2-methoxyphenoxy}-3-(trifluoromethyl)benzonitrile or a salt thereof.

12. A prodrug of the compound according to claim 1 or a salt thereof.

13. A medicament containing the compound according to claim 1 or a salt thereof or a prodrug thereof.

14. The medicament according to claim 13, which is an estrogen-related receptor-α inverse agonist.

15. The medicament according to claim 13, which is a prophylactic or therapeutic drug for cancer.

16. A method for the prophylaxis or treatment of cancer in a mammal, comprising administering an effective amount of the compound according to claim 1 or a salt thereof or a prodrug thereof to the mammal.

17. Use of the compound according to claim 1 or a salt thereof or a prodrug thereof for the production of a prophylactic or therapeutic agent for cancer.
